# EUROPEAN PATENT APPLICATION

(11) **EP 3 722 416 A1**
(43) Date of publication of application: **14.10.2020**
(21) Application number: 20162827.8
(22) Date of filing: 13.08.2013
(51) Int. Cl.: C12N 5/0783, A61K 35/17, A61P 35/00, A61P 35/02

(54) **NATURAL KILLER CELLS AND USES THEREOF**

(30) Priority: 13.08.2012 US 201261682706 P; 15.03.2013 US 201361799211 P
(62) Divisional of application: 13829894.8
(71) Applicant: Celularity, Inc., Warren, New Jersey 07059 (US)
(72) Inventor: LAW, Eric, East Brunswick, NJ 08816 (US); KANG, Lin, Edison, NJ 08820 (US); JANKOVIC, Vladimir, New York, NY 10044 (US); ZHANG, Xiaokui, Livingston, NJ 07039 (US); ABBOT, Stewart, Warren, NJ 07059 (US); HARIRI, Robert, J., Warren, NJ 07059 (US)
(74) Representative: Mewburn Ellis LLP

(57) **Abstract**

Provided herein are methods of producing natural killer (NK) cells and NK progenitor cell populations using a two-step expansion and differentiation method. Also provided herein are methods of producing populations of NK cells and NK progenitor cell populations using a three-step expansion and differentiation method. Also provided herein are methods of suppressing tumor cell proliferation using the NK cells, the NK cell populations, and the NK progenitor call populations produced by the methods described herein, as well as methods of treating individuals having cancer or a viral infection, comprising administering the NK cells, the NK cell populations, and the NK progenitor cell populations produced by the methods described herein to an individual having the cancer or viral infection.

## Description

### 1. FIELD

Provided herein are methods of producing populations of natural killer (NK) cells and populations of NK progenitor cells from a population of hematopoietic progenitor cells, *e.g.,* methods of producing NK cells and NK progenitor cells from cells of the placenta, for example, from placental perfusate (*e.g.,* human placental perfusate) such as placenta-derived intermediate NK cells, or other tissues, for example, umbilical cord blood or peripheral blood. Also provided herein are expanded NK cell populations, as well as isolated populations of NK cells and NK progenitor cells produced by the methods presented herein. Further provided herein are methods of using the placental perfusate, the NK cells and/or NK progenitor cells described herein, to suppress the proliferation of tumor cells. In certain embodiments, the NK cells and/or NK progenitor cells produced by the methods described herein are used in combination with, and/or treated with, one or more immunomodulatory compounds, *e.g.,* immunomodulatory compounds referred to herein as IMiDs®.

### 2. BACKGROUND

Natural killer (NK) cells are cytotoxic lymphocytes that constitute a major component of the innate immune system. NK cells do not express T-cell antigen receptors (TCR), CD3 or surface immunoglobulins (Ig) B cell receptor. NK cells generally express the surface markers CD16 (FcγRIII) and CD56 in humans, but a subclass of human NK cells is CD16⁻. NK cells are cytotoxic; small granules in their cytoplasm contain special proteins such as perforin and proteases known as granzymes. Upon release in close proximity to a cell targeted for killing, perforin forms pores in the cell membrane of the target cell through which the granzymes and associated molecules can enter, inducing apoptosis. One granzyme, granzyme B (also known as granzyme 2 and cytotoxic T-lymphocyte-associated serine esterase 1), is a serine protease crucial for rapid induction of target cell apoptosis in the cell-mediated immune response.

NK cells are activated in response to interferons or macrophage-derived cytokines. Activated NK cells are sometimes referred to as lymphokine activated killer (LAK) cells, although LAK cells are typically a heterogeneous population of NK cells, CD56+ CD3+ NK-like T cells, and possibly other cytotoxic cells. The cytotoxic activity of NK cells are largely regulated by two types of surface receptors, which may be considered "activating receptors" or "inhibitory receptors," although some receptors, *e.g.,* CD94 and 2B4, can work either way depending on ligand interactions.

Among other activities, NK cells play a role in the host rejection of tumors and have been shown capable of killing virus-infected cells. Cancer cells with altered or reduced level of self-class I MHC expression result in induction of NK cell sensitivity. Accumulating clinical data suggest that haploidentical transplantation of human NK cells isolated from peripheral blood mononuclear cells (PBMC) or bone marrow mediate potent anti-leukemia effects without incurring detectable graft versus host disease (GVHD). *See* Ruggeri et al., Science 295:2097-2100 (2002)). Natural killer cells can become activated by cells lacking, or displaying reduced levels of, major histocompatibility complex (MHC) proteins. Additionally, the activating receptors expressed on NK cells are known to mediate detection of "stressed" or transformed cells with express ligands to activating receptors and therefore trigger the NK cell activation. For instance, NCR1 (NKp46) binds viral hemagglutinins. NKG2D ligands include CMV UL16-binding protein 1 (ULB1), ULB2, ULB3 and MHC-class-I-polypeptide-related sequence A (MICA) and MICB proteins. NK protein 2B4 binds CD48, and DNAM-1 binds Poliovirus receptor (PVR) and Nectin-2, both are consistently detected in acute myeloid leukemia (AML). See Penda et al., Blood 105: 2066-2073 (2004). Moreover, lysis of AML has been described to be mainly natural cytotoxicity receptor (NCR) dependent. See Fauriat et al., Blood 109: 323-330 (2007). Activated and expanded NK cells, and in some cases LAK cells, from peripheral blood have been used in both *ex vivo* therapy and *in vivo* treatment of patients having advanced cancer, with some success against bone marrow related diseases, such as leukemia; breast cancer; and certain types of lymphoma. NK cell treatment, or in some instances LAK cell treatment, requires that the patient first receive IL-2, followed by leukopheresis and adoptive transfer. In some instances, adoptive transfer is preceded by an ex vivo incubation and culture of the harvested autologous blood cells in the presence of IL-2 for a few days. The NK cells, or in some instances LAK cells, must be reinfused along with relatively high doses of IL-2 to complete the therapy. This purging treatment is expensive and can cause serious side effects. These include fluid retention, pulmonary edema, drop in blood pressure, and high fever.

In spite of the advantageous properties of NK cells in killing tumor cells and virus-infected cells, they remain difficult to work with and to apply in immunotherapy, primarily due to the difficulty in maintaining their tumor-targeting and tumoricidal capabilities during culture and expansion. Thus, there is a need in the art to develop an efficient method to produce and expand natural killer cells that retain tumoricidal functions.

### 3. SUMMARY

Provided herein are methods of expanding and differentiating cells, for example, hematopoietic cells, such as hematopoietic stem cells, *e.g.,* CD34⁺ hematopoietic stem cells, to produce natural killer (NK) cells and NK progenitor cells.

In one aspect, provided herein is a method of producing NK cells comprising culturing hematopoietic stem cells or progenitor cells, *e.g.,* CD34⁺ stem cells or progenitor cells, in a first medium to produce expanded and differentiated cells (differentiation as demonstrated, *e.g.,* by loss of progenitor status of the cells and/or loss of CD34 expression), and subsequently culturing said expanded cells in a second medium in which said cells expand further and differentiate into natural killer cells. The first and second steps comprise culturing the cells in media with a unique combination of cellular factors. Generally, the medium used in the first step is different from the medium used in the second step. In certain embodiments, said cellular factors (*e.g.,* cytokines) are not within an undefined component of the media (*e.g.,* serum), for example, the cellular factors (*e.g.,* cytokines, for example, particular concentrations of cytokines) are exogenous to the undefined component of the media (*e.g.,* serum). In certain embodiments, said method is a two-step method. In certain embodiments, medium for the second step is added to the cell culture from the first step without changing the medium from the first step. In certain embodiments, said method does not comprise any third or intermediate step in which the cells are contacted (or cultured). Natural killer cells produced by the methods provided herein that use these two steps (*e.g.,* two-step method) are referred to herein as TSNK cells.

In a specific embodiment, provided herein is a method of producing a population of activated natural killer (NK) cells, comprising: (a) seeding a population of hematopoietic stem or progenitor cells in a first medium comprising exogenous interleukin-15 (IL-15) and, optionally, one or both of stem cell factor (SCF) and interleukin-7 (IL-7), wherein said exogenous IL-15 and optional SCF and IL-7 are in addition to whatever, if any, amount of IL-15, SCF or IL-7 are present in an undefined component of said medium, such that the population expands, and a plurality of hematopoietic stem or progenitor cells within said population of hematopoietic stem or progenitor cells differentiate into NK cells; and (b) expanding the cells from the first step in a second medium comprising exogenous interleukin-2 (IL-2), wherein said exogenous IL-2 is in addition to whatever, if any, amount of IL-2 is present in an undefined component of said medium, to produce a population of activated NK cells.

In certain embodiments, said first medium comprises medium comprising interleukin-15 (IL-15), *e.g.,* 1 ng/mL to 50 ng/mL, and one or more of human serum (*e.g.,* human serum AB), fetal bovine serum (FBS) or fetal calf serum (FCS), *e.g.,* 1% to 20 % v/v, *e.g.,* 5% to 20% v/v, stem cell factor (SCF), *e.g.,* 1 ng/mL to 50 ng/mL, FMS-like tyrosine kinase-3 ligand (Flt-3 ligand), *e.g.,* 1 ng/ml to 20 ng/mL; interleukin-7 (IL-7), *e.g.,* 1 ng/mL to 50 ng/mL; thrombopoietin (TPO), *e.g.,* 1 ng/mL to 100 ng/mL, for example, 1 ng/mL to 50 ng/mL; interleukin-2 (IL-2), *e.g.,* up to 2000 IU/mL, for example, 50 IU/mL to 500 IU/mL; and/or heparin, *e.g.,* 0.1 IU/mL to 10 IU/mL. In a specific embodiment, said first medium comprises IL-15 and stem cell factor (SCF) or interleukin-7 (IL-7). In another specific embodiment, said first medium comprises IL-15, SCF, and IL-7. In another specific embodiment, said first medium comprises growth medium, serum (e.g., human serum (*e.g.,* human serum AB), FBS or FCS), SCF, IL-7 and IL-15. In another specific embodiment, said first medium further comprises Flt-3 ligand (Flt3-L), TPO, IL-2, and/or heparin. In another specific embodiment, said first medium comprises growth medium, 10% human serum or fetal bovine serum, 20 ng/mL SCF, 10 ng/ml Flt3-L, 20 ng/mL IL-7, 20 ng/mL TPO, 200 IU/mL IL-2, 10 ng/mL IL-15, and 1.5 IU/mL heparin. In another specific embodiment, said first medium does not comprise IL-2.

In certain embodiments, said first medium comprises GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640. In certain embodiments, said medium comprises O-acetyl-carnitine (also referred to as acetylcamitine, O-acetyl-L-carnitine, OAC or ALCAR), *e.g.,* about 0.5 mM-10 mM. In one embodiment, said medium comprises STEMSPAN® H3000, and/or DMEM:F12. In another embodiment, said medium comprises OAC, *e.g.,* about 0.5, 1, 2, 3, 4, 5, 6, 7, 8, 9, or 10 mM, and STEMSPAN® H3000, and/or DMEM:F12. In a specific embodiment, said medium comprises GBGM®. In another specific embodiment, said medium comprises DMEM:F12 and about 5 mM of OAC. In another specific embodiment, said medium comprises STEMSPAN® H3000 and about 5 mM of OAC.

In certain embodiments, said culturing in said first medium comprises culturing using feeder cells, *e.g.,* K562 cells, *e.g.,* mitomycin C-treated K562 cells, peripheral blood mononuclear cells (PBMCs), *e.g.,* mitomycin C-treated PBMCs or tissue culture-adherent stem cells, *e.g.,* mitomycin C-treated tissue culture-adherent stem cells. In another embodiment, said culturing in said first medium does not comprise culturing using feeder cells.

In certain embodiments, said second medium comprises cell growth medium comprising IL-2, *e.g.,* 10 IU/mL to 1000 IU/mL, and one or more of: human serum (*e.g.,* human serum AB), fetal bovine serum (FBS) or fetal calf serum (FCS), *e.g.,* 5%-15% FCS v/v; transferrin, *e.g.,* 10 µg/mL to 50 µg/mL; insulin, *e.g.,* 5 µg/mL to 20 µg/mL; ethanolamine, *e.g.,* 5 x 10⁻⁴ to 5 x 10⁻⁵ M; oleic acid, *e.g.,* 0.1 µg/mL to 5 µg/mL; linoleic acid, *e.g.,* 0.1 µg/mL to 5 µg/mL; palmitic acid, *e.g.,* 0.05 µg/mL to 2 µg/mL; bovine serum albumin (BSA), *e.g.,* 1 µg/mL to 5 µg/mL; and/or phytohemagglutinin, e.g., 0.01 µg/mL to 1 µg/mL. In a more specific embodiment, said second medium comprises cell growth medium comprising human serum, FBS or FCS, *e.g.,* 10% v/v, IL-2, transferrin, insulin, ethanolamine, oleic acid, linoleic acid, palmitic acid, bovine serum albumin (BSA) and/or phytohemagglutinin. In a more specific embodiment, said second medium comprises Iscove's Modified Dulbecco's Medium (IMDM), 10% human serum, FBS or FCS, 400 IU/ml IL-2, 35 µg/mL transferrin, 5 µg/mL insulin, 2 x 10⁻⁵ M ethanolamine, 1 µg/mL oleic acid, 1 µg/mL linoleic acid, 0.2 µg/mL palmitic acid, 2.5 µg/mL BSA and 0.1 µg/mL phytohemagglutinin.

In certain embodiments, said second medium comprises GBGM®, AIM-V®, X-VIVO™ 10, X-VIVO™ 15, OPTMIZER, STEMSPAN® H3000, CELLGRO COMPLETE™, DMEM:Ham's F12 ("F12") *(e.g.,* 2:1 ratio, or high glucose or low glucose DMEM), EL08-1D2, Advanced DMEM (Gibco), Myelocult™ H5100, IMDM, and/or RPMI-1640. In certain embodiments, said medium comprises one or more of O-acetyl-carnitine, or a compound that affects acetyl-CoA cycling in mitochondria, thiazovivin, Y-27632, pyintegrin, Rho kinase (ROCK) inhibitors, caspase inhibitors or other anti-apoptotic compounds/peptides, NOVA-RS (Sheffield Bio-Science) or other small-molecule growth enhancers. In certain embodiments, said medium comprises one or more anti-oxidants, *e.g.,* holo-transferrin, insulin solution, reduced glutathione, sodium selenite, ethanolamine, ascorbic acid, b-mercaptoethanol, O-acetyl-L-carnitine, N-acetylcysteine, (+/-) lipoic acid, nicotinamide, or resveratrol. In certain embodiments, said medium comprises about 0.5 mM-10 mM OAC. In one embodiment, said medium comprises STEMSPAN® H3000, and/or DMEM:F12. In another embodiment, said medium comprises OAC, *e.g.,* about 0.5, 1, 2, 3, 4, 5, 6, 7, 8, 9, or 10 mM, and STEMSPAN® H3000, and/or DMEM:F12. In a specific embodiment, said medium comprises GBGM®. In another specific embodiment, said medium comprises DMEM:F12 and about 5 mM of OAC. In another specific embodiment, said medium comprises Stemspan® H3000 and about 5 mM of OAC.

In certain embodiments, said culturing in said second medium comprises culturing using feeder cells, *e.g.,* K562 cells *(e.g.,* mitomycin C-treated K562 cells) or PBMCs (*e.g.,* mitomycin C-treated PBMC), *e.g.,* at the time the cells are started in said second medium, or 1, 2, 3, 4, 5, 6, 7, 8, 9 or 10 days thereafter. In another embodiment, said culturing in said second medium does not comprise culturing using feeder cells. In certain embodiments, the second medium is added to the cells from the first culture step without removing the first culture medium.

In a specific embodiment, provided herein is a method of producing a population of activated natural killer (NK) cells, comprising: (a) seeding a population of hematopoietic stem or progenitor cells in a first medium as described herein, e.g., one comprising interleukin-15 (IL-15) and, optionally, one or more of stem cell factor (SCF) and interleukin-7 (IL-7), wherein said IL-15 and optional SCF and IL-7 are in addition to whatever, if any, amount of IL-15, SCF or IL-7 are present in an undefined component of said medium, such that the population expands, and a plurality of hematopoietic stem or progenitor cells within said population of hematopoietic stem or progenitor cells differentiate into NK cells during said expanding; and (b) expanding the cells from step (a) in a second medium comprising interleukin-2 (IL-2), wherein said IL-2 is in addition to whatever to whatever, if any, amount of IL-2 is present in an undefined component of said medium, to produce a population of activated NK cells.

In another specific embodiment, provided herein is a two-step method of producing a population of activated natural killer (NK) cells, wherein a first step of said method comprises expanding a population of hematopoietic stem or progenitor cells in a first medium comprising one or more of SCF, IL-7 and IL-15, wherein said SCF, IL-7 and IL-15 are in addition to whatever, if any, amount of IL-15, SCF or IL-7 are present in an undefined component of said medium (*e.g.,* serum), and wherein a plurality of hematopoietic stem or progenitor cells within said population of hematopoietic stem or progenitor cells differentiate into NK cells during said expanding; and wherein a second step of said method comprises expanding the cells from the first step in a second medium comprising IL-2, wherein said IL-2 is in addition to whatever, if any, amount of IL-2 is present in an undefined component of said medium, to produce activated NK cells. In another specific embodiment, said first medium further comprises one or more of Fms-like-tyrosine kinase 3 ligand (Flt3-L), thrombopoietin (Tpo), interleukin-2 (IL-2), and/or heparin (in addition to whatever, if any, amount is present in an undefined component of said medium). In another specific embodiment, said first medium further comprises about 5%-20% fetal bovine serum or human serum. In another specific embodiment, the SCF is present at a concentration of about 1 to about 150 ng/mL in the first medium. In another specific embodiment, the Flt3-L is present at a concentration of about 1 to about 150 ng/mL in the first medium. In another specific embodiment, the IL-2 is present at a concentration of about 50 to about 1500 IU/mL in the first medium. In another specific embodiment, the IL-7 is present at a concentration of about 1 to about 150 ng/mL in the first medium. In another specific embodiment, the IL-15 is present at a concentration 1 to about 150 ng/mL in the first medium. In another specific embodiment, the Tpo is present at a concentration of about 1 to about 150 ng/mL in the first medium. In another specific embodiment, the heparin is present at a concentration of about 0.1 to about 30 U/mL in the first medium. In another specific embodiment, the IL-2 in the second step is present at a concentration 50 to about 1500 IU/mL in the second medium. In another specific embodiment, said second medium additionally comprises one or more of fetal calf serum (FCS), transferrin, insulin, ethanolamine, oleic acid, linoleic acid, palmitic acid, bovine serum albumin (BSA) and phytohemagglutinin (in addition to whatever, if any, amount is present in an undefined component of said medium).

In certain specific embodiments, said hematopoietic stem or progenitor cells are cultured in said first medium for 1, 2, 3, 4, 5, 6, 7, 8, 9, 10, 11, 12, 13, 14, 15, 16, 17, 18, 19, 20, 21, 22, 23, 24, 25, 26, 27 or 28 days before said culturing in said second medium. In certain other specific embodiments, said cells are cultured in said second medium for 1, 2, 3, 4, 5, 6, 7, 8, 9, 10, 11, 12, 13, 14, 15, 16, 17, 18, 19, 20, 21, 22, 23, 24, 25, 26, 27 or 28 days. In a more specific embodiment, said hematopoietic stem or progenitor cells are cultured in said first medium for 21 days, and then cultured in said second medium for 21 days. In another specific embodiment, said hematopoietic stem or progenitor cells are cultured in said first medium for 21 days, and then cultured in said second medium for 14 days.

Further provided herein is a population of natural killer cells produced by the method described above, referred to herein as TSNK cells. In a specific embodiment, said NK cells (*e.g.,* TSNK cells) are CD3⁻CD56⁺. In a specific embodiment, said NK cells (*e.g.,* TSNK cells) are CD3⁻CD56⁺CD16⁻. In another specific embodiment, said NK cells (*e.g.,* TSNK cells) are additionally CD94⁺CD117⁺. In another specific embodiment, said NK cells (*e.g.,* TSNK cells) are additionally CD161⁻. In another specific embodiment, said NK cells (*e.g.,* TSNK cells) are additionally NKG2D⁺. In another specific embodiment, said NK cells are additionally NKp46⁺. In another specific embodiment, said NK cells are additionally CD226⁺.

In certain embodiments, greater than 90%, 92%, 94%, 96% or 98% of said TSNK cells are CD56⁺ and CD16⁻. In some embodiments, at least 80%, 82%, 84%, 86%, 88% or 90% of said TSNK cells are CD3⁻ and CD56⁺. In other embodiments, greater than 90%, 92%, 94%, 96% or 98% of said TSNK cells are CD56⁺, CD16⁻ and CD3⁻. In other embodiments, at least 50%, 52%, 54%, 56%, 58% or 60% of said TSNK cells are NKG2D⁺. In other embodiments, fewer than 10%, 9%, 8%, 7%, 6%, 5%, 4% or 3% of said TSNK cells are NKB1⁺. In certain other embodiments, fewer than 10%, 8%, 6%, 4% or 2% of said TSNK cells are NKAT2⁺. In certain other embodiments, fewer than 10%, 8%, 6%, 4% or 2% of said TSNK cells are CD56⁺ and CD16⁺. In more specific embodiments, at least 50%, 55%, 60%, 65% or 70% of said CD3⁻, CD56⁺ TSNK cells are NKp46⁺. In other more specific embodiments, at least 50%, 55%, 60%, 65%, 70%, 75%, 80% or 85% of said CD3⁻, CD56⁺ TSNK cells are CD117⁺. In other more specific embodiments, at least 20%, 25%, 30%, 35%, 40% or 45% of said CD3⁻, CD56⁺ TSNK cells are CD94⁺. In other more specific embodiments, at least 10%, 20%, 25%, 30%, 35%, 40%, 45% or 50% of said CD3⁻, CD56⁺ TSNK cells are CD161⁻. In other more specific embodiments, at least 10%, 12%, 14%, 16%, 18% or 20% of said CD3⁻, CD56⁺ TSNK cells are CD226⁺. In more specific embodiments, at least 20%, 25%, 30%, 35% or 40% of said CD3⁻, CD56⁺ TSNK cells are CD7⁺. In more specific embodiments, at least 30%, 35%, 40%, 45%, 50%, 55% or 60% of said CD3⁻, CD56⁺ TSNK cells are CD5⁺.

In one embodiment, a TSNK cell population comprises cells which are CD117+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% CD117⁺ cells. In one embodiment, a TSNK cell population comprises cells which are NKG2D+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% NKG2D⁺ cells. In one embodiment, a TSNK cell population comprises cells which are NKp44+. In a specific embodiment, said TSNK cell population comprise no more than about 5%, 10%, 15%, 20%, 25%, 30%, 35%, 40%, 45%, 50%, 55%, 60%, 65%, 70%, 75%, 80%, 85%, or 90% NKp44⁺ cells. In one embodiment, a TSNK cell population comprises cells which are CD52+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% CD52⁺ cells. In a particular embodiment, a TSNK cell population comprises cells which are CD52+ CD117+. In one embodiment, a TSNK cell population comprises cells which are CD244+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% CD244⁺ cells. In a particular embodiment, a TSNK cell population comprises cells which are CD244+ CD117+. In one embodiment, a TSNK cell population comprises cells which are LFA-1+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% LFA-1+ cells. In one embodiment, a TSNK cell population comprises cells which are CD94+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% CD94+ cells.

In another aspect, provided herein is a method that comprises the three stages described herein (and referred to herein as the "three-stage method") of producing NK cell populations or NK progenitor cell populations. Natural killer cells produced by the methods provided herein that use these three stages (*e.g.,* three-stage method) are referred to herein as NK progenitor cells or NK cells produced by the three-stage method. In certain embodiments, said method does not comprise any fourth or intermediate step in which the cells are contacted (or cultured).

In certain aspects, the three-stage method comprises a first stage ("stage 1") comprising culturing hematopoietic stem cells or progenitor cells, *e.g.,* CD34⁺ stem cells or progenitor cells, in a first medium for a specified time period, *e.g.,* as described herein. In certain embodiments, the first medium contains one or more factors that promote expansion of hematopoietic progenitor cells, one or more factors for initiation of lymphoid differentiation within the expanding hematopoietic progenitor population, and/or one or more factors that mimic stromal feeder support. In certain embodiments, the first medium comprises one or more cytokines (for example, Flt3L, TPO, SCF). In certain embodiments, the first medium comprises IL-7. In certain embodiments, the first medium comprises sub-ng/mL concentrations of G-CSF, IL-6 and/or GM-CSF. In a specific embodiment, the first medium comprises the cytokines Flt3L, TPO, and SCF, IL-7, and sub-ng/mL concentrations of G-CSF, IL-6 and GM-CSF. In specific embodiments, in the first medium, CD34+ cells undergo expansion into lineage specific progenitors, which then become CD34-. In certain embodiments, in the first medium, expansion of CD34+ cells is coupled with differentiation of the cells (differentiation as demonstrated, *e.g.,* by loss of multipotency, loss of progenitor cell status, loss of CD34 expression, and/or increase in CD34- lineage specific progenitors). In certain embodiments, in the first medium, CD34-lineage specific progenitors become predominant. In certain embodiments, the CD34- cells comprise more than 50%, more than 55%, more than 60%, more than 65%, more than 70%, more than 75%, more than 80%, or more of the total population at the end of stage 1. In a more specific embodiment, CD34- cells comprise more than 80% of the total population at the end of stage 1.

Subsequently, in "stage 2" said cells are cultured in a second medium for a specified time period, *e.g.,* as described herein. In certain embodiments, the second medium contains factors that can promote further expansion of lymphoid progenitors, factors that can contribute to development along the NK lineage, and/or factors that mimic stromal feeder support. In certain embodiments, the second medium comprises one or more cytokines (*e.g.,* Flt3L, SCF, IL-15, and/or IL-7). In certain embodiments, the second medium comprises IL-17 and/or IL-15. In certain embodiments, the second medium comprises sub-ng/mL concentrations of G-CSF, IL-6 and/or GM-CSF. In a specific embodiment, the second medium comprises the cytokines Flt3L, SCF, IL-15, and IL-7, IL-17 and IL-15, and sub-ng/mL concentrations of G-CSF, IL-6 and GM-CSF.

Subsequently, in "stage 3" said cells are cultured in a third medium for a specified time period, *e.g.,* as described herein. In certain embodiments, the third medium comprises factors that promote differentiation and functional activation of CD56+CD3-CD16- cells. In one embodiment, such factors comprise IL2 and IL12 and IL18, IL12 and IL15, IL12 and IL18, IL2 and IL12 and IL15 and IL18, or IL2 and IL15 and IL18. In certain embodiments, the third medium comprises factors that mimic stromal feeder support. In certain embodiments, the third medium comprises one or more cytokines (*e.g.,* SCF, IL-15, IL-7, IL-2). In certain embodiments, the third medium comprises sub-ng/mL concentrations of G-CSF, IL-6 and/or GM-CSF. In a specific embodiment, the third medium comprises the cytokines SCF, IL-15, IL-7, IL-2, and sub-ng/mL concentrations of G-CSF, IL-6 and GM-CSF.

In a specific embodiment, the three-stage method is used to produce NK progenitor cells. In another specific embodiment, the three-stage method is used to produce NK cells. In certain embodiments, the three-stage method is used to produce NK cells which are activated. In certain embodiments, the three-stage method is conducted in the absence of stromal feeder cell support.

In certain embodiments, the stage 2 medium and/or the stage 3 medium is added to the culture without removal of medium from one or more prior stages. In certain embodiments, stage 2 medium is added to stage 1 culture without removal of medium from the stage 1 culture, followed by the addition of stage 3 medium to the stage 2 culture without removal of medium from the stage 2 culture.

In certain aspects, the first medium used in the three-stage methods described herein may contain any of the components of the first medium and second medium described above in connection with the two-step method. In certain embodiments, said first medium used in the three-stage method comprises medium comprising one or more of: animal serum, e.g., human serum (*e.g.,* human serum AB), fetal bovine serum (FBS) or fetal calf serum (FCS), e.g., 1% to 20 % v/v serum, e.g., 5% to 20% v/v serum; stem cell factor (SCF), *e.g.,* 1 ng/mL to 50 ng/mL SCF; FMS-like tyrosine kinase-3 ligand (Flt-3 ligand), *e.g.,* 1 ng/ml to 30 ng/mL Flt-3 ligand; interleukin-7 (IL-7), *e.g.,* 1 ng/mL to 50 ng/mL IL-7; thrombopoietin (TPO), *e.g.,* 1 ng/mL to 100 ng/mL, for example, 1 ng/mL to 50 ng/mL TPO; interleukin-2 (IL-2), *e.g.,* up to 2000 IU/mL, for example, 50 IU/mL to 500 IU/mL; and/or heparin, *e.g.,* low-weight heparin (LWH), *e.g.,* 0.1 IU/mL to 10 IU/mL heparin. In certain embodiments, said first medium additionally comprises one or more of the following: antibiotics such as gentamycin; antioxidants such as transferrin, insulin, and/or beta-mercaptoethanol; sodium selenite; ascorbic acid; ethanolamine; and glutathione. In certain embodiments, said first medium additionally comprises OAC. In certain embodiments, said first medium additionally comprises interleukin-6 (IL-6), leukemia inhibitory factor (LIF), G-CSF, GM-CSF, and/or MIP-1α. In certain embodiments, said first medium additionally comprises one or more anti-oxidants, *e.g.,* holo-transferrin, insulin solution, reduced glutathione, sodium selenite, ethanolamine, ascorbic acid, b-mercaptoethanol, O-acetyl-L-carnitine, N-acetylcysteine, (+/-) lipoic Acid, nicotinamide, or resveratrol. In certain embodiments, the medium that provides the base for the first medium is a cell/tissue culture medium known to those of skill in the art, e.g., a commercially available cell/tissue culture medium such as GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640; or is a medium that comprises components generally included in known cell/tissue culture media, such as the components included in GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640.

The second medium used in the three-stage methods described herein may contain any of the components of the first medium and second medium described above in connection with the two-step method. In certain embodiments, said second medium used in the three-stage method comprises medium comprising one or more of: animal serum, e.g., human serum (*e.g.,* human serum AB), FBS or FCS, e.g., 5% to 20% v/v serum; SCF, *e.g.,* 1 ng/mL to 50 ng/mL SCF; Flt-3 ligand, *e.g.,* 1 ng/ml to 30 ng/mL Flt-3 ligand; IL-7, *e.g.,* 1 ng/mL to 50 ng/mL IL-7; interleukin-15 (IL-15), e.g., 1 ng/mL to 50 ng/mL IL-15; and/or heparin, e.g., LWH, *e.g.,* 0.1 IU/mL to 10 IU/mL heparin. In certain embodiments, said second medium additionally comprises one or more of the following: antibiotics such as gentamycin; antioxidants such as transferrin, insulin, and/or beta-mercaptoethanol; sodium selenite; ascorbic acid; ethanolamine; and glutathione. In certain embodiments, said second medium additionally comprises OAC. In certain embodiments, said second medium additionally comprises interleukin-6 (IL-6), leukemia inhibitory factor (LIF), G-CSF, GM-CSF, and/or MIP-1α. In certain embodiments, said second medium additionally comprises one or more anti-oxidants, *e.g.,* holo-transferrin, insulin solution, reduced glutathione, sodium selenite, ethanolamine, ascorbic acid, b-mercaptoethanol, O-acetyl-L-camitine, N-acetylcysteine, (+/-) lipoic acid, nicotinamide, or resveratrol. In certain embodiments, the medium that provides the base for the second medium is a cell/tissue culture medium known to those of skill in the art, e.g., a commercially available cell/tissue culture medium such as GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640; or is a medium that comprises components generally included in known cell/tissue culture media, such as the components included in GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640.

The third medium used in the three-stage methods described herein may contain any of the components of the first medium and second medium described above in connection with the two-step method. In certain embodiments, said third medium used in the three-stage method comprises medium comprising one or more of: animal serum, e.g., human serum (*e.g.,* human serum AB), FBS or FCS, e.g., 5% to 20% v/v serum; SCF, *e.g.,* 1 ng/mL to 50 ng/mL SCF; Flt-3 ligand, *e.g.,* 1 ng/ml to 30 ng/mL Flt-3 ligand; IL-7, *e.g.,* 1 ng/mL to 50 ng/mL IL-7; IL-15, e.g., 1 ng/mL to 50 ng/mL IL-15; and interleukin-2 (IL-2), e.g., in the range from 0 to 2000 IU/mL, for example, 50 IU/mL to 1000 IU/mL IL-2. In certain embodiments, said third medium additionally comprises one or more of the following: antibiotics such as gentamycin; antioxidants such as transferrin, insulin, and/or beta-mercaptoethanol; sodium selenite; ascorbic acid; ethanolamine; and glutathione. In certain embodiments, said third medium additionally comprises OAC. In certain embodiments, said third medium additionally comprises interleukin-6 (IL-6), leukemia inhibitory factor (LIF), G-CSF, GM-CSF, and/or MIP-1α. In certain embodiments, said third medium additionally comprises one or more anti-oxidants, *e.g.,* holo-transferrin, insulin solution, reduced glutathione, sodium selenite, ethanolamine, ascorbic acid, b-mercaptoethanol, O-acetyl-L-carnitine, N-acetylcysteine, (+/-) lipoic acid, nicotinamide, or resveratrol. In certain embodiments, the medium that provides the base for the third medium is a cell/tissue culture medium known to those of skill in the art, e.g., a commercially available cell/tissue culture medium such as GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), EL08-1D2, Advanced DMEM (Gibco), Myelocult™ H5100, IMDM, and/or RPMI-1640; or is a medium that comprises components generally included in known cell/tissue culture media, such as the components included in GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640.

In certain embodiments, in the three-stage methods described herein, said hematopoietic stem or progenitor cells are cultured in said first medium for 1, 2, 3, 4, 5, 6, 7, 8, 9, 10, 11, 12, 13, 14, 15, 16, 17, 18, 19, or 20 days before said culturing in said second medium. In certain embodiments, cells cultured in said first medium are cultured in said second medium for 1, 2, 3, 4, 5, 6, 7, 8, 9, 10, 11, 12, 13, 14, 15, 16, 17, 18, 19, or 20 days before said culturing in said third medium. In certain embodiments, cells cultured in said first medium and said second medium are cultured in said third medium for 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days, or for more than 30 days.

In certain embodiments, in the three-stage methods described herein, said hematopoietic stem or progenitor cells are cultured in said first medium for 2-12 days, 3-11 days, for example, 3-5, 4-6, 5-7, 6-8, 7-9, 8-10, or 9-11 days, before said culturing in said second medium. In certain embodiments, cells cultured in said first medium are cultured in said second medium for 1-10 days, for example, 1-3, 2-4, 3-5, 4-6, 5-7, 6-8, or 7-9 days, before said culturing in said third medium. In certain embodiments, cells cultured in said first medium and said second medium are cultured in said third medium for 2-27 days, for example, 3-25 days, e.g., for 3-5, 4-6, 5-7, 6-8, 7-9, 8-10, 9-11, 10-12, 11-13, 12-14, 13-15, 14-16, 15-17, 16-18, 17-19, 18-20, 19-21, 20-22, 21-23, 22-24, or 23-25 days.

In one embodiment, in the three-stage methods described herein, said hematopoietic stem or progenitor cells are cultured in said first medium for 7-9 days before said culturing in said second medium; cultured in said second medium for 5-7 days before said culturing in said third medium; and cultured in said third medium for 5-9 days, i.e., the cells are cultured a total of 17-25 days.

In a specific embodiment, in the three-stage methods described herein, said hematopoietic stem or progenitor cells are cultured in said first medium for 9 days before said culturing in said second medium; cultured in said second medium for 5 days before said culturing in said third medium; and cultured in said third medium for 7 days, i.e., the cells are cultured a total of 21 days.

In one embodiment, in the three-stage methods described herein, said hematopoietic stem or progenitor cells are cultured in said first medium for 7-9 days before said culturing in said second medium; cultured in said second medium for 5-7 days before said culturing in said third medium; and cultured in said third medium for 21-31 days, i.e., the cells are cultured a total of 33-47 days.

In a specific embodiment, in the three-stage methods described herein, said hematopoietic stem or progenitor cells are cultured in said first medium for 9 days before said culturing in said second medium; cultured in said second medium for 5 days before said culturing in said third medium; and cultured in said third medium for 21 days, i.e., the cells are cultured a total of 35 days.

In one embodiment, provided herein is an isolated NK progenitor cell population, wherein said NK progenitor cells are produced according to the three-stage method described herein.

In another embodiment, provided herein is an isolated NK cell population, wherein said NK cells are produced according to the three-stage method described herein.

In another embodiment, provided herein is an isolated NK cell population, wherein said NK cells are activated, wherein said activated NK cells are produced according to the three-stage method described herein.

Without being bound by any particular theory of operation, it has been discovered by the inventors that at certain time points of the three-stage method, cell populations can be isolated that possess characteristics, e.g., structural and functional characteristics, that are distinct from the characteristics of cell populations isolated at other time points of the three-stage method. For example, cell populations generated using a three-stage method as described herein having a shorter third culture step have characteristics distinct from cell populations generated using a three-stage method as described herein having a longer third step. For example, all else being equal, cell populations isolated from a three-stage method having a short third culture step, e.g., a third culture step of 4-6, 5-7, 6-8, or 7-9 days are distinct from cell populations isolated from a three-stage method having a long third culture step, e.g., a third culture step of 18-20, 19-21, 20-22, or 21-23 days. Such distinct cell populations isolated from a three-stage method having a short third culture step constitute NK progenitor cell populations (that is, constitute cell populations that comprise a higher percentage of NK progenitor cells than NK cells), whereas cell populations isolated from a three-stage method having a long third culture step constitute NK cell populations (that is, constitute cell populations that comprise a higher percentage of NK cells than NK progenitor cells).

Accordingly, provided herein is an isolated NK progenitor cell population produced by a three-stage method described herein. In a specific embodiment, said NK progenitor cell population comprises a low percentage of CD3⁻CD56⁺ cells as compared to the percentage of CD3⁻CD56⁺ cells associated with NK cell populations, such as NK cell populations produced by the three-stage methods described herein, e.g., the NK progenitor cell population comprises about 5%, 10%, 15%, 20%, 25%, 30%, 35%, 40%, 45%, or 50% CD3⁻CD56⁺ cells. In another specific embodiment, said NK progenitor cell population comprises no more than 5%, 10%, 15%, 20%, 25%, 30%, 35%, 40%, 45%, or 50% CD3⁻CD56⁺ cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% CD3⁻CD56⁺ cells. In some embodiments, said NK progenitor cell populations, e.g., a NK progenitor cell populations that comprise a low percentage of CD3⁻CD56⁺ cells as compared to the percentage of CD3⁻CD56⁺ cells associated with NK cell populations, comprise no more than 1%, no more than 2%, no more than 3%, no more than 4%, no more than 5%, no more than 10%, or no more than 15% CD3⁻CD56⁺ cells. In another specific embodiment, said NK progenitor cell populations produced by a three-stage method described herein are produced using a three-stage method that comprises a short third culture step, e.g., a third culture step of 4-6, 5-7, 6-8, or 7-9 days. In particular embodiments, an NK progenitor cell population produced by a three-stage method described herein comprises a greater proportion of CD56- cells than CD56+ cells. In particular embodiments, an NK progenitor cell population produced by a three-stage method described herein differentiates in vivo or ex vivo into a population with an increased proportion of CD56+ cells.

In certain embodiments, said CD3⁻CD56⁺ cells in said NK progenitor cell populations are additionally CD117⁺. In a specific embodiment, about 65%, 70%, 75%, 80%, 85%, 90%, 95%, 98%, or 99% of said CD3⁻CD56⁺ cells in said NK progenitor cell populations are CD117⁺. In another specific embodiment, no less than 65%, 70%, 75%, 80%, 85%, 90%, 95%, 98%, or 99% of said CD3⁻CD56⁺ cells in said NK progenitor cell populations are CD117⁺. In another specific embodiment, between 65%-70%, 70%-75%, 75%-80%, 80%-85%, 85%-90%, 90%-95%, or 95%-99% of said CD3⁻CD56⁺ cells in said NK progenitor cell populations are CD117⁺.

In certain embodiments, said CD3⁻CD56⁺ cells in said NK progenitor cell populations are additionally CD161⁺. In a specific embodiment, about 40%, 45%, 50%, 55%, 60%, 65%, 70%, or 75% of said CD3⁻CD56⁺ cells in said NK progenitor cell populations are CD161⁺. In another specific embodiment, no less than 40%, 45%, 50%, 55%, 60%, 65%, 70%, or 75% of said CD3⁻CD56⁺ cells in said NK progenitor cell populations are CD161⁺. In another specific embodiment, between 40%-45%, 45%-50%, 50%-55%, 55%-60%, 60%-65%, 65%-70%, or 70%-75% of said CD3⁻CD56⁺ cells in said NK progenitor cell populations are CD161⁺.

In certain embodiments, said CD3⁻CD56⁺ cells in said NK progenitor cell populations are additionally NKp46⁺. In a specific embodiment, about 25%, 30%, 35%, 40%, 45%, 50%, 55%, 60%, 65%, 70%, 75%, 80%, 85%, 90% or more of said CD3⁻CD56⁺ cells in said NK progenitor cell populations are NKp46⁺. In a more specific embodiment, about 25%, 30%, 35%, 40%, 45%, 50%, or 55% of said CD3⁻CD56⁺ cells in said NK progenitor cell populations are NKp46⁺. In another specific embodiment, no more than 25%, 30%, 35%, 40%, 45%, 50%, or 55% of said CD3⁻CD56⁺ cells in said NK progenitor cell populations are NKp46⁺. In another specific embodiment, between 25%-30%, 30%-35%, 35%-40%, 40%-45%, 45%-50%, 50%-55%, 55%-60%, 60%-65%, 65%-70%, 70%-75%, 75%-80%, or 85%-90% of said CD3⁻CD56⁺ cells in said NK progenitor cell populations are NKp46⁺. In a more specific embodiment, between 25%-30%, 30%-35%, 35%-40%, 40%-45%, 45%-50%, or 50%-55% of said CD3⁻CD56⁺ cells in said NK progenitor cell populations are NKp46⁺.

In certain embodiments, said NK progenitor cell population contains cells that are CD56⁺CD16⁻. In certain embodiments, CD3⁻CD56⁺ cells in said NK progenitor cell populations are CD16⁻. In certain embodiments, CD3⁻CD56⁺ cells in said NK progenitor cell populations are CD16⁺. In a specific embodiment, said NK progenitor cell populations comprise no more than 5%, 10%, 15%, 20%, 25%, 30%, 35%, 40%, 45%, or 50% CD16⁺ cells. In another specific embodiment, said NK progenitor cell populations comprise between 0%-5%, 5%-10%, 10%-15%, 15%-20%, or 20%-25% CD16⁺ cells. In some embodiments, said NK progenitor cell populations comprise no more than 1%, no more than 2%, no more than 3%, no more than 4%, no more than 5%, no more than 10%, or no more than 15% CD16⁺ cells.

In certain embodiments, said CD3⁻CD56⁺ cells in said NK progenitor cell populations are additionally CD16⁻. In certain embodiments, said CD3⁻CD56⁺ cells in said NK progenitor cell populations are additionally CD117⁺ and CD161⁺. In certain embodiments, said CD3⁻CD56⁺ cells in said NK progenitor cell populations are additionally CD16⁻, CD117⁺ and CD161⁺. In certain embodiments, said CD3⁻CD56⁺ cells in said NK progenitor cell populations are additionally CD16⁻, CD117⁺, CD161⁺, and NKp46⁺. In one embodiment, an NK progenitor cell population produced by a three-stage method described herein comprises no more than about 40% CD3-CD56+ cells.

In one embodiment, an NK progenitor cell population produced by a three-stage method described herein comprises cells which are CD117+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% CD117⁺ cells. In one embodiment, an NK progenitor cell population produced by a three-stage method described herein comprises cells which are NKG2D+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% NKG2D⁺ cells. In one embodiment, an NK progenitor cell population produced by a three-stage method described herein comprises cells which are NKp44+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% NKp44⁺ cells. In one embodiment, an NK progenitor cell population produced by a three-stage method described herein comprises cells which are CD52+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% CD52⁺ cells. In a particular embodiment, said NK progenitor cell population produced by a three-stage method described herein comprises cells which are CD52+ CD117+. In one embodiment, an NK progenitor cell population produced by a three-stage method described herein comprises cells which are CD244+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% CD244⁺ cells. In a particular embodiment, said NK progenitor cell population produced by a three-stage method described herein comprises cells which are CD244+ CD117+. In one embodiment, an NK progenitor cell population produced by a three-stage method described herein comprises cells which are LFA-1+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% LFA-1+ cells. In one embodiment, an NK progenitor cell population produced by a three-stage method described herein comprises cells which are CD94+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% CD94+ cells.

In a specific embodiment, an NK progenitor cell population produced by a three-stage method described herein comprises a low percentage of CD34⁻CD117⁺ cells as compared to the percentage of CD34⁻CD117⁺ cells associated with an NK cell population, e.g., the NK progenitor cell population comprises about 5%, 10%, 15%, 20%, 25%, 30%, 35%, 40%, 45%, or 50% CD34⁻CD117⁺ cells. In another specific embodiment, said NK progenitor cell population comprises no more than 5%, 10%, 15%, 20%, 25%, 30%, 35%, 40%, 45%, or 50% CD34⁻CD117⁺ cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% CD34⁻CD117⁺ cells. In some embodiments, said NK progenitor cell population comprises no more than 1%, no more than 2%, no more than 3%, no more than 4%, no more than 5%, no more than 10%, or no more than 15% CD34⁻CD117⁺ cells. In another specific embodiment, said NK progenitor cell population produced by a three-stage method described herein is produced using a three-stage method that comprises a short third culture step, e.g., a third culture step of 4-6, 5-7, 6-8, or 7-9 days. In certain embodiments, NK progenitor cells produced using a three-stage method that comprises a third culture step, e.g., a third culture step of 4-6, 5-7, 6-8, or 7-9 days engraft bone marrow (e.g., in vivo) at a higher efficiency than NK cells produced using a three-stage method that comprises a longer third culture step, e.g., a third culture step of 18-20, 19-21, 20-22, or 21-23 days.

In a specific embodiment, an NK progenitor cell population produced by a three-stage method described herein comprises a low percentage of CD161+ cells as compared to the percentage of CD161⁺ cells associated with an NK cell population, e.g., the NK progenitor cell population comprises about 5%, 10%, 15%, 20%, 25%, 30%, 35%, 40%, 45%, or 50% CD161⁺ cells. In another specific embodiment, said NK progenitor cell population comprises no more than 5%, 10%, 15%, 20%, 25%, 30%, 35%, 40%, 45%, or 50% CD161⁺ cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% CD161⁺ cells. In some embodiments, said NK progenitor cell population comprises no more than 1%, no more than 2%, no more than 3%, no more than 4%, no more than 5%, no more than 10%, or no more than 15% CD161⁺ cells. In another specific embodiment, said NK progenitor cell population produced by a three-stage method described herein is produced using a three-stage method that comprises a short third culture step, e.g., a third culture step of 4-6, 5-7, 6-8, or 7-9 days.

In a specific embodiment, an NK progenitor cell population produced by a three-stage method described herein comprises a low percentage of NKp46⁺ cells as compared to the percentage of NKp46⁺ cells associated with an NK cell population, e.g., the NK progenitor cell population comprises about 1%, 5%, 10%, 15%, 20%, 25%, 30%, 35%, 40%, 45%, or 50% NKp46⁺ cells. In another specific embodiment, said NK progenitor cell population comprises no more than 1%, 5%, 10%, 15%, 20%, 25%, 30%, 35%, 40%, 45%, or 50% NKp46⁺ cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% NKp46⁺ cells. In some embodiments, said NK progenitor cell population comprises no more than 1%, no more than 2%, no more than 3%, no more than 4%, no more than 5%, no more than 10%, or no more than 15% NKp46⁺ cells. In another specific embodiment, said NK progenitor cell population produced by a three-stage method described herein is produced using a three-stage method that comprises a short third culture step, e.g., a third culture step of 4-6, 5-7, 6-8, or 7-9 days.

In a specific embodiment, an NK progenitor cell population produced by a three-stage method described herein comprises a low percentage of CD56⁺CD16- cells as compared to the percentage of CD56⁺CD16- cells associated with an NK cell population, e.g., the NK progenitor cell population comprises about 1%, 5%, 10%, 15%, 20%, 25%, 30%, 35%, 40%, 45%, or 50% CD56⁺CD16- cells. In another specific embodiment, said NK progenitor cell population comprises no more than 1%, 5%, 10%, 15%, 20%, 25%, 30%, 35%, 40%, 45%, or 50% CD56⁺CD16- cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% CD56⁺CD16- cells. In some embodiments, said NK progenitor cell population comprises no more than 1%, no more than 2%, no more than 3%, no more than 4%, no more than 5%, no more than 10%, or no more than 15% CD56⁺CD16- cells. In another specific embodiment, said NK progenitor cell population produced by a three-stage method described herein is produced using a three-stage method that comprises a short third culture step, e.g., a third culture step of 4-6, 5-7, 6-8, or 7-9 days.

In one embodiment, an NK progenitor cell population produced by a three-stage method described herein comprises cells that are CD52+CD117+. In a specific embodiment, an NK progenitor cell population produced by a three-stage method described herein comprises a higher percentage of CD52⁺CD117+ cells as compared to the percentage of CD52⁺CD117⁺ cells associated with a hematopoietic progenitor cell population. In a specific embodiment, an NK progenitor cell population produced by a three-stage method described herein comprises a higher percentage of CD52⁺CD117+ cells as compared to the percentage of CD52⁺CD117+ cells associated with an NK cell population, e.g., the NK progenitor cell population comprises about 50%, 55%, 60%, 65%, 70%, 75%, 80%, 85%, 90% or more CD52⁺CD117+ cells. In another specific embodiment, said NK progenitor cell population comprises no less than 50%, 55%, 60%, 65%, 70%, 75%, 80%, 85%, or 90% CD52⁺CD117+ cells. In another specific embodiment, said NK progenitor cell population comprises between 50%-55%, 55%-60%, 60%-65%, 65%-70%, 70%-75%, 75%-80%, 80%-85%, 85%-90%, 90%-95% or more CD52⁺CD117+ cells. In another specific embodiment, said NK progenitor cell population which comprises CD52⁺CD117+ cells produced by a three-stage method described herein is produced using a three-stage method that comprises a short third culture step, e.g., a third culture step of 4-6, 5-7, 6-8, or 7-9 days. In a specific embodiment, said NK progenitor cell population which comprises CD52⁺CD117+ cells is produced using a three-stage method that comprises a total of 12 days or more, 13 days or more, 14 days or more, 15 days or more, 16 days or more, 17 days or more, 18 days or more, 19 days or more, 20 days or more, or 21 days or more of culture. In a specific embodiment, said NK progenitor cell population which comprises CD52⁺CD117+ cells is produced using a three-stage method that comprises a total of at least 12 days, 13 days, or 14 days of culture but not more than 21-25 days, 25-30 days, or 30-35 days of culture. In a specific embodiment, said NK progenitor cell population which comprises CD52⁺CD117+ cells is produced using a three-stage method that comprises a total of 21 days of culture.

Further provided herein is an isolated NK cell population produced by a three-stage method described herein, wherein said NK cell population comprises a greater percentage of CD3⁻CD56⁺ cells than an NK progenitor cell population produced by a three-stage method described herein, e.g., an NK progenitor cell population produced by the same three-stage method with the exception that the third culture step used to produce the NK progenitor cell population was of shorter duration than the third culture step used to produce the NK cell population. In a specific embodiment, said NK cell population comprises about 65%, 70%, 75%, 80%, 85%, 90%, 95%, 98%, or 99% CD3⁻CD56⁺ cells. In another specific embodiment, said NK cell population comprises no less than 65%, 70%, 75%, 80%, 85%, 90%, 95%, 98%, or 99% CD3⁻CD56⁺ cells. In another specific embodiment, said NK cell population comprises between 65%-70%, 70%-75%, 75%-80%, 80%-85%, 85%-90%, 90%-95%, or 95%-99% CD3⁻CD56⁺ cells. In another specific embodiment, said NK cell population produced by a three-stage method described herein is produced using a three-stage method that comprises a long third culture step, e.g., a third culture step of 18-20, 19-21, 20-22, or 21-23 days.

In certain embodiments, said CD3⁻CD56⁺ cells in said NK cell population comprises CD3⁻CD56⁺ cells that are additionally CD117⁺, wherein said NK cell population comprises a lesser percentage of CD3⁻CD56⁺CD117⁺ cells than an NK progenitor cell population produced by a three-stage method described herein, e.g., an NK progenitor cell population produced by the same three-stage method with the exception that the third culture step used to produce the NK progenitor cell population was of shorter duration than the third culture step used to produce the NK cell population.

In certain embodiments, said CD3⁻CD56⁺ cells in said NK cell population comprises CD3⁻CD56⁺ cells that are additionally CD161⁺, wherein said NK cell population comprises a lesser percentage of CD3⁻CD56⁺CD161⁺ cells than an NK progenitor cell population produced by a three-stage method described herein, e.g., an NK progenitor cell population produced by the same three-stage method with the exception that the third culture step used to produce the NK progenitor cell population was of shorter duration than the third culture step used to produce the NK cell population.

In certain embodiments, said CD3⁻CD56⁺ cells in said NK cell population comprises CD3⁻CD56⁺ cells that are additionally NKp46⁺, wherein said NK cell population comprises a greater percentage of CD3⁻CD56⁺NKp46⁺ cells than an NK progenitor cell population produced by a three-stage method described herein, e.g., an NK progenitor cell population produced by the same three-stage method with the exception that the third culture step used to produce the NK progenitor cell population was of shorter duration than the third culture step used to produce the NK cell population.

In one embodiment, an NK cell population produced by a three-stage method described herein comprises cells which are CD117+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% CD117⁺ cells. In one embodiment, an NK cell population produced by a three-stage method described herein comprises cells which are NKG2D+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% NKG2D⁺ cells. In one embodiment, an NK cell population produced by a three-stage method described herein comprises cells which are NKp44+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% NKp44⁺ cells. In one embodiment, an NK cell population produced by a three-stage method described herein comprises cells which are CD52+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% CD52⁺ cells. In a particular embodiment, said NK cell population produced by a three-stage method described herein comprises cells which are CD52+ CD117+. In one embodiment, an NK cell population produced by a three-stage method described herein comprises cells which are CD244+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% CD244⁺ cells. In a particular embodiment, said NK cell population produced by a three-stage method described herein comprises cells which are CD244+ CD117+. In one embodiment, an NK cell population produced by a three-stage method described herein comprises cells which are LFA-1+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% LFA-1+ cells. In one embodiment, an NK cell population produced by a three-stage method described herein comprises cells which are CD94+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% CD94+ cells.

In a specific embodiment, the NK progenitor cell populations described herein possess a greater ability to engraft bone marrow (e.g., in vivo) than NK cell populations, for example, the NK cell populations described herein, e.g., NK cell populations having a higher percentage of CD56+ cells than the NK progenitor cell population. In a specific embodiment, the NK progenitor cell populations described herein possess a greater ability to engraft bone marrow (e.g., in vivo) than NK cell populations, for example, the NK cell populations described herein, e.g., NK cell populations having a high percentage of CD16+ cells. In certain embodiments, NK progenitor cells and/or NK cells cultured for a shorter period of time engraft bone marrow at a higher efficiency than NK progenitor cells and/or NK cells cultured for a longer period of time. For example, in certain embodiments, NK progenitor cell populations produced using a three-stage method that comprises a short third culture step, e.g., a third culture step of 4-6, 5-7, 6-8, or 7-9 days, engraft bone marrow (e.g., in vivo) at a higher efficiency than NK cell populations produced using a three-stage method that comprises a long third culture step, e.g., a third culture step of 18-20, 19-21, 20-22, or 21-23 days. The engrafting cells of the NK progenitor cell populations described herein also are capable of persisting and replicating in vivo, e.g., in bone marrow. Without being bound by any particular theory of operation, it is believed that the ability of the engrafting cells of the NK progenitor cell populations described herein to both engraft bone marrow in vivo to a greater degree than the cells of NK cell populations and to perpetuate/replicate in vivo indicates that such cells are ideal for use in in vivo methods for which NK cell activity is beneficial. For example, in certain embodiments, the NK progenitor cell populations described herein can be used in methods of treatment, e.g., treatment of hematologic cancer, wherein said methods comprise administration of said NK progenitor cell populations to patients in need of such treatment. In accordance with such embodiments, said cells from such NK progenitor cell populations can persist in high numbers in vivo, ultimately maturing into NK cells that yield the desired therapeutic effect. In certain embodiments, said NK progenitor cell populations can be co-administered with a second agent that promotes differentiation/maturation of the cells of the NK progenitor cell populations into NK cells, for example, a second agent capable off inducing NK cell maturation/differentiation can be administered before, concurrently with, or following the administration of the NK progenitor cell populations to the patients.

Accordingly, in another aspect, provided herein is the use of TSNK cells; and/or NK progenitor cell populations described herein and/or NK cell populations isolated using the three-stage methods described herein to suppress tumor cell proliferation, treat viral infection, or treat cancer, e.g., blood cancers and solid tumors. In certain embodiments, the TSNK cells, NK progenitor cell populations, and/or NK cell populations are contacted with, or used in combination with, an immunomodulatory compound, e.g., an immunomodulatory compound described herein, or thalidomide. In certain embodiments, the TSNK cells, NK progenitor cell populations, and/or NK cell populations are treated with, or used in combination with, an immunomodulatory compound, e.g., an immunomodulatory compound described herein, or thalidomide.

In a specific embodiment, said cancer is a solid tumor. In another embodiment, said cancer is a blood cancer. In specific embodiments, the cancer is glioblastoma, primary ductal carcinoma, leukemia, acute T cell leukemia, chronic myeloid lymphoma (CML), acute myelogenous leukemia (AML), chronic myelogenous leukemia (CML), lung carcinoma, colon adenocarcinoma, histiocytic lymphoma, colorectal carcinoma, colorectal adenocarcinoma, prostate cancer, multiple myeloma, or retinoblastoma.

In another specific embodiment, the hematopoietic cells, *e.g.,* hematopoietic stem cells or progenitor cells, from which the TSNK cells are produced and/or from which the NK progenitor cell populations, and/or NK cell populations are produced, are obtained from placental perfusate, umbilical cord blood or peripheral blood. In one embodiment, the hematopoietic cells, *e.g.,* hematopoietic stem cells or progenitor cells, from which the TSNK cells are produced and/or from which the NK progenitor cell populations, and/or NK cell populations are produced, are obtained from placenta, *e.g.,* placental perfusate. In one embodiment, the hematopoietic cells, *e.g.,* hematopoietic stem cells or progenitor cells, from which the TSNK cells are produced and/or from which the NK progenitor cell populations, and/or NK cell populations are produced, are not obtained from umbilical cord blood. In one embodiment, the hematopoietic cells, *e.g.,* hematopoietic stem cells or progenitor cells, from which the TSNK cells are produced and/or from which the NK progenitor cell populations, and/or NK cell populations are produced, are not obtained from peripheral blood. In another specific embodiment, the hematopoietic cells, *e.g.,* hematopoietic stem cells or progenitor cells, from which the TSNK cells are produced and/or from which the NK progenitor cell populations and/or NK cell populations are produced, are combined cells from placental perfusate and cord blood, *e.g.,* cord blood from the same placenta as the perfusate. In another specific embodiment, said umbilical cord blood is isolated from a placenta other than the placenta from which said placental perfusate is obtained. In certain embodiments, the combined cells can be obtained by pooling or combining the cord blood and placental perfusate. In certain embodiments, the cord blood and placental perfusate are combined at a ratio of 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like by volume to obtain the combined cells. In a specific embodiment, the cord blood and placental perfusate are combined at a ratio of from 10:1 to 1:10, from 5:1 to 1:5, or from 3:1 to 1:3. In another specific embodiment, the cord blood and placental perfusate are combined at a ratio of 10:1, 5:1, 3:1, 1:1, 1:3, 1:5 or 1:10. In a more specific embodiment, the cord blood and placental perfusate are combined at a ratio of 8.5:1.5 (85%:15%).

In certain embodiments, the cord blood and placental perfusate are combined at a ratio of 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like by total nucleated cells (TNC) content to obtain the combined cells. In a specific embodiment, the cord blood and placental perfusate are combined at a ratio of from 10:1 to 10:1, from 5:1 to 1:5, or from 3:1 to 1: 3. In another specific embodiment, the cord blood and placental perfusate are combined at a ratio of 10:1, 5:1, 3:1, 1:1, 1:3, 1:5 or 1:10.

In one embodiment, therefore, provided herein is a method of treating an individual having cancer or a viral infection, comprising administering to said individual an effective amount of isolated TSNK cells. In another embodiment, provided herein is a method of treating an individual having cancer or a viral infection, comprising administering to said individual an effective amount of an isolated NK progenitor cell population described herein, e.g., an isolated NK progenitor cell population produced using the three-stage methods described. In another embodiment, provided herein is a method of treating an individual having cancer or a viral infection, comprising administering to said individual an effective amount of an isolated NK cell population produced using the three-stage methods described herein. In certain embodiments, the cancer is a solid tumor. In certain embodiments, the cancer is a hematological cancer. In a specific embodiment, the hematological cancer is leukemia. In another specific embodiment, the hematological cancer is lymphoma. In another specific embodiment, the hematological cancer is acute myeloid leukemia. In another specific embodiment, the hematological cancer is chronic lymphocytic leukemia. In another specific embodiment, the hematological cancer is chronic myelogenous leukemia.

In a specific embodiment, the isolated TSNK cells, and/or the isolated NK progenitor cell populations described herein and/or NK cell populations isolated using the three-stage methods described herein have been treated with an immunomodulatory compound, *e.g.* an immunomodulatory compound described herein, or thalidomide, prior to said administration. In a specific embodiment, the isolated TSNK cells, and/or the isolated NK progenitor cell populations described herein and/or NK cell populations isolated using the three-stage methods described herein have been treated with IL2 and IL12 and IL18, IL12 and IL15, IL12 and IL18, IL2 and IL12 and IL15 and IL18, or IL2 and IL15 and IL18 prior to said administration. In another specific embodiment, the method comprises administering to the individual (1) an effective amount of isolated TSNK cells, an effective amount of an isolated NK progenitor cell population, or an effective amount of an isolated NK cell population produced using a three-stage method described herein; and (2) an effective amount of an immunomodulatory compound or thalidomide. An "effective amount" in this context means an amount of TSNK cells, or an amount of cells in an NK progenitor cell population, or NK cell population, and optionally immunomodulatory compound or thalidomide, that results in a detectable improvement in one or more symptoms of said cancer or said infection, compared to an individual having said cancer or said infection who has not been administered said TSNK cells, said NK progenitor cell population, or said NK cell population and, optionally, an immunomodulatory compound or thalidomide. In a specific embodiment, said immunomodulatory compound is lenalidomide or pomalidomide. In another embodiment, the method additionally comprises administering an anticancer compound to the individual, *e.g.,* one or more of the anticancer compounds described below.

In another embodiment, provided herein is a method of suppressing the proliferation of tumor cells comprising bringing a therapeutically effective amount of TSNK cells into proximity with the tumor cells, *e.g.,* contacting the tumor cells with the TSNK cells. Hereinafter, unless noted otherwise, the term "proximity" refers to sufficient proximity to elicit the desired result; *e.g.,* in certain embodiments, the term proximity refers to contact. In another embodiment, provided herein is a method of suppressing the proliferation of tumor cells comprising bringing a therapeutically effective amount of an isolated NK progenitor cell population produced using the three-stage methods described herein into proximity with the tumor cells, *e.g.,* contacting the tumor cells with the NK progenitor cells.

In a specific embodiment, the isolated TSNK cells, and/or the isolated NK progenitor cell population or NK cell population produced using the three-stage methods described herein have been treated with an immunomodulatory compound, *e.g.* an immunomodulatory compound described herein, below, or thalidomide, and/or IL2 and IL12 and IL18, IL12 and IL15, IL12 and IL18, IL2 and IL12 and IL15 and IL18, or IL2 and IL15 and IL18, prior to said contacting or bringing into proximity. In another specific embodiment, an effective amount of an immunomodulatory compound, *e.g.* an immunomodulatory compound described in herein, below, or thalidomide is additionally brought into proximity with the tumor cells *e.g.,* the tumor cells are contacted with the immunomodulatory compound or thalidomide. An "effective amount" in this context means an amount of TSNK cells, cells in an NK progenitor cell population, or cells in an NK cell population, and optionally an immunomodulatory compound or thalidomide, that results in a detectable suppression of said tumor cells compared to an equivalent number of tumor cells not contacted or brought into proximity with said TSNK cells, cells in an NK progenitor cell population, or cells in an NK cell population, and optionally an immunomodulatory compound or thalidomide. In another specific embodiment, the method further comprises bringing an effective amount of an anticancer compound, *e.g.,* an anticancer compound described below, into proximity with the tumor cells, *e.g.,* contacting the tumor cells with the anticancer compound.

In a specific embodiment of this method, the tumor cells are blood cancer cells. In another specific embodiment, the tumor cells are solid tumor cells. In another embodiment, the tumor cells are primary ductal carcinoma cells, leukemia cells, acute T cell leukemia cells, chronic myeloid lymphoma (CML) cells, acute myelogenous leukemia cells (AML), chronic myelogenous leukemia (CML) cells, glioblastoma cells, lung carcinoma cells, colon adenocarcinoma cells, histiocytic lymphoma cells, multiple myeloma cells, retinoblastoma cell, colorectal carcinoma cells, prostate cancer cells, or colorectal adenocarcinoma cells. In another specific embodiment, said contacting or bringing into proximity takes place *in vitro.* In another specific embodiment, said contacting or bringing into proximity takes place *in vivo.* In a more specific embodiment, said *in vivo* contacting or bringing into proximity takes place in a human.

In another aspect, provided herein is a method of treating an individual having multiple myeloma, comprising administering to the individual (1) lenalidomide; (2) melphalan; and (3) expanded NK cells, wherein said NK cells are effective to treat multiple myeloma in said individual. In a specific embodiment, said NK cells are cord blood NK cells, or NK cells produced from cord blood hematopoietic cells, *e.g.,* hematopoietic stem cells. In another embodiment, said NK cells have been produced by any of the methods described herein for producing NK cells, *e.g.,* for producing TSNK cells or for producing NK cell populations using a three-stage method. In another embodiment, said NK cells have been expanded prior to said administering. In another embodiment, said lenalidomide, melphalan, and/or NK cells are administered separately from each other. In certain specific embodiments of the method of treating an individual with multiple myeloma, said NK cells are produced by a two-step method of producing a population of activated natural killer (NK) cells, wherein a first step of said method comprises expanding a population of hematopoietic stem or progenitor cells in a first medium comprising one or more of stem cell factor (SCF), interleukin-7 (IL-7) and interleukin-15 (IL-15), and wherein said SCF, IL-7 and IL-15 are not comprised within an undefined component of said medium, and wherein a plurality of hematopoietic stem or progenitor cells within said population of hematopoietic stem or progenitor cells differentiate into NK cells during said expanding; and wherein a second step of said method comprises expanding the cells from the first step in a second medium comprising interleukin-2 (IL-2), to produce activated NK cells. In certain specific embodiments of the method of treating an individual with multiple myeloma, said NK cell populations are produced by a three-stage method, as described herein.

In other specific embodiments of the method of treating an individual with multiple myeloma, said NK cells are produced by a method comprising: (a) seeding a population of hematopoietic stem or progenitor cells in a first medium comprising interleukin-15 (IL-15) and, optionally, one or more of stem cell factor (SCF) and interleukin-7 (IL-7), wherein said IL-15 and optional SCF and IL-7 are not comprised within an undefined component of said medium, such that the population expands, and a plurality of hematopoietic stem or progenitor cells within said population of hematopoietic stem or progenitor cells differentiate into NK cells during said expanding; and (b) expanding the cells from step (a) in a second medium comprising interleukin-2 (IL-2), to produce a population of activated NK cells.

In another aspect, provided herein is a method of treating an individual having acute myelogenous leukemia (AML), comprising administering to the individual expanded NK cells (optionally activated by pretreatment with IL2 and IL12 and IL18, IL12 and IL15, IL12 and IL18, IL2 and IL12 and IL15 and IL18, or IL2 and IL15 and IL18), wherein said NK cells are effective to treat AML in said individual. In a specific embodiment, said NK cells are cord blood NK cells, or NK cells produced from cord blood hematopoietic cells, *e.g.,* hematopoietic stem cells. In another embodiment, said NK cells have been produced by any of the methods described herein for producing NK cells, *e.g.,* for producing TSNK cells or for producing NK cell populations using a three-stage method as set forth herein. In another embodiment, said NK cells have been expanded prior to said administering. In certain specific embodiments of the method of treating an individual with AML, said NK cells are produced by a two-step method of producing a population of activated natural killer (NK) cells, wherein a first step of said method comprises expanding a population of hematopoietic stem or progenitor cells in a first medium comprising one or more of stem cell factor (SCF), interleukin-7 (IL-7) and interleukin-15 (IL-15), and wherein said SCF, IL-7 and IL-15 are not comprised within an undefined component of said medium, and wherein a plurality of hematopoietic stem or progenitor cells within said population of hematopoietic stem or progenitor cells differentiate into NK cells during said expanding; and wherein a second step of said method comprises expanding the cells from the first step in a second medium comprising interleukin-2 (IL-2), to produce activated NK cells. In certain specific embodiments of the method of treating an individual with AML, said NK cell populations are produced by a three-stage method, as described herein. In a particular embodiment, the AML to be treated by the foregoing methods comprises refractory AML, poor-prognosis AML, or childhood AML.

In other specific embodiments of the method of treating an individual with AML, said NK cells are produced by a method comprising: (a) seeding a population of hematopoietic stem or progenitor cells in a first medium comprising interleukin-15 (IL-15) and, optionally, one or more of stem cell factor (SCF) and interleukin-7 (IL-7), wherein said IL-15 and optional SCF and IL-7 are not comprised within an undefined component of said medium, such that the population expands, and a plurality of hematopoietic stem or progenitor cells within said population of hematopoietic stem or progenitor cells differentiate into NK cells during said expanding; and (b) expanding the cells from step (a) in a second medium comprising interleukin-2 (IL-2), to produce a population of activated NK cells.

In another aspect, provided herein is a method of treating an individual having chronic lymphocytic leukemia (CLL), comprising administering to the individual a therapeutically effective dose of (1) lenalidomide; (2) melphalan; (3) fludarabine; and (4) expanded NK cells, *e.g.,* TSNK cells or a NK cell population produced using a three-stage method described herein, wherein said NK cells are effective to treat said CLL in said individual. In a specific embodiment, said NK cells are cord blood NK cells, or NK cells produced from cord blood hematopoietic cells, *e.g.,* hematopoietic stem cells. In another embodiment, said NK cells have been produced by any of the methods described herein for producing NK cells, *e.g.,* for producing TSNK cells or for producing NK cell populations using a three-stage method described herein. In a specific embodiment of any of the above methods, said NK cells have been expanded for at least 10 days prior to said administering. In a specific embodiment of any of the above methods, said lenalidomide, melphalan, fludarabine, and expanded NK cells are administered to said individual separately. In certain specific embodiments of the method of treating an individual with CLL, said NK cells are produced by a two-step method of producing a population of activated natural killer (NK) cells, wherein a first step of said method comprises expanding a population of hematopoietic stem or progenitor cells in a first medium comprising one or more of stem cell factor (SCF), interleukin-7 (IL-7) and interleukin-15 (IL-15), and wherein said SCF, IL-7 and IL-15 are not comprised within an undefined component of said medium, and wherein a plurality of hematopoietic stem or progenitor cells within said population of hematopoietic stem or progenitor cells differentiate into NK cells during said expanding; and wherein a second step of said method comprises expanding the cells from the first step in a second medium comprising interleukin-2 (IL-2), to produce activated NK cells. In certain specific embodiments of the method of treating an individual with CLL, said NK cell populations are produced by a three-stage method, as described herein.

In other specific embodiments of the method of treating an individual with CLL, said NK cells are produced by a method comprising: (a) seeding a population of hematopoietic stem or progenitor cells in a first medium comprising interleukin-15 (IL-15) and, optionally, one or more of stem cell factor (SCF) and interleukin-7 (IL-7), wherein said IL-15 and optional SCF and IL-7 are not comprised within an undefined component of said medium, such that the population expands, and a plurality of hematopoietic stem or progenitor cells within said population of hematopoietic stem or progenitor cells differentiate into NK cells during said expanding; and (b) expanding the cells from step (a) in a second medium comprising interleukin-2 (IL-2), to produce a population of activated NK cells.

In one aspect, provided herein is a method of cryopreserving a population of NK cells, *e.g.,* TSNK cells. In one embodiment, said method comprises: (a) seeding a population of hematopoietic stem or progenitor cells in a first medium comprising interleukin-15 (IL-15) and, optionally, one or more of stem cell factor (SCF) and interleukin-7 (IL-7), wherein said IL-15 and optional SCF and IL-7 are not comprised within an undefined component of said medium, such that the population expands, and a plurality of hematopoietic stem or progenitor cells within said population of hematopoietic stem or progenitor cells differentiate into NK cells during said expanding; (b) expanding the cells from step (a) in a second medium comprising interleukin-2 (IL-2), to produce a population of activated NK cells, and (c) cryopreserving the NK cells from step (b) in a cryopreservation medium. In a specific embodiment, said step (c) further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain embodiments, the method includes no intermediary steps between step (a) and (b), and between step (b) and (c).

In another embodiment, said method of cryopreserving a population of NK cells, e.g., TSNK cells, comprises: (a) expanding a population of hematopoietic stem or progenitor cells in a first medium comprising one or more of stem cell factor (SCF), IL-2, interleukin-7 (IL-7), interleukin-15 (IL-15) and heparin, and wherein said SCF, IL-2, IL-7 and IL-15 are not comprised within an undefined component of said medium, and wherein a plurality of hematopoietic stem or progenitor cells within said population of hematopoietic stem or progenitor cells differentiate into NK cells during said expanding; (b) expanding the cells from step (a) in a second medium comprising interleukin-2 (IL-2), to produce activated NK cells; and (c) cryopreserving the NK cells from step (b) in a cryopreservation medium. In a specific embodiment, said step (c) further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain embodiments, the method includes no intermediary steps between step (a) and (b), and between step (b) and (c), and/or no additional culturing steps prior to step (a).

In certain embodiments, the NK progenitor cell populations and/or NK cell populations produced using a three-stage method described herein are cryopreserved, e.g., cryopreserved using a method described herein. In a certain embodiments, the NK progenitor cell populations and/or NK cell populations produced using a three-stage method described herein are cryopreserved in a cryopreservation medium, e.g., a cryopreservation medium described herein. In a specific embodiment, cryopreservation of the NK progenitor cell populations and/or NK cell populations produced using a three-stage method described herein comprises (1) preparing a cell suspension solution comprising an NK progenitor cell population and/or an NK cell population produced using a three-stage method described herein; (2) adding cryopreservation medium to the cell suspension solution from step (1) to obtain a cryopreserved cell suspension; (3) cooling the cryopreserved cell suspension from step (3) to obtain a cryopreserved sample; and (4) storing the cryopreserved sample below -80 °C.

In another specific embodiment, the hematopoietic cells, e.g., hematopoietic stem or progenitor cells from which the TSNK cells are produced express one or more of the microRNAs hsa-miR-380, hsa-miR-512, hsa-miR-517, hsa-miR-518c, hsa-miR-519b, hsa-miR-520a, hsa-miR-337, hsa-miR-422a, hsa-miR-549, and hsa-miR-618 at a detectably higher level than peripheral blood natural killer cells, as determined, *e.g.,* by quantitative real-time PCR (qRT-PCR). In another specific embodiment, the hematopoietic cells, *e.g.,* hematopoietic stem or progenitor cells from which the NK progenitor cell populations and/or NK cell populations produced using a three-stage method described herein are produced express one or more of the microRNAs hsa-miR-380, hsa-miR-512, hsa-miR-517, hsa-miR-518c, hsa-miR-519b, hsa-miR-520a, hsa-miR-337, hsa-miR-422a, hsa-miR-549, and hsa-miR-618 at a detectably higher level than peripheral blood natural killer cells, as determined, *e.g.,* by quantitative real-time PCR (qRT-PCR).

In another specific embodiment, an immunomodulatory compound or thalidomide is brought into proximity with said TSNK cells in an amount and for a time sufficient for said natural killer cells to express detectably more granzyme B or perforin than an equivalent number of natural killer cells, *e.g.,* TSNK cells, not contacted or brought into proximity with said immunomodulatory compound or thalidomide. In another specific embodiment, an immunomodulatory compound, *e.g.,* lenalidomide or pomalidomide, or thalidomide is brought into proximity with said TSNK cells in an amount and for a time sufficient for said cells to exhibit detectably more cytotoxicity towards said tumor cells than an equivalent number of natural killer cells, *e.g.,* TSNK cells, not contacted or brought into proximity with said immunomodulatory compound, *e.g.,* lenalidomide or pomalidomide, or with thalidomide. In another specific embodiment, said TSNK cells express one or more of BAX, CCL5, CCR5, CSF2, FAS, GUSB, IL2RA, or TNFRSF18 at a higher level than an equivalent number of natural killer cells, *e.g.* TSNK cells, not contacted or brought into proximity with said immunomodulatory compound or thalidomide. In another specific embodiment, said TSNK cells express one or more of ACTB, BAX, CCL2, CCL3, CCL5, CCR5, CSF1, CSF2, ECE1, FAS, GNLY, GUSB, GZMB, ILIA, IL2RA, IL8, IL10, LTA, PRF1, PTGS2, SKI, and/or TBX21 at a higher level than an equivalent number of natural killer cells, *e.g.,* TSNK cells, not contacted or brought into proximity with said immunomodulatory compound or thalidomide.

In another specific embodiment, an immunomodulatory compound or thalidomide is brought into proximity with NK progenitor cells or NK cells produced using a three-stage method described herein in an amount and for a time sufficient for said natural killer cells to express detectably more granzyme B or perforin than an equivalent number of natural killer cells, *e.g.,* NK progenitor cells or NK cells, not contacted or brought into proximity with said immunomodulatory compound or thalidomide. In another specific embodiment, an immunomodulatory compound, *e.g.,* lenalidomide or pomalidomide, or thalidomide is brought into proximity with said NK progenitor cells or NK cells in an amount and for a time sufficient for said cells to exhibit detectably more cytotoxicity towards said tumor cells than an equivalent number of natural killer cells, *e.g.,* NK progenitor cells or NK cells, not contacted or brought into proximity with said immunomodulatory compound, *e.g.,* lenalidomide or pomalidomide, or with thalidomide. In another specific embodiment, said NK progenitor cells or NK cells produced using a three-stage method described herein express one or more of BAX, CCL5, CCR5, CSF2, FAS, GUSB, IL2RA, or TNFRSF18 at a higher level than an equivalent number of natural killer cells, *e.g.* NK progenitor cells or NK cells, not contacted or brought into proximity with said immunomodulatory compound or thalidomide. In another specific embodiment, said NK progenitor cells or NK cells express one or more of ACTB, BAX, CCL2, CCL3, CCL5, CCR5, CSF1, CSF2, ECE1, FAS, GNLY, GUSB, GZMB, ILIA, IL2RA, IL8, IL10, LTA, PRF1, PTGS2, SKI, and/or TBX21 at a higher level than an equivalent number of natural killer cells, *e.g.,* NK progenitor cells or NK cells, not contacted or brought into proximity with said immunomodulatory compound or thalidomide.

In certain embodiments of the methods of treatment or tumor suppression above, TSNK cells are combined with other natural killer cells, e.g., natural killer cells isolated from placental perfusate, umbilical cord blood or peripheral blood, or produced from hematopoietic cells by a different method. In a specific embodiment, TSNK cells are combined with an NK progenitor cell population produced using a three-stage method described herein. In another specific embodiment, TSNK cells are combined with an NK cell population produced using a three-stage method described herein. In specific embodiments, the TSNK cells are combined with natural killer cells from another source, or made by a different method (e.g., NK progenitor cell populations and/or NK cell populations produced using a three-stage method described herein), in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In other embodiments of the methods of treatment or tumor suppression above, NK progenitor cell populations produced using a three-stage method described herein are combined with other natural killer cells, *e.g.,* natural killer cells isolated from placental perfusate, umbilical cord blood or peripheral blood, or produced from hematopoietic cells by a different method. In specific embodiments, the NK progenitor cells are combined with natural killer cells from another source, or made by a different method, in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In certain embodiments of the methods of treatment or tumor suppression above, NK cell populations produced using a three-stage method described herein are combined with other natural killer cells, *e.g.,* natural killer cells isolated from placental perfusate, umbilical cord blood or peripheral blood, or produced from hematopoietic cells by a different method. In specific embodiments, the NK cells are combined with natural killer cells from another source, or made by a different method, in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another aspect, provided herein is a composition comprising isolated TSNK cells. In a specific embodiment, said TSNK cells are produced from hematopoietic cells, *e.g.,* hematopoietic stem or progenitor cells isolated from placental perfusate, umbilical cord blood, and/or peripheral blood. In another specific embodiment, said TSNK cells comprise at least 50% of cells in the composition. In another specific embodiment, said TSNK cells comprise at least 80%, 85%, 90%. 95%, 98% or 99% of cells in the composition. In certain embodiments, greater than 90%, 92%, 94%, 96% or 98% of TSNK cells in said composition are CD56⁺ and CD16⁻. In other embodiments, at least 80%, 82%, 84%, 86%, 88% or 90% of TSNK cells in said composition are CD3⁻ and CD56⁺. In other embodiments, at least 50%, 52%, 54%, 56%, 58% or 60% of said cells are NKG2D⁺. In other embodiments, fewer than 10%, 9%, 8%, 7%, 6%, 5%, 4% or 3% of said cells are NKB1⁺. In certain other embodiments, fewer than 10%, 8%, 6%, 4% or 2% of said TSNK cells are NKAT2⁺. In certain other embodiments, fewer than 10%, 8%, 6%, 4% or 2% of said TSNK cells are CD56⁺ and CD16⁺. In more specific embodiments, at least 50%, 55%, 60%, 65% or 70% of said CD3⁻, CD56⁺ TSNK cells are NKp46⁺. In other more specific embodiments, at least 50%, 55%, 60%, 65%, 70%, 75%, 80% or 85% of said CD3⁻, CD56⁺ TSNK cells are CD117⁺. In other more specific embodiments, at least 20%, 25%, 30%, 35%, 40% or 45% of said CD3⁻, CD56⁺ TSNK cells are CD94⁺. In other more specific embodiments, at least 10%, 12%, 14%, 16%, 18% or 20% of said CD3⁻, CD56⁺ TSNK cells are CD226⁺. In more specific embodiments, at least 20%, 25%, 30%, 35% or 40% of said CD3⁻, CD56⁺ TSNK cells are CD7⁺. In more specific embodiments, at least 30%, 35%, 40%, 45%, 50%, 55% or 60% of said CD3⁻, CD56⁺ TSNK cells are CD5⁺.

In another specific embodiment, said isolated CD56⁺, CD16⁻ TSNK cells are from a single individual. In a more specific embodiment, said isolated CD56⁺, CD16⁻ natural killer cells (TSNK cells) comprise natural killer cells from at least two different individuals. In another specific embodiment, said TSNK cells are from a different individual than the individual for whom treatment with the TSNK cells is intended. In another specific embodiment, said TSNK cells have been contacted or brought into proximity with an immunomodulatory compound or thalidomide in an amount and for a time sufficient for said TSNK cells to express detectably more granzyme B or perforin than an equivalent number of natural killer cells, *i.e.* TSNK cells, not contacted or brought into proximity with said immunomodulatory compound or thalidomide. In another specific embodiment, said composition additionally comprises an immunomodulatory compound or thalidomide. In certain embodiments, the immunomodulatory compound is a compound described below, *e.g.,* an amino-substituted isoindoline compound.

In another specific embodiment, the composition additionally comprises one or more anticancer compounds, *e.g.,* one or more of the anticancer compounds described below.

In a more specific embodiment, the composition comprises TSNK cells and natural killer cells from another source, or made by another method. In a specific embodiment, said other source is placental blood and/or umbilical cord blood. In another specific embodiment, said other source is peripheral blood. In more specific embodiments, the TSNK cells are combined with natural killer cells from another source, or made by another method in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific embodiment, the composition comprises TSNK cells and either isolated placental perfusate or isolated placental perfusate cells. In a more specific embodiment, said placental perfusate is from the same individual as said TSNK cells. In another more specific embodiment, said placental perfusate comprises placental perfusate from a different individual than said TSNK cells. In another specific embodiment, all, or substantially all (*e.g.,* greater than 90%, 95%, 98% or 99%) of cells in said placental perfusate are fetal cells. In another specific embodiment, the placental perfusate or placental perfusate cells, comprise fetal and maternal cells. In a more specific embodiment, the fetal cells in said placental perfusate comprise less than about 90%, 80%, 70%, 60% or 50% of the cells in said perfusate. In another specific embodiment, said perfusate is obtained by passage of a 0.9% NaCl solution through the placental vasculature. In another specific embodiment, said perfusate comprises a culture medium. In another specific embodiment, said perfusate has been treated to remove erythrocytes. In another specific embodiment, said composition comprises an immunomodulatory compound, *e.g.,* an immunomodulatory compound described in Section 5.2.1.1 below, *e.g.,* an amino-substituted isoindoline compound. In another specific embodiment, the composition additionally comprises one or more anticancer compounds, *e.g.,* one or more of the anticancer compounds described below.

In another specific embodiment, the composition comprises TSNK cells and placental perfusate cells. In a more specific embodiment, said placental perfusate cells are from the same individual as said TSNK cells. In another more specific embodiment, said placental perfusate cells are from a different individual than said TSNK cells. In another specific embodiment, the composition comprises isolated placental perfusate and isolated placental perfusate cells, wherein said isolated perfusate and said isolated placental perfusate cells are from different individuals. In another more specific embodiment of any of the above embodiments comprising placental perfusate, said placental perfusate comprises placental perfusate from at least two individuals. In another more specific embodiment of any of the above embodiments comprising placental perfusate cells, said isolated placental perfusate cells are from at least two individuals. In another specific embodiment, said composition comprises an immunomodulatory compound. In another specific embodiment, the composition additionally comprises one or more anticancer compounds, *e.g.,* one or more of the anticancer compounds described below.

In another aspect, provided herein is a composition comprising isolated NK progenitor cells or NK cells produced by a three-stage method described herein. In a specific embodiment, said NK progenitor cells or NK cells are produced from hematopoietic cells, *e.g.,* hematopoietic stem or progenitor cells isolated from placental perfusate, umbilical cord blood, and/or peripheral blood. In another specific embodiment, said NK progenitor cells or NK cells comprise at least 50% of cells in the composition. In another specific embodiment, said NK progenitor cells or NK cells comprise at least 80%, 85%, 90%. 95%, 98% or 99% of cells in the composition. In certain embodiments, greater than 90%, 92%, 94%, 96% or 98% of NK progenitor cells or NK cells in said composition are CD56⁺ and CD16⁻. In other embodiments, at least 80%, 82%, 84%, 86%, 88% or 90% of NK progenitor cells or NK cells in said composition are CD3⁻ and CD56⁺. In other embodiments, at least 50%, 52%, 54%, 56%, 58% or 60% of said cells are NKG2D⁺. In other embodiments, fewer than 10%, 9%, 8%, 7%, 6%, 5%, 4% or 3% of said cells are NKB1⁺. In certain other embodiments, fewer than 10%, 8%, 6%, 4% or 2% of said NK progenitor cells or NK cells are NKAT2⁺. In certain other embodiments, fewer than 10%, 8%, 6%, 4% or 2% of said NK progenitor cells or NK cells are CD56⁺ and CD16⁺. In more specific embodiments, at least 50%, 55%, 60%, 65% or 70% of said CD3⁻, CD56⁺ NK progenitor cells or NK cells are NKp46⁺. In other more specific embodiments, at least 50%, 55%, 60%, 65%, 70%, 75%, 80% or 85% of said CD3⁻, CD56⁺ NK progenitor cells or NK cells are CD117⁺. In other more specific embodiments, at least 20%, 25%, 30%, 35%, 40% or 45% of said CD3⁻, CD56⁺ NK progenitor cells or NK cells are CD94⁺. In other more specific embodiments, at least 10%, 12%, 14%, 16%, 18% or 20% of said CD3⁻, CD56⁺ NK progenitor cells or NK cells are CD226⁺. In more specific embodiments, at least 20%, 25%, 30%, 35% or 40% of said CD3⁻, CD56⁺ NK progenitor cells or NK cells are CD7⁺. In more specific embodiments, at least 30%, 35%, 40%, 45%, 50%, 55% or 60% of said CD3⁻, CD56⁺ NK progenitor cells or NK cells are CD5⁺.

In a specific embodiment, said isolated CD56⁺, CD16⁻ NK progenitor cells or NK cells are from a single individual. In a more specific embodiment, said isolated CD56⁺, CD16⁻ natural killer cells (NK progenitor cells or NK cells) comprise natural killer cells from at least two different individuals. In another specific embodiment, said NK progenitor cells or NK cells are from a different individual than the individual for whom treatment with the NK progenitor cells or NK cells is intended. In another specific embodiment, said NK progenitor cells or NK cells have been contacted or brought into proximity with an immunomodulatory compound or thalidomide in an amount and for a time sufficient for said NK progenitor cells or NK cells to express detectably more granzyme B or perforin than an equivalent number of natural killer cells, *i.e.* NK progenitor cells or NK cells, not contacted or brought into proximity with said immunomodulatory compound or thalidomide. In another specific embodiment, said composition additionally comprises an immunomodulatory compound or thalidomide. In certain embodiments, the immunomodulatory compound is a compound described below, *e.g.,* an amino-substituted isoindoline compound.

In another specific embodiment, the composition additionally comprises one or more anticancer compounds, *e.g.,* one or more of the anticancer compounds described below.

In a more specific embodiment, the composition comprises NK progenitor cells or NK cells produced by a three-stage method described herein and natural killer cells from another source, or made by another method. In a specific embodiment, said other source is placental blood and/or umbilical cord blood. In another specific embodiment, said other source is peripheral blood. In more specific embodiments, the NK progenitor cells or NK cells are combined with natural killer cells from another source, or made by another method in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific embodiment, the composition comprises NK progenitor cells or NK cells produced using a three-stage method described herein and either isolated placental perfusate or isolated placental perfusate cells. In a more specific embodiment, said placental perfusate is from the same individual as said NK progenitor cells or NK cells. In another more specific embodiment, said placental perfusate comprises placental perfusate from a different individual than said NK progenitor cells or NK cells. In another specific embodiment, all, or substantially all (*e.g.,* greater than 90%, 95%, 98% or 99%) of cells in said placental perfusate are fetal cells. In another specific embodiment, the placental perfusate or placental perfusate cells, comprise fetal and maternal cells. In a more specific embodiment, the fetal cells in said placental perfusate comprise less than about 90%, 80%, 70%, 60% or 50% of the cells in said perfusate. In another specific embodiment, said perfusate is obtained by passage of a 0.9% NaCl solution through the placental vasculature. In another specific embodiment, said perfusate comprises a culture medium. In another specific embodiment, said perfusate has been treated to remove erythrocytes. In another specific embodiment, said composition comprises an immunomodulatory compound, *e.g.,* an immunomodulatory compound described in Section 5.2.1.1 below, *e.g.,* an amino-substituted isoindoline compound. In another specific embodiment, the composition additionally comprises one or more anticancer compounds, e.g., one or more of the anticancer compounds described below.

In another specific embodiment, the composition comprises NK progenitor cells or NK cells produced using a three-stage method described herein and placental perfusate cells. In a more specific embodiment, said placental perfusate cells are from the same individual as said NK progenitor cells or NK cells. In another more specific embodiment, said placental perfusate cells are from a different individual than said NK progenitor cells or NK cells. In another specific embodiment, the composition comprises isolated placental perfusate and isolated placental perfusate cells, wherein said isolated perfusate and said isolated placental perfusate cells are from different individuals. In another more specific embodiment of any of the above embodiments comprising placental perfusate, said placental perfusate comprises placental perfusate from at least two individuals. In another more specific embodiment of any of the above embodiments comprising placental perfusate cells, said isolated placental perfusate cells are from at least two individuals. In another specific embodiment, said composition comprises an immunomodulatory compound. In another specific embodiment, the composition additionally comprises one or more anticancer compounds, *e.g.,* one or more of the anticancer compounds described below.

In another aspect, provided herein is a composition, e.g., a pharmaceutical composition, comprising an isolated NK progenitor cell population, *e.g.,* produced by the three-stage method described herein. In a specific embodiment, said isolated NK progenitor cell population is produced from hematopoietic cells, *e.g.,* hematopoietic stem or progenitor cells isolated from placental perfusate, umbilical cord blood, and/or peripheral blood. In another specific embodiment, said isolated NK progenitor cell population comprises at least 50% of cells in the composition. In another specific embodiment, said isolated NK progenitor cell population comprises at least 80%, 85%, 90%. 95%, 98% or 99% of cells in the composition. In certain embodiments, no more than 5%, 10%, 15%, 20%, 25%, 30%, 35%, or 40% of the cells in said isolated NK progenitor cell population are CD3⁻CD56⁺ cells. In certain embodiments, no less than 65%, 70%, 75%, 80%, 85%, 90%, 95%, 98%, or 99% of said CD3⁻CD56⁺ cells are CD117⁺. In certain embodiments, no less than 40%, 45%, 50%, 55%, 60%, 65%, 70%, or 75% of said CD3⁻CD56⁺ cells are CD161⁺. In certain embodiments, no more than 25%, 30%, 35%, 40%, 45%, 50%, 55%, 60%, 65%, 70%, 75%, 80%, 85%, or 90% of said CD3⁻CD56⁺ cells are NKp46⁺. In more specific embodiments, no more than 25%, 30%, 35%, 40%, 45%, 50%, or 55% of said CD3⁻CD56⁺ cells are NKp46⁺. In certain embodiments, said CD3⁻CD56⁺ cells are CD16⁻.

In another specific embodiment, said isolated NK progenitor cells in said composition are from a single individual. In a more specific embodiment, said isolated NK progenitor cells comprise NK progenitor cells from at least two different individuals. In another specific embodiment, said isolated NK progenitor cells in said composition are from a different individual than the individual for whom treatment with the NK progenitor cells is intended. In another specific embodiment, said NK progenitor cells have been contacted or brought into proximity with an immunomodulatory compound or thalidomide in an amount and for a time sufficient for said NK progenitor cells to express detectably more granzyme B or perforin than an equivalent number of natural killer cells, *i.e.* NK progenitor cells not contacted or brought into proximity with said immunomodulatory compound or thalidomide. In another specific embodiment, said composition additionally comprises an immunomodulatory compound or thalidomide. In certain embodiments, the immunomodulatory compound is a compound described below, *e.g.,* an amino-substituted isoindoline compound.

In another specific embodiment, the composition additionally comprises one or more anticancer compounds, *e.g.,* one or more of the anticancer compounds described below.

In a more specific embodiment, the composition comprises NK progenitor cells from another source, or made by another method. In a specific embodiment, said other source is placental blood and/or umbilical cord blood. In another specific embodiment, said other source is peripheral blood. In more specific embodiments, the NK progenitor cell population in said composition is combined with NK progenitor cells from another source, or made by another method in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific embodiment, the composition comprises an NK progenitor cell population and either isolated placental perfusate or isolated placental perfusate cells. In a more specific embodiment, said placental perfusate is from the same individual as said NK progenitor cell population. In another more specific embodiment, said placental perfusate comprises placental perfusate from a different individual than said NK progenitor cell population. In another specific embodiment, all, or substantially all (*e.g.,* greater than 90%, 95%, 98% or 99%) of cells in said placental perfusate are fetal cells. In another specific embodiment, the placental perfusate or placental perfusate cells, comprise fetal and maternal cells. In a more specific embodiment, the fetal cells in said placental perfusate comprise less than about 90%, 80%, 70%, 60% or 50% of the cells in said perfusate. In another specific embodiment, said perfusate is obtained by passage of a 0.9% NaCl solution through the placental vasculature. In another specific embodiment, said perfusate comprises a culture medium. In another specific embodiment, said perfusate has been treated to remove erythrocytes. In another specific embodiment, said composition comprises an immunomodulatory compound, e.g., an immunomodulatory compound described below, *e.g.,* an amino-substituted isoindoline compound. In another specific embodiment, the composition additionally comprises one or more anticancer compounds, *e.g.,* one or more of the anticancer compounds described below.

In another specific embodiment, the composition comprises an NK progenitor cell population and placental perfusate cells. In a more specific embodiment, said placental perfusate cells are from the same individual as said NK progenitor cell population. In another more specific embodiment, said placental perfusate cells are from a different individual than said NK progenitor cell population. In another specific embodiment, the composition comprises isolated placental perfusate and isolated placental perfusate cells, wherein said isolated perfusate and said isolated placental perfusate cells are from different individuals. In another more specific embodiment of any of the above embodiments comprising placental perfusate, said placental perfusate comprises placental perfusate from at least two individuals. In another more specific embodiment of any of the above embodiments comprising placental perfusate cells, said isolated placental perfusate cells are from at least two individuals. In another specific embodiment, said composition comprises an immunomodulatory compound. In another specific embodiment, the composition additionally comprises one or more anticancer compounds, *e.g.,* one or more of the anticancer compounds described below.

### 3.1. Definitions

As used herein, the terms "immunomodulatory compound" and "IMiD™" do not encompass thalidomide.

As used herein, "lenalidomide" means 3-(4'aminoisoindoline-1'-one)-1-piperidine-2,6-dione (Chemical Abstracts Service name) or 2,6-Piperidinedione,3-(4-amino-1,3-dihydro-1-oxo-2H-isoindol-2-yl)- (International Union of Pure and Applied Chemistry (IUPAC) name). As used herein, "pomalidomide" means 4-amino-2-(2,6-dioxopiperidin-3-yl)isoindole-1,3-dione.

As used herein, "multipotent," when referring to a cell, means that the cell has the capacity to differentiate into a cell of another cell type. In certain embodiments, "a multipotent cell" is a cell that has the capacity to grow into any subset of the mammalian body's approximately 260 cell types. Unlike a pluripotent cell, a multipotent cell does not have the capacity to form all of the cell types.

As used herein, "feeder cells" refers to cells of one type that are co-cultured with cells of a second type, to provide an environment in which the cells of the second type can be maintained, and perhaps proliferate. Without being bound by any theory, feeder cells can provide, for example, peptides, polypeptides, electrical signals, organic molecules (*e.g.,* steroids), nucleic acid molecules, growth factors (*e.g.,* bFGF), other factors (*e.g.,* cytokines), and metabolic nutrients to target cells. In certain embodiments, feeder cells grow in a mono-layer.

As used herein, "natural killer cell" or "NK cells" without further modification, includes natural killer cells from any tissue source. In one embodiment, the NK cells are characterized as an NK cell population comprising cells with the mature NK cell markers CD 16, low expression of CD56 ("CD56dim"), and KIRs.

As used herein, the term "NK progenitor cell population" refers to a population of cells comprising cells of the natural killer cell lineage that have yet to develop into mature NK cells, as indicated by, e.g., the level(s) of expression one or more phenotypic markers, e.g., CD56, CD16, and KIRs. In one embodiment, the NK progenitor cell population comprises cells with low CD16 and high CD56.

As used herein, "placental perfusate" means perfusion solution that has been passed through at least part of a placenta, *e.g.,* a human placenta, *e.g.,* through the placental vasculature, including a plurality of cells collected by the perfusion solution during passage through the placenta.

As used herein, "placental perfusate cells" means nucleated cells, *e.g.,* total nucleated cells, isolated from, or isolatable from, placental perfusate.

As used herein, "tumor cell suppression," "suppression of tumor cell proliferation," and the like, includes slowing the growth of a population of tumor cells, *e.g.,* by killing one or more of the tumor cells in said population of tumor cells, for example, by contacting or bringing, *e.g.,* TSNK cells or a population of cells comprising TSNK cells, an NK progenitor cell population, or an NK cell population produced using a three-stage method described herein into proximity with the population of tumor cells, *e.g.,* contacting the population of tumor cells with TSNK cells or a population of cells comprising TSNK cells, an NK progenitor cell population, or an NK cell population produced using a three-stage method described herein.

As used herein, the term "hematopoietic cells" includes hematopoietic stem cells and hematopoietic progenitor cells.

As used herein, the "undefined component" is a term of art in the culture medium field that refers to components whose constituents are not generally provided or quantified. Examples of an "undefined component" include, without limitation, human serum (*e.g.,* human serum AB) and fetal serum (*e.g.,* fetal bovine serum or fetal calf serum).

As used herein, "+", when used to indicate the presence of a particular cellular marker, means that the cellular marker is detectably present in fluorescence activated cell sorting over an isotype control; or is detectable above background in quantitative or semi-quantitative RT-PCR.

As used herein, "-", when used to indicate the presence of a particular cellular marker, means that the cellular marker is not detectably present in fluorescence activated cell sorting over an isotype control; or is not detectable above background in quantitative or semi-quantitative RT-PCR.

### 4. BRIEF DESCRIPTION OF THE FIGURES

FIG. 1: Fold expansion of NK cells differentiated from hematopoietic stem cells (HSCs) with various medium formulations. Error bars represent standard derivation from three donors. X axis: day (D) of culture. Y axis: fold-expansion compared to day 0 (start of culture).
FIG. 2: Phenotypic characterization of cultivated NK cells with NK2A medium. Cells were triple-labeled with PE-antiCD56, FITC-antiCD3, PerCP-antiCD16. Horizontal, vertical lines: level of fluorescent label significantly above background.
FIG. 3: Fold expansion of NK cells cultivated with NK2A (FF), NK2A (placental stem cells as feeder cells), NK2A (MSC as feeder cells) or Two-stage NK medium. X axis: day (D) of culture. Y axis: fold-expansion compared to day 0 (start of culture).

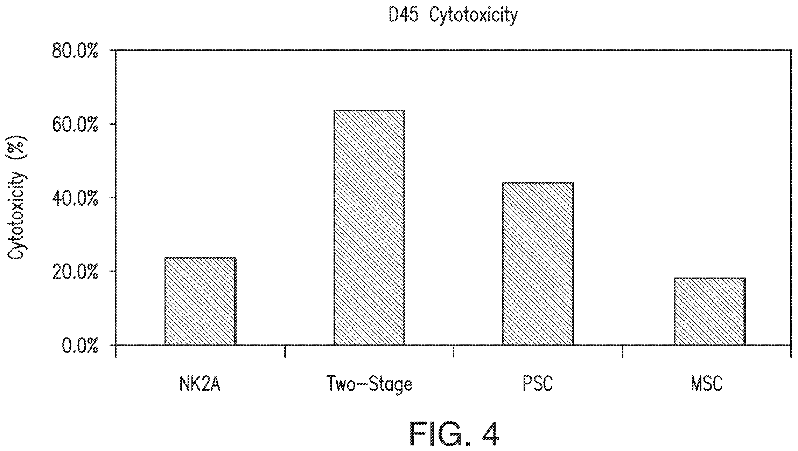

FIG. 4: Cytotoxicity of NK cells cultivated with NK2A (without feeder cells), Two-stage NK medium; NK2A with CD34⁻, CD10⁺, CD105⁺, CD200⁺ tissue culture plastic-adherent placental stem cells (PSC) as feeder cells, NK2A with bone marrow-derived mesenchymal stem cells (MSC) as feeder cells, at Day 45 post-culture initiation. Representative data from three donors are shown in FIG 4.

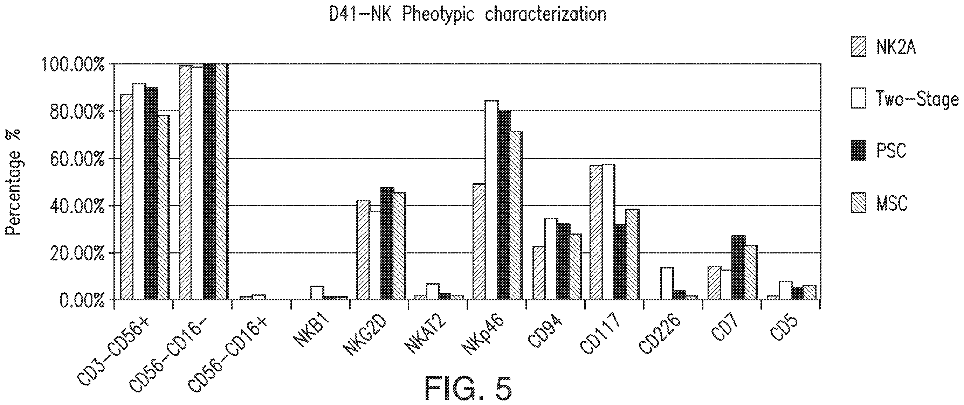

FIG. 5: Phenotypic characterization of NK cells on Day 41 (D41) of culture. Representative data from 3 individual donors are shown. X axis: percentage of NK cells, produced by the two-stage method, that are CD3⁻CD56⁺, CD16⁻CD56⁺, or CD16⁺CD56⁺; or that express NKB1, NKG2D, NKp46, CD94, CD117, CD226, CD7 or CD5. Cells were cultured in NK2A (without feeder cells), Two-stage NK medium; NK2A with CD34⁻, CD10⁺, CD105⁺, CD200⁺ tissue culture plastic-adherent placental stem cells (PSC) as feeder cells, NK2A with bone marrow-derived mesenchymal stem cells (MSC) as feeder cells, at Day 41 post-culture initiation.

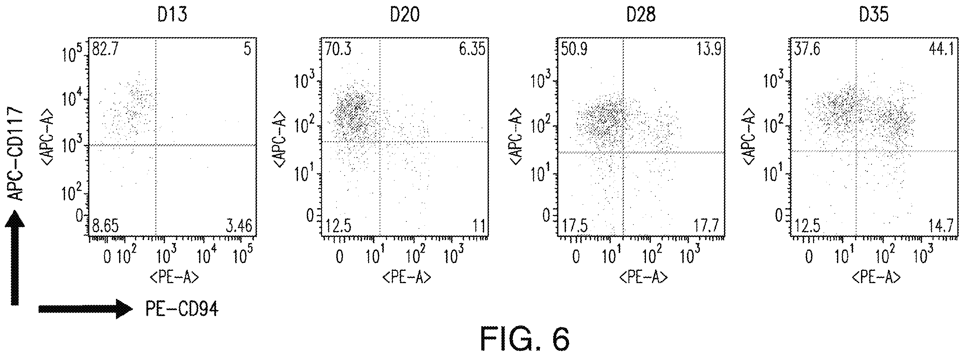

FIG. 6: Expression of CD94 and CD117 in the CD56⁺CD3⁻ NK cell population during NK cultivation in NK2A medium. The dominant population of CD56⁺CD94⁺CD117⁺ cells was identified from cultured NK cells in NK2A medium, which is distinguishable from embryonic stem cell (ESC)-derived NK cells (CD56⁺CD94⁺CD117^{low/-}). Representative data from three donors are shown in FIG 6. X axis: fluorescence from phycoerythrin (PE)-labeled anti-CD94. Y-axis: fluorescence from APC-labeled antiCD117. Horizontal, vertical lines: level of fluorescent label significantly above background. D13, D20, D28, D35: Days 13, 20, 28 and 35 post CD34⁺ cell culture initiation.

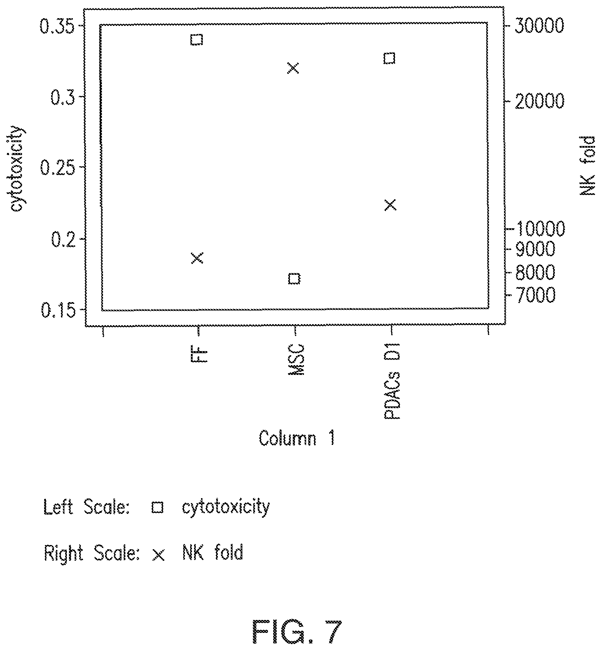

FIG. 7: Effects of placental stem cells on cultured NK cells in comparison with MSC and NK2A medium alone. X axis: NK cells cultured in NK2A medium without a feeder layer (FF); NK cells cultured in NK2A medium with bone marrow-derived mesenchymal stem cells (MCS) as a feeder layer; or NK2A medium with CD10⁺, CD34⁻, CD105⁺, CD200⁺ tissue culture plastic-adherent placental stem cells (PDACs) as a feeder layer. Y axis (left): cytotoxicity, expressed as a percentage of tumor cells remaining (1.0 = 100%); cytotoxicity indicated by open squares. Y axis (right): fold expansion of NK cells using the two-step method; fold expansion expressed as asterisks.
FIGS. 8A-8B: Effects of relative ratios of umbilical cord blood (UCB) and human placental perfusate (HPP) on the purity of Post-thaw CD34⁺ cells. FIG 8A: Effects of HPP volumetric content (vol %) on CD34⁺Lin⁻ purity. X axis: volumetric fraction of HPP in the pooled UCB and HPP (Combo). Y axis: the percentage of CD34⁺Lin⁻ cells. FIG. 8B. Effects of HPP TNC content (TNC%) on CD34⁺Lin⁻ purity. Y axis: the percentage of CD34⁺Lin⁻ cells.
FIG. 9: FACS-based cytotoxicity assay of day 35 cultured (D35) NK cells against various tumor cell lines labeled with PKH26-TOPRO3. Error bars represent the standard deviation among experiments conducted in triplicate.
FIG. 10: LDH release assay to assess D35 NK cell cytotoxicity against HCT-116 cells. Effector (NK cells) to tumor (HCT-116 cells) cell ratios are shown on the X-axis.
FIG. 11: D35 NK cells in vivo cytotoxicity in a K562 tumor xenograft model.
FIG. 12: D35 NK cells in vivo cytotoxicity in a HCT-116 tumor xenograft model.
FIG. 13: D35 NK cells in vivo cytotoxicity in a U-87MG tumor xenograft model.
FIGS. 14A-14B: D35 NK cells in vivo cytotoxicity in a BT474 tumor xenograft model in the presence of IL-2 (A) or IL-15 or in the absence of cytokine (B).
FIGS. 15A-15C: Hematoxylin and eosin (H&E; A, left panel) and immunohistochemistry (B, C) staining of series sections of BT474 tumor xenograft recovered at endpoint of *in vivo* study depicted in Figure 14B. In B and C, human nuclei are depicted, and the arrows point to cell stained with CD56 antibody (B, middle panel) and NKp46 antibody (C, right panel).
FIGS. 16A-16B: Immunofluorescence analysis of K562 cells (A, top panel) and D35 NK cells (B, bottom panel). Nuclei are stained in all images; images on left = anti-CD56; images at center = anti-NKG2D; images at right = anti-NKp46. Bars = 50 µm.
FIGS. 17A-17F: Immunofluorescence analysis of human tumor xenografts from mice. A-D: K562 xenografts imaged following administration of vehicle (A); D35 NK cells administered intravenously (I.V.) (B); vehicle + IL-2 I.V. (C); and D35 NK cells + IL-2 I.V. (D). E and F: BT474 xenografts imaged following administration of vehicle + IL-2 I.V. (E); and D35 NK cells + IL-2 I.V (F). Nuclei are stained in all images; images on left = anti-CD56; images at center = anti-NKG2d; images at right = anti-NKp46. Bars = 50 µm.
FIGS. 18A-18D: Biodistribution and persistence of Two-Stage D35 NK cells *in vivo* in human hematopoietic cytokine (IL-2 and IL-15)-expressing NSGS mice. Shown are the percentages of human CD45 (as a marker for Two-Stage D35 NK cells) compared to mouse CD45 in IL-2 (A, C) and IL-15 (B, D) treated mice at different time points (d=days) following transfer. A, BS = analysis of blood; C, D = analysis of liver.
FIG 19: Two-Stage D35 NK cells cultured *in vitro* analyzed for the indicated markers by fluorescence-assisted cell sorting (FACS): Top = CD56+ vs. CD16+; Bottom left = CD56+ vs. KIR2DL2/DL3/DS2+; Bottom right = CD56+ vs. KIR3DL1/DS1+.
FIG 20: *In vivo* maturation of Two-Stage D35 NK cells in human hematopoietic cytokine (IL-15)-expressing NSGS mice detected on day 28 following *in vivo* adoptive transfer. Top left = mouse CD45 vs. human CD45. Right panels demonstrate further analysis of the human CD45 cell population: Top = CD56+ vs. CD16+; Bottom = KIR2DL2/DL3/DS2+ vs. KIR3DL1/DS1+.
FIG. 21: *In vitro* killing activity of the D35 NK cell population against genetically induced primary leukemic stem cells. Results of liquid co-culture assay of the D35 NK cell population cytotoxicity. MLL = engineered CD34+ cells expressing MLL-AF9; THP1 = human acute monocytic leukemia cell line; K562 = K562 tumor cell line. Values on y-axis are x 10⁶.
FIG. 22: Methylcellulose assay of cytotoxicity of the D35 NK cell population. Depicted is the killing of AML colony forming cells using *ex vivo* isolated cells of the D35 NK cell population from the biodistribution study shown in Figure 18. MLL = engineered CD34+ cells expressing MLL-AF9; K562 = K562 tumor cell line.

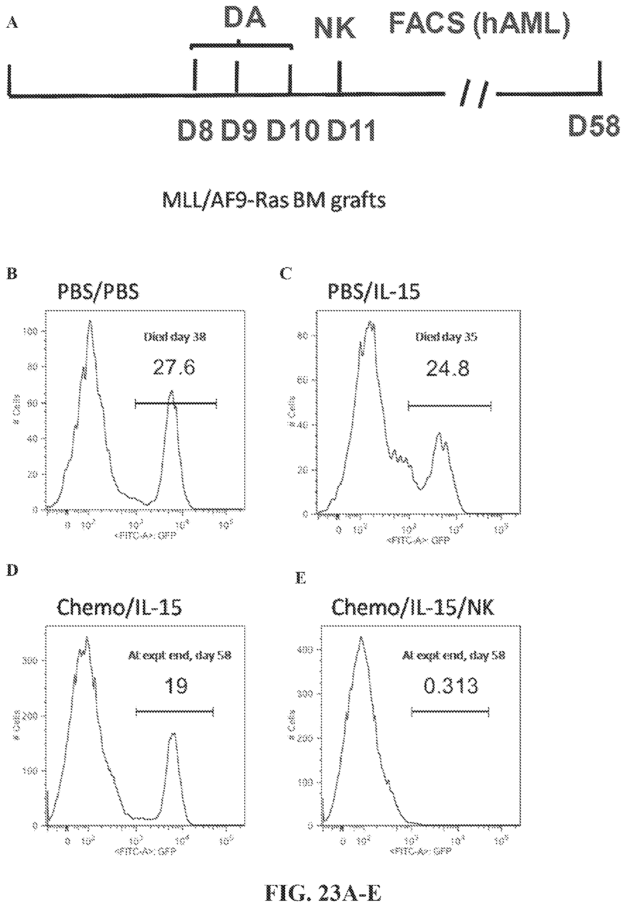

FIGS. 23A-23F: Frequency of leukemic cell detection over 8 weeks following MLL transplant. A. Schematic of treatments following transplant of MLL cells. DA = doxorubicin and docetaxel; D = timepoint of experiment in days (day 58 = endpoint, unless the mice died sooner, as indicated on the graphs for B and C); NK = D35 NK cell population. B. Graphs show numbers of fluorescent cells in control groups - mice administered PBS (B) or IL-15 (C) - and groups administered either IL-15 and chemotherapy alone (D) or the combination of the D35 NK cell population NK cells plus IL-15 and chemotherapy (E). (F) provides a summary of the results shown in parts C, D, and E. The amounts of AML cells remaining are shown on the Y-axes; values represent the percentage of AML cells in peripheral blood.
FIG. 24: Bone marrow engrafting human cell population generated from Day 21 (D21) NK progenitor cell population 4 weeks following *in vivo* adoptive transfer.
FIG. 25: *In vivo* cell cycle analysis of the bone marrow engrafting D21 NK progenitor cell population 4 weeks following *in vivo* adoptive transfer.
FIG. 26: *Ex vivo* differentiation of D21 NK progenitor cell population derived bone marrow engrafting CD56-CD16- progenitors.
FIG. 27: Cytotoxic activity of D21 NK progenitor cell population *ex vivo* differentiated into mature NK cells.
FIG. 28: Relative telomere length of D21 NK progenitor cells (NK-D21) and D35 NK cells (NK-D35) cells and other cells including peripheral blood (PB) NK cells, as compared to HL60 cells.

### 5. DETAILED DESCRIPTION

Provided herein are novel methods of producing and expanding NK cells from hematopoietic cells, e.g., hematopoietic stem cells or progenitor cells. Also provided herein are methods, e.g., three-stage methods, of producing NK progenitor cell populations and NK cell populations from hematopoietic cells, e.g., hematopoietic stem cells or progenitor cells. The hematopoietic cells used to produce the NK cells, and NK cell populations, as well as the NK progenitor cell populations may be isolated from any source, for example, without limitation, placenta, umbilical cord blood, placental blood, peripheral blood, spleen or liver. In certain embodiments, the NK cells, NK cell populations, or NK progenitor cell populations are produced from expanded hematopoietic cells, *e.g.,* hematopoietic stem cells and/or hematopoietic progenitor cells. In one embodiment, hematopoietic cells are collected from a source of such cells, *e.g.,* placental perfusate, umbilical cord blood, placental blood, peripheral blood, spleen, liver and/or bone marrow. In a specific embodiment, the hematopoietic cells are expanded and differentiated, continuously, in a first medium without the use of feeder cells. The cells are then cultured in a second medium in the presence of feeder cells. In an alternative embodiment, the culture is in the second medium in the absence of feeder cells. Such isolation, expansion and differentiation can be performed in a central facility, which provides expanded hematopoietic cells for shipment to decentralized expansion and differentiation at points of use, e.g., hospital, military base, military front line, or the like.

### 5.1. Hematopoietic Cells

Hematopoietic cells useful in the methods disclosed herein can be any hematopoietic cells able to differentiate into NK cells and/or NK progenitor cells, e.g., precursor cells, hematopoietic progenitor cells, hematopoietic stem cells, or the like. Hematopoietic cells can be obtained from tissue sources such as, *e.g.,* bone marrow, cord blood, placental blood, peripheral blood, liver or the like, or combinations thereof. Hematopoietic cells can be obtained from placenta. In a specific embodiment, the hematopoietic cells are obtained from placental perfusate. In one embodiment, the hematopoietic cells are not obtained from umbilical cord blood. In one embodiment, the hematopoietic cells are not obtained from peripheral blood. Hematopoietic cells from placental perfusate can comprise a mixture of fetal and maternal hematopoietic cells, *e.g.,* a mixture in which maternal cells comprise greater than 5% of the total number of hematopoietic cells. Preferably, hematopoietic cells from placental perfusate comprise at least about 90%, 95%, 98%, 99% or 99.5% fetal cells.

In another specific embodiment, the hematopoietic cells, *e.g.,* hematopoietic stem cells or progenitor cells, from which the TSNK cells are produced, or from which the NK progenitor cell populations or NK cell populations produced using a three-stage method described herein are produced, are obtained from placental perfusate, umbilical cord blood or peripheral blood. In another specific embodiment, the hematopoietic cells, *e.g.,* hematopoietic stem cells or progenitor cells, from which the TSNK cells are produced, or from which the NK progenitor cell populations or NK cell populations produced using a three-stage method described herein are produced, are combined cells from placental perfusate and cord blood, *e.g.,* cord blood from the same placenta as the perfusate. In another specific embodiment, said umbilical cord blood is isolated from a placenta other than the placenta from which said placental perfusate is obtained. In certain embodiments, the combined cells can be obtained by pooling or combining the cord blood and placental perfusate. In certain embodiments, the cord blood and placental perfusate are combined at a ratio of 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like by volume to obtain the combined cells. In a specific embodiment, the cord blood and placental perfusate are combined at a ratio of from 10:1 to 1:10, from 5:1 to 1:5, or from 3:1 to 1:3. In another specific embodiment, the cord blood and placental perfusate are combined at a ratio of 10:1, 5:1, 3:1, 1:1, 1:3, 1:5 or 1:10. In a more specific embodiment, the cord blood and placental perfusate are combined at a ratio of 8.5:1.5 (85%:15%).

In certain embodiments, the cord blood and placental perfusate are combined at a ratio of 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like by total nucleated cells (TNC) content to obtain the combined cells. In a specific embodiment, the cord blood and placental perfusate are combined at a ratio of from 10:1 to 10:1, from 5:1 to 1:5, or from 3:1 to 1: 3. In another specific embodiment, the cord blood and placental perfusate are combined at a ratio of 10:1, 5:1, 3:1, 1:1, 1:3, 1:5 or 1:10.

In another specific embodiment, the hematopoietic cells, *e.g.,* hematopoietic stem cells or progenitor cells from which said TSNK cells are produced, or from which the NK progenitor cell populations or NK cell populations produced using a three-stage method described herein are produced, are from both umbilical cord blood and placental perfusate, but wherein said umbilical cord blood is isolated from a placenta other than the placenta from which said placental perfusate is obtained.

In certain embodiments, the hematopoietic cells are CD34⁺ cells. In specific embodiments, the hematopoietic cells useful in the methods disclosed herein are CD34⁺CD38⁺ or CD34⁺CD38⁻. In a more specific embodiment, the hematopoietic cells are CD34⁺CD38⁻Lin⁻. In another specific embodiment, the hematopoietic cells are one or more of CD2⁻, CD3⁻, CD11b⁻, CD11c⁻, CD14⁻, CD16⁻, CD19⁻, CD24⁻, CD56⁻, CD66b⁻ and/or glycophorin A⁻. In another specific embodiment, the hematopoietic cells are CD2⁻, CD3⁻, CD11b⁻, CD11c⁻, CD14⁻, CD16⁻, CD19⁻, CD24⁻, CD56⁻, CD66b⁻ and glycophorin A⁻. In another more specific embodiment, the hematopoietic cells are CD34⁺CD38⁻CD33⁻CD117⁻. In another more specific embodiment, the hematopoietic cells are CD34⁺CD38⁻CD33⁻CD117⁻CD235⁻CD36⁻.

In another embodiment, the hematopoietic cells are CD45⁺. In another specific embodiment, the hematopoietic cells are CD34⁺CD45⁺. In another embodiment, the hematopoietic cell is Thy-1⁺. In a specific embodiment, the hematopoietic cell is CD34⁺Thy-1⁺. In another embodiment, the hematopoietic cells are CD133⁺. In specific embodiments, the hematopoietic cells are CD34+CD133⁺ or CD133Thy-1⁺. In another specific embodiment, the CD34⁺ hematopoietic cells are CXCR4⁺. In another specific embodiment, the CD34⁺ hematopoietic cells are CXCR4⁻. In another embodiment, the hematopoietic cells are positive for KDR (vascular growth factor receptor 2). In specific embodiments, the hematopoietic cells are CD34⁺KDR⁺, CD133+KDR⁺ or Thy-1⁺KDR⁺. In certain other embodiments, the hematopoietic cells are positive for aldehyde dehydrogenase (ALDH+), *e.g.,* the cells are CD34⁺ ALDH⁺.

In certain other embodiments, the CD34⁺ cells are CD45⁻. In specific embodiments, the CD34⁺ cells, *e.g.,* CD34⁺, CD45⁻ cells express one or more, or all, of the miRNAs hsa-miR-380, hsa-miR-512, hsa-miR-517, hsa-miR-518c, hsa-miR-519b, hsa-miR-520a, hsa-miR-337, hsa-miR-422a, hsa-miR-549, and/or hsa-miR-618.

In certain embodiments, the hematopoietic cells are CD34⁻.

The hematopoietic cells can also lack certain markers that indicate lineage commitment, or a lack of developmental naiveté. For example, in another embodiment, the hematopoietic cells are HLA-DR . In specific embodiments, the hematopoietic cells are CD34⁺HLA-DR⁻, CD133⁺HLA-DR⁻, Thy-1⁺HLA-DR⁻ or ALDH⁺HLA-DR⁻ In another embodiment, the hematopoietic cells are negative for one or more, preferably all, of lineage markers CD2, CD3, CD11b, CD11c, CD14, CD16, CD19, CD24, CD56, CD66b and glycophorin A.

Thus, hematopoietic cells can be selected for use in the methods disclosed herein on the basis of the presence of markers that indicate an undifferentiated state, or on the basis of the absence of lineage markers indicating that at least some lineage differentiation has taken place. Methods of isolating cells, including hematopoietic cells, on the basis of the presence or absence of specific markers is discussed in detail below.

Hematopoietic cells used in the methods provided herein can be a substantially homogeneous population, *e.g.,* a population comprising at least about 95%, at least about 98% or at least about 99% hematopoietic cells from a single tissue source, or a population comprising hematopoietic cells exhibiting the same hematopoietic cell-associated cellular markers. For example, in various embodiments, the hematopoietic cells can comprise at least about 95%, 98% or 99% hematopoietic cells from bone marrow, cord blood, placental blood, peripheral blood, or placenta, *e.g.,* placenta perfusate.

Hematopoietic cells used in the methods provided herein can be obtained from a single individual, *e.g.,* from a single placenta, or from a plurality of individuals, *e.g.,* can be pooled. Where the hematopoietic cells are obtained from a plurality of individuals and pooled, the hematopoietic cells may be obtained from the same tissue source. Thus, in various embodiments, the pooled hematopoietic cells are all from placenta, *e.g.,* placental perfusate, all from placental blood, all from umbilical cord blood, all from peripheral blood, and the like.

Hematopoietic cells used in the methods disclosed herein can, in certain embodiments, comprise hematopoietic cells from two or more tissue sources. For example, in certain embodiments, when hematopoietic cells from two or more sources are combined for use in the methods herein, a plurality of the hematopoietic cells used to produce TSNK cells comprise hematopoietic cells from placenta, *e.g.,* placenta perfusate. In various embodiments, the hematopoietic cells used to produce TSNK cells, or NK progenitor cell populations or NK cell populations produced using a three-stage method described herein, comprise hematopoietic cells from placenta and from cord blood; from placenta and peripheral blood; from placenta and placental blood, or placenta and bone marrow. In a preferred embodiment, the hematopoietic cells comprise hematopoietic cells from placental perfusate in combination with hematopoietic cells from cord blood, wherein the cord blood and placenta are from the same individual, *i.e.,* wherein the perfusate and cord blood are matched. In embodiments in which the hematopoietic cells comprise hematopoietic cells from two tissue sources, the hematopoietic cells from the sources can be combined in a ratio of, for example, 1:10, 2:9, 3:8, 4:7:, 5:6, 6:5, 7:4, 8:3, 9:2, 1:10, 1:9, 1:8, 1:7, 1:6, 1:5, 1:4, 1:3, 1:2, 1:1, 2:1, 3:1, 4:1, 5:1, 6:1, 7:1, 8:1 or 9:1.

### 5.1.1. Placental Hematopoietic Stem Cells

In certain embodiments, the hematopoietic cells used in the methods provided herein are placental hematopoietic cells. As used herein, "placental hematopoietic cells" means hematopoietic cells obtained from the placenta itself, and not from placental blood or from umbilical cord blood. In one embodiment, placental hematopoietic cells are CD34⁺. In a specific embodiment, the placental hematopoietic cells are predominantly (*e.g.,* at least about 50%, 55%, 60%, 65%, 70%, 75%, 80%, 85%, 90%, 95% or 98%) CD34⁺CD38⁻ cells. In another specific embodiment, the placental hematopoietic cells are predominantly (*e.g.,* at least about 50%, 55%, 60%, 65%, 70%, 75%, 80%, 85%, 90%, 95% or 98%) CD34⁺CD38⁺ cells. Placental hematopoietic cells can be obtained from a post-partum mammalian (*e.g.,* human) placenta by any means known to those of skill in the art, *e.g.,* by perfusion.

In another embodiment, the placental hematopoietic cell is CD45⁻. In a specific embodiment, the hematopoietic cell is CD34⁺CD45⁻. In another specific embodiment, the placental hematopoietic cells are CD34⁺CD45⁺.

### 5.2. Production of Natural Killer Cells, Natural Killer Cell Populations and Natural Killer Progenitor Cell Populations

Production of NK cells, NK cell populations, and NK progenitor cell populations by the present methods comprises expanding a population of hematopoietic cells. During cell expansion, a plurality of hematopoietic cells within the hematopoietic cell population differentiate into NK cells.

### 5.2.1. Production of TSNK Cells

In one embodiment, provided herein is a method of producing a population of activated natural killer (NK) cells, comprising: (a) seeding a population of hematopoietic stem or progenitor cells in a first medium comprising interleukin-15 (IL-15) and, optionally, one or more of stem cell factor (SCF) and interleukin-7 (IL-7), wherein said IL-15 and optional SCF and IL-7 are not comprised within an undefined component of said medium, such that the population expands, and a plurality of hematopoietic stem or progenitor cells within said population of hematopoietic stem or progenitor cells differentiate into NK cells during said expanding; and (b) expanding the cells from step (a) in a second medium comprising interleukin-2 (IL-2), to produce a population of activated NK cells.

In another embodiment, NK cells provided herein are produced by a two-step process of expansion/ differentiation and maturation of NK cells. The first and second steps comprise culturing the cells in media with a unique combination of cellular factors. In certain embodiments, the process involves (a) culturing and expanding a population of hematopoietic cells in a first medium, wherein a plurality of hematopoietic stem or progenitor cells within the hematopoietic cell population differentiate into NK cells; and (b) expanding the NK cells from step (a) in a second medium, wherein the NK cells are further expanded and differentiated, and wherein the NK cells are maturated (*e.g.,* activated or otherwise possessing cytotoxic activity). In certain embodiments, the method includes no intermediary steps between step (a) and (b), no additional culturing steps prior to step (a), and/or no additional steps (*e.g.,* maturation step) after step (b).

### 5.2.1.1. First Culturing Step

In certain embodiments, the methods provided herein comprises a first step of culturing and expanding a population of hematopoietic cells in a first medium, wherein a plurality of hematopoietic stem or progenitor cells within the hematopoietic cell population differentiate into NK cells.

Without wishing to be bound by any parameter, mechanism or theory, culture of the hematopoietic cells as provided herein results in continuous expansion of the hematopoietic cells and differentiation of NK cells and/or NK progenitor cell populations from said cells. In certain embodiments, hematopoietic cells, *e.g.,* stem cells or progenitor cells, used in the methods provided herein are expanded and differentiated in the first step using a feeder layer. In other embodiments, hematopoietic cells, *e.g.,* stem cells or progenitor cells, are expanded and differentiated in the first step without the use of a feeder layer.

Feeder cell-independent expansion and differentiation of hematopoietic cells can take place in any container compatible with cell culture and expansion, *e.g.,* flask, tube, beaker, dish, multiwell plate, bag or the like. In a specific embodiment, feeder cell-independent expansion of hematopoietic cells takes place in a bag, *e.g.,* a flexible, gas-permeable fluorocarbon culture bag (for example, from American Fluoroseal). In a specific embodiment, the container in which the hematopoietic cells are expanded is suitable for shipping, *e.g.,* to a site such as a hospital or military zone wherein the expanded NK cells are further expanded and differentiated.

In certain embodiments, hematopoietic cells are expanded and differentiated, *e.g.,* in a continuous fashion, in a first culture medium. In one embodiment, the first culture medium is an animal-component free medium. Exemplary animal component-free media useful in the methods provided herein include, but are not limited to, Basal Medium Eagle (BME), Dulbecco's Modified Eagle's Medium (DMEM), Glasgow Minimum Essential Medium (GMEM), Dulbecco's Modified Eagle's Medium/Nutrient Mixture F-12 Ham (DMEM/F-12), Minimum Essential Medium (MEM), Iscove's Modified Dulbecco's Medium (IMDM), Nutrient Mixture F-10 Ham (Ham's F-10), Nutrient Mixture F-12 Ham (Ham's F-12), RPMI-1640 Medium, Williams' Medium E, STEMSPAN® (Cat. No. Stem Cell Technologies, Vancouver, Canada), Glycostem Basal Growth Medium (GBGM®), AIM-V® medium (Invitrogen), X-VIVO™ 10 (Lonza), X-VIVO™ 15 (Lonza), OPTMIZER (Invitrogen), STEMSPAN® H3000 (STEMCELL Technologies), CELLGRO COMPLETE™ (Mediatech), EL08-1D2, Myelocult™ H5100, or any modified variants or combinations thereof.

In preferred embodiments, the first culture medium comprises interleukin-15 (IL-15), and one or more other medium supplements (*e.g.,* nutrients, cytokines and/or factors). Medium supplements suitable for use in the methods provided herein include, for example without limitation, serum (such as human serum AB, fetal bovine serum (FBS) or fetal calf serum (FCS)), vitamins, bovine serum albumin (BSA), amino acids (*e.g.,* L-glutamine), fatty acids (*e.g.,* oleic acid, linoleic acid or palmitic acid), insulin (*e.g.,* recombinant human insulin), transferrin (iron saturated human transferrin), β-mercaptoethanol, stem cell factor (SCF), Fms-like-tyrosine kinase 3 ligand (Flt3-L), cytokines such as interleukin-2 (IL-2), interleukin-7 (IL-7), thrombopoietin (Tpo), heparin, or O-acetyl-carnitine (also referred to as acetylcarnitine, O-acetyl-L-carnitine or OAC). In a specific embodiment, the medium used herein comprises human serum AB. In another specific embodiment, the medium used herein comprises FBS. In another specific embodiment, the medium used herein comprises OAC.

In certain embodiments, the first medium does not comprise one or more of, granulocyte colony-stimulating factor (G-CSF), granulocyte/macrophage colony stimulating factor (GM-CSF), interleukin-6 (IL-6), macrophage inflammatory Protein 1 α (MIP1α), or leukemia inhibitory factor (LIF).

Thus, in one aspect, provided herein is a two-step method of producing NK cells, wherein said first step comprises expanding and differentiating a population of hematopoietic cells in a first culture medium in the absence of feeder cells, wherein a plurality of hematopoietic cells within said population of hematopoietic cells differentiate into NK cells during said expanding, and wherein the medium comprises IL-15 at a concentration 1 to about 150 ng/mL, SCF at a concentration of about 1 to about 150 ng/mL, IL-2 at a concentration of about 50 to about 1500 IU/mL, IL-7 at a concentration of about 1 to about 150 ng/mL, and heparin at a concentration of about 0.1 to about 30 IU/mL, and wherein said SCF, IL-2, IL-7, IL-15 and heparin are not comprised within an undefined component of said medium (*e.g.,* serum). In certain embodiments, said medium comprises one or more of O-acetyl-carnitine (also referred to as acetylcarnitine, O-acetyl-L-carnitine or OAC), or a compound that affects acetyl-CoA cycling in mitochondria, thiazovivin, Y-27632, pyintegrin, Rho kinase (ROCK) inhibitors, caspase inhibitors or other anti-apoptotic compounds/peptides, NOVA-RS (Sheffield Bio-Science) or other small-molecule growth enhancers. In certain embodiments, said medium comprises one or more anti-oxidants, *e.g.,* holo-transferrin, insulin solution, reduced glutathione, sodium selenite, ethanolamine, ascorbic acid, b-mercaptoethanol, O-acetyl-L-carnitine, N-acetylcysteine, (+/-) lipoic acid, nicotinamide, or resveratrol. In certain embodiments, said medium comprises about 0.5 mM-10 mM OAC. In one embodiment, said medium comprises Stemspan® H3000, and/or DMEM:F12 and about 0.5, 1, 2, 3, 4, 5, 6, 7, 8, 9, or 10 mM OAC. In a specific embodiment of the method, said medium is GBGM®. In another specific embodiment, said medium comprises STEMSPAN® H3000 and about 5 mM of OAC. In another specific embodiment, said medium comprises DMEM:F12 and about 5 mM of OAC. The OAC can be added anytime during the culturing methods provided herein. In certain embodiments, said OAC is added to the first medium and/or during the first culturing step. In some embodiments, said OAC is added to the first medium on Day 0, 1, 2, 3, 4, 5, 6, 7, 8, 9, 10, 11, 12, 13, 14, 15, 16, 17, 18, 19, 20, 21 of the culture. In a specific embodiment, said OAC is added to the first medium on Day 7 of the first culturing step. In a more specific embodiment, said OAC is added to the first medium on Day 7 of the culture and is present throughout the first and second culturing steps. In certain embodiments, said OAC is added to the second medium and/or during the second culturing step. In some embodiments, said OAC is added to the second medium on Day 22, 23, 24, 25, 26, 27, 28, 29, 30, 31, 32, 33, 34, 35 of the culture.

In another specific embodiment, said medium is IMDM supplemented with about 5-20% BSA, about 1-10 µg/mL recombinant human insulin, about 10-50 µg/mL iron saturated human transferrin and about 10-50 µM β-mercaptoethanol. In another specific embodiment, said medium does not comprise one or more, or any, of IL-11, IL-3, homeobox-B4 (HoxB4), and/or methylcellulose.

In other specific embodiments, said medium comprises SCF at a concentration of about 0.1 to about 500 ng/mL; about 5 to about 100 ng/mL; or about 20 ng/mL. In other specific embodiments, said medium comprises IL-2 at a concentration of about 10 to about 2000 IU/mL; or about 100 to about 500 IU/mL; or about 200 IU/mL. In other specific embodiments, said medium comprises IL-7 at a concentration of about 0.1 to about 500 ng/mL; about 5 to about 100 ng/mL; or about 20 ng/mL. In other specific embodiments, said medium comprises IL-15 at a concentration of about 0.1 to about 500 ng/mL; about 5 to about 100 ng/mL; or about 10 ng/mL. In other specific embodiments, said medium comprises heparin at concentration of about 0.05 to about 100 U/mL; or about 0.5 to about 20 U/ml; or about 1.5 U/mL.

In yet other specific embodiment of the method, said medium further comprises Fms-like-tyrosine kinase 3 ligand (Flt-3L) at a concentration of about 1 to about 150 ng/mL, thrombopoietin (Tpo) at a concentration of about 1 to about 150 ng/mL, or a combination of both. In other specific embodiments, said medium comprises Flt-3L at a concentration of about 0.1 to about 500 ng/mL; about 5 to about 100 ng/mL; or about 20 ng/mL. In other specific embodiments, said medium comprises Tpo at a concentration of about 0.1 to about 500 ng/mL; about 5 to about 100 ng/mL; or about 20 ng/mL.

In a more specific embodiment of the method, the first culture medium is GBGM®, which comprises about 20 ng/mL SCF, about 20 ng/mL IL-7, about 10 ng/mL IL-15. In another more specific embodiment of the method, the first culture medium is GBGM®, which comprises about 20 ng/mL SCF, about 20 ng/mL Flt3-L, about 200 IU/mL IL-2, about 20 ng/mL IL-7, about 10 ng/mL IL-15, about 20 ng/mL Tpo, and about 1.5 U/mL heparin. In another specific embodiment, said first culture medium further comprises 10% human serum (*e.g.,* human serum AB) or fetal serum (*e.g.,* FBS).

In another embodiment, hematopoietic cells are expanded by culturing said cells, *e.g.,* in said first medium, in contact or proximity with an immunomodulatory compound, *e.g.,* a TNF-α inhibitory compound, for a time and in an amount sufficient to cause a detectable increase in the proliferation of the hematopoietic cells over a given time, compared to an equivalent number of hematopoietic cells not contacted or brought into proximity with the immunomodulatory compound. *See, e.g.,* U.S. Patent Application Publication No. 2003/0235909, issued as U.S. Patent No. 7,498,171, the disclosures of which are hereby incorporated by reference in their entirety. In certain embodiments, the immunomodulatory compound is an amino-substituted isoindoline. In a preferred embodiment, the immunomodulatory compound is 3-(4-amino-1-oxo-1,3-dihydroisoindol-2-yl)-piperidine-2,6-dione; 3-(4'aminoisolindoline-1'-one)-1-piperidine-2,6-dione; 4-(amino)-2-(2,6-dioxo(3-piperidyl))-isoindoline-1,3-dione; or 4-Amino-2-(2,6-dioxopiperidin-3-yl)isoindole-1,3-dione. In another preferred embodiment, the immunomodulatory compound is pomalidomide, or lenalidomide. In another embodiment, said immunomodulatory compound is a compound having the structure 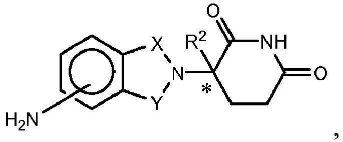 wherein one of X and Y is C=O, the other of X and Y is C=O or CH₂, and R² is hydrogen or lower alkyl, or a pharmaceutically acceptable salt, hydrate, solvate, clathrate, enantiomer, diastereomer, racemate, or mixture of stereoisomers thereof. In another embodiment, said immunomodulatory compound is a compound having the structure 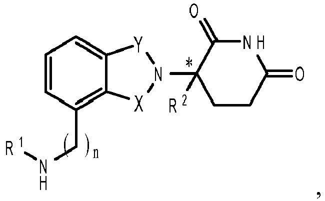
wherein one of X and Y is C=O and the other is CH₂ or C=O;
R¹ is H, (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, C(O)R³, C(S)R³, C(O)OR⁴ , (C₁-C₈)alkyl-N(R⁶)₂, (C₁-C₈)alkyl-OR⁵, (C₁-C₈)alkyl-C(O)OR⁵, C(O)NHR³, C(S)NHR³, C(O)NR³R^{3'}, C(S)NR³R^{3'} or (C₁-C₈)alkyl-O(CO)R⁵;
R² is H, F, benzyl, (C₁-C₈)alkyl, (C₂-C₈)alkenyl, or (C₂-C₈)alkynyl;
R³ and R^{3'} are independently (C₁-C₈)alkyl, (C₃-C₇)cycloalkyl, (C₂-C₈)alkenyl, (C₂-C₈)alkynyl, benzyl, aryl, (C₀-C₄)alkyl-(C₁-C₆)heterocycloalkyl, (C₀-C₄)alkyl-(C₂-C₅)heteroaryl, (C₀-C₈)alkyl-N(R⁶)₂, (C₁-C₈)alkyl-OR⁵, (C₁-C₈)alkyl-C(O)OR⁵, (C₁-C₈)alkyl-O(CO)R⁵, or C(O)OR⁵;
R⁴ is (C₁-C₈)alkyl, (C₂-C₈)alkenyl, (C₂-C₈)alkynyl, (C₁-C₄)alkyl-OR⁵, benzyl, aryl, (C₀-C₄)alkyl-(C₁-C₆)heterocycloalkyl, or (C₀-C₄)alkyl-(C₂-C₅)heteroaryl;
R⁵ is (C₁-C₈)alkyl, (C₂-C₈)alkenyl, (C₂-C₈)alkynyl, benzyl, aryl, or (C₂-C₅)heteroaryl;
each occurrence of R⁶ is independently H, (C₁-C₈)alkyl, (C₂-C₈)alkenyl, (C₂-C₈)alkynyl, benzyl, aryl, (C₂-C₅)heteroaryl, or (C₀-C₈)alkyl-C(O)O-R⁵ or the R⁶ groups can join to form a heterocycloalkyl group;
n is 0 or 1; and
* represents a chiral-carbon center;
or a pharmaceutically acceptable salt, hydrate, solvate, clathrate, enantiomer, diastereomer, racemate, or mixture of stereoisomers thereof. In another embodiment, said immunomodulatory compound is a compound having the structure 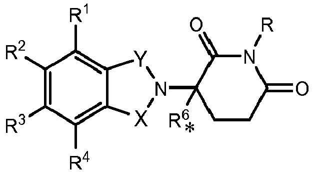 wherein:
one of X and Y is C=O and the other is CH₂ or C=O;
R is H or CH₂OCOR';
(i) each of R¹, R², R³, or R⁴, independently of the others, is halo, alkyl of 1 to 4 carbon atoms, or alkoxy of 1 to 4 carbon atoms or (ii) one of R¹, R², R³, or R⁴ is nitro or -NHR⁵ and the remaining of R¹, R², R³, or R⁴ are hydrogen;
R⁵ is hydrogen or alkyl of 1 to 8 carbons
R⁶ hydrogen, alkyl of 1 to 8 carbon atoms, benzo, chloro, or fluoro;
R' is R⁷-CHR¹⁰-N(R⁸R⁹);
R⁷ is m-phenylene or p-phenylene or -(CₙH₂ₙ)- in which n has a value of 0 to 4;
each of R⁸ and R⁹ taken independently of the other is hydrogen or alkyl of 1 to 8 carbon atoms, or R⁸ and R⁹ taken together are tetramethylene, pentamethylene, hexamethylene, or -CH₂CH₂X₁CH₂CH₂- in which X₁ is -O-, -S-, or -NH-;
R¹⁰ is hydrogen, alkyl of to 8 carbon atoms, or phenyl; and
* represents a chiral-carbon center;
or a pharmaceutically acceptable salt, hydrate, solvate, clathrate, enantiomer, diastereomer, racemate, or mixture of stereoisomers thereof.

In a specific embodiment, expansion of the hematopoietic cells is performed in IMDM supplemented with 20% BITS (bovine serum albumin, recombinant human insulin and transferrin), SCF, Flt-3 ligand, IL-3, and 4-(Amino)-2-(2,6-dioxo(3-piperidyl))-isoindoline-1,3-dione (10 µM in 0.05% DMSO). In a more specific embodiment, about 5 x 10⁷ hematopoietic cells, *e.g.,* CD34⁺ cells, are expanded in the medium to from about 5 x 10¹⁰ cells to about 5 x 10¹² cells, which are resuspended in 100 mL of IMDM to produce a population of expanded hematopoietic cells. The population of expanded hematopoietic cells is preferably cryopreserved to facilitate shipping.

In various specific embodiments, at least 50%, 55%, 60%, 65%, 70%. 75%, 80%, 85%, 90%, 95%, 97%, 98%, or 99% of the hematopoietic cells are differentiated to NK cells.

In certain embodiments, the method of expansion and differentiation of the hematopoietic cells, as described herein, comprises maintaining the cell population comprising said hematopoietic cells at between about 2 x 10⁴ and about 6 x 10⁶ cells per milliliter, *e.g.,* between about 2 x 10⁴ and about 2 x 10⁵ cells per milliliter, during expansion and differentiation. In certain other embodiments, the method of expansion and differentiation of the hematopoietic cells, as described herein, comprises maintaining the cell population comprising said hematopoietic cells at no more than about 1 x 10⁵ cells, 1 x 10⁴ cells, or 1 x 10³ cells per milliliter. In certain other embodiments, the method of expansion and differentiation of the hematopoietic cells, as described herein, comprises maintaining the cell population comprising said hematopoietic cells at no more than about 1 x 10⁵ cells per milliliter, 2 x 10⁵ cells per milliliter, 3 x 10⁵ cells per milliliter, 4 x 10⁵ cells per milliliter, 5 x 10⁵ cells per milliliter, 6 x 10⁵ cells per milliliter, 7 x 10⁵ cells per milliliter, 8 x 10⁵ cells per milliliter, 9 x 10⁵ cells per milliliter, 1 x 10⁶ cells per milliliter, 2 x 10⁶ cells per milliliter, 3 x 10⁶ cells per milliliter, 4 x 10⁶ cells per milliliter, 5 x 10⁶ cells per milliliter, 6 x 10⁶ cells per milliliter, 7 x 10⁶ cells per milliliter, 8 x 10⁶ cells per milliliter, or 9 x 10⁶ cells per milliliter. In certain embodiments, provided herein is a method of culturing cells described herein, wherein a density of about 1 x 10³ to 5 x 10³, 5 x 10³ to 1 x 10⁴, 1 x 10⁴ to 5 x 10⁴, 5 x 10⁴ to 1 x 10⁵, 1 x 10⁵ to 5 x 10⁵, 5 x 10⁵ to 1 x 10⁶, 1 x 10⁶ to 5 x 10⁶, 5 x 10⁶ to 1 x 10⁷, 1 x 10⁷ to 5 x 10⁷ , or more cells per cm² is achieved.

The time for expansion and differentiation of hematopoietic cells into NK cells can be, for example, from about 3 days to about 120 days. In one embodiment, the differentiation time is about 7 days to about 75 days. In another embodiment, the differentiation time is about 14 days to about 50 days. In a specific embodiment, the differentiation time is about 21 days to about 28 days.

### 5.2.1.2. Second Step

In the method provided herein, the hematopoietic cells, *e.g.,* stem cells or progenitor cells, and natural killer cells, resulting from the first step, are further expanded and differentiated in a second step, *e.g.,* without the use of feeder layer or in the presence of feeder cells. Culture of the cells as provided herein results in continuous expansion, differentiation as well as maturation of the NK cells from the first step. In the second step, the NK cells are expanded, differentiated and maturated, in a continuous fashion, in a second culture medium, *e.g.,* comprising different cytokines and/or bioactive molecules than said first medium. In certain embodiments, the second culture medium is an animal component-free medium. Exemplary animal component-free cell culture media are described above.

Thus, in one aspect, provided herein is a method of producing NK cells, comprising expanding the NK cells from the first step, described above, in a second medium in the presence of feeder cells and in contact or proximity with interleukin-2 (IL-2). In specific embodiments, said second medium comprises cell growth medium comprising IL-2, *e.g.,* 10 IU/mL to 1000 IU/mL, and one or more of: human serum (*e.g.,* human serum AB), fetal bovine serum (FBS) or fetal calf serum (FCS), *e.g.,* 5%-15% FCS v/v; transferrin, *e.g.,* 10 µg/mL to 50 µg/mL; insulin, *e.g.,* 5 µg/mL to 20 µg/mL; ethanolamine, *e.g.,* 5 x 10⁻⁴ to 5 x 10⁻⁵ M; oleic acid, *e.g.,* 0.1 µg/mL to 5 µg/mL; linoleic acid, *e.g.,* 0.1 µg/mL to 5 µg/mL; palmitic acid, *e.g.,* 0.05 µg/mL to 2 µg/mL; bovine serum albumin (BSA), *e.g.,* 1 µg/mL to 5 µg/mL; and/or phytohemagglutinin, *e.g.,* 0.01 µg/mL to 1 µg/mL. In a more specific embodiment, said second medium comprises cell growth medium comprising FBS or FCS, *e.g.,* 10% FCS v/v, IL-2, transferrin, insulin, ethanolamine, oleic acid, linoleic acid, palmitic acid, bovine serum albumin (BSA) and phytohemagglutinin. In a more specific embodiment, said second medium comprises Iscove's Modified Dulbecco's Medium (IMDM), 10% FBS or FCS, 400 IU IL-2, 35 µg/mL transferrin, 5 µg/mL insulin, 2 x 10⁻⁵ M ethanolamine, 1 µg/mL oleic acid, 1 µg/mL linoleic acid

(Sigma-Aldrich), 0.2 µg/mL palmitic acid (Sigma-Aldrich), 2.5 µg/mL BSA (Sigma-Aldrich) and 0.1 µg/mL phytohemagglutinin.

In certain embodiments, the second medium does not comprise one or more of, granulocyte colony-stimulating factor (G-CSF), granulocyte/macrophage colony stimulating factor (GM-CSF), interleukin-6 (IL-6), macrophage inflammatory Protein 1 α (MIP1α), or leukemia inhibitory factor (LIF).

In addition to the method, provided herein are any of the media described above as compositions.

Feeder cells, when used, can be established from various cell types. Examples of these cell types include, without limitation, fibroblasts, stem cells (*e.g.,* tissue culture-adherent placental stem cells), blood cells (*e.g.,* peripheral blood mononuclear cells (PBMC)), and cancerous cells (*e.g.,* chronic myelogenous leukemia (CML) cells such as K562). In a specific embodiment, said culturing in said second medium comprises culturing using feeder cells, *e.g.,* K562 cells and/or peripheral blood mononuclear cells (PBMCs), *e.g.,* at the time the cells are started in said second medium, 1, 2, 3, 4, 5, 6, 7, 8, 9 or 10 days thereafter. In certain embodiments, feeder cells are optionally from a different species as the cells they are supporting. For example, human NK cells can be supported by mouse embryonic fibroblasts (from primary culture or a telomerized line).

In certain embodiments, feeder cells are optionally inactivated by irradiation (*e.g.,* γ-irradiation) or treatment with an anti-mitotic agent such as mitomycin C, to prevent them from outgrowing the cells they are supporting, but permit synthesis of important factors that support the NK cells. For example, cells can be irradiated at a dose to inhibit proliferation but permit synthesis of important factors that support human embryonic stem (hES) cells (about 4000 rads gamma irradiation).

Culture of NK cells for the second step can take place in any container compatible with cell culture and expansion, *e.g.,* flask, tube, beaker, dish, multiwell plate, bag or the like. In a specific embodiment, feeder cell-dependent culture of NK cells takes place in a bag, *e.g.,* a flexible, gas-permeable fluorocarbon culture bag (for example, from American Fluoroseal). In a specific embodiment, the container in which the NK cells are cultured is suitable for shipping, *e.g.,* to a site such as a hospital or military zone wherein the expanded NK cells are further expanded, differentiated and maturated.

Differentiation of the cells from step 1 into TSNK cells can be assessed by detecting NK cell-specific markers, *e.g.,* by flow cytometry. NK cell-specific markers include, but are not limited to, CD56, CD94, CD117 and NKp46. Differentiation can also be assessed by the morphological characteristics of NK cells, *e.g.,* large size, high protein synthesis activity in the abundant endoplasmic reticulum (ER), and/or preformed granules.

The time for expansion and differentiation of cells from step 1 into TSNK cells can be, for example, from about 3 days to about 120 days. In one embodiment, the differentiation time is about 7 days to about 75 days. In another embodiment, the differentiation time is about 14 days to about 50 days. In a specific embodiment, the differentiation time is about 10 days to about 21 days.

Differentiation of hematopoietic cells into NK cells can be assessed by detecting markers, *e.g.,* CD56, CD94, CD117, NKG2D, DNAM-1 and NKp46, by, for example, flow cytometry. Differentiation can also be assessed by the morphological characteristics of NK cells, *e.g.,* large size, high protein synthesis activity in the abundant endoplasmic reticulum (ER), and/or preformed granules. Maturation of NK cells (*e.g.,* TSNK cells) can be assessed by detecting one or more functionally relevant makers, for example, CD94, CD161, NKp44, DNAM-1, 2B4, NKp46, CD94, KIR, and the NKG2 family of activating receptors (*e.g.,* NKG2D). Maturation of NK cells (*e.g.,* TSNK cells) can also be assessed by detecting specific markers during different developmental stages. For example, in one embodiment, NK progenitor cells are CD34⁺, CD45RA⁺, CD10⁺, CD117⁻ and/or CD161⁻. In another embodiment, NK progenitor cells are CD34⁺, CD45RA⁺, CD10⁻, CD117⁺, and/or CD161⁻. In another embodiment, immature NK cells are CD34⁻, CD117⁺, CD161⁺, NKp46⁻ and/or CD94/NKG2A⁻. In another embodiment, CD56^{bright} NK cells are CD117⁺, NKp46⁺, CD94/NKG2A⁺, CD16⁻, and/or KIR^{+/-}. In another embodiment, CD56^{dim} NK cells are CD117⁻, NKp46⁺, CD94/NKG2A^{+/-}, CD16+, and/or KIR⁺. In a specific embodiment, maturation of NK cells (*e.g.,* TSNK cells) is determined by the percentage of NK cells (*e.g.,* TSNK cells) that are CD161⁻, CD94⁺ and/or NKp46⁺. In a more specific embodiment, at least 10%, 20%, 25%, 30%, 35%, 40%, 50%, 55%, 60%, 65% or 70% of mature NK cells (*e.g.,* TSNK cells) are NKp46⁺. In another more specific embodiment, at least 10%, 20%, 25%, 30%, 35%, 40%, 45% or 50% of mature NK cells (*e.g.,* TSNK cells) are CD94⁺. In another more specific embodiment, at least 10%, 20%, 25%, 30%, 35%, 40%, 45% or 50% of mature NK cells (*e.g.,* TSNK cells) are CD161⁻.

In certain embodiments, the differentiation of hematopoietic cells into NK cells are assessed by detecting the expression level of, *e.g.,* CD3, CD7 or CD127, CD10, CD14, CD15, CD16, CD33, CD34, CD56, CD94, CD117, CD161, NKp44, NKp46, NKG2D, DNAM-1, 2B4 or TO-PRO-3, using, *e.g.,* antibodies to one or more of these cell markers. Such antibodies can be conjugated to a detectable label, for example, as fluorescent label, *e.g.,* FITC, R-PE, PerCP, PerCP-Cy5.5, APC, APC-Cy7 or APC-H7.

### 5.2.2. Production of NK Progenitor Cell Populations and NK Cell Populations Using a Three-Stage Method

In one embodiment, provided herein is a three-stage method of producing NK cell populations or NK progenitor cell populations. In certain embodiments, the method of expansion and differentiation of the hematopoietic cells, as described herein, to produce NK progenitor cell populations or NK cell populations according to a three-stage method described herein comprises maintaining the cell population comprising said hematopoietic cells at between about 2 x 10⁴ and about 6 x 10⁶ cells per milliliter, *e.g.,* between about 2 x 10⁴ and about 2 x 10⁵ cells per milliliter, during expansion and differentiation. In certain other embodiments, the method of expansion and differentiation of the hematopoietic cells, as described herein, comprises maintaining the cell population comprising said hematopoietic cells at no more than about 1 x 10⁵ cells per milliliter. In certain other embodiments, the method of expansion and differentiation of the hematopoietic cells, as described herein, comprises maintaining the cell population comprising said hematopoietic cells at no more than about 1 x 10⁵ cells per milliliter, 2 x 10⁵ cells per milliliter, 3 x 10⁵ cells per milliliter, 4 x 10⁵ cells per milliliter, 5 x 10⁵ cells per milliliter, 6 x 10⁵ cells per milliliter, 7 x 10⁵ cells per milliliter, 8 x 10⁵ cells per milliliter, 9 x 10⁵ cells per milliliter, 1 x 10⁶ cells per milliliter, 2 x 10⁶ cells per milliliter, 3 x 10⁶ cells per milliliter, 4 x 10⁶ cells per milliliter, 5 x 10⁶ cells per milliliter, 6 x 10⁶ cells per milliliter, 7 x 10⁶ cells per milliliter, 8 x 10⁶ cells per milliliter, or 9 x 10⁶ cells per milliliter.

In a certain embodiment, the three-stage method comprises a first stage ("stage 1") comprising culturing hematopoietic stem cells or progenitor cells, *e.g.,* CD34⁺ stem cells or progenitor cells, in a first medium for a specified time period, *e.g.,* as described herein. In certain embodiments, the first medium contains one or more factors that promote expansion of hematopoietic progenitor cells, one or more factors for initiation of lymphoid differentiation within the expanding hematopoietic progenitor population, and/or one or more factors that mimic stromal feeder support. In certain embodiments, the first medium comprises one or more cytokines (for example, Flt3L, TPO, SCF). In certain embodiments, the first medium comprises IL-7. In certain embodiments, the first medium comprises sub-ng/mL concentrations of G-CSF, IL-6 and/or GM-CSF. In a specific embodiment, the first medium comprises the cytokines Flt3L, TPO, and SCF, IL-7, and sub-ng/mL concentrations of G-CSF, IL-6 and GM-CSF. In specific embodiments, in the first medium, CD34+ cells undergo expansion into lineage specific progenitors, which then become CD34-. In certain embodiments, this expansion occurs rapidly. In certain embodiments, the CD34- cells comprise more than 50%, more than 55%, more than 60%, more than 65%, more than 70%, more than 75%, more than 80%, or more of the total population at the end of stage 1. In a more specific embodiment, CD34- cells comprise more than 80% of the total population at the end of stage 1.

In certain embodiments, subsequently, in "stage 2" said cells are cultured in a second medium for a specified time period, *e.g.,* as described herein. In certain embodiments, the second medium contains factors that may promote further expansion of lymphoid progenitors, factors that may contribute to development along the NK lineage, and/or factors that mimic stromal feeder support. In certain embodiments, the second medium comprises one or more cytokines (*e.g.,* Flt3L, SCF, IL-15, and/or IL-7). In certain embodiments, the second medium comprises IL-17 and/or IL-15. In certain embodiments, the second medium comprises sub-ng/mL concentrations of G-CSF, IL-6 and/or GM-CSF. In a specific embodiment, the second medium comprises the cytokines Flt3L, SCF, IL-15, and IL-7, IL-17 and IL-15, and sub-ng/mL concentrations of G-CSF, IL-6 and GM-CSF.

In certain embodiments, subsequently, in "stage 3" said cells are cultured in a third medium for a specified time period, *e.g.,* as described herein. In certain embodiments, the third medium comprises factors that promote differentiation and functional activation of CD56+CD3-CD16- cells, which may be NK progenitor cells. In one embodiment, such factors comprise IL2 and IL12 and IL18, IL12 and IL15, IL12 and IL18, IL2 and IL12 and IL15 and IL18, or IL2 and IL15 and IL18. In certain embodiments, the third medium comprises factors that mimic stromal feeder support. In certain embodiments, the third medium comprises one or more cytokines (*e.g.,* SCF, IL-15, IL-7, IL-2). In certain embodiments, the third medium comprises sub-ng/mL concentrations of G-CSF, IL-6 and/or GM-CSF. In a specific embodiment, the third medium comprises the cytokines SCF, IL-15, IL-7, IL-2, and sub-ng/mL concentrations of G-CSF, IL-6 and GM-CSF.

In a specific embodiment, the three-stage method is used to produce NK progenitor cell populations. In another specific embodiment, the three-stage method is used to produce NK cell populations. In certain embodiments, the three-stage method is conducted in the absence of stromal feeder cell support. In certain embodiments, the three-stage method is conducted in the absence of exogenously added steroids (*e.g.,* cortisone, hydrocortisone, or derivatives thereof).

In certain embodiments, the first medium used in the three-stage methods described herein may contain any of the components of the first or second medium described above in connection with the two-step method. In certain embodiments, said first medium used in the three-stage method comprises medium comprising one or more of: animal serum, e.g., human serum (*e.g.,* human serum AB), fetal bovine serum (FBS) or fetal calf serum (FCS), e.g., 1% to 20 % v/v serum, e.g., 5% to 20% v/v serum; stem cell factor (SCF), *e.g.,* 1 ng/mL to 50 ng/mL SCF; FMS-like tyrosine kinase-3 ligand (Flt-3 ligand), *e.g.,* 1 ng/ml to 30 ng/mL Flt-3 ligand; interleukin-7 (IL-7), *e.g.,* 1 ng/mL to 50 ng/mL IL-7; thrombopoietin (TPO), *e.g.,* 1 ng/mL to 100 ng/mL, for example, 1 ng/mL to 50 ng/mLTPO; interleukin-2 (IL-2), *e.g.,* up to 2000 IU/mL, for example, 50 IU/mL to 500 IU/mL; and/or heparin, *e.g.,* low-weight heparin (LWH), *e.g.,* 0.1 IU/mL to 10 IU/mL heparin. In certain embodiments, said first medium additionally comprises one or more of the following: antibiotics such as gentamycin; antioxidants such as transferrin, insulin, and/or beta-mercaptoethanol; sodium selenite; ascorbic acid; ethanolamine; and glutathione. In certain embodiments, said first medium additionally comprises OAC. In certain embodiments, said first medium additionally comprises interleukin-6 (IL-6), leukemia inhibitory factor (LIF), G-CSF, GM-CSF, and/or MIP-1α. In certain embodiments, said first medium additionally comprises one or more anti-oxidants, *e.g.,* holo-transferrin, insulin solution, reduced glutathione, sodium selenite, ethanolamine, ascorbic acid, b-mercaptoethanol, O-acetyl-L-carnitine, N-acetylcysteine, (+/-) lipoic acid, nicotinamide, or resveratrol. In certain embodiments, the medium that provides the base for the first medium is a cell/tissue culture medium known to those of skill in the art, e.g., a commercially available cell/tissue culture medium such as GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640; or is a medium that comprises components generally included in known cell/tissue culture media, such as the components included in GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640.

In certain embodiments, the second medium used in the three-stage methods described herein may contain any of the components of the first or second medium described above in connection with the two-step method. In certain embodiments, said second medium used in the three-stage method comprises medium comprising one or more of: animal serum, e.g., human serum (*e.g.,* human serum AB), FBS or FCS, e.g., 5% to 20% v/v serum; SCF, *e.g.,* 1 ng/mL to 50 ng/mL SCF; Flt-3 ligand, *e.g.,* 1 ng/ml to 30 ng/mL Flt-3 ligand; IL-7, *e.g.,* 1 ng/mL to 50 ng/mL IL-7; interleukin-15 (IL-15), e.g., 1 ng/mL to 50 ng/mL IL-15; and/or heparin, e.g., LWH, *e.g.,* 0.1 IU/mL to 10 IU/mL heparin. In certain embodiments, said second medium additionally comprises one or more of the following: antibiotics such as gentamycin; antioxidants such as transferrin, insulin, and/or beta-mercaptoethanol; sodium selenite; ascorbic acid; ethanolamine; and glutathione. In certain embodiments, said second medium additionally comprises OAC. In certain embodiments, said second medium additionally comprises interleukin-6 (IL-6), leukemia inhibitory factor (LIF), G-CSF, GM-CSF, and/or MIP-1α. In certain embodiments, said second medium additionally comprises one or more anti-oxidants, *e.g.,* holo-transferrin, insulin solution, reduced glutathione, sodium selenite, ethanolamine, ascorbic acid, b-mercaptoethanol, O-acetyl-L-carnitine, N-acetylcysteine, (+/-) lipoic acid, nicotinamide, or resveratrol. In certain embodiments, the medium that provides the base for the second medium is a cell/tissue culture medium known to those of skill in the art, e.g., a commercially available cell/tissue culture medium such as GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640; or is a medium that comprises components generally included in known cell/tissue culture media, such as the components included in GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640.

In certain embodiments, the third medium used in the three-stage methods described herein may contain any of the components of the first or second medium described above in connection with the two-step method. In certain embodiments, said third medium used in the three-stage method comprises medium comprising one or more of: animal serum, e.g., human serum (*e.g.,* human serum AB), FBS or FCS, e.g., 5% to 20% v/v serum; SCF, *e.g.,* 1 ng/mL to 50 ng/mL SCF; Flt-3 ligand, *e.g.,* 1 ng/ml to 30 ng/mL Flt-3 ligand; IL-7, *e.g.,* 1 ng/mL to 50 ng/mL IL-7; IL-15, e.g., 1 ng/mL to 50 ng/mL IL-15; and interleukin-2 (IL-2), e.g., in the range from 0 to 2000 IU/mL, for example, 50 IU/mL to 1000 IU/mL IL-2. In certain embodiments, said third medium additionally comprises one or more of the following: antibiotics such as gentamycin; antioxidants such as transferrin, insulin, and/or beta-mercaptoethanol; sodium selenite; ascorbic acid; ethanolamine; and glutathione. In certain embodiments, said third medium additionally comprises OAC. In certain embodiments, said third medium additionally comprises interleukin-6 (IL-6), leukemia inhibitory factor (LIF), G-CSF, GM-CSF, and/or MIP-1α. In certain embodiments, said third medium additionally comprises one or more anti-oxidants, *e.g.,* holo-transferrin, insulin solution, reduced glutathione, sodium selenite, ethanolamine, ascorbic acid, b-mercaptoethanol, O-acetyl-L-carnitine, N-acetylcysteine, (+/-) lipoic acid, nicotinamide, or resveratrol. In certain embodiments, the medium that provides the base for the third medium is a cell/tissue culture medium known to those of skill in the art, e.g., a commercially available cell/tissue culture medium such as GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640; or is a medium that comprises components generally included in known cell/tissue culture media, such as the components included in GBGM®, AIM-V®, X-VIVO™ 10, X-VIVO™ 15, OPTMIZER, STEMSPAN® H3000, CELLGRO COMPLETE™, DMEM:Ham's F12 ("F12") (*e.g.,* 2:1 ratio, or high glucose or low glucose DMEM), Advanced DMEM (Gibco), EL08-1D2, Myelocult™ H5100, IMDM, and/or RPMI-1640..

In certain embodiments, in the three-stage methods described herein, said hematopoietic stem or progenitor cells are cultured in said first medium for 1, 2, 3, 4, 5, 6, 7, 8, 9, 10, 11, 12, 13, 14, 15, 16, 17, 18, 19, or 20 days before said culturing in said second medium. In certain embodiments, cells cultured in said first medium are cultured in said second medium for 1, 2, 3, 4, 5, 6, 7, 8, 9, 10, 11, 12, 13, 14, 15, 16, 17, 18, 19, or 20 days before said culturing in said third medium. In certain embodiments, cells cultured in said first medium and said second medium are cultured in said third medium for 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days, or for more than 30 days.

In certain embodiments, in the three-stage methods described herein, said hematopoietic stem or progenitor cells are cultured in said first medium for 2-12 days, 3-11 days, for example, 3-5, 4-6, 5-7, 6-8, 7-9, 8-10, or 9-11 days, before said culturing in said second medium. In certain embodiments, cells cultured in said first medium are cultured in said second medium for 1-10 days, for example, 1-3, 2-4, 3-5, 4-6, 5-7, 6-8, or 7-9 days, before said culturing in said third medium. In certain embodiments, cells cultured in said first medium and said second medium are cultured in said third medium for 2-27 days, for example, 3-25 days, e.g., for 3-5, 4-6, 5-7, 6-8, 7-9, 8-10, 9-11, 10-12, 11-13, 12-14, 13-15, 14-16, 15-17, 16-18, 17-19, 18-20, 19-21, 20-22, 21-23, 22-24, or 23-25 days.

In a specific embodiment, in the three-stage methods described herein, said hematopoietic stem or progenitor cells are cultured in said first medium for 9 days before said culturing in said second medium; cultured in said second medium for 5 days before said culturing in said third medium; and cultured in said third medium for 7 days, i.e., the cells are cultured a total of 21 days.

In a specific embodiment, in the three-stage methods described herein, said hematopoietic stem or progenitor cells are cultured in said first medium for 7-9 days before said culturing in said second medium; cultured in said second medium for 5-7 days before said culturing in said third medium; and cultured in said third medium for 21-35 days, i.e., the cells are cultured a total of 35 days. In a more specific embodiment, in the three-stage methods described herein, said hematopoietic stem or progenitor cells are cultured in said first medium for 9 days before said culturing in said second medium; cultured in said second medium for 5 days before said culturing in said third medium; and cultured in said third medium for 21 days, i.e., the cells are cultured a total of 35 days.

### 5.3. Isolation of NK Cells

Methods of isolating natural killer cells are known in the art and can be used to isolate the natural killer cells, e.g., TSNK cells or NK progenitor cells or NK cells produced using the three-stage method , described herein. NK cells can be isolated or enriched by staining cells from a tissue source, *e.g.,* peripheral blood, with antibodies to CD56 and CD3, and selecting for CD56⁺CD3 cells. NK cells, e.g., TSNK cells, cells can be isolated using a commercially available kit, for example, the NK Cell Isolation Kit (Miltenyi Biotec). NK cells, e.g., TSNK cells, can also be isolated or enriched by removal of cells other than NK cells in a population of cells that comprise the NK cells, e.g., TSNK cells. For example, NK cells, e.g., TSNK cells, may be isolated or enriched by depletion of cells displaying non-NK cell markers using, *e.g.,* antibodies to one or more of CD3, CD4, CD14, CD19, CD20, CD36, CD66b, CD123, HLA DR and/or CD235a (glycophorin A). Negative isolation can be carried out using a commercially available kit, *e.g.,* the NK Cell Negative Isolation Kit (Dynal Biotech). Cells isolated by these methods may be additionally sorted, *e.g.,* to separate CD16⁺ and CD16⁻ cells.

Cell separation can be accomplished by, *e.g.,* flow cytometry, fluorescence-activated cell sorting (FACS), or, preferably, magnetic cell sorting using microbeads conjugated with specific antibodies. The cells may be isolated, *e.g.,* using a magnetic activated cell sorting (MACS) technique, a method for separating particles based on their ability to bind magnetic beads (*e.g.,* about 0.5-100 µm diameter) that comprise one or more specific antibodies, *e.g.,* anti-CD56 antibodies. Magnetic cell separation can be performed and automated using, *e.g.,* an AUTOMACS™ Separator (Miltenyi). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

### 5.4. Placental Perfusate

NK cells, e.g., TSNK cells, and NK progenitor cell and NK cell populations produced according to the three-stage method described herein may be produced from hematopoietic cells, *e.g.,* hematopoietic stem or progenitors from any source, *e.g.,* placental tissue, placental perfusate, umbilical cord blood, placental blood, peripheral blood, spleen, liver, or the like. In certain embodiments, the hematopoietic stem cells are combined hematopoietic stem cells from placental perfusate and from cord blood from the same placenta used to generate the placental perfusate. Placental perfusate comprising placental perfusate cells that can be obtained, for example, by the methods disclosed in U.S. Patent Nos. 7,045,148 and 7,468,276, the disclosures of which are hereby incorporated in their entireties.

### 5.4.1. Cell Collection Composition

The placental perfusate and perfusate cells, from which hematopoietic stem or progenitors may be isolated, or useful in tumor suppression or the treatment of an individual having tumor cells, cancer or a viral infection, *e.g.,* in combination with the NK cells, e.g., TSNK cells, or NK progenitor cell or NK cell populations produced according to the three-stage method provided herein, can be collected by perfusion of a mammalian, *e.g.,* human post-partum placenta using a placental cell collection composition. Perfusate can be collected from the placenta by perfusion of the placenta with any physiologically-acceptable solution, *e.g.,* a saline solution, culture medium, or a more complex cell collection composition. A cell collection composition suitable for perfusing a placenta, and for the collection and preservation of perfusate cells is described in detail in related U.S. Application Publication No. 2007/0190042, which is incorporated herein by reference in its entirety.

The cell collection composition can comprise any physiologically-acceptable solution suitable for the collection and/or culture of stem cells, for example, a saline solution (*e.g.,* phosphate-buffered saline, Kreb's solution, modified Kreb's solution, Eagle's solution, 0.9% NaCl. *etc*.), a culture medium (*e.g.,* DMEM, H.DMEM, *etc*.), and the like.

The cell collection composition can comprise one or more components that tend to preserve placental cells, that is, prevent the placental cells from dying, or delay the death of the placental cells, reduce the number of placental cells in a population of cells that die, or the like, from the time of collection to the time of culturing. Such components can be, *e.g.,* an apoptosis inhibitor (*e.g.,* a caspase inhibitor or JNK inhibitor); a vasodilator (*e.g.,* magnesium sulfate, an antihypertensive drug, atrial natriuretic peptide (ANP), adrenocorticotropin, corticotropin-releasing hormone, sodium nitroprusside, hydralazine, adenosine triphosphate, adenosine, indomethacin or magnesium sulfate, a phosphodiesterase inhibitor, *etc*.); a necrosis inhibitor (*e.g.,* 2-(1H-Indol-3-yl)-3-pentylamino-maleimide, pyrrolidine dithiocarbamate, or clonazepam); a TNF-α inhibitor; and/or an oxygen-carrying perfluorocarbon (*e.g.,* perfluorooctyl bromide, perfluorodecyl bromide, *etc*.).

The cell collection composition can comprise one or more tissue-degrading enzymes, *e.g.,* a metalloprotease, a serine protease, a neutral protease, a hyaluronidase, an RNase, or a DNase, or the like. Such enzymes include, but are not limited to, collagenases (*e.g.,* collagenase I, II, III or IV, a collagenase from *Clostridium histolyticum, etc*.); dispase, thermolysin, elastase, trypsin, LIBERASE, hyaluronidase, and the like.

The cell collection composition can comprise a bacteriocidally or bacteriostatically effective amount of an antibiotic. In certain non-limiting embodiments, the antibiotic is a macrolide (*e.g.,* tobramycin), a cephalosporin (*e.g.,* cephalexin, cephradine, cefuroxime, cefprozil, cefaclor, cefixime or cefadroxil), a clarithromycin, an erythromycin, a penicillin (*e.g.,* penicillin V) or a quinolone (*e.g.,* ofloxacin, ciprofloxacin or norfloxacin), a tetracycline, a streptomycin, *etc.* In a particular embodiment, the antibiotic is active against Gram(+) and/or Gram(-) bacteria, *e.g., Pseudomonas aeruginosa, Staphylococcus aureus,* and the like.

The cell collection composition can also comprise one or more of the following compounds: adenosine (about 1 mM to about 50 mM); D-glucose (about 20 mM to about 100 mM); magnesium ions (about 1 mM to about 50 mM); a macromolecule of molecular weight greater than 20,000 daltons, in one embodiment, present in an amount sufficient to maintain endothelial integrity and cellular viability (*e.g.,* a synthetic or naturally occurring colloid, a polysaccharide such as dextran or a polyethylene glycol present at about 25 g/l to about 100 g/l, or about 40 g/l to about 60 g/l); an antioxidant (*e.g.,* butylated hydroxyanisole, butylated hydroxytoluene, glutathione, vitamin C or vitamin E present at about 25 µM to about 100 µM); a reducing agent (*e.g.,* N-acetylcysteine present at about 0.1 mM to about 5 mM); an agent that prevents calcium entry into cells (*e.g.,* verapamil present at about 2 µM to about 25 µM); nitroglycerin (*e.g.,* about 0.05 g/L to about 0.2 g/L); an anticoagulant, in one embodiment, present in an amount sufficient to help prevent clotting of residual blood (*e.g.,* heparin or hirudin present at a concentration of about 1000 units/l to about 100,000 units/l); or an amiloride containing compound (*e.g.,* amiloride, ethyl isopropyl amiloride, hexamethylene amiloride, dimethyl amiloride or isobutyl amiloride present at about 1.0 µM to about 5 µM).

### 5.4.2. Collection and Handling of Placenta

Generally, a human placenta is recovered shortly after its expulsion after birth. In a preferred embodiment, the placenta is recovered from a patient after informed consent and after a complete medical history of the patient is taken and is associated with the placenta. Preferably, the medical history continues after delivery.

Prior to recovery of perfusate, the umbilical cord blood and placental blood are removed. In certain embodiments, after delivery, the cord blood in the placenta is recovered. The placenta can be subjected to a conventional cord blood recovery process. Typically a needle or cannula is used, with the aid of gravity, to exsanguinate the placenta (*see, e.g.,* Anderson, U.S. Patent No. 5,372,581; Hessel et al., U.S. Patent No. 5,415,665). The needle or cannula is usually placed in the umbilical vein and the placenta can be gently massaged to aid in draining cord blood from the placenta. Such cord blood recovery may be performed commercially, *e.g.,* LifeBank Inc., Cedar Knolls, N.J., ViaCord, Cord Blood Registry and CryoCell. Preferably, the placenta is gravity drained without further manipulation so as to minimize tissue disruption during cord blood recovery.

Typically, a placenta is transported from the delivery or birthing room to another location, *e.g.,* a laboratory, for recovery of cord blood and collection of perfusate. The placenta is preferably transported in a sterile, thermally insulated transport device (maintaining the temperature of the placenta between 20-28 °C), for example, by placing the placenta, with clamped proximal umbilical cord, in a sterile zip-lock plastic bag, which is then placed in an insulated container. In another embodiment, the placenta is transported in a cord blood collection kit substantially as described in U.S. Patent No. 7,147,626. Preferably, the placenta is delivered to the laboratory four to twenty-four hours following delivery. In certain embodiments, the proximal umbilical cord is clamped, preferably within 4-5 cm (centimeter) of the insertion into the placental disc prior to cord blood recovery. In other embodiments, the proximal umbilical cord is clamped after cord blood recovery but prior to further processing of the placenta.

The placenta, prior to collection of the perfusate, can be stored under sterile conditions and at either room temperature or at a temperature of 5 to 25 °C (centigrade). The placenta may be stored for a period of longer than forty eight hours, and preferably for a period of four to twenty-four hours prior to perfusing the placenta to remove any residual cord blood. The placenta is preferably stored in an anticoagulant solution at a temperature of 5 °C to 25 °C (centigrade). Suitable anticoagulant solutions are well known in the art. For example, a solution of heparin or warfarin sodium can be used. In a preferred embodiment, the anticoagulant solution comprises a solution of heparin (*e.g.,* 1% w/w in 1:1000 solution). The exsanguinated placenta is preferably stored for no more than 36 hours before placental perfusate is collected.

### 5.4.3. Placental Perfusion

Methods of perfusing mammalian placentae and obtaining placental perfusate are disclosed, *e.g.,* in Hariri, U.S. Patent Nos. 7,045,148 and 7,255,879, and in U.S. Application Publication Nos. 2007/0190042 and 20070275362, issued as U.S. Pat No. 8,057,788, the disclosures of which are hereby incorporated by reference herein in their entireties.

Perfusate can be obtained by passage of perfusion solution, *e.g.,* saline solution, culture medium or cell collection compositions described above, through the placental vasculature. In one embodiment, a mammalian placenta is perfused by passage of perfusion solution through either or both of the umbilical artery and umbilical vein. The flow of perfusion solution through the placenta may be accomplished using, *e.g.,* gravity flow into the placenta. Preferably, the perfusion solution is forced through the placenta using a pump, *e.g.,* a peristaltic pump. The umbilical vein can be, *e.g.,* cannulated with a cannula, *e.g.,* a TEFLON® or plastic cannula, that is connected to a sterile connection apparatus, such as sterile tubing. The sterile connection apparatus is connected to a perfusion manifold.

In preparation for perfusion, the placenta is preferably oriented in such a manner that the umbilical artery and umbilical vein are located at the highest point of the placenta. The placenta can be perfused by passage of a perfusion solution through the placental vasculature, or through the placental vasculature and surrounding tissue. In one embodiment, the umbilical artery and the umbilical vein are connected simultaneously to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. In another embodiment, the perfusion solution is passed through the umbilical veins and collected from the umbilical artery, or is passed through the umbilical artery and collected from the umbilical veins, that is, is passed through only the placental vasculature (fetal tissue).

In one embodiment, for example, the umbilical artery and the umbilical vein are connected simultaneously, *e.g.,* to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. Placental cells that are collected by this method, which can be referred to as a "pan" method, are typically a mixture of fetal and maternal cells.

In another embodiment, the perfusion solution is passed through the umbilical veins and collected from the umbilical artery, or is passed through the umbilical artery and collected from the umbilical veins. Placental cells collected by this method, which can be referred to as a "closed circuit" method, are typically almost exclusively fetal.

The closed circuit perfusion method can, in one embodiment, be performed as follows. A post-partum placenta is obtained within about 48 hours after birth. The umbilical cord is clamped and cut above the clamp. The umbilical cord can be discarded, or can processed to recover, *e.g.,* umbilical cord stem cells, and/or to process the umbilical cord membrane for the production of a biomaterial. The amniotic membrane can be retained during perfusion, or can be separated from the chorion, *e.g.,* using blunt dissection with the fingers. If the amniotic membrane is separated from the chorion prior to perfusion, it can be, *e.g.,* discarded, or processed, *e.g.,* to obtain stem cells by enzymatic digestion, or to produce, *e.g.,* an amniotic membrane biomaterial, *e.g.,* the biomaterial described in U.S. Application Publication No. 2004/0048796. After cleaning the placenta of all visible blood clots and residual blood, *e.g.,* using sterile gauze, the umbilical cord vessels are exposed, *e.g.,* by partially cutting the umbilical cord membrane to expose a cross-section of the cord. The vessels are identified, and opened, *e.g.,* by advancing a closed alligator clamp through the cut end of each vessel. The apparatus, *e.g.,* plastic tubing connected to a perfusion device or peristaltic pump, is then inserted into each of the placental arteries. The pump can be any pump suitable for the purpose, *e.g.,* a peristaltic pump. Plastic tubing, connected to a sterile collection reservoir, *e.g.,* a blood bag such as a 250 mL collection bag, is then inserted into the placental vein. Alternatively, the tubing connected to the pump is inserted into the placental vein, and tubes to a collection reservoir(s) are inserted into one or both of the placental arteries. The placenta is then perfused with a volume of perfusion solution, *e.g.,* about 750 ml of perfusion solution. Cells in the perfusate are then collected, *e.g.,* by centrifugation.

In one embodiment, the proximal umbilical cord is clamped during perfusion, and more preferably, is clamped within 4-5 cm (centimeter) of the cord's insertion into the placental disc.

The first collection of perfusion fluid from a mammalian placenta during the exsanguination process is generally colored with residual red blood cells of the cord blood and/or placental blood. The perfusion fluid becomes more colorless as perfusion proceeds and the residual cord blood cells are washed out of the placenta. Generally from 30 to 100 mL of perfusion fluid is adequate to initially flush blood from the placenta, but more or less perfusion fluid may be used depending on the observed results.

The volume of perfusion liquid used to perfuse the placenta may vary depending upon the number of placental cells to be collected, the size of the placenta, the number of collections to be made from a single placenta, *etc.* In various embodiments, the volume of perfusion liquid may be from 50 mL to 5000 mL, 50 mL to 4000 mL, 50 mL to 3000 mL, 100 mL to 2000 mL, 250 mL to 2000 mL, 500 mL to 2000 mL, or 750 mL to 2000 mL. Typically, the placenta is perfused with 700-800 mL of perfusion liquid following exsanguination.

The placenta can be perfused a plurality of times over the course of several hours or several days. Where the placenta is to be perfused a plurality of times, it may be maintained or cultured under aseptic conditions in a container or other suitable vessel, and perfused with a cell collection composition, or a standard perfusion solution (*e.g.,* a normal saline solution such as phosphate buffered saline ("PBS") with or without an anticoagulant (*e.g.,* heparin, warfarin sodium, coumarin, bishydroxycoumarin), and/or with or without an antimicrobial agent (*e.g.,* β-mercaptoethanol (0.1 mM); antibiotics such as streptomycin (*e.g.,* at 40-100 µg/ml), penicillin (*e.g.,* at 40 U/ml), amphotericin B (*e.g.,* at 0.5 µg/ml). In one embodiment, an isolated placenta is maintained or cultured for a period of time without collecting the perfusate, such that the placenta is maintained or cultured for 1, 2, 3, 4, 5, 6, 7, 8, 9, 10, 11, 12, 13, 14, 15, 16, 17, 18, 19, 20, 21, 22, 23, or 24 hours, or 2 or 3 or more days before perfusion and collection of perfusate. The perfused placenta can be maintained for one or more additional time(s), *e.g.,* 1, 2, 3, 4, 5, 6, 7, 8, 9, 10, 11, 12, 13, 14, 15, 16, 17, 18, 19, 20, 21, 22, 23, 24 or more hours, and perfused a second time with, *e.g.,* 700-800 mL perfusion fluid. The placenta can be perfused 1, 2, 3, 4, 5 or more times, for example, once every 1, 2, 3, 4, 5 or 6 hours. In a preferred embodiment, perfusion of the placenta and collection of perfusion solution, *e.g.,* placental cell collection composition, is repeated until the number of recovered nucleated cells falls below 100 cells/ml. The perfusates at different time points can be further processed individually to recover time-dependent populations of cells, *e.g.,* total nucleated cells. Perfusates from different time points can also be pooled.

### 5.4.4. Placental Perfusate and Placental Perfusate Cells

Typically, placental perfusate from a single placental perfusion comprises about 100 million to about 500 million nucleated cells, including hematopoietic cells from which NK cells, e.g., TSNK cells, or NK progenitor cells or NK cells produced according to the three-stage method described herein, may be produced by the method disclosed herein. In certain embodiments, the placental perfusate or perfusate cells comprise CD34⁺ cells, *e.g.,* hematopoietic stem or progenitor cells. Such cells can, in a more specific embodiment, comprise CD34⁺CD45⁻ stem or progenitor cells, CD34⁺CD45⁺ stem or progenitor cells, or the like. In certain embodiments, the perfusate or perfusate cells are cryopreserved prior to isolation of hematopoietic cells therefrom. In certain other embodiments, the placental perfusate comprises, or the perfusate cells comprise, only fetal cells, or a combination of fetal cells and maternal cells.

### 5.5. NK and NK Progenitor Cells

### 5.5.1. TSNK Cells

In one aspect, provided herein are TSNK cells, which are NK cells produced by a two-step method described herein (see, *e.g.,* Section 5.2.1). Further provided herein is a population of cells comprising the TSNK cells produced by the methods described herein (*e.g.,* two-step method). In a specific embodiment, said NK cells (*e.g.,* TSNK cells) are CD3⁻CD56⁺. In a specific embodiment, said NK cells (*e.g.,* TSNK cells) are CD3⁻CD56⁺CD16⁻. In another specific embodiment, said NK cells (*e.g.,* TSNK cells) are additionally CD94⁺CD117⁺. In another specific embodiment, said NK cells (*e.g.,* TSNK cells) are additionally CD161⁻. In another specific embodiment, said NK cells (*e.g.,* TSNK cells) are additionally NKG2D⁺. In another specific embodiment, said NK cells are additionally NKp46⁺. In another specific embodiment, said NK cells are additionally CD226⁺.

In certain embodiments, greater than 50%, 60%, 70%, 80%, 90%, 92%, 94%, 96%, 98% of said TSNK cells are CD56⁺ and CD16⁻. In other embodiments, at least 50%, 60%, 70%, 80%, 82%, 84%, 86%, 88% or 90% of said TSNK cells are CD3⁻ and CD56⁺. In other embodiments, at least 50%, 52%, 54%, 56%, 58% or 60% of said TSNK cells are NKG2D⁺. In other embodiments, fewer than 30%, 20%, 10%, 9%, 8%, 7%, 6%, 5%, 4% or 3% of said cells are NKB1⁺. In certain other embodiments, fewer than 30%, 20%, 10%, 8%, 6%, 4% or 2% of said TSNK cells are NKAT2⁺. In certain other embodiments, fewer than 30%, 20%, 10%, 8%, 6%, 4% or 2% of said TSNK cells are CD56⁺ and CD16⁺. In more specific embodiments, at least 10%, 20%, 25%, 30%, 35%, 40%, 50%, 55%, 60%, 65% or 70% of said CD3⁻, CD56⁺ TSNK cells are NKp46⁺. In other more specific embodiments, at least 10%, 20%, 25%, 30%, 35%, 40%, 50%, 55%, 60%, 65%, 70%, 75%, 80% or 85% of said CD3⁻, CD56⁺ TSNK cells are CD117⁺. In other more specific embodiments, at least 10%, 20%, 25%, 30%, 35%, 40%, 45% or 50% of said CD3⁻, CD56⁺ TSNK cells are CD94+. In other more specific embodiments, at least 10%, 20%, 25%, 30%, 35%, 40%, 45% or 50% of said CD3⁻, CD56⁺ TSNK cells are CD161⁻. In other more specific embodiments, at least 10%, 12%, 14%, 16%, 18% or 20% of said CD3⁻, CD56⁺ TSNK cells are CD226⁺. In more specific embodiments, at least 20%, 25%, 30%, 35% or 40% of said CD3⁻, CD56⁺ TSNK cells are CD7⁺. In more specific embodiments, at least 30%, 35%, 40%, 45%, 50%, 55% or 60% of said CD3⁻, CD56⁺ TSNK cells are CD5⁺.

In particular embodiments, a TSNK cell population comprises a greater proportion of CD56- cells than CD56+ cells. In particular embodiments, a TSNK cell population differentiates in vivo or ex vivo into a population with an increased proportion of CD56+ cells.

In one embodiment, a TSNK cell population comprises cells which are CD117+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% CD117⁺ cells. In one embodiment, a TSNK cell population comprises cells which are NKG2D+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% NKG2D⁺ cells. In one embodiment, a TSNK cell population comprises cells which are NKp44+. In a specific embodiment, said TSNK cell population comprise no more than about 5%, 10%, 15%, 20%, 25%, 30%, 35%, 40%, 45%, 50%, 55%, 60%, 65%, 70%, 75%, 80%, 85%, or 90% NKp44⁺ cells. In one embodiment, a TSNK cell population comprises cells which are CD52+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% CD52⁺ cells. In a particular embodiment, a TSNK cell population comprises cells which are CD52+ CD117⁺. In one embodiment, a TSNK cell population comprises cells which are CD244+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% CD244⁺ cells. In a particular embodiment, a TSNK cell population comprises cells which are CD244+ CD117⁺. In one embodiment, a TSNK cell population comprises cells which are LFA-1+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% LFA-1+ cells. In one embodiment, a TSNK cell population comprises cells which are CD94+. In a specific embodiment, said TSNK cell populations comprise no more than about 5%, 10%, 15%, 20%, 25%, 30%, 35%, 40%, 45%, 50%, 55%, 60%, 65%, 70%, 75%, 80%, 85%, or 90% CD94+ cells.

In various other embodiments, TSNK cells can be combined with, *e.g.,* NK cells, wherein said NK cells have been isolated from a tissue source and have not been expanded; NK cells isolated from a tissue source and expanded, or NK cells produced by a different method, *e.g.,* CD56⁺CD16⁺ natural killer cells, *e.g.,* in ratios of, for example, about 1:10, 2:9, 3:8, 4:7:, 5:6, 6:5, 7:4, 8:3, 9:2, 1:10, 1:9, 1:8, 1:7, 1:6, 1:5, 1:4, 1:3, 1:2, 1:1, 2:1, 3:1, 4:1, 5:1, 6:1, 7:1, 8:1 or about 9:1. As used in this context, "isolated" means that the cells have been removed from their normal tissue environment.

TSNK cells can have a fetal genotype or a maternal genotype. For example, because the post-partum placenta, as a source of hematopoietic cells suitable for producing TSNK cells, comprises tissue and cells from the fetus and from the mother, placental perfusate can comprise fetal cells only, or a substantial majority of fetal cells (*e.g.,* greater than about 90%, 95%, 98% or 99%), or can comprise a mixture of fetal and maternal cells (*e.g.,* the fetal cells comprise less than about 90%, 80%, 70%, 60%, or 50% of the total nucleated cells of the perfusate). In one embodiment, the TSNK cells are derived only from fetal placental hematopoietic cells, *e.g.,* cells obtained from closed-circuit perfusion of the placenta wherein the perfusion produces perfusate comprising a substantial majority, or only, fetal placental hematopoietic cells. In another embodiment, the TSNK cells are derived from fetal and maternal cells, *e.g.,* cells obtained by perfusion by the pan method (*see* above), wherein the perfusion produced perfusate comprising a mix of fetal and maternal placental cells. Thus, in one embodiment, provided herein is a population of placenta-derived intermediate natural killer cells, the substantial majority of which have the fetal genotype. In another embodiment, provided herein is a population of placenta-derived intermediate natural killer cells that comprise natural killer cells having the fetal genotype and natural killer cells having the maternal phenotype.

Also provided herein are populations of TSNK cells that comprise natural killer cells not produced by the methods described herein. For example, in one embodiment, provided herein is a population of TSNK cells that also comprises natural killer cells isolated from, *e.g.,* umbilical cord blood, peripheral blood, bone marrow, or a combination of two or more of the foregoing, or NK cells expanded by a method other than the methods described herein. Such populations of TSNK cells can comprise the TSNK cells and other NK cells in, *e.g.,* a ratio of about 1:10, 2:9, 3:8, 4:7:, 5:6, 6:5, 7:4, 8:3, 9:2, 10:1, 1:9, 1:8, 1:7, 1:6, 1:5, 1:4, 1:3, 1:2, 1:1, 2:1, 3:1, 4:1, 5:1, 6:1, 7:1, 8:1, 9:1, 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, or about 1:100, or the like.

In certain embodiments, the isolated natural killer cells (*e.g.,* TSNK cells) or populations enriched for natural killer cells (*e.g.,* TSNK cells) can be assessed by detecting one or more functionally relevant markers, for example, CD94, CD161, NKp44, DNAM-1, 2B4, NKp46, CD94, KIR, and the NKG2 family of activating receptors (*e.g.,* NKG2D). In some embodiments, the purity of the isolated or enriched natural killer cells can be confirmed by detecting one or more of CD56, CD3 and CD16.

Optionally, the cytotoxic activity isolated or enriched natural killer cells can be assessed, *e.g.,* in a cytotoxicity assay using tumor cells, *e.g.,* cultured K562, LN-18, U937, WERI-RB-1, U-118MG, HT-29, HCC2218, KG-1, or U266 tumor cells, or the like as target cells.

### 5.5.2. NK Progenitor Cells

In one embodiment, provided herein is an isolated NK progenitor cell population, wherein said NK progenitor cells are produced according to the three-stage method described in Section 5.2.2 above.

In one embodiment, said isolated NK progenitor cell population comprises a low percentage of CD3-CD56+ cells as compared to the percentage of CD3-CD56+ cells associated with NK cell populations, such as NK cell populations produced by the three-stage methods described herein, e.g., the NK progenitor cell population comprises about 5%, 10%, 15%, 20%, 25%, 30%, 35%, 40%, 45%, or 50% CD3-CD56+ cells. In another specific embodiment, said NK progenitor cell population comprises no more than 5%, 10%, 15%, 20%, 25%, 30%, 35%, 40%, 45%, or 50% CD3-CD56+ cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% CD3-CD56+ cells. In some embodiments, said NK progenitor cell populations, e.g., a NK progenitor cell populations that comprise a low percentage of CD3-CD56+ cells as compared to the percentage of CD3-CD56+ cells associated with NK cell populations, comprise no more than 1%, no more than 2%, no more than 3%, no more than 4%, no more than 5%, no more than 10%, or no more than 15% CD3-CD56+ cells. In another specific embodiment, said NK progenitor cell populations produced by a three-stage method described herein are produced using a three-stage method that comprises a short third culture step, e.g., a third culture step of 4-6, 5-7, 6-8, or 7-9 days.

In certain embodiments, said CD3⁻CD56⁺ cells in said NK progenitor cell populations are additionally CD117⁺. In a specific embodiment, about 65%, 70%, 75%, 80%, 85%, 90%, 95%, 98%, or 99% of said CD3⁻CD56⁺ cells in said NK progenitor cell populations are CD117⁺. In another specific embodiment, no less than 65%, 70%, 75%, 80%, 85%, 90%, 95%, 98%, or 99% of said CD3⁻CD56⁺ cells in said NK progenitor cell populations are CD117⁺. In another specific embodiment, between 65%-70%, 70%-75%, 75%-80%, 80%-85%, 85%-90%, 90%-95%, or 95%-99% of said CD3⁻CD56⁺ cells in said NK progenitor cell populations are CD117⁺.

In certain embodiments, said CD3-CD56+ cells in said NK progenitor cell populations are additionally CD161+. In a specific embodiment, about 40%, 45%, 50%, 55%, 60%, 65%, 70%, or 75% of said CD3-CD56+ cells in said NK progenitor cell populations are CD161+. In another specific embodiment, no less than 40%, 45%, 50%, 55%, 60%, 65%, 70%, or 75% of said CD3-CD56+ cells in said NK progenitor cell populations are CD161+. In another specific embodiment, between 40%-45%, 45%-50%, 50%-55%, 55%-60%, 60%-65%, 65%-70%, or 70%-75% of said CD3-CD56+ cells in said NK progenitor cell populations are CD161+.

In certain embodiments, said CD3-CD56+ cells in said NK progenitor cell populations are additionally NKp46+. In a specific embodiment, about 25%, 30%, 35%, 40%, 45%, 50%, 55%, 60%, 65%, 70%, 75%, 80%, 85%, 90% or more of said CD3-CD56+ cells in said NK progenitor cell populations are NKp46+. In a more specific embodiment, about 25%, 30%, 35%, 40%, 45%, 50%, or 55% of said CD3-CD56+ cells in said NK progenitor cell populations are NKp46+. In another specific embodiment, no more than 25%, 30%, 35%, 40%, 45%, 50%, or 55% of said CD3-CD56+ cells in said NK progenitor cell populations are NKp46+. In another specific embodiment, between 25%-30%, 30%-35%, 35%-40%, 40%-45%, 45%-50%, 50%-55%, 55%-60%, 60%-65%, 65%-70%, 70%-75%, 75%-80%, 80%-85%, 85%-90% or more of said CD3-CD56+ cells in said NK progenitor cell populations are NKp46+. In a more specific embodiment, between 25%-30%, 30%-35%, 35%-40%, 40%-45%, 45%-50%, or 50%-55% of said CD3-CD56+ cells in said NK progenitor cell populations are NKp46+.

In certain embodiments, said NK progenitor cell population contains cells that are CD56⁺CD16⁻. In certain embodiments, CD3⁻CD56⁺ cells in said NK progenitor cell populations are CD16⁻. In certain embodiments, CD3⁻CD56⁺ cells in said NK progenitor cell populations are CD16⁺. In a specific embodiment, said NK progenitor cell populations comprise no more than 5%, 10%, 15%, 20%, 25%, 30%, 35%, 40%, 45%, or 50% CD16⁺ cells. In another specific embodiment, said NK progenitor cell populations comprise between 0%-5%, 5%-10%, 10%-15%, 15%-20%, or 20%-25% CD16⁺ cells. In some embodiments, said NK progenitor cell populations comprise no more than 1%, no more than 2%, no more than 3%, no more than 4%, no more than 5%, no more than 10%, or no more than 15% CD16⁺ cells.

In certain embodiments, said CD3-CD56+ cells in said NK progenitor cell populations are additionally CD16⁻. In certain embodiments, said CD3-CD56+ cells in said NK progenitor cell populations are additionally CD117⁺ and CD161⁺. In certain embodiments, said CD3-CD56+ cells in said NK progenitor cell populations are additionally CD16-, CD117+ and CD161+. In certain embodiments, said CD3-CD56+ cells in said NK progenitor cell populations are additionally CD16-, CD117+, CD161+, and NKp46+.

In one embodiment, an NK progenitor cell population produced by a three-stage method described herein comprises no more than about 40% CD3-CD56+ cells. In one embodiment, an NK progenitor cell population produced by a three-stage method described herein comprises cells which are CD117+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% CD117+ cells. In one embodiment, an NK progenitor cell population produced by a three-stage method described herein comprises cells which are CD52+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% CD52+ cells. In a particular embodiment, said NK progenitor cell population produced by a three-stage method described herein comprises cells which are CD52+ CD117+. In one embodiment, an NK progenitor cell population produced by a three-stage method described herein comprises cells which are CD244+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% CD244+ cells. In a particular embodiment, said NK progenitor cell population produced by a three-stage method described herein comprises cells which are CD244+ CD117⁺. In one embodiment, an NK progenitor cell population produced by a three-stage method described herein comprises cells which are LFA-1+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% LFA-1+ cells. In one embodiment, an NK progenitor cell population produced by a three-stage method described herein comprises cells which are CD94+. In a specific embodiment, said NK progenitor cell populations comprise no more than about 5%, 10%, 15%, 20%, 25%, 30%, 35%, 40%, 45%, 50%, 55%, 60%, 65%, 70%, 75%, 80%, 85%, or 90% CD94+ cells.

In particular embodiments, an NK progenitor cell population produced by a three-stage method described herein comprises a greater proportion of CD56- cells than CD56+ cells. In particular embodiments, an NK progenitor cell population produced by a three-stage method described herein differentiates in vivo or ex vivo into a population with an increased proportion of CD56+ cells.

In a specific embodiment, an NK progenitor cell population produced by a three-stage method described herein comprises a low percentage of CD34⁻CD117⁺ cells as compared to the percentage of CD34⁻CD117⁺ cells associated with an NK cell population, e.g., the NK progenitor cell population comprises about 5%, 10%, 15%, 20%, 25%, 30%, 35%, 40%, 45%, or 50% CD34⁻CD117⁺ cells. In another specific embodiment, said NK progenitor cell population comprises no more than 5%, 10%, 15%, 20%, 25%, 30%, 35%, 40%, 45%, or 50% CD34⁻CD117 ⁺ cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% CD34⁻CD117⁺ cells. In some embodiments, said NK progenitor cell population comprises no more than 1%, no more than 2%, no more than 3%, no more than 4%, no more than 5%, no more than 10%, or no more than 15% CD34⁻CD117⁺ cells. In another specific embodiment, said NK progenitor cell population produced by a three-stage method described herein is produced using a three-stage method that comprises a short third culture step, e.g., a third culture step of 4-6, 5-7, 6-8, or 7-9 days.

In a specific embodiment, an NK progenitor cell population produced by a three-stage method described herein comprises a low percentage of CD161+ cells as compared to the percentage of CD161⁺ cells associated with an NK cell population, e.g., the NK progenitor cell population comprises about 5%, 10%, 15%, 20%, 25%, 30%, 35%, 40%, 45%, or 50% CD161⁺ cells. In another specific embodiment, said NK progenitor cell population comprises no more than 5%, 10%, 15%, 20%, 25%, 30%, 35%, 40%, 45%, or 50% CD161⁺ cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% CD161⁺ cells. In some embodiments, said NK progenitor cell population comprises no more than 1%, no more than 2%, no more than 3%, no more than 4%, no more than 5%, no more than 10%, or no more than 15% CD161⁺ cells. In another specific embodiment, said NK progenitor cell population produced by a three-stage method described herein is produced using a three-stage method that comprises a short third culture step, e.g., a third culture step of 4-6, 5-7, 6-8, or 7-9 days.

In a specific embodiment, an NK progenitor cell population produced by a three-stage method described herein comprises a low percentage of NKp46⁺ cells as compared to the percentage of NKp46⁺ cells associated with an NK cell population, e.g., the NK progenitor cell population comprises about 1%, 5%, 10%, 15%, 20%, 25%, 30%, 35%, 40%, 45%, or 50% NKp46⁺ cells. In another specific embodiment, said NK progenitor cell population comprises no more than 1%, 5%, 10%, 15%, 20%, 25%, 30%, 35%, 40%, 45%, or 50% NKp46⁺ cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% NKp46⁺ cells. In some embodiments, said NK progenitor cell population comprises no more than 1%, no more than 2%, no more than 3%, no more than 4%, no more than 5%, no more than 10%, or no more than 15% NKp46⁺ cells. In another specific embodiment, said NK progenitor cell population produced by a three-stage method described herein is produced using a three-stage method that comprises a short third culture step, e.g., a third culture step of 4-6, 5-7, 6-8, or 7-9 days.

In a specific embodiment, an NK progenitor cell population produced by a three-stage method described herein comprises a low percentage of CD56⁺CD16- cells as compared to the percentage of CD56⁺CD16- cells associated with an NK cell population, e.g., the NK progenitor cell population comprises about 1%, 5%, 10%, 15%, 20%, 25%, 30%, 35%, 40%, 45%, or 50% CD56⁺CD16- cells. In another specific embodiment, said NK progenitor cell population comprises no more than 1%, 5%, 10%, 15%, 20%, 25%, 30%, 35%, 40%, 45%, or 50% CD56⁺CD16- cells. In another specific embodiment, said NK progenitor cell population comprises between 0%-5%, 5%-10%, 10%-15%, 15%-20%, 20%-25%, 25%-30%, 30%-35%, 35%-40%, 40%-45%, or 45%-50% CD56⁺CD16- cells. In some embodiments, said NK progenitor cell population comprises no more than 1%, no more than 2%, no more than 3%, no more than 4%, no more than 5%, no more than 10%, or no more than 15% CD56⁺CD16- cells. In another specific embodiment, said NK progenitor cell population produced by a three-stage method described herein is produced using a three-stage method that comprises a short third culture step, e.g., a third culture step of 4-6, 5-7, 6-8, or 7-9 days.

In one embodiment, an NK progenitor cell population produced by a three-stage method described herein comprises cells that are CD52+CD117+. In a specific embodiment, an NK progenitor cell population produced by a three-stage method described herein comprises a higher percentage of CD52⁺CD117+ cells as compared to the percentage of CD52⁺CD117⁺ cells associated with a hematopoietic progenitor cell population. In a specific embodiment, an NK progenitor cell population produced by a three-stage method described herein comprises a higher percentage of CD52⁺CD117+ cells as compared to the percentage of CD52⁺CD117+ cells associated with an NK cell population, e.g., the NK progenitor cell population comprises about 50%, 55%, 60%, 65%, 70%, 75%, 80%, 85%, 90% or more CD52⁺CD117+ cells. In another specific embodiment, said NK progenitor cell population comprises no less than 50%, 55%, 60%, 65%, 70%, 75%, 80%, 85%, or 90% CD52⁺CD117+ cells. In another specific embodiment, said NK progenitor cell population comprises between 50%-55%, 55%-60%, 60%-65%, 65%-70%, 70%-75%, 75%-80%, 80%-85%, 85%-90%, 90%-95% or more CD52⁺CD117+ cells. In another specific embodiment, said NK progenitor cell population which comprises CD52⁺CD117+ cells produced by a three-stage method described herein is produced using a three-stage method that comprises a short third culture step, e.g., a third culture step of 4-6, 5-7, 6-8, or 7-9 days. In a specific embodiment, said NK progenitor cell population which comprises CD52⁺CD117+ cells is produced using a three-stage method that comprises a total of 12 days or more, 13 days or more, 14 days or more, 15 days or more, 16 days or more, 17 days or more, 18 days or more, 19 days or more, 20 days or more, or 21 days or more of culture. In a specific embodiment, said NK progenitor cell population which comprises CD52⁺CD117+ cells is produced using a three-stage method that comprises a total of at least 12 days, 13 days, or 14 days of culture but not more than 21-25 days, 25-30 days, or 30-35 days of culture. In a specific embodiment, said NK progenitor cell population which comprises CD52⁺CD117+ cells is produced using a three-stage method that comprises a total of 21 days of culture.

In a specific embodiment, the NK progenitor cells described herein possess a greater ability to engraft bone marrow (e.g., in vivo) than NK cells, e.g., NK cells produced using a comparable method. For example, in certain embodiments, NK progenitor cells produced using a three-stage method that comprises a short third culture step, e.g., a third culture step of 4-6, 5-7, 6-8, or 7-9 days engraft bone marrow (e.g., in vivo) at a higher efficiency than NK cells produced using a three-stage method that comprises a longer third culture step, e.g., a third culture step of 18-20, 19-21, 20-22, or 21-23 days. In another embodiment, the NK progenitor cells described herein possess longer telomeres than peripheral blood (PB) derived NK cells.

### 5.5.3. NK Cells Produced by Three-Step Method

In another embodiment, provided herein is an isolated NK cell population, wherein said NK cells are produced according to the method described in Section 5.2.2 above.

In one embodiment, provided herein is an isolated NK cell population produced by a three-stage method described herein, wherein said NK cell population comprises a greater percentage of CD3-CD56+ cells than an NK progenitor cell population produced by a three-stage method described herein, e.g., an NK progenitor cell population produced by the same three-stage method with the exception that the third culture step used to produce the NK progenitor cell population was of shorter duration than the third culture step used to produce the NK cell population. In a specific embodiment, said NK cell population comprises about 65%, 70%, 75%, 80%, 85%, 90%, 95%, 98%, or 99% CD3-CD56+ cells. In another specific embodiment, said NK cell population comprises no less than 65%, 70%, 75%, 80%, 85%, 90%, 95%, 98%, or 99% CD3-CD56+ cells. In another specific embodiment, said NK cell population comprises between 65%-70%, 70%-75%, 75%-80%, 80%-85%, 85%-90%, 90%-95%, or 95%-99% CD3-CD56+ cells. In another specific embodiment, said NK cell population produced by a three-stage method described herein is produced using a three-stage method that comprises a long third culture step, e.g., a third culture step of 18-20, 19-21, 20-22, or 21-23 days.

In certain embodiments, said CD3⁻CD56⁺ cells in said NK cell population comprises CD3⁻CD56⁺ cells that are additionally CD117⁺, wherein said NK cell population comprises a lesser percentage of CD3⁻CD56⁺CD117⁺ cells than an NK progenitor cell population produced by a three-stage method described herein, e.g., an NK progenitor cell population produced by the same three-stage method with the exception that the third culture step used to produce the NK progenitor cell population was of shorter duration than the third culture step used to produce the NK cell population.

In certain embodiments, said CD3⁻CD56⁺ cells in said NK cell population comprises CD3⁻CD56⁺ cells that are additionally CD161⁺, wherein said NK cell population comprises a lesser percentage of CD3⁻CD56⁺CD161⁺ cells than an NK progenitor cell population produced by a three-stage method described herein, e.g., an NK progenitor cell population produced by the same three-stage method with the exception that the third culture step used to produce the NK progenitor cell population was of shorter duration than the third culture step used to produce the NK cell population.

In certain embodiments, said CD3⁻CD56⁺ cells in said NK cell population comprises CD3⁻CD56⁺ cells that are additionally NKp46⁺, wherein said NK cell population comprises a greater percentage of CD3⁻CD56⁺NKp46⁺ cells than an NK progenitor cell population produced by a three-stage method described herein, e.g., an NK progenitor cell population produced by the same three-stage method with the exception that the third culture step used to produce the NK progenitor cell population was of shorter duration than the third culture step used to produce the NK cell population.

In certain embodiments, said CD3⁻CD56⁺ cells in said NK cell population comprises CD3⁻CD56⁺ cells that are additionally CD16-, wherein said NK cell population comprises a greater percentage of CD3⁻CD56⁺CD16- cells than an NK progenitor cell population produced by a three-stage method described herein, e.g., an NK progenitor cell population produced by the same three-stage method with the exception that the third culture step used to produce the NK progenitor cell population was of shorter duration than the third culture step used to produce the NK cell population. In another embodiment, the NK cells produced using the three-stage method described herein possess longer telomeres than peripheral blood (PB) derived NK cells.

In one embodiment, an NK cell population produced by a three-stage method described herein comprises cells which are CD117+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% CD117⁺ cells. In one embodiment, an NK cell population produced by a three-stage method described herein comprises cells which are NKG2D+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% NKG2D⁺ cells. In one embodiment, an NK cell population produced by a three-stage method described herein comprises cells which are NKp44+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% NKp44⁺ cells. In one embodiment, an NK cell population produced by a three-stage method described herein comprises cells which are CD52+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% CD52⁺ cells. In a particular embodiment, said NK cell population produced by a three-stage method described herein comprises cells which are CD52+ CD117+. In one embodiment, an NK cell population produced by a three-stage method described herein comprises cells which are CD244+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% CD244⁺ cells. In a particular embodiment, said NK cell population produced by a three-stage method described herein comprises cells which are CD244+ CD117+. In one embodiment, an NK cell population produced by a three-stage method described herein comprises cells which are LFA-1+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% LFA-1+ cells. In one embodiment, an NK cell population produced by a three-stage method described herein comprises cells which are CD94+. In a specific embodiment, said NK cell populations comprise no more than about 5%, 10%, 15%, 20%, 25%, 30%, 35%, 40%, 45%, 50%, 55%, 60%, 65%, 70%, 75%, 80%, 85%, or 90% CD94+ cells.

### 5.6. NK and NK Progenitor Cells In Combination With Placental Perfusate

Further provided herein are compositions comprising NK cells produced using the methods described herein, e.g., TSNK cells, NK cell populations, or NK progenitor cell populations produced according to the three-stage method described herein, in combination with placental perfusate, placental perfusate cells and/or adherent placental cells, *e.g.,* for use in suppressing the proliferation of a tumor cell or plurality of tumor cells.

### 5.6.1. Combinations of NK Cells and Perfusate or Perfusate Cells

Further provided herein are compositions comprising combinations of NK cells produced using the methods described herein, e.g., TSNK cells, or NK cell populations, or NK progenitor cell populations produced according to the three-stage method described herein, and placental perfusate and/or placental perfusate cells. In one embodiment, for example, provided herein is a volume of placental perfusate supplemented with NK cells produced using the methods described herein, e.g., TSNK cells, or NK cell populations, or NK progenitor cell populations. In specific embodiments, for example, each milliliter of placental perfusate is supplemented with about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more NK cells produced using the methods described herein, e.g., TSNK cells, or NK cell populations, or NK progenitor cell populations. In another embodiment, placental perfusate cells are supplemented with NK cells produced using the methods described herein, e.g., TSNK cells, or NK cell populations, or NK progenitor cell populations. In certain other embodiments, when placental perfusate cells are combined with NK cells produced using the methods described herein, e.g., TSNK cells, NK cell populations, or NK progenitor cell populations, the placental perfusate cells generally comprise about, greater than about, or fewer than about, 50%, 45%, 40%, 35%, 30%, 25%, 20%, 15%, 10%, 8%, 6%, 4%, 2% or 1% of the total number of cells. In certain other embodiments, when NK cells produced using the methods described herein, e.g., TSNK cells, or NK cell populations, or NK progenitor cell populations are combined with a plurality of placental perfusate cells and/or combined natural killer cells, the NK cells or NK cell populations, or NK progenitor cell populations generally comprise about, greater than about, or fewer than about, 50%, 45%, 40%, 35%, 30%, 25%, 20%, 15%, 10%, 8%, 6%, 4%, 2% or 1% of the total number of cells. In certain other embodiments, when NK cells produced using the methods described herein, e.g., TSNK cells, or NK cell populations, or NK progenitor cell populations are used to supplement placental perfusate, the volume of solution (*e.g.,* saline solution, culture medium or the like) in which the cells are suspended comprises about, greater than about, or less than about, 50%, 45%, 40%, 35%, 30%, 25%, 20%, 15%, 10%, 8%, 6%, 4%, 2% or 1% of the total volume of perfusate plus cells, where the NK cells or NK progenitor cells are suspended to about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more cells per milliliter prior to supplementation.

In other embodiments, any of the above combinations of cells is, in turn, combined with umbilical cord blood or nucleated cells from umbilical cord blood.

Further provided herein is pooled placental perfusate that is obtained from two or more sources, *e.g.,* two or more placentas, and combined, *e.g.,* pooled. Such pooled perfusate can comprise approximately equal volumes of perfusate from each source, or can comprise different volumes from each source. The relative volumes from each source can be randomly selected, or can be based upon, *e.g.,* a concentration or amount of one or more cellular factors, e.g., cytokines, growth factors, hormones, or the like; the number of placental cells in perfusate from each source; or other characteristics of the perfusate from each source. Perfusate from multiple perfusions of the same placenta can similarly be pooled.

Similarly, provided herein are placental perfusate cells, and placenta-derived intermediate natural killer cells, that are obtained from two or more sources, e.g., two or more placentas, and pooled. Such pooled cells can comprise approximately equal numbers of cells from the two or more sources, or different numbers of cells from one or more of the pooled sources. The relative numbers of cells from each source can be selected based on, *e.g.,* the number of one or more specific cell types in the cells to be pooled, *e.g.,* the number of CD34⁺ cells, *etc.*

Further provided herein are NK cells produced using the methods described herein, e.g., TSNK cells, or NK cell populations, or NK progenitor cell populations, and combinations of such cells with placental perfusate and/or placental perfusate cells, that have been assayed to determine the degree or amount of tumor suppression (that is, the potency) to be expected from, *e.g.,* a given number of NK cells or NK cell populations, or NK progenitor cell populations, or a given volume of perfusate. For example, an aliquot or sample number of cells is contacted or brought into proximity with a known number of tumor cells under conditions in which the tumor cells would otherwise proliferate, and the rate of proliferation of the tumor cells in the presence of placental perfusate, perfusate cells, placental natural killer cells, or combinations thereof, over time (*e.g.,* 1, 2, 3, 4, 5, 6, 7, 8, 9, or 10 weeks, or longer) is compared to the proliferation of an equivalent number of the tumor cells in the absence of perfusate, perfusate cells, placental natural killer cells, or combinations thereof. The potency of the cells can be expressed, *e.g.,* as the number of cells or volume of solution required to suppress tumor cell growth, *e.g.,* by about 10%, 15%, 20%, 25%, 30%, 35%, 40%, 45%, 50%, or the like.

In certain embodiments, NK cells produced using the methods described herein, e.g., TSNK cells, or NK cell populations, or NK progenitor cell populations are provided as pharmaceutical grade administrable units. Such units can be provided in discrete volumes, *e.g.,* 15 mL, 20 mL, 25 mL, 30 nL. 35 mL, 40 mL, 45 mL, 50 mL, 55 mL, 60 mL, 65 mL, 70 mL, 75 mL, 80 mL, 85 mL, 90 mL, 95 mL, 100 mL, 150 mL, 200 mL, 250 mL, 300 mL, 350 mL, 400 mL, 450 mL, 500 mL, or the like. Such units can be provided so as to contain a specified number of cells, *e.g.,* NK cells or NK cell populations, or NK progenitor cell populations in combination with other NK cells or perfusate cells, *e.g.,* 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more cells per unit. In specific embodiments, the units can comprise about, at least about, or at most about 1 x 10⁴, 5 x 10⁴, 1 x 10⁵, 5 x 10⁵, 1 x 10⁶, 5 x 10⁶ or more NK cells or NK progenitor cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more cells per unit. Such units can be provided to contain specified numbers of NK cells or NK cell populations, or NK progenitor cell populations, and/or any of the other cells.

In the above embodiments, the NK cells or NK cell populations, or NK progenitor cell populations or combinations of NK cells or NK cell populations, or NK progenitor cell populations with other NK cells, perfusate cells or perfusate can be autologous to a recipient (that is, obtained from the recipient), or allogeneic to a recipient (that is, obtained from at last one other individual from said recipient).

In certain embodiments, each unit of cells is labeled to specify one or more of volume, number of cells, type of cells, whether the unit has been enriched for a particular type of cell, and/or potency of a given number of cells in the unit, or a given number of milliliters of the unit, that is, whether the cells in the unit cause a measurable suppression of proliferation of a particular type or types of tumor cell.

### 5.6.2. Combinations of NK Cells or NK Progenitor Cells With Adherent Placental Stem Cells

In other embodiments, the NK cells produced using the methods described herein, e.g., TSNK cells, or NK cell populations or NK progenitor cell populations produced using the three-stage method described herein, either alone or in combination with placental perfusate or placental perfusate cells, are supplemented with isolated adherent placental cells, *e.g.,* placental stem cells and placental multipotent cells as described, *e.g.,* in Hariri U.S. Patent Nos. 7,045,148 and 7,255,879, and in U.S. Patent Application Publication No. 2007/0275362, the disclosures of which are incorporated herein by reference in their entireties. "Adherent placental cells" means that the cells are adherent to a tissue culture surface, *e.g.,* tissue culture plastic. The adherent placental cells useful in the compositions and methods disclosed herein are not trophoblasts, embryonic germ cells or embryonic stem cells. In certain embodiments, adherent placental stem cells are used as feeder cells during the processes (*e.g.,* two-step method) as described above.

The NK cells produced using the methods described herein, e.g., TSNK cells, or NK cell populations, or NK progenitor cell populations, either alone or in combination with placental perfusate or placental perfusate cells can be supplemented with, *e.g.,* 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more adherent placental cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more adherent placental cells. The adherent placental cells in the combinations can be, *e.g.,* adherent placental cells that have been cultured for, *e.g.,* 1, 2, 3, 4, 5, 6, 7, 8, 9, 10, 12, 14, 16, 18, 20, 22, 24, 26, 28, 30, 32, 34, 36, 38, or 40 population doublings, or more.

Isolated adherent placental cells, when cultured in primary cultures or expanded in cell culture, adhere to the tissue culture substrate, *e.g.,* tissue culture container surface (*e.g.,* tissue culture plastic). Adherent placental cells in culture assume a generally fibroblastoid, stellate appearance, with a number of cytoplasmic processes extending from the central cell body. Adherent placental cells are, however, morphologically distinguishable from fibroblasts cultured under the same conditions, as the adherent placental cells exhibit a greater number of such processes than do fibroblasts. Morphologically, adherent placental cells are also distinguishable from hematopoietic stem cells, which generally assume a more rounded, or cobblestone, morphology in culture.

The isolated adherent placental cells, and populations of adherent placental cells, useful in the compositions and methods provided herein, express a plurality of markers that can be used to identify and/or isolate the cells, or populations of cells that comprise the adherent placental cells. The adherent placental cells, and adherent placental cell populations useful in the compositions and methods provided herein include adherent placental cells and adherent placental cell-containing cell populations obtained directly from the placenta, or any part thereof (*e.g.,* amnion, chorion, amnion-chorion plate, placental cotyledons, umbilical cord, and the like). The adherent placental stem cell population, in one embodiment, is a population (that is, two or more) of adherent placental stem cells in culture, *e.g.,* a population in a container, *e.g.,* a bag.

The adherent placental cells generally express the markers CD73, CD105, and CD200, and/or OCT-4, and do not express CD34, CD38, or CD45. Adherent placental stem cells can also express HLA-ABC (MHC-1) and HLA-DR. These markers can be used to identify adherent placental cells, and to distinguish the adherent placental cells from other cell types. Because the adherent placental cells can express CD73 and CD105, they can have mesenchymal stem cell-like characteristics. Lack of expression of CD34, CD38 and/or CD45 identifies the adherent placental stem cells as non-hematopoietic stem cells.

In certain embodiments, the isolated adherent placental cells described herein detectably suppress cancer cell proliferation or tumor growth.

In certain embodiments, the isolated adherent placental cells are isolated placental stem cells. In certain other embodiments, the isolated adherent placental cells are isolated placental multipotent cells. In a specific embodiment, the isolated adherent placental cells are CD34⁻, CD10⁺ and CD105⁺ as detected by flow cytometry. In a more specific embodiment, the isolated CD34⁻, CD10⁺, CD105⁺ adherent placental cells are placental stem cells. In another more specific embodiment, the isolated CD34⁻, CD10⁺, CD105⁺ placental cells are multipotent adherent placental cells. In another specific embodiment, the isolated CD34⁻, CD10⁺, CD105⁺ placental cells have the potential to differentiate into cells of a neural phenotype, cells of an osteogenic phenotype, or cells of a chondrogenic phenotype. In a more specific embodiment, the isolated CD34⁻, CD10⁺, CD105⁺ adherent placental cells are additionally CD200⁺. In another more specific embodiment, the isolated CD34⁻, CD10⁺, CD105⁺ adherent placental cells are additionally CD90⁺ or CD45⁻, as detected by flow cytometry. In another more specific embodiment, the isolated CD34⁻, CD10⁺, CD105⁺ adherent placental cells are additionally CD90⁺ or CD45⁻, as detected by flow cytometry. In a more specific embodiment, the CD34⁻, CD10⁺, CD105⁺, CD200⁺ adherent placental cells are additionally CD90⁺ or CD45⁻, as detected by flow cytometry. In another more specific embodiment, the CD34⁻, CD10⁺, CD105⁺, CD200⁺ adherent placental cells are additionally CD90⁺ and CD45⁻, as detected by flow cytometry. In another more specific embodiment, the CD34⁻, CD10⁺, CD105⁺, CD200⁺, CD90⁺, CD45⁻ adherent placental cells are additionally CD80⁻ and CD86⁻, as detected by flow cytometry.

In one embodiment, the isolated adherent placental cells are CD200⁺, HLA-G⁺. In a specific embodiment, said isolated adherent placental cells are also CD73⁺ and CD105⁺. In another specific embodiment, said isolated adherent placental cells are also CD34⁻, CD38⁻ or CD45⁻. In a more specific embodiment, said isolated adherent placental cells are also CD34⁻, CD38⁻, CD45⁻, CD73⁺ and CD105⁺. In another embodiment, said isolated adherent placental cells produce one or more embryoid-like bodies when cultured under conditions that allow the formation of embryoid-like bodies.

In another embodiment, the isolated adherent placental cells are CD73⁺, CD105⁺, CD200⁺. In a specific embodiment of said populations, said isolated adherent placental cells are also HLA-G⁺. In another specific embodiment, said isolated adherent placental cells are also CD34⁻, CD38⁻ or CD45⁻. In another specific embodiment, said isolated adherent placental cells are also CD34⁻, CD38⁻ and CD45⁻. In a more specific embodiment, said isolated adherent placental cells are also CD34⁻, CD38⁻, CD45⁻, and HLA-G⁺. In another specific embodiment, said isolated adherent placental cells produce one or more embryoid-like bodies when cultured under conditions that allow the formation of embryoid-like bodies.

In another embodiment, the isolated adherent placental cells are CD200⁺, OCT-4⁺. In a specific embodiment, said isolated adherent placental cells are also CD73⁺ and CD105⁺. In another specific embodiment, said isolated adherent placental cells are also HLA-G⁺. In another specific embodiment, said isolated adherent placental cells are also CD34⁻, CD38⁻ and CD45⁻. In a more specific embodiment, said isolated adherent placental cells are also CD34⁻, CD38⁻, CD45⁻, CD73⁺, CD105⁺ and HLA-G⁺. In another specific embodiment, the isolated adherent placental cells also produce one or more embryoid-like bodies when cultured under conditions that allow the formation of embryoid-like bodies.

In another embodiment, the isolated adherent placental cells are CD73⁺, CD105⁺ and HLA-G⁺. In a specific embodiment, said isolated adherent placental cells are also CD34⁻, CD38⁻ or CD45⁻. In another specific embodiment, said isolated adherent placental cells also CD34⁻, CD38⁻ and CD45⁻. In another specific embodiment, said adherent stem cells are also OCT-4⁺. In another specific embodiment, said adherent stem cells are also CD200⁺. In a more specific embodiment, said adherent stem cells are also CD34⁻, CD38⁻, CD45⁻, OCT-4⁺ and CD200⁺.

In another embodiment, the isolated adherent placental cells are CD73⁺, CD105⁺ stem cells, wherein said cells produce one or more embryoid-like bodies under conditions that allow formation of embryoid-like bodies. In a specific embodiment, said isolated adherent placental cells are also CD34⁻, CD38⁻ or CD45⁻. In another specific embodiment, isolated adherent placental cells are also CD34⁻, CD38⁻ and CD45⁻. In another specific embodiment, isolated adherent placental cells are also OCT-4⁺. In a more specific embodiment, said isolated adherent placental cells are also OCT-4⁺, CD34⁻, CD38⁻ and CD45⁻.

In another embodiment, the adherent placental stem cells are OCT-4⁺ stem cells, wherein said adherent placental stem cells produce one or more embryoid-like bodies when cultured under conditions that allow the formation of embryoid-like bodies, and wherein said stem cells have been identified as detectably suppressing cancer cell proliferation or tumor growth.

In various embodiments, at least 10%, at least 20%, at least 30%, at least 40%, at least 50% at least 60%, at least 70%, at least 80%, at least 90%, or at least 95% of said isolated adherent placental cells are OCT-4⁺. In a specific embodiment of the above populations, said isolated adherent placental cells are also CD73⁺ and CD105⁺. In another specific embodiment, said isolated adherent placental cells are also CD34⁻, CD38⁻, or CD45⁻. In another specific embodiment, said stem cells are CD200⁺. In a more specific embodiment, said isolated adherent placental cells are also CD73⁺, CD105⁺, CD200⁺, CD34⁻, CD38⁻, and CD45⁻. In another specific embodiment, said isolated adherent placental cells have been expanded, for example, passaged at least once, at least three times, at least five times, at least 10 times, at least 15 times, or at least 20 times.

In a more specific embodiment of any of the above embodiments, the isolated adherent placental cells express ABC-p (a placenta-specific ABC transporter protein; *see, e.g.,* Allikmets et al., Cancer Res. 58(23):5337-9 (1998)).

In another embodiment, the isolated adherent placental cells CD29⁺, CD44⁺, CD73⁺, CD90⁺, CD105⁺, CD200⁺, CD34⁻ and CD133⁻. In another embodiment, the isolated adherent placental cells constitutively secrete IL-6, IL-8 and monocyte chemoattractant protein (MCP-1).

Each of the above-referenced isolated adherent placental cells can comprise cells obtained and isolated directly from a mammalian placenta, or cells that have been cultured and passaged at least 1, 2, 3, 4, 5, 6, 7, 8, 9, 10, 12, 14, 16, 18, 20, 25, 30 or more times, or a combination thereof. Tumor cell suppressive pluralities of the isolated adherent placental cells described above can comprise about, at least, or no more than, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated adherent placental cells.

### 5.6.3. Compositions Comprising Adherent Placental Cell Conditioned Media

Also provided herein is the use of a composition comprising NK cells produced using the methods described herein, e.g., TSNK cells, or NK cell populations or NK progenitor cell populations produced using the three-stage method described herein, and additionally conditioned medium, wherein said composition is tumor suppressive, or is effective in the treatment of cancer or viral infection. Adherent placental cells as described herein can be used to produce conditioned medium that is tumor cell suppressive, anti-cancer or anti-viral that is, medium comprising one or more biomolecules secreted or excreted by the cells that have a detectable tumor cell suppressive effect, anti-cancer effect or antiviral effect. In various embodiments, the conditioned medium comprises medium in which the cells have proliferated (that is, have been cultured) for at least 1, 2, 3, 4, 5, 6, 7, 8, 9, 10, 11, 12, 13, 14 or more days. In other embodiments, the conditioned medium comprises medium in which such cells have grown to at least 30%, 40%, 50%, 60%, 70%, 80%, 90% confluence, or up to 100% confluence. Such conditioned medium can be used to support the culture of a separate population of cells, *e.g.,* placental cells, or cells of another kind. In another embodiment, the conditioned medium provided herein comprises medium in which isolated adherent placental cells, *e.g.,* isolated adherent placental stem cells or isolated adherent placental multipotent cells, and cells other than isolated adherent placental cells, e.g., non-placental stem cells or multipotent cells, have been cultured.

Such conditioned medium can be combined with any of, or any combination of NK cells produced using the methods described herein, e.g., TSNK cells, or NK cell populations, or NK progenitor cell populations, placental perfusate, placental perfusate cells to form a composition that is tumor cell suppressive, anticancer or antiviral. In certain embodiments, the composition comprises less than half conditioned medium by volume, e.g., about, or less than about, 50%, 45%, 40%, 35%, 30%, 25%, 20%, 15%, 10%, 5%, 4%, 3%, 2%, or 1% by volume.

Thus, in one embodiment, provided herein is a composition comprising NK cells produced using the methods described herein, e.g., TSNK cells, or NK cell populations, or NK progenitor cell populations and culture medium from a culture of isolated adherent placental cells, wherein said isolated adherent placental cells (a) adhere to a substrate; and (b) are CD34⁻, CD10⁺ and CD105⁺; wherein said composition detectably suppresses the growth or proliferation of tumor cells, or is anti-cancer or antiviral. In a specific embodiment, the isolated adherent placental cells are CD34⁻, CD10⁺ and CD105⁺ as detected by flow cytometry. In a more specific embodiment, the isolated CD34⁻, CD10⁺, CD105⁺ adherent placental cells are placental stem cells. In another more specific embodiment, the isolated CD34⁻, CD10⁺, CD105⁺ placental cells are multipotent adherent placental cells. In another specific embodiment, the isolated CD34⁻, CD10⁺, CD105⁺ placental cells have the potential to differentiate into cells of a neural phenotype, cells of an osteogenic phenotype, or cells of a chondrogenic phenotype. In a more specific embodiment, the isolated CD34⁻, CD10⁺, CD105⁺ adherent placental cells are additionally CD200⁺. In another more specific embodiment, the isolated CD34⁻, CD10⁺, CD105⁺ adherent placental cells are additionally CD90⁺ or CD45⁻, as detected by flow cytometry. In another more specific embodiment, the isolated CD34⁻, CD10⁺, CD105⁺ adherent placental cells are additionally CD90⁺ or CD45⁻, as detected by flow cytometry. In a more specific embodiment, the CD34⁻, CD10⁺, CD105⁺, CD200⁺ adherent placental cells are additionally CD90⁺ or CD45⁻, as detected by flow cytometry. In another more specific embodiment, the CD34⁻, CD10⁺, CD105⁺, CD200⁺ adherent placental cells are additionally CD90⁺ and CD45⁻, as detected by flow cytometry. In another more specific embodiment, the CD34⁻, CD10⁺, CD105⁺, CD200⁺, CD90⁺, CD45⁻ adherent placental cells are additionally CD80⁻ and CD86⁻, as detected by flow cytometry.

In another embodiment, provided herein is a composition comprising NK cells produced using the methods described herein, e.g., TSNK cells, or NK cell populations, or NK progenitor cell populations and culture medium from a culture of isolated adherent placental cells, wherein said isolated adherent placental cells (a) adhere to a substrate; and (b) express CD200 and HLA-G, or express CD73, CD105, and CD200, or express CD200 and OCT-4, or express CD73, CD105, and HLA-G, or express CD73 and CD105 and facilitate the formation of one or more embryoid-like bodies in a population of placental cells that comprise the placental stem cells when said population is cultured under conditions that allow formation of embryoid-like bodies, or express OCT-4 and facilitate the formation of one or more embryoid-like bodies in a population of placental cells that comprise the placental stem cells when said population is cultured under conditions that allow formation of embryoid-like bodies; wherein said composition detectably suppresses the growth or proliferation of tumor cells, or is anti-cancer or antiviral. In a specific embodiment, the composition further comprises a plurality of said isolated placental adherent cells. In another specific embodiment, the composition comprises a plurality of non-placental cells. In a more specific embodiment, said non-placental cells comprise CD34⁺ cells, *e.g.,* hematopoietic progenitor cells, such as peripheral blood hematopoietic progenitor cells, cord blood hematopoietic progenitor cells, or placental blood hematopoietic progenitor cells. The non-placental cells can also comprise stem cells, such as mesenchymal stem cells, *e.g.,* bone marrow-derived mesenchymal stem cells. The non-placental cells can also be one or more types of adult cells or cell lines. In another specific embodiment, the composition comprises an anti-proliferative agent, *e.g.,* an anti-MIP-la or anti-MIP-1β antibody.

In a specific embodiment, culture medium conditioned by one of the cells or cell combinations described above is obtained from a plurality of isolated adherent placental cells co-cultured with a plurality of tumor cells at a ratio of about 1:1, about 2:1, about 3:1, about 4:1, or about 5:1 isolated adherent placental cells to tumor cells. For example, the conditioned culture medium or supernatant can be obtained from a culture comprising about 1 x 10⁵ isolated adherent placental cells, about 1 x 10⁶ isolated adherent placental cells, about 1 x 10⁷ isolated adherent placental cells, or about 1 x 10⁸ isolated adherent placental cells, or more. In another specific embodiment, the conditioned culture medium or supernatant is obtained from a co-culture comprising about 1 x 10⁵ to about 5 x 10⁵ isolated adherent placental cells and about 1 x 10⁵ tumor cells; about 1 x 10⁶ to about 5 x 10⁶ isolated adherent placental cells and about 1 x 10⁶ tumor cells; about 1 x 10⁷ to about 5 x 10⁷ isolated adherent placental cells and about 1 x 10⁷ tumor cells; or about 1 x 10⁸ to about 5 x 10⁸ isolated adherent placental cells and about 1 x 10⁸ tumor cells.

### 5.7. Preservation of Cells

Cells, *e.g.,* NK cells produced using the methods described herein, e.g., TSNK cells, or NK cell populations or NK progenitor cell populations produced using the three-stage method described herein, or placental perfusate cells comprising hematopoietic stem cells or progenitor cells, can be preserved, that is, placed under conditions that allow for long-term storage, or under conditions that inhibit cell death by, *e.g.,* apoptosis or necrosis.

Placental perfusate can be produced by passage of a cell collection composition through at least a part of the placenta, *e.g.,* through the placental vasculature. The cell collection composition comprises one or more compounds that act to preserve cells contained within the perfusate. Such a placental cell collection composition can comprise an apoptosis inhibitor, necrosis inhibitor and/or an oxygen-carrying perfluorocarbon, as described in related U.S. Application Publication No. 20070190042, the disclosure of which is hereby incorporated by reference in its entirety.

In one embodiment, perfusate or a population of placental cells are collected from a mammalian, *e.g.,* human, post-partum placenta by bringing the perfusate or population of cells into proximity with a cell collection composition comprising an inhibitor of apoptosis and an oxygen-carrying perfluorocarbon, wherein said inhibitor of apoptosis is present in an amount and for a time sufficient to reduce or prevent apoptosis in the population of placental cells, *e.g.,* adherent placental cells, for example, placental stem cells or placental multipotent cells, as compared to a population of cells not contacted or brought into proximity with the inhibitor of apoptosis. For example, the placenta can be perfused with the cell collection composition, and placental cells, *e.g.,* total nucleated placental cells, are isolated therefrom. In a specific embodiment, the inhibitor of apoptosis is a caspase inhibitor. In another specific embodiment, said inhibitor of apoptosis is a JNK inhibitor. In a more specific embodiment, said JNK inhibitor does not modulate differentiation or proliferation of adherent placental cells, *e.g.,* adherent placental stem cells or adherent placental multipotent cells. In another embodiment, the cell collection composition comprises said inhibitor of apoptosis and said oxygen-carrying perfluorocarbon in separate phases. In another embodiment, the cell collection composition comprises said inhibitor of apoptosis and said oxygen-carrying perfluorocarbon in an emulsion. In another embodiment, the cell collection composition additionally comprises an emulsifier, *e.g.,* lecithin. In another embodiment, said apoptosis inhibitor and said perfluorocarbon are between about 0 °C and about 25 °C at the time of bringing the placental cells into proximity with the cell collection composition. In another more specific embodiment, said apoptosis inhibitor and said perfluorocarbon are between about 2 °C and 10 °C, or between about 2 °C and about 5 °C, at the time of bringing the placental cells into proximity with the cell collection composition. In another more specific embodiment, said bringing into proximity is performed during transport of said population of cells. In another more specific embodiment, said bringing into proximity is performed during freezing and thawing of said population of cells.

In another embodiment, placental perfusate and/or placental cells can be collected and preserved by bringing the perfusate and/or cells into proximity with an inhibitor of apoptosis and an organ-preserving compound, wherein said inhibitor of apoptosis is present in an amount and for a time sufficient to reduce or prevent apoptosis of the cells, as compared to perfusate or placental cells not contacted or brought into proximity with the inhibitor of apoptosis. In a specific embodiment, the organ-preserving compound is UW solution (described in U.S. Patent No. 4,798,824; also known as VIASPAN™; *see also* Southard et al., Transplantation 49(2):251-257 (1990) or a solution described in Stern et al., U.S. Patent No. 5,552,267, the disclosures of which are hereby incorporated by reference in their entireties. In another embodiment, said organ-preserving composition is hydroxyethyl starch, lactobionic acid, raffinose, or a combination thereof. In another embodiment, the placental cell collection composition additionally comprises an oxygen-carrying perfluorocarbon, either in two phases or as an emulsion.

In another embodiment of the method, placental cells are brought into proximity with a cell collection composition comprising an apoptosis inhibitor and oxygen-carrying perfluorocarbon, organ-preserving compound, or combination thereof, during perfusion. In another embodiment, placental cells are brought into proximity with said cell collection compound after collection by perfusion.

Typically, during placental cell collection, enrichment and isolation, it is preferable to minimize or eliminate cell stress due to hypoxia and mechanical stress. In another embodiment of the method, therefore, placental perfusate or a population of placental cells is exposed to a hypoxic condition during collection, enrichment or isolation for less than six hours during said preservation, wherein a hypoxic condition is a concentration of oxygen that is less than normal blood oxygen concentration. In a more specific embodiment, said perfusate or population of placental cells is exposed to said hypoxic condition for less than two hours during said preservation. In another more specific embodiment, said population of placental cells is exposed to said hypoxic condition for less than one hour, or less than thirty minutes, or is not exposed to a hypoxic condition, during collection, enrichment or isolation. In another specific embodiment, said population of placental cells is not exposed to shear stress during collection, enrichment or isolation.

Cells, *e.g.,* placental perfusate cells, hematopoietic cells, *e.g.,* CD34⁺ hematopoietic stem cells; NK cells produced using the methods described herein, e.g., TSNK cells, or NK cell populations, or NK progenitor cell populations; isolated adherent placental cells provided herein can be cryopreserved, *e.g.,* in cryopreservation medium in small containers, *e.g.,* ampoules or septum vials. In certain embodiments, cells provided herein are cryopreserved at a concentration of about 1 x 10⁴ - 5 x 10⁸ cells per mL. In specific embodiments, cells provided herein are cryopreserved at a concentration of about 1 x 10⁶ - 1.5 x 10⁷ cells per mL. In more specific embodiments, cells provided herein are cryopreserved at a concentration of about 1 x 10⁴, 5 x 10⁴, 1 x 10⁵, 5 x 10⁵, 1 x 10⁶, 5 x 10⁶, 1 x 10⁷, 1.5 x 10⁷ cells per mL.

Suitable cryopreservation medium includes, but is not limited to, normal saline, culture medium including, *e.g.,* growth medium, or cell freezing medium, for example commercially available cell freezing medium, *e.g.,* C2695, C2639 or C6039 (Sigma); CryoStor® CS2, CryoStor® CS5 or CryoStor®CS10 (BioLife Solutions). In one embodiment, cryopreservation medium comprises DMSO (dimethylsulfoxide), at a concentration of, *e.g.,* about 1, 2, 3, 4, 5, 6, 7, 8, 9 or 10% (v/v). Cryopreservation medium may comprise additional agents, for example, methylcellulose, dextran, albumin (*e.g.,* human serum albumin), trehalose, and/or glycerol. In certain embodiments, the cryopreservation medium comprises about 1%-10% DMSO, about 25%-75% dextran and/or about 20-60% human serum albumin (HSA). In certain embodiments, the cryopreservation medium comprises about 1%-10% DMSO, about 25%-75% trehalose and/or about 20-60% human HSA. In a specific embodiment, the cryopreservation medium comprises 5% DMSO, 55% dextran and 40% HSA. In a more specific embodiment, the cryopreservation medium comprises 5% DMSO, 55% dextran (10% w/v in normal saline) and 40% HSA. In another specific embodiment, the cryopreservation medium comprises 5% DMSO, 55% trehalose and 40% HSA. In a more specific embodiment, the cryopreservation medium comprises 5% DMSO, 55% trehalose (10% w/v in normal saline) and 40% HSA. In another specific embodiment, the cryopreservation medium comprises CryoStor® CS5. In another specific embodiment, the cryopreservation medium comprises CryoStor®CS10.

Cells provided herein can be cryopreserved by any of a variety of methods, and at any stage of cell culturing, expansion or differentiation. For example, cells provided herein can be cryopreserved right after isolation from the origin tissues or organs, *e.g.,* placental perfusate or umbilical cord blood, or during, or after either the first or second step of the methods outlined above. In certain embodiments, the hematopoietic cells, *e.g.,* hematopoietic stem or progenitor cells are cryopreserved within about 1, 5, 10, 15, 20, 30, 45 minutes or within about 1, 2, 4, 6, 10, 12, 18, 20 or 24 hours after isolation from the origin tissues or organs. In certain embodiments, said cells are cryopreserved within 1, 2 or 3 days after isolation from the origin tissues or organs. In certain embodiments, said cells are cryopreserved after being cultured in a first medium as described above, for about 1, 2, 3, 4, 5, 6, 7, 8, 9, 10, 11, 12, 13, 14, 15, 16, 17, 18, 19, 20, 21, 22, 23, 24, 25, 26, 27 or 28 days. In some embodiments, said cells are cryopreserved after being cultured in a first medium as described above, for about 1, 2, 3, 4, 5, 6, 7, 8, 9, 10, 11, 12, 13, 14, 15, 16, 17, 18, 19, 20, 21, 22, 23, 24, 25, 26, 27 or 28 days, and in a second medium for about 1, 2, 3, 4, 5, 6, 7, 8, 9, 10, 11, 12, 13, 14, 15, 16, 17, 18, 19, 20, 21, 22, 23, 24, 25, 26, 27 or 28 days as described above. In some embodiments, when NK cells or NK progenitor cells are made using a three-stage method described herein, said cells are cryopreserved after being cultured in a first medium about 1, 2, 3, 4, 5, 6, 7, 8, 9, 10, 11, 12, 13, 14, 15, 16, 17, 18, 19, 20, 21, 22, 23, 24, or 25 days; and/or after being cultured in a second medium about 1, 2, 3, 4, 5, 6, 7, 8, 9, 10, 11, 12, 13, 14, 15, 16, 17, 18, 19, 20, 21, 22, 23, 24, or 25 days; and/or after being cultured in a third medium about 1, 2, 3, 4, 5, 6, 7, 8, 9, 10, 11, 12, 13, 14, 15, 16, 17, 18, 19, 20, 21, 22, 23, 24, or 25 days. In a specific embodiment, NK progenitor cells are made using a three-stage method described herein, and said cells are cryopreserved after being cultured in a first medium for 9 days; after being cultured in a second medium for 5 days; and after being cultured in a third medium for 7 days.

In one aspect, provided herein is a method of cryopreserving a population of NK cells, *e.g.,* TSNK cells. In one embodiment, said method comprises: (a) seeding a population of hematopoietic stem or progenitor cells in a first medium comprising interleukin-15 (IL-15) and, optionally, one or more of stem cell factor (SCF) and interleukin-7 (IL-7), wherein said IL-15 and optional SCF and IL-7 are not comprised within an undefined component of said medium, such that the population expands, and a plurality of hematopoietic stem or progenitor cells within said population of hematopoietic stem or progenitor cells differentiate into NK cells during said expanding; (b) expanding the cells from step (a) in a second medium comprising interleukin-2 (IL-2), to produce a population of activated NK cells, and (c) cryopreserving the NK cells from step (b) in a cryopreservation medium. In a specific embodiment, said step (c) further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain embodiments, the method includes no intermediary steps between step (a) and (b), and between step (b) and (c), and/or no additional culturing steps prior to step (a).

In another embodiment, said method of cryopreserving a population of NK cells, e.g., TSNK cells comprises: (a) expanding a population of hematopoietic stem or progenitor cells in a first medium comprising one or more of stem cell factor (SCF), IL-2, interleukin-7 (IL-7), interleukin-15 (IL-15) and heparin, and wherein said SCF, IL-2, IL-7 and IL-15 are not comprised within an undefined component of said medium, and wherein a plurality of hematopoietic stem or progenitor cells within said population of hematopoietic stem or progenitor cells differentiate into NK cells during said expanding; (b) expanding the cells from step (a) in a second medium comprising interleukin-2 (IL-2), to produce activated NK cells; and (c) cryopreserving the NK cells from step (b) in a cryopreservation medium. In a specific embodiment, said step (c) further comprises (1) preparing a cell suspension solution; (2) adding cryopreservation medium to the cell suspension solution from step (1) to obtain cryopreserved cell suspension; (3) cooling the cryopreserved cell suspension from step (3) to obtain a cryopreserved sample; and (4) storing the cryopreserved sample below -80 °C. In certain embodiments, the method includes no intermediary steps between step (a) and (b), and between step (b) and (c).

Cells provided herein are preferably cooled in a controlled-rate freezer, *e.g.,* at about 0.1, 0.3, 0.5, 1, or 2 °C/min during cryopreservation. A preferred cryopreservation temperature is about -80 °C to about -180 °C, preferably about -125 °C to about -140 °C. Cryopreserved cells can be transferred to liquid nitrogen prior to thawing for use. In some embodiments, for example, once the ampoules have reached about -90 °C, they are transferred to a liquid nitrogen storage area. Cryopreserved cells preferably are thawed at a temperature of about 25 °C to about 40 °C, preferably to a temperature of about 37 °C. In certain embodiments, the cryopreserved cells are thawed after being cryopreserved for about 1, 2, 4, 6, 10, 12, 18, 20 or 24 hours, or for about 1, 2, 3, 4, 5, 6, 7, 8, 9, 10, 11, 12, 13, 14, 15, 16, 17, 18, 19, 20, 21, 22, 23, 24, 25, 26, 27 or 28 days. In certain embodiments, the cryopreserved cells are thawed after being cryopreserved for about 1, 2, 3, 4, 5, 6, 7, 8, 9, 10, 11, 12, 13, 14, 15, 16, 17, 18, 19, 20, 21, 22, 23, 24, 25, 26, 27 or 28 months. In certain embodiments, the cryopreserved cells are thawed after being cryopreserved for about 1, 2, 3, 4, 5, 6, 7, 8, 9 or 10 years.

Suitable thawing medium includes, but is not limited to, normal saline, plasmalyte culture medium including, for example, growth medium, e.g., RPMI medium. In preferred embodiments, the thawing medium comprises one or more of medium supplements (e.g., nutrients, cytokines and/or factors). Medium supplements suitable for thawing cells provided herein include, for example without limitation, serum such as human serum AB, fetal bovine serum (FBS) or fetal calf serum (FCS), vitamins, human serum albumin (HSA), bovine serum albumin (BSA), amino acids (*e.g.,* L-glutamine), fatty acids (*e.g.,* oleic acid, linoleic acid or palmitic acid), insulin (e.g., recombinant human insulin), transferrin (iron saturated human transferrin), β-mercaptoethanol, stem cell factor (SCF), Fms-like-tyrosine kinase 3 ligand (Flt3-L), cytokines such as interleukin-2 (IL-2), interleukin-7 (IL-7), interleukin-15 (IL-15), thrombopoietin (Tpo) or heparin. In a specific embodiment, the thawing medium useful in the methods provided herein comprises RPMI. In another specific embodiment, said thawing medium comprises plasmalyte. In another specific embodiment, said thawing medium comprises about 0.5-20% FBS. In another specific embodiment, said thawing medium comprises about 1, 2, 5, 10, 15 or 20% FBS. In another specific embodiment, said thawing medium comprises about 0.5%-20% HSA. In another specific embodiment, said thawing medium comprises about 1, 2.5, 5, 10, 15, or 20% HSA. In a more specific embodiment, said thawing medium comprises RPMI and about 10% FBS. In another more specific embodiment, said thawing medium comprises plasmalyte and about 5% HSA.

The cryopreservation methods provided herein can be optimized to allow for long-term storage, or under conditions that inhibit cell death by, e.g., apoptosis or necrosis. In one embodiments, the post-thaw cells comprise greater than 60%, 65%, 70%, 75%, 80%, 85%, 90%, 95% or 98% of viable cells, as determined by, e.g., automatic cell counter or trypan blue method. In another embodiment, the post-thaw cells comprise about 0.5, 1, 5, 10, 15, 20 or 25% of dead cells. In another embodiment, the post-thaw cells comprise about 0.5, 1, 5, 10, 15, 20 or 25% of early apoptotic cells. In another embodiment, about 0.5, 1, 5, 10, 15 or 20% of post-thaw cells undergo apoptosis after 1, 2, 3, 4, 5, 6, 7, 8, 9, 10, 11, 12, 13, 14, 15, 16, 17, 18, 19, 20, 21, 22, 23, 24, 25, 26, 27 or 28 days after being thawed, e.g., as determined by an apoptosis assay (*e.g.,* TO-PRO3 or AnnV/PI Apoptosis assay kit). In certain embodiments, the post-thaw cells are re-cryopreserved after being cultured, expanded or differentiated using methods provided herein.

### 5.8. Compositions Comprising NK Cells

### 5.8.1. TSNK Cells

In some embodiments, provided herein is a composition comprising isolated TSNK cells. In a specific embodiment, said TSNK cells are produced from hematopoietic cells, e.g., hematopoietic stem or progenitor cells isolated from placental perfusate, umbilical cord blood, and/or peripheral blood. In another specific embodiment, said TSNK cells comprise at least 50% of cells in the composition. In another specific embodiment, said TSNK cells comprise at least 80%, 85%, 90%. 95%, 98% or 99% of cells in the composition. In certain embodiments, greater than 90%, 92%, 94%, 96% or 98% of TSNK cells in said composition are CD56⁺ and CD16⁻. In other embodiments, at least 80%, 82%, 84%, 86%, 88% or 90% of TSNK cells in said composition are CD3⁻ and CD56⁺. In other embodiments, at least 50%, 52%, 54%, 56%, 58% or 60% of said cells are NKG2D⁺. In other embodiments, fewer than 10%, 9%, 8%, 7%, 6%, 5%, 4% or 3% of said cells are NKB1⁺. In certain other embodiments, fewer than 10%, 8%, 6%, 4% or 2% of said TSNK cells are NKAT2⁺. In certain other embodiments, fewer than 10%, 8%, 6%, 4% or 2% of said TSNK cells are CD56⁺ and CD16⁺. In more specific embodiments, at least 50%, 55%, 60%, 65% or 70% of said CD3⁻, CD56⁺ TSNK cells are NKp46⁺. In other more specific embodiments, at least 50%, 55%, 60%, 65%, 70%, 75%, 80% or 85% of said CD3⁻, CD56⁺ TSNK cells are CD117⁺. In other more specific embodiments, at least 20%, 25%, 30%, 35%, 40% or 45% of said CD3⁻, CD56⁺ TSNK cells are CD94⁺. In other more specific embodiments, at least 10%, 12%, 14%, 16%, 18% or 20% of said CD3⁻, CD56⁺ TSNK cells are CD226⁺. In more specific embodiments, at least 20%, 25%, 30%, 35% or 40% of said CD3⁻, CD56⁺ TSNK cells are CD7⁺. In more specific embodiments, at least 30%, 35%, 40%, 45%, 50%, 55% or 60% of said CD3⁻, CD56⁺ TSNK cells are CD5⁺.

In another specific embodiment, said isolated CD56⁺, CD16⁻ TSNK cells are from a single individual. In a more specific embodiment, said isolated CD56⁺, CD16⁻ natural killer cells (TSNK cells) comprise natural killer cells from at least two different individuals. In another specific embodiment, said TSNK cells have been contacted or brought into proximity with an immunomodulatory compound or thalidomide in an amount and for a time sufficient for said TSNK cells to express detectably more granzyme B or perforin than an equivalent number of natural killer cells, *i.e.* TSNK cells, not contacted or brought into proximity with said immunomodulatory compound or thalidomide. In another specific embodiment, said composition additionally comprises an immunomodulatory compound or thalidomide. In certain embodiments, the immunomodulatory compound is a compound described below, *e.g.,* an amino-substituted isoindoline compound.

In another specific embodiment, the composition additionally comprises one or more anticancer compounds, *e.g.,* one or more of the anticancer compounds described below.

In a more specific embodiment, the composition comprises TSNK cells and natural killer cells from another source, or made by another method. In a specific embodiment, said other source is placental blood and/or umbilical cord blood. In another specific embodiment, said other source is peripheral blood. In more specific embodiments, the TSNK cells are combined with natural killer cells from another source, or made by another method in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific embodiment, the composition comprises TSNK cells and either isolated placental perfusate or isolated placental perfusate cells. In a more specific embodiment, said placental perfusate is from the same individual as said TSNK cells. In another more specific embodiment, said placental perfusate comprises placental perfusate from a different individual than said TSNK cells. In another specific embodiment, all, or substantially all (*e.g.,* greater than 90%, 95%, 98% or 99%) of cells in said placental perfusate are fetal cells. In another specific embodiment, the placental perfusate or placental perfusate cells, comprise fetal and maternal cells. In a more specific embodiment, the fetal cells in said placental perfusate comprise less than about 90%, 80%, 70%, 60% or 50% of the cells in said perfusate. In another specific embodiment, said perfusate is obtained by passage of a 0.9% NaCl solution through the placental vasculature. In another specific embodiment, said perfusate comprises a culture medium. In another specific embodiment, said perfusate has been treated to remove erythrocytes. In another specific embodiment, said composition comprises an immunomodulatory compound, *e.g.,* an immunomodulatory compound described in Section 5.2.1.1 below, *e.g.,* an amino-substituted isoindoline compound. In another specific embodiment, the composition additionally comprises one or more anticancer compounds, *e.g.,* one or more of the anticancer compounds described below.

In another specific embodiment, the composition comprises TSNK cells and placental perfusate cells. In a more specific embodiment, said placental perfusate cells are from the same individual as said TSNK cells. In another more specific embodiment, said placental perfusate cells are from a different individual than said TSNK cells. In another specific embodiment, the composition comprises isolated placental perfusate and isolated placental perfusate cells, wherein said isolated perfusate and said isolated placental perfusate cells are from different individuals. In another more specific embodiment of any of the above embodiments comprising placental perfusate, said placental perfusate comprises placental perfusate from at least two individuals. In another more specific embodiment of any of the above embodiments comprising placental perfusate cells, said isolated placental perfusate cells are from at least two individuals. In another specific embodiment, said composition comprises an immunomodulatory compound. In another specific embodiment, the composition additionally comprises one or more anticancer compounds, e.g., one or more of the anticancer compounds described below.

### 5.8.2. NK Progenitor Cells Produced Using The Three-Stage Method

In some embodiments, provided herein is a composition, e.g., a pharmaceutical composition, comprising an isolated NK progenitor cell population produced using the three-stage method described herein. In a specific embodiment, said isolated NK progenitor cell population is produced from hematopoietic cells, *e.g.,* hematopoietic stem or progenitor cells isolated from placental perfusate, umbilical cord blood, and/or peripheral blood. In another specific embodiment, said isolated NK progenitor cell population comprises at least 50% of cells in the composition. In another specific embodiment, said isolated NK progenitor cell population comprises at least 80%, 85%, 90%. 95%, 98% or 99% of cells in the composition. In certain embodiments, no more than 5%, 10%, 15%, 20%, 25%, 30%, 35%, or 40% of the cells in said isolated NK progenitor cell population are CD3⁻CD56⁺ cells. In certain embodiments, no less than 65%, 70%, 75%, 80%, 85%, 90%, 95%, 98%, or 99% of said CD3⁻CD56⁺ cells are CD117⁺. In certain embodiments, no less than 40%, 45%, 50%, 55%, 60%, 65%, 70%, or 75% of said CD3⁻CD56⁺ cells are CD161⁺. In certain embodiments, no more than 25%, 30%, 35%, 40%, 45%, 50%, 55%, 60%, 65%, 70%, 75%, 80%, 85%, or 90% of said CD3⁻CD56⁺ cells are NKp46⁺. In more specific embodiments, no more than 25%, 30%, 35%, 40%, 45%, 50%, or 55% of said CD3⁻CD56⁺ cells are NKp46⁺. In certain embodiments, said CD3⁻CD56⁺ cells are CD16⁻.

In another specific embodiment, said isolated NK progenitor cells in said composition are from a single individual. In a more specific embodiment, said isolated NK progenitor cells comprise NK progenitor cells from at least two different individuals. In another specific embodiment, said isolated NK progenitor cells in said composition are from a different individual than the individual for whom treatment with the NK progenitor cells is intended. In another specific embodiment, said NK progenitor cells have been contacted or brought into proximity with an immunomodulatory compound or thalidomide in an amount and for a time sufficient for said NK progenitor cells to express detectably more granzyme B or perforin than an equivalent number of natural killer cells, *i.e.* NK progenitor cells not contacted or brought into proximity with said immunomodulatory compound or thalidomide. In another specific embodiment, said composition additionally comprises an immunomodulatory compound or thalidomide. In certain embodiments, the immunomodulatory compound is a compound described below, *e.g.,* an amino-substituted isoindoline compound.

In another specific embodiment, the composition additionally comprises one or more anticancer compounds, *e.g*., one or more of the anticancer compounds described below.

In a more specific embodiment, the composition comprises NK progenitor cells from another source, or made by another method. In a specific embodiment, said other source is placental blood and/or umbilical cord blood. In another specific embodiment, said other source is peripheral blood. In more specific embodiments, the NK progenitor cell population in said composition is combined with NK progenitor cells from another source, or made by another method in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific embodiment, the composition comprises an NK progenitor cell population produced using the three-stage method described herein and either isolated placental perfusate or isolated placental perfusate cells. In a more specific embodiment, said placental perfusate is from the same individual as said NK progenitor cell population. In another more specific embodiment, said placental perfusate comprises placental perfusate from a different individual than said NK progenitor cell population. In another specific embodiment, all, or substantially all (*e.g.,* greater than 90%, 95%, 98% or 99%) of cells in said placental perfusate are fetal cells. In another specific embodiment, the placental perfusate or placental perfusate cells, comprise fetal and maternal cells. In a more specific embodiment, the fetal cells in said placental perfusate comprise less than about 90%, 80%, 70%, 60% or 50% of the cells in said perfusate. In another specific embodiment, said perfusate is obtained by passage of a 0.9% NaCl solution through the placental vasculature. In another specific embodiment, said perfusate comprises a culture medium. In another specific embodiment, said perfusate has been treated to remove erythrocytes. In another specific embodiment, said composition comprises an immunomodulatory compound, *e.g.,* an immunomodulatory compound described below, *e.g.,* an amino-substituted isoindoline compound. In another specific embodiment, the composition additionally comprises one or more anticancer compounds, e.g., one or more of the anticancer compounds described below.

In another specific embodiment, the composition comprises an NK progenitor cell population and placental perfusate cells. In a more specific embodiment, said placental perfusate cells are from the same individual as said NK progenitor cell population. In another more specific embodiment, said placental perfusate cells are from a different individual than said NK progenitor cell population. In another specific embodiment, the composition comprises isolated placental perfusate and isolated placental perfusate cells, wherein said isolated perfusate and said isolated placental perfusate cells are from different individuals. In another more specific embodiment of any of the above embodiments comprising placental perfusate, said placental perfusate comprises placental perfusate from at least two individuals. In another more specific embodiment of any of the above embodiments comprising placental perfusate cells, said isolated placental perfusate cells are from at least two individuals. In another specific embodiment, said composition comprises an immunomodulatory compound. In another specific embodiment, the composition additionally comprises one or more anticancer compounds, *e.g*., one or more of the anticancer compounds described below.

### 5.8.3. NK Cells Produced Using The Three-Stage Method

In some embodiments, provided herein is a composition, e.g., a pharmaceutical composition, comprising an isolated NK cell population produced using the three-stage method described herein. In a specific embodiment, said isolated NK cell population is produced from hematopoietic cells, e.g., hematopoietic stem or progenitor cells isolated from placental perfusate, umbilical cord blood, and/or peripheral blood. In another specific embodiment, said isolated NK cell population comprises at least 50% of cells in the composition. In another specific embodiment, said isolated NK cell population comprises at least 80%, 85%, 90%. 95%, 98% or 99% of cells in the composition. In certain embodiments, no more than 5%, 10%, 15%, 20%, 25%, 30%, 35%, or 40% of the cells in said isolated NK cell population are CD3⁻CD56⁺ cells. In certain embodiments, no less than 65%, 70%, 75%, 80%, 85%, 90%, 95%, 98%, or 99% of said CD3⁻CD56⁺ cells are CD117⁺. In certain embodiments, no less than 40%, 45%, 50%, 55%, 60%, 65%, 70%, or 75% of said CD3⁻CD56⁺ cells are CD161⁺. In certain embodiments, no more than 25%, 30%, 35%, 40%, 45%, 50%, 55%, 60%, 65%, 70%, 75%, 80%, 85%, or 90% of said CD3⁻CD56⁺ cells are NKp46⁺. In more specific embodiments, no more than 25%, 30%, 35%, 40%, 45%, 50%, or 55% of said CD3⁻CD56⁺ cells are NKp46⁺. In certain embodiments, said CD3⁻CD56⁺ cells are CD16⁻.

In another specific embodiment, said isolated NK cells in said composition are from a single individual. In a more specific embodiment, said isolated NK cells comprise NK cells from at least two different individuals. In another specific embodiment, said isolated NK cells in said composition are from a different individual than the individual for whom treatment with the NK cells is intended. In another specific embodiment, said NK cells have been contacted or brought into proximity with an immunomodulatory compound or thalidomide in an amount and for a time sufficient for said NK cells to express detectably more granzyme B or perforin than an equivalent number of natural killer cells, *i.e.* NK cells not contacted or brought into proximity with said immunomodulatory compound or thalidomide. In another specific embodiment, said composition additionally comprises an immunomodulatory compound or thalidomide. In certain embodiments, the immunomodulatory compound is a compound described below, *e.g.,* an amino-substituted isoindoline compound.

In another specific embodiment, the composition additionally comprises one or more anticancer compounds, *e.g*., one or more of the anticancer compounds described below.

In a more specific embodiment, the composition comprises NK cells from another source, or made by another method. In a specific embodiment, said other source is placental blood and/or umbilical cord blood. In another specific embodiment, said other source is peripheral blood. In more specific embodiments, the NK cell population in said composition is combined with NK cells from another source, or made by another method in a ratio of about 100:1, 95:5, 90:10, 85:15, 80:20, 75:25, 70:30, 65:35, 60:40, 55:45: 50:50, 45:55, 40:60, 35:65, 30:70, 25:75, 20:80, 15:85, 10:90, 5:95, 100:1, 95:1, 90:1, 85:1, 80:1, 75:1, 70:1, 65:1, 60:1, 55:1, 50:1, 45:1, 40:1, 35:1, 30:1, 25:1, 20:1, 15:1, 10:1, 5:1, 1:1, 1:5, 1:10, 1:15, 1:20, 1:25, 1:30, 1:35, 1:40, 1:45, 1:50, 1:55, 1:60, 1:65, 1:70, 1:75, 1:80, 1:85, 1:90, 1:95, 1:100, or the like.

In another specific embodiment, the composition comprises an NK cell population produced using the three-stage method described herein and either isolated placental perfusate or isolated placental perfusate cells. In a more specific embodiment, said placental perfusate is from the same individual as said NK cell population. In another more specific embodiment, said placental perfusate comprises placental perfusate from a different individual than said NK cell population. In another specific embodiment, all, or substantially all *(e.g.,* greater than 90%, 95%, 98% or 99%) of cells in said placental perfusate are fetal cells. In another specific embodiment, the placental perfusate or placental perfusate cells, comprise fetal and maternal cells. In a more specific embodiment, the fetal cells in said placental perfusate comprise less than about 90%, 80%, 70%, 60% or 50% of the cells in said perfusate. In another specific embodiment, said perfusate is obtained by passage of a 0.9% NaCl solution through the placental vasculature. In another specific embodiment, said perfusate comprises a culture medium. In another specific embodiment, said perfusate has been treated to remove erythrocytes. In another specific embodiment, said composition comprises an immunomodulatory compound, *e.g.,* an immunomodulatory compound described below, *e.g.,* an amino-substituted isoindoline compound. In another specific embodiment, the composition additionally comprises one or more anticancer compounds, *e.g.,* one or more of the anticancer compounds described below.

In another specific embodiment, the composition comprises an NK cell population and placental perfusate cells. In a more specific embodiment, said placental perfusate cells are from the same individual as said NK cell population. In another more specific embodiment, said placental perfusate cells are from a different individual than said NK cell population. In another specific embodiment, the composition comprises isolated placental perfusate and isolated placental perfusate cells, wherein said isolated perfusate and said isolated placental perfusate cells are from different individuals. In another more specific embodiment of any of the above embodiments comprising placental perfusate, said placental perfusate comprises placental perfusate from at least two individuals. In another more specific embodiment of any of the above embodiments comprising placental perfusate cells, said isolated placental perfusate cells are from at least two individuals. In another specific embodiment, said composition comprises an immunomodulatory compound. In another specific embodiment, the composition additionally comprises one or more anticancer compounds, *e.g.,* one or more of the anticancer compounds described below.

### 5.9. Uses of TSNK Cells and NK Cells and NK Progenitor Cells Produced Using the Three-Stage Method

The NK cells produced using the methods described herein, e.g., TSNK cells, or NK cell populations or NK progenitor cell populations produced according to the three-stage method described herein, provided herein can be used in methods of treating individuals having cancer, e.g., individuals having solid tumor cells and/or blood cancer cells, or persons having a viral infection. In some such embodiments, an effective dosage of NK cells produced using the methods described herein ranges from 1 x 10⁴ to 5 x 10⁴, 5 x 10⁴ to 1 x 10⁵, 1 x 10⁵ to 5 x 10⁵, 5 x 10⁵ to 1 x 10⁶, 1 x 10⁶ to 5 x 10⁶, 5 x 10⁶ to 1 x 10⁷, or more cells/kilogram body weight. The NK cells produced using the methods described herein, e.g., TSNK cells, or NK cell populations, or NK progenitor cell populations, provided herein can also be used in methods of suppressing proliferation of tumor cells.

### 5.9.1. Treatment of Individuals Having Cancer

In one embodiment, provided herein is a method of treating an individual having a cancer, for example, a blood cancer or a solid tumor, comprising administering to said individual a therapeutically effective amount of NK cells produced using the methods described herein, e.g., TSNK cells, or NK cell populations or NK progenitor cell populations produced using the three-stage method described herein. In certain embodiments, the individual has a deficiency of natural killer cells, *e.g.,* a deficiency of NK cells active against the individual's cancer. In a specific embodiment, the method additionally comprises administering to said individual isolated placental perfusate or isolated placental perfusate cells, *e.g.,* a therapeutically effective amount of placental perfusate or isolated placental perfusate cells. In another specific embodiment, the method comprises additionally administering to said individual an effective amount of an immunomodulatory compound, *e.g.,* an immunomodulatory compound described above, or thalidomide. As used herein, an "effective amount" is an amount that, *e.g.,* results in a detectable improvement of, lessening of the progression of, or elimination of, one or more symptoms of a cancer from which the individual suffers.

In a specific embodiment, the cancer is a blood cancer, *e.g.,* a leukemia or a lymphoma. In more specific embodiments, the cancer is an acute leukemia, *e.g.,* acute T cell leukemia, acute myelogenous leukemia (AML), acute promyelocytic leukemia, acute myeloblastic leukemia, acute megakaryoblastic leukemia, precursor B acute lymphoblastic leukemia, precursor T acute lymphoblastic leukemia, Burkitt's leukemia (Burkitt's lymphoma), or acute biphenotypic leukemia; a chronic leukemia, *e.g.,* chronic myeloid lymphoma, chronic myelogenous leukemia (CML), chronic monocytic leukemia, chronic lymphocytic leukemia (CLL)/Small lymphocytic lymphoma, or B-cell prolymphocytic leukemia; hairy cell lymphoma; T-cell prolymphocytic leukemia; or a lymphoma, *e.g.,* histiocytic lymphoma, lymphoplasmacytic lymphoma (*e.g*., Waldenstrom macroglobulinemia), splenic marginal zone lymphoma, plasma cell neoplasm (*e.g*., plasma cell myeloma, plasmacytoma, a monoclonal immunoglobulin deposition disease, or a heavy chain disease), extranodal marginal zone B cell lymphoma (MALT lymphoma), nodal marginal zone B cell lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, T cell large granular lymphocytic leukemia, aggressive NK cell leukemia, adult T cell leukemia/lymphoma, extranodal NK/T cell lymphoma, nasal type, enteropathy-type T cell lymphoma, hepatosplenic T cell lymphoma, blastic NK cell lymphoma, mycosis fungoides (Sezary syndrome), a primary cutaneous CD30-positive T cell lymphoproliferative disorder (*e.g*., primary cutaneous anaplastic large cell lymphoma or lymphomatoid papulosis), angioimmunoblastic T cell lymphoma, peripheral T cell lymphoma, unspecified, anaplastic large cell lymphoma, a Hodgkin's lymphoma or a nodular lymphocyte-predominant Hodgkin's lymphoma. In another specific embodiment, the cancer is multiple myeloma or myelodysplastic syndrome.

In certain other specific embodiments, the cancer is a solid tumor, *e.g.,* a carcinoma, such as an adenocarcinoma, an adrenocortical carcinoma, a colon adenocarcinoma, a colorectal adenocarcinoma, a colorectal carcinoma, a ductal cell carcinoma, a lung carcinoma, a thyroid carcinoma, a nasopharyngeal carcinoma, a melanoma (*e.g*., a malignant melanoma), a non-melanoma skin carcinoma, or an unspecified carcinoma; a desmoid tumor; a desmoplastic small round cell tumor; an endocrine tumor; an Ewing sarcoma; a germ cell tumor (*e.g*., testicular cancer, ovarian cancer, choriocarcinoma, endodermal sinus tumor, germinoma, *etc*.); a hepatosblastoma; a hepatocellular carcinoma; a neuroblastoma; a non-rhabdomyosarcoma soft tissue sarcoma; an osteosarcoma; a retinoblastoma; a rhabdomyosarcoma; or a Wilms tumor. In another embodiment, the solid tumor is pancreatic cancer or breast cancer. In other embodiments, the solid tumor is an acoustic neuroma; an astrocytoma (*e.g*., a grade I pilocytic astrocytoma, a grade II low-grade astrocytoma; a grade III anaplastic astrocytoma; or a grade IV glioblastoma multiforme); a chordoma; a craniopharyngioma; a glioma (*e.g.,* a brain stem glioma; an ependymoma; a mixed glioma; an optic nerve glioma; or a subependymoma); a glioblastoma; a medulloblastoma; a meningioma; a metastatic brain tumor; an oligodendroglioma; a pineoblastoma; a pituitary tumor; a primitive neuroectodermal tumor; or a schwannoma. In another embodiment, the cancer is prostate cancer.

In certain embodiments, the individual having a cancer, for example, a blood cancer or a solid tumor, *e.g.,* an individual having a deficiency of natural killer cells, is an individual that has received a bone marrow transplant before said administering. In certain embodiments, the bone marrow transplant was in treatment of said cancer. In certain other embodiments, the bone marrow transplant was in treatment of a condition other than said cancer. In certain embodiments, the individual received an immunosuppressant in addition to said bone marrow transplant. In certain embodiments, the individual who has had a bone marrow transplant exhibits one or more symptoms of graft-versus-host disease (GVHD) at the time of said administration. In certain other embodiments, the individual who has had a bone marrow transplant is administered said cells before a symptom of graft-versus-host disease (GVHD) has manifested.

In certain specific embodiments, the individual having a cancer, for example, a blood cancer, has received at least one dose of a TNFα inhibitor, e.g., ETANERCEPT® (Enbrel), prior to said administering. In specific embodiments, said individual received said dose of a TNFα inhibitor within 1, 2, 3, 4, 5, 6, 7, 8, 9, 10, 11 or 12 months of diagnosis of said cancer. In a specific embodiment, the individual who has received a dose of a TNFα inhibitor exhibits acute myeloid leukemia. In a more specific embodiment, the individual who has received a dose of a TNFα inhibitor and exhibits acute myeloid leukemia further exhibits deletion of the long arm of chromosome 5 in blood cells. In another embodiment, the individual having a cancer, for example, a blood cancer, exhibits a Philadelphia chromosome.

In certain other embodiments, the cancer, for example, a blood cancer or a solid tumor, in said individual is refractory to one or more anticancer drugs. In a specific embodiment, the cancer is refractory to GLEEVEC® (imatinib mesylate).

In certain embodiments, the cancer, for example, a blood cancer, in said individual responds to at least one anticancer drug; in this embodiment, placental perfusate, isolated placental perfusate cells, isolated natural killer cells, *e.g.,* placental natural killer cells, e.g., placenta-derived intermediate natural killer cells, isolated combined natural killer cells, or TSNK, NK progenitor cells, or NK cells described herein, and/or combinations thereof, and optionally an immunomodulatory compound, are added as adjunct treatments or as a combination therapy with said anticancer drug. In certain other embodiments, the individual having a cancer, for example, a blood cancer, has been treated with at least one anticancer drug, and has relapsed, prior to said administering. In certain embodiments, the individual to be treated has a refractory cancer. In one embodiment, the cancer treatment method with the cells described herein protects against (*e.g.,* prevents or delays) relapse of cancer. In one embodiment, the cancer treatment method described herein results in remission of the cancer for 1 month or more, 2, 3, 4, 5, 6, 7, 8, 9, 10, 11, or 12 months or more, 1 year or more, 2 years or more, 3 years or more, or 4 years or more.

In one embodiment, provided herein is a method of treating an individual having multiple myeloma, comprising administering to the individual (1) lenalidomide; (2) melphalan; and (3) expanded NK cells, wherein said NK cells are effective to treat multiple myeloma in said individual. In a specific embodiment, said NK cells are cord blood NK cells, or NK cells produced from cord blood hematopoietic cells, *e.g.,* hematopoietic stem cells. In another embodiment, said NK cells have been produced by any of the methods described herein for producing NK cells, *e.g.,* for producing TSNK cells. In another embodiment, said NK cells have been expanded prior to said administering. In another embodiment, said lenalidomide, melphalan, and/or NK cells are administered separately from each other. In certain specific embodiments of the method of treating an individual with multiple myeloma, said NK cells are produced by a method comprising: expanding a population of hematopoietic stem or progenitor cells in a first medium comprising one or more of stem cell factor (SCF), IL-2, interleukin-7 (IL-7), interleukin-15 (IL-15) and heparin, and wherein said SCF, IL-2, IL-7 and IL-15 are not comprised within an undefined component of said medium, and wherein a plurality of hematopoietic stem or progenitor cells within said population of hematopoietic stem or progenitor cells differentiate into NK cells during said expanding; and expanding the cells from step (a) in a second medium comprising interleukin-2 (IL-2).

In another embodiment, provided herein is a method of treating an individual having acute myelogenous leukemia (AML), comprising administering to the individual expanded NK cells (optionally activated by pretreatment with IL2 and IL12 and IL18, IL12 and IL15, IL12 and IL18, IL2 and IL12 and IL15 and IL18, or IL2 and IL15 and IL18), wherein said NK cells are effective to treat AML in said individual. In a specific embodiment, said NK cells are cord blood NK cells, or NK cells produced from cord blood hematopoietic cells, *e.g.,* hematopoietic stem cells. In another embodiment, said NK cells have been produced by any of the methods described herein for producing NK cells, *e.g.,* for producing TSNK cells or for producing NK cell populations using a three-stage method. In another embodiment, said NK cells have been expanded prior to said administering. In certain specific embodiments of the method of treating an individual with AML, said NK cells are produced by a two-step method of producing a population of activated natural killer (NK) cells, wherein a first step of said method comprises expanding a population of hematopoietic stem or progenitor cells in a first medium comprising one or more of stem cell factor (SCF), interleukin-7 (IL-7) and interleukin-15 (IL-15), and wherein said SCF, IL-7 and IL-15 are not comprised within an undefined component of said medium, and wherein a plurality of hematopoietic stem or progenitor cells within said population of hematopoietic stem or progenitor cells differentiate into NK cells during said expanding; and wherein a second step of said method comprises expanding the cells from the first step in a second medium comprising interleukin-2 (IL-2), to produce activated NK cells. In certain specific embodiments of the method of treating an individual with AML, said NK cell populations are produced by a three-stage method, as described herein. In a particular embodiment, the AML to be treated by the foregoing methods comprises refractory AML, poor-prognosis AML, or childhood AML. Methods known in the art for administering NK cells for the treatment of refractory AML, poor-prognosis AML, or childhood AML may be adapted for this purpose; see, *e.g.,* Miller et al., 2005, Blood 105:3051-3057; Rubnitz et al., 2010, J Clin Oncol. 28:955-959, each of which is incorporated herein by reference in its entirety.

In other specific embodiments of the method of treating an individual with AML, said NK cells are produced by a method comprising: (a) seeding a population of hematopoietic stem or progenitor cells in a first medium comprising interleukin-15 (IL-15) and, optionally, one or more of stem cell factor (SCF) and interleukin-7 (IL-7), wherein said IL-15 and optional SCF and IL-7 are not comprised within an undefined component of said medium, such that the population expands, and a plurality of hematopoietic stem or progenitor cells within said population of hematopoietic stem or progenitor cells differentiate into NK cells during said expanding; and (b) expanding the cells from step (a) in a second medium comprising interleukin-2 (IL-2), to produce a population of activated NK cells.

In another embodiment, provided herein is a method of treating an individual having chronic lymphocytic leukemia (CLL), comprising administering to the individual a therapeutically effective dose of (1) lenalidomide; (2) melphalan; (3) fludarabine; and (4) expanded NK cells, *e.g.,* TSNK cells, wherein said NK cells are effective to treat said CLL in said individual. In a specific embodiment, said NK cells are cord blood NK cells, or NK cells produced from cord blood hematopoietic stem cells. In another embodiment, said NK cells have been produced by any of the methods described herein for producing NK cells, *e.g.,* for producing TSNK cells. In a specific embodiment of any of the above methods, said NK cells have been expanded for at least 10 days prior to said administering. In a specific embodiment of any of the above methods, said lenalidomide, melphalan, fludarabine, and expanded NK cells are administered to said individual separately. In certain specific embodiments of the method of treating an individual with CLL, said NK cells are produced by a method comprising: expanding a population of hematopoietic stem or progenitor cells in a first medium comprising one or more of stem cell factor (SCF), IL-2, interleukin-7 (IL-7), interleukin-15 (IL-15) and heparin, and wherein said SCF, IL-2, IL-7 and IL-15 are not comprised within an undefined component of said medium, and wherein a plurality of hematopoietic stem or progenitor cells within said population of hematopoietic stem or progenitor cells differentiate into NK cells during said expanding; and expanding the cells from step (a) in a second medium comprising interleukin-2 (IL-2), to produce activated NK cells.

### 5.9.2. Suppression of Tumor Cell Proliferation

Further provided herein is a method of suppressing the proliferation of tumor cells, comprising bringing NK cells produced using the methods described herein, e.g., TSNK cells, or NK cell populations or NK progenitor cell populations produced using the three-stage method described herein, into proximity with the tumor cells, *e.g.,* contacting the tumor cells with NK cells produced using the methods described herein, *e.g.,* TSNK cells, or NK cell populations, or NK progenitor cell populations. Optionally, isolated placental perfusate or isolated placental perfusate cells is brought into proximity with the tumor cells and/or NK cells produced using the methods described herein, e.g., TSNK cells, or NK cell populations, or NK progenitor cell populations. In another specific embodiment, an immunomodulatory compound, *e.g.,* an immunomodulatory compound described above, or thalidomide is additionally brought into proximity with the tumor cells and/or NK cells produced using the methods described herein, e.g., TSNK cells, or NK cell populations, or NK progenitor cell populations, such that proliferation of the tumor cells is detectably reduced compared to tumor cells of the same type not brought into proximity with NK cells produced using the methods described herein, e.g., TSNK cells, or NK cell populations, or NK progenitor cell populations. Optionally, isolated placental perfusate or isolated placental perfusate cells are brought into proximity with the tumor cells and/or NK cells produced using the methods described herein, e.g., TSNK cells, or NK cell populations, or NK progenitor cell populations contacted or brought into proximity with an immunomodulatory compound.

As used herein, in certain embodiments, "contacting," with respect to cells, in one embodiment encompasses direct physical, *e.g.,* cell-cell, contact between placental perfusate, placental perfusate cells, natural killer cells, *e.g.,* TSNK cells, NK cell populations or NK progenitor cell populations produced according to the three-stage method described herein, and/or isolated combined natural killer cells and the tumor cells. In another embodiment, "contacting" encompasses presence in the same physical space, *e.g.,* placental perfusate, placental perfusate cells, natural killer cells, *e.g.,* placental intermediate natural killer cells, natural killer cells described herein, *e.g.,* TSNK cells, NK cell populations or NK progenitor cell populations produced according to the three-stage method described herein, and/or isolated combined natural killer cells are placed in the same container (*e.g*., culture dish, multiwell plate) as tumor cells. In another embodiment, "contacting" placental perfusate, placental perfusate cells, combined natural killer cells, placental intermediate natural killer cells, or natural killer cells described herein, *e.g.,* TSNK cells, NK cell populations or NK progenitor cell populations produced according to the three-stage method described herein, and tumor cells is accomplished, *e.g.,* by injecting or infusing the placental perfusate or cells, *e.g.,* placental perfusate cells, combined natural killer cells or natural killer cells, *e.g.,* placental intermediate natural killer cells into an individual, *e.g.,* a human comprising tumor cells, *e.g.,* a cancer patient. "Contacting," in the context of immunomodulatory compounds and/or thalidomide, means, *e.g.,* that the cells and the immunomodulatory compound and/or thalidomide are directly physically contacted with each other, or are placed within the same physical volume (*e.g*., a cell culture container or an individual).

In a specific embodiment, the tumor cells are blood cancer cells, *e.g.,* leukemia cells or lymphoma cells. In more specific embodiments, the cancer is an acute leukemia, *e.g.,* acute T cell leukemia cells, acute myelogenous leukemia (AML) cells, acute promyelocytic leukemia cells, acute myeloblastic leukemia cells, acute megakaryoblastic leukemia cells, precursor B acute lymphoblastic leukemia cells, precursor T acute lymphoblastic leukemia cells, Burkitt's leukemia (Burkitt's lymphoma) cells, or acute biphenotypic leukemia cells; chronic leukemia cells, *e.g.,* chronic myeloid lymphoma cells, chronic myelogenous leukemia (CML) cells, chronic monocytic leukemia cells, chronic lymphocytic leukemia (CLL)/Small lymphocytic lymphoma cells, or B-cell prolymphocytic leukemia cells; hairy cell lymphoma cells; T-cell prolymphocytic leukemia cells; or lymphoma cells, *e.g.,* histiocytic lymphoma cells, lymphoplasmacytic lymphoma cells (*e.g.,* Waldenstrom macroglobulinemia cells), splenic marginal zone lymphoma cells, plasma cell neoplasm cells (*e.g.,* plasma cell myeloma cells, plasmacytoma cells, monoclonal immunoglobulin deposition disease, or a heavy chain disease), extranodal marginal zone B cell lymphoma (MALT lymphoma) cells, nodal marginal zone B cell lymphoma (NMZL) cells, follicular lymphoma cells, mantle cell lymphoma cells, diffuse large B cell lymphoma cells, mediastinal (thymic) large B cell lymphoma cells, intravascular large B cell lymphoma cells, primary effusion lymphoma cells, T cell large granular lymphocytic leukemia cells, aggressive NK cell leukemia cells, adult T cell leukemia/lymphoma cells, extranodal NK/T cell lymphoma - nasal type cells, enteropathy-type T cell lymphoma cells, hepatosplenic T cell lymphoma cells, blastic NK cell lymphoma cells, mycosis fungoides (Sezary syndrome), primary cutaneous CD30-positive T cell lymphoproliferative disorder (*e.g.,* primary cutaneous anaplastic large cell lymphoma or lymphomatoid papulosis) cells, angioimmunoblastic T cell lymphoma cells, peripheral T cell lymphoma - unspecified cells, anaplastic large cell lymphoma cells, Hodgkin lymphoma cells or nodular lymphocyte-predominant Hodgkin lymphoma cells. In another specific embodiment, the tumor cells are multiple myeloma cells or myelodysplastic syndrome cells.

In specific embodiments, the tumor cells are solid tumor cells, e.g., carcinoma cells, for example, adenocarcinoma cells, adrenocortical carcinoma cells, colon adenocarcinoma cells, colorectal adenocarcinoma cells, colorectal carcinoma cells, ductal cell carcinoma cells, lung carcinoma cells, thyroid carcinoma cells, nasopharyngeal carcinoma cells, melanoma cells (*e.g.,* malignant melanoma cells), non-melanoma skin carcinoma cells, or unspecified carcinoma cells; desmoid tumor cells; desmoplastic small round cell tumor cells; endocrine tumor cells; Ewing sarcoma cells; germ cell tumor cells (*e.g*., testicular cancer cells, ovarian cancer cells, choriocarcinoma cells, endodermal sinus tumor cells, germinoma cells, *etc.*); hepatosblastoma cells; hepatocellular carcinoma cells; neuroblastoma cells; non-rhabdomyosarcoma soft tissue sarcoma cells; osteosarcoma cells; retinoblastoma cells; rhabdomyosarcoma cells; or Wilms tumor cells. In another embodiment, the tumor cells are pancreatic cancer cells or breast cancer cells. In other embodiments, the solid tumor cells are acoustic neuroma cells; astrocytoma cells (*e.g.,* grade I pilocytic astrocytoma cells, grade II low-grade astrocytoma cells; grade III anaplastic astrocytoma cells; or grade IV glioblastoma multiforme cells); chordoma cells; craniopharyngioma cells; glioma cells (*e.g.,* brain stem glioma cells; ependymoma cells; mixed glioma cells; optic nerve glioma cells; or subependymoma cells); glioblastoma cells; medulloblastoma cells; meningioma cells; metastatic brain tumor cells; oligodendroglioma cells; pineoblastoma cells; pituitary tumor cells; primitive neuroectodermal tumor cells; or schwannoma cells. In another embodiment, the tumor cells are prostate cancer cells.

As used herein, "therapeutically beneficial" and "therapeutic benefits" include, but are not limited to, *e.g.,* reduction in the size of a tumor; lessening or cessation of expansion of a tumor; reducing or preventing metastatic disease; reduction in the number of cancer cells in a tissue sample, *e.g.,* a blood sample, per unit volume; the clinical improvement in any symptom of the particular cancer or tumor said individual has, the lessening or cessation of worsening of any symptom of the particular cancer the individual has, *etc.*

### 5.9.3. Treatment of cancers using NK cells, NK Cell Populations, and NK Progenitor Cell Populations and other anticancer agents

Treatment of an individual having cancer using the NK cells produced using the methods described herein, e.g., TSNK cells, or NK cell populations or NK progenitor cell populations produced using the three-stage method described herein, can be part of an anticancer therapy regimen that includes one or more other anticancer agents. In addition or alternatively, treatment of an individual having cancer using the NK cells produced using the methods described herein, e.g., TSNK cells, or NK cell populations, or NK progenitor cell populations described herein, can be used to supplement an anticancer therapy that includes one or more other anticancer agents. Such anticancer agents are well-known in the art. Specific anticancer agents that may be administered to an individual having cancer, *e.g.,* an individual having tumor cells, in addition to the NK cells produced using the methods described herein, e.g., TSNK cells, or NK cell populations, or NK progenitor cell populations, and optionally perfusate, perfusate cells, natural killer cells other than NK cells produced using the methods described herein, e.g., TSNK cells, or NK cell populations, or NK progenitor cell populations, include, but are not limited to: acivicin; aclarubicin; acodazole hydrochloride; acronine; adozelesin; adriamycin; adrucil; aldesleukin; altretamine; ambomycin; ametantrone acetate; amsacrine; anastrozole; anthramycin; asparaginase (e.g., from Erwinia chrysan; Erwinaze); asperlin; avastin (bevacizumab); azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; celecoxib (COX-2 inhibitor); Cerubidine; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflomithine hydrochloride; elsamitrucin; Elspar; enloplatin; enpromate; epipropidine; epirubicin hydrochloride; erbulozole; esorubicin hydrochloride; estramustine; estramustine phosphate sodium; etanidazole; etoposide; etoposide phosphate; Etopophos; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; Idamycin; idarubicin hydrochloride; ifosfamide; ilmofosine; iproplatin; irinotecan; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; Proleukin; Purinethol; puromycin; puromycin hydrochloride; pyrazofurin; Rheumatrex; riboprine; safingol; safingol hydrochloride; semustine; simtrazene; sparfosate sodium; sparsomycin; spirogermanium hydrochloride; spiromustine; spiroplatin; streptonigrin; streptozocin; sulofenur; Tabloid; talisomycin; tecogalan sodium; taxotere; tegafur; teloxantrone hydrochloride; temoporfin; teniposide; teroxirone; testolactone; thiamiprine; thioguanine; thiotepa; tiazofurin; tirapazamine; Toposar; toremifene citrate; trestolone acetate; Trexall; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; and zorubicin hydrochloride.

Other anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptosar (also called Campto; irinotecan) camptothecin derivatives; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidenmin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; doxorubicin; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine (*e.g*., Fludara); fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imatinib (*e.g*., GLEEVEC®), imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide + estrogen + progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mitoguazone; mitolactol; mitomycin analogues; mitonafide; mitotoxin fibroblast growth factor-saporin; mitoxantrone; mofarotene; molgramostim; anti-EGFR antibody (*e.g*., Erbitux (cetuximab)); anti-CD19 antibody; anti-CD20 antibody (*e.g.,* rituximab); anti-disialoganglioside (GD2) antibody (*e.g.,* monoclonal antibody 3F8 or ch14>18); anti-ErbB2 antibody (*e.g*., herceptin); human chorionic gonadotrophin; monophosphoryl lipid A+myobacterium cell wall sk; mopidamol; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; nilutamide; nisamycin; nitric oxide modulators; nitroxide antioxidant; nitrullyn; oblimersen (GENASENSE®); O⁶-benzylguanine; octreotide; okicenone; oligonucleotides; onapristone; ondansetron; ondansetron; oracin; oral cytokine inducer; ormaplatin; osaterone; oxaliplatin (*e.g*., Floxatin); oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stipiamide; stromelysin inhibitors; sulfinosine; superactive vasoactive intestinal peptide antagonist; suradista; suramin; swainsonine; tallimustine; tamoxifen methiodide; tauromustine; tazarotene; tecogalan sodium; tegafur; tellurapyrylium; telomerase inhibitors; temoporfin; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; Vectibix (panitumumab)velaresol; veramine; verdins; verteporfin; vinorelbine; vinxaltine; vitaxin; vorozole; Welcovorin (leucovorin); Xeloda (capecitabine); zanoterone; zeniplatin; zilascorb; and zinostatin stimalamer.

In some embodiments, treatment of an individual having cancer using the NK cells produced using the methods described herein, e.g., TSNK cells, or NK cell populations, or NK progenitor cell populations described herein is part of an anticancer therapy regimen for antibody-dependent cell-mediated cytotoxicity (ADCC). In one embodiment, the ADCC regimen comprises administration of one or more antibodies (*e.g*., an antibody described in the foregoing paragraph) in combination with NK cells produced using the methods described herein, e.g., TSNK cells, or NK cell populations, or NK progenitor cell populations described herein. Several types of cancer can be treated using such ADCC methods, including but not limited to acute lymphoblastic leukemia (ALL) or other B-cell malignancies (lymphomas and leukemias), neuroblastoma, melanoma, breast cancers, and head and neck cancers. In specific embodiments, the ADCC therapy comprises administration of one or more of the following antibodies anti-EGFR antibody (*e.g.,* Erbitux (cetuximab)), anti-CD19 antibody, anti-CD20 antibody (*e.g.,* rituximab), anti-disialoganglioside (GD2) antibody (*e.g*., monoclonal antibody 3F8 or ch14>18), or anti-ErbB2 antibody (*e.g*., herceptin), in combination with NK cells produced using the methods described herein, *e.g*., TSNK cells, or NK cell populations, or NK progenitor cell populations described herein.

### 5.9.4. Treatment of Viral Infection

In another embodiment, provided herein is a method of treating an individual having a viral infection, comprising administering to said individual a therapeutically effective amount of NK cells produced using the methods described herein, e.g., TSNK cells, or NK cell populations or NK progenitor cell populations produced using the three-stage method described herein. In certain embodiments, the individual has a deficiency of natural killer cells, *e.g.,* a deficiency of NK cells active against the individual's viral infection. In certain specific embodiments, said administering additionally comprises administering to the individual one or more of isolated placental perfusate, isolated placental perfusate cells, isolated natural killer cells, *e.g.,* placental natural killer cells, *e.g.,* placenta-derived intermediate natural killer cells, isolated combined natural killer cells, and/or combinations thereof. In certain specific embodiments, the NK cells produced using the methods described herein, e.g., TSNK cells, or NK cell populations, or NK progenitor cell populations are contacted or brought into proximity with an immunomodulatory compound, *e.g.,* an immunomodulatory compound above, or thalidomide, prior to said administration. In certain other specific embodiments, said administering comprises administering an immunomodulatory compound, *e.g.,* an immunomodulatory compound described above, or thalidomide, to said individual in addition to said NK cells produced using the methods described herein, e.g., TSNK cells, or NK cell populations, or NK progenitor cell populations, wherein said amount is an amount that, *e.g.,* results in a detectable improvement of, lessening of the progression of, or elimination of, one or more symptoms of said viral infection. In specific embodiments, the viral infection is an infection by a virus of the Adenoviridae, Picornaviridae, Herpesviridae, Hepadnaviridae, Flaviviridae, Retroviridae, Orthomyxoviridae, Paramyxoviridae, Papilommaviridae, Rhabdoviridae, or Togaviridae family. In more specific embodiments, said virus is human immunodeficiency virus (HIV).coxsackievirus, hepatitis A virus (HAV), poliovirus, Epstein-Barr virus (EBV), herpes simplex type 1 (HSV1), herpes simplex type 2 (HSV2), human cytomegalovirus (CMV), human herpesvirus type 8 (HHV8), herpes zoster virus (varicella zoster virus (VZV) or shingles virus), hepatitis B virus (HBV), hepatitis C virus (HCV), hepatitis D virus (HDV), hepatitis E virus (HEV), influenza virus (*e.g*., influenza A virus, influenza B virus, influenza C virus, or thogotovirus), measles virus, mumps virus, parainfluenza virus, papillomavirus, rabies virus, or rubella virus.

In other more specific embodiments, said virus is adenovirus species A, serotype 12, 18, or 31; adenovirus species B, serotype 3, 7, 11, 14, 16, 34, 35, or 50; adenovirus species C, serotype 1, 2, 5, or 6; species D, serotype 8, 9, 10, 13, 15, 17, 19, 20, 22, 23, 24, 25, 26, 27, 28, 29, 30, 32, 33, 36, 37, 38, 39, 42, 43, 44, 45, 46, 47, 48, 49, or 51; species E, serotype 4; or species F, serotype 40 or 41.

In certain other more specific embodiments, the virus is Apoi virus (APOIV), Aroa virus (AROAV), bagaza virus (BAGV), Banzi virus (BANV), Bouboui virus (BOUV), Cacipacore virus (CPCV), Carey Island virus (CIV), Cowbone Ridge virus (CRV), Dengue virus (DENV), Edge Hill virus (EHV), Gadgets Gully virus (GGYV), Ilheus virus (ILHV), Israel turkey meningoencephalomyelitis virus (ITV), Japanese encephalitis virus (JEV), Jugra virus (JUGV), Jutiapa virus (JUTV), kadam virus (KADV), Kedougou virus (KEDV), Kokobera virus (KOKV), Koutango virus (KOUV), Kyasanur Forest disease virus (KFDV), Langat virus (LGTV), Meaban virus (MEAV), Modoc virus (MODV), Montana myotis leukoencephalitis virus (MMLV), Murray Valley encephalitis virus (MVEV), Ntaya virus (NTAV), Omsk hemorrhagic fever virus (OHFV), Powassan virus (POWV), Rio Bravo virus (RBV), Royal Farm virus (RFV), Saboya virus (SABV), St. Louis encephalitis virus (SLEV), Sal Vieja virus (SVV), San Perlita virus (SPV), Saumarez Reef virus (SREV), Sepik virus (SEPV), Tembusu virus (TMUV), tick-borne encephalitis virus (TBEV), Tyuleniy virus (TYUV), Uganda S virus (UGSV), Usutu virus (USUV), Wesselsbron virus (WESSV), West Nile virus (WNV), Yaounde virus (YAOV), Yellow fever virus (YFV), Yokose virus (YOKV), or Zika virus (ZIKV).

In other embodiments, the NK cells produced using the methods described herein, e.g., TSNK cells, or NK cell populations, or NK progenitor cell populations, and optionally placental perfusate and/or perfusate cells, are administered to an individual having a viral infection as part of an antiviral therapy regimen that includes one or more other antiviral agents. Specific antiviral agents that may be administered to an individual having a viral infection include, but are not limited to: imiquimod, podofilox, podophyllin, interferon alpha (IFNα), reticolos, nonoxynol-9, acyclovir, famciclovir, valaciclovir, ganciclovir, cidofovir; amantadine, rimantadine; ribavirin; zanamavir and oseltaumavir; protease inhibitors such as indinavir, nelfinavir, ritonavir, or saquinavir; nucleoside reverse transcriptase inhibitors such as didanosine, lamivudine, stavudine, zalcitabine, or zidovudine; and non-nucleoside reverse transcriptase inhibitors such as nevirapine, or efavirenz.

### 5.9.5. Administration

Determination of the number of cells, *e.g.,* placental perfusate cells, *e.g.,* nucleated cells from placental perfusate, combined natural killer cells, and/or isolated natural killer cells, *e.g.,* TSNK cells, or NK cell populations or NK progenitor cell populations produced using the three-stage method described herein, and determination of the amount of an immunomodulatory compound, *e.g.,* an immunomodulatory compound, or thalidomide, can be performed independently of each other.

### 5.9.5.1. Administration of Cells

In certain embodiments, NK cells produced using the methods described herein, e.g., TSNK cells, or NK cell populations or NK progenitor cell populations produced using the three-stage method described herein, are used, *e.g.,* administered to an individual, in any amount or number that results in a detectable therapeutic benefit to the individual, *e.g.,* an effective amount, wherein the individual has a viral infection, cancer, or tumor cells, for example, an individual having tumor cells, a solid tumor or a blood cancer, *e.g.,* a cancer patient. Such cells can be administered to such an individual by absolute numbers of cells, *e.g.,* said individual can be administered at about, at least about, or at most about, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, or 1 x 10¹¹ NK cells produced using the methods described herein, e.g., TSNK cells, or NK cell populations, or NK progenitor cell populations. In other embodiments, NK cells produced using the methods described herein, e.g., TSNK cells, or NK cell populations, or NK progenitor cell populations can be administered to such an individual by relative numbers of cells, *e.g.,* said individual can be administered at about, at least about, or at most about, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, or 1 x 10¹¹ NK cells produced using the methods described herein, e.g., TSNK cells, or NK cell populations, or NK progenitor cell populations per kilogram of the individual. In other embodiments, NK cells produced using the methods described herein, e.g., TSNK cells, or NK cell populations, or NK progenitor cell populations can be administered to such an individual by relative numbers of cells, *e.g.,* said individual can be administered at about, at least about, or at most about, 1 x 10⁵, 5 x 10⁵, 1 x 10⁶, 5 x 10⁶, 1 x 10⁷, 5 x 10⁷, 1 x 10⁸, or 5 x 10⁸ NK cells produced using the methods described herein, e.g., TSNK cells, or NK cell populations, or NK progenitor cell populations per kilogram of the individual. NK cells produced using the methods described herein, e.g., TSNK cells, or NK cell populations, or NK progenitor cell populations can be administered to such an individual according to an approximate ratio between a number of NK cells produced using the methods described herein, e.g., TSNK cells, or NK cell populations, or NK progenitor cell populations, and optionally placental perfusate cells and/or natural killer cells other than NK cells produced using the methods described herein, e.g., TSNK cells, or NK cell populations, or NK progenitor cell populations, and a number of tumor cells in said individual (*e.g*., an estimated number). For example, NK cells produced using the methods described herein, e.g., TSNK cells, or NK cell populations, or NK progenitor cell populations can be administered to said individual in a ratio of about, at least about or at most about 1:1, 1:1, 3:1, 4:1, 5:1, 6:1, 7:1, 8:1, 9:1, 10:1, 15:1, 20:1, 25:1, 30:1, 35:1, 40:1, 45:1, 50:1, 55:1, 60:1, 65:1, 70:1, 75:1, 80:1, 85:1, 90:1, 95:1 or 100:1 to the number of tumor cells in the individual. The number of tumor cells in such an individual can be estimated, *e.g.,* by counting the number of tumor cells in a sample of tissue from the individual, *e.g.,* blood sample, biopsy, or the like. In specific embodiments, *e.g.,* for solid tumors, said counting is performed in combination with imaging of the tumor or tumors to obtain an approximate tumor volume. In a specific embodiment, an immunomodulatory compound or thalidomide, *e.g.,* an effective amount of an immunomodulatory compound or thalidomide, are administered to the individual in addition to the NK cells produced using the methods described herein, e.g., TSNK cells, or NK cell populations, or NK progenitor cell populations, optionally placental perfusate cells and/or natural killer cells other than NK cells produced using the methods described herein, e.g., TSNK cells, or NK cell populations, or NK progenitor cell populations.

In certain embodiments, the method of suppressing the proliferation of tumor cells, *e.g.,* in an individual; treatment of an individual having a deficiency in the individual's natural killer cells; or treatment of an individual having a viral infection; or treatment of an individual having cancer, *e.g.,* an individual having tumor cells, a blood cancer or a solid tumor, comprises bringing the tumor cells into proximity with, or administering to said individual, a combination of NK cells produced using the methods described herein, e.g., TSNK cells, or NK cell populations, or NK progenitor cell populations and one or more of placental perfusate and/or placental perfusate cells. In specific embodiments, the method additionally comprises bringing the tumor cells into proximity with, or administering to the individual, an immunomodulatory compound or thalidomide.

In a specific embodiment, for example, treatment of an individual having a deficiency in the individual's natural killer cells (*e.g.,* a deficiency in the number of NK cells or in the NK cells' reactivity to a cancer, tumor or virally-infected cells); or treatment of an individual having a cancer or a viral infection, or suppression of tumor cell proliferation, comprises bringing said tumor cells into proximity with, or administering to said individual, NK cells produced using the methods described herein, e.g., TSNK cells, or NK cell populations, or NK progenitor cell populations supplemented with isolated placental perfusate cells or placental perfusate. In specific embodiments, about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more NK cells produced using the methods described herein, e.g., TSNK cells, or NK cell populations, or NK progenitor cell populations per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more NK cells produced using the methods described herein, e.g., TSNK cells, or NK cell populations, or NK progenitor cell populations, are supplemented with about, or at least about, 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more isolated placental perfusate cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more isolated placental perfusate cells. In other more specific embodiments, about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more NK cells produced using the methods described herein, e.g., TSNK cells, or NK cell populations, or NK progenitor cell populations per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more NK cells produced using the methods described herein, e.g., TSNK cells, or NK cell populations, or NK progenitor cell populations are supplemented with about, or at least about, 1, 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, 500, 550, 600, 650, 700, 750, 800, 850, 900, 950 or 1000 mL of perfusate, or about 1 unit of perfusate.

In another specific embodiment, treatment of an individual having a deficiency in the individual's natural killer cells; treatment of an individual having cancer; treatment of an individual having a viral infection; or suppression of tumor cell proliferation, comprises bringing the tumor cells into proximity with, or administering to the individual, NK cells produced using the methods described herein, e.g., TSNK cells, or NK cell populations, or NK progenitor cell populations, wherein said cells are supplemented with adherent placental cells, e.g., adherent placental stem cells or multipotent cells, *e.g.,* CD34⁻, CD10⁺, CD105⁺, CD200⁺ tissue culture plastic-adherent placental cells. In specific embodiments, the NK cells produced using the methods described herein, e.g., TSNK cells, or NK cell populations, or NK progenitor cell populations are supplemented with about 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸ or more adherent placental stem cells per milliliter, or 1 x 10⁴, 5 x 10⁴, 1 x 10⁵, 5 x 10⁵, 1 x 10⁶, 5 x 10⁶, 1 x 10⁷, 5 x 10⁷, 1 x 10⁸, 5 x 10⁸, 1 x 10⁹, 5 x 10⁹, 1 x 10¹⁰, 5 x 10¹⁰, 1 x 10¹¹ or more adherent placental cells, e.g., adherent placental stem cells or multipotent cells.

In another specific embodiment, treatment of an individual having a deficiency in the individual's natural killer cells; treatment of an individual having cancer; treatment of an individual having a viral infection; or suppression of tumor cell proliferation, is performed using an immunomodulatory compound or thalidomide in combination with NK cells produced using the methods described herein, e.g., TSNK cells, or NK cell populations, or NK progenitor cell populations, wherein said cells are supplemented with conditioned medium, e.g., medium conditioned by CD34⁻, CD10⁺, CD105⁺, CD200⁺ tissue culture plastic-adherent placental cells, e.g., 0.1, 0.2, 0.3, 0.4, 0.5, 0.6, 0.1, 0.8, 0.9, 1, 2, 3, 4, 5, 6, 7, 8, 9, 10 mL of stem cell-conditioned culture medium per unit of perfusate, or per 10⁴, 10⁵, 10⁶, 10⁷, 10⁸, 10⁹, 10¹⁰, or 10¹¹ NK cells produced using the methods described herein, e.g., TSNK cells, or NK cell populations, or NK progenitor cell populations. In certain embodiments, the tissue culture plastic-adherent placental cells are the multipotent adherent placental cells described in U.S. Patent No. 7,468,276 and U.S. Patent Application Publication No. 2007/0275362, the disclosures of which are incorporated herein by reference in their entireties. In another specific embodiment, the method additionally comprises bringing the tumor cells into proximity with, or administering to the individual, an immunomodulatory compound or thalidomide.

In another specific embodiment, treatment of an individual having a deficiency in the individual's natural killer cells; treatment of an individual having cancer; treatment of an individual having a viral infection; or suppression of tumor cell proliferation, in which said NK cells produced using the methods described herein, e.g., TSNK cells, or NK cell populations, or NK progenitor cell populations are supplemented with placental perfusate cells, the perfusate cells are brought into proximity with interleukin-2 (IL-2) for a period of time prior to said bringing into proximity. In certain embodiments, said period of time is about, at least, or at most 1, 2, 3, 4, 5, 6, 7, 8, 9, 10, 12, 14, 16, 18, 20, 22, 24, 26, 28, 30, 32, 34, 36, 38, 40, 42, 44, 46 or 48 hours prior to said bringing into proximity.

The NK cells produced using the methods described herein, e.g., TSNK cells, or NK cell populations, or NK progenitor cell populations, and optionally perfusate or perfusate cells, can be administered once to an individual having a viral infection, an individual having cancer, or an individual having tumor cells, during a course of anticancer therapy; or can be administered multiple times, e.g., once every 1, 2, 3, 4, 5, 6, 7, 8, 9, 10, 11, 12, 13, 14, 15, 16, 17, 18, 19, 20, 21, 22 or 23 hours, or once every 1, 2, 3, 4, 5, 6 or 7 days, or once every 1, 2, 3, 4, 5, 6, 7, 8, 9, 10, 24, 36 or more weeks during therapy. In embodiments in which cells and an immunomodulatory compound or thalidomide are used, the immunomodulatory compound or thalidomide, and cells or perfusate, can be administered to the individual together, *e.g.,* in the same formulation; separately, *e.g.,* in separate formulations, at approximately the same time; or can be administered separately, *e.g.,* on different dosing schedules or at different times of the day. Similarly, in embodiments in which cells and an antiviral compound or anticancer compound are used, the antiviral compound or anticancer compound, and cells or perfusate, can be administered to the individual together, *e.g.,* in the same formulation; separately, *e.g.,* in separate formulations, at approximately the same time; or can be administered separately, *e.g.,* on different dosing schedules or at different times of the day. The NK cells produced using the methods described herein, e.g., TSNK cells, or NK cell populations, or NK progenitor cell populations, and perfusate or perfusate cells, can be administered without regard to whether NK cells produced using the methods described herein, e.g., TSNK cells, or NK cell populations, or NK progenitor cell populations, perfusate, or perfusate cells have been administered to the individual in the past.

### 6. KITS

Provided herein is a pharmaceutical pack or kit comprising one or more containers filled with one or more of the compositions described herein, e.g., a composition comprising NK cells produced by a method described herein, e.g., TSNK cells, or NK cell populations or NK progenitor cell populations produced using the three-stage method described herein. Optionally associated with such container(s) can be a notice in the form prescribed by a governmental agency regulating the manufacture, use or sale of pharmaceuticals or biological products, which notice reflects approval by the agency of manufacture, use or sale for human administration.

The kits encompassed herein can be used in accordance with the methods described herein, e.g., methods of suppressing the growth of tumor cells and/or methods of treating cancer, e.g., hematologic cancer, and/or methods of treating viral infection. In one embodiment, a kit comprises NK cells produced by a method described herein, e.g., TSNK cells, or NK cell populations, or NK progenitor cell populations, or a composition thereof, in one or more containers. In a specific embodiment, provided herein is a kit comprising TSNK cells, or a composition thereof. In another specific embodiment, provided herein is a kit comprising an NK cell population produced by a three-stage method described herein, or a composition thereof. In another specific embodiment, provided herein is a kit comprising an NK progenitor cell population or a composition thereof. In specific embodiments, said NK progenitor cell population in said kit are produced by a method described herein, e.g., a three-stage method described herein.

### 7. EXAMPLES

### 7.1. Example 1: Recovery of Hematopoietic Stem Cells from Human Placental Perfusate and Umbilical Cord Blood

Human placental perfusate (HPP) and umbilical cord blood (UCB) cells were generally purified using either Ficoll or ammonium chloride to obtain total nucleated cells (TNCs). TNCs were then used in a positive selection procedure to isolate CD34⁺ cells using anti-CD34 beads and RoboSep according to the manufacturer's protocol (StemCell Technologies, Inc.). In this experiment, CD34⁺ cells were isolated with greater than 90% purity. Alternatively, EasySep® Human Progenitor Cell Enrichment Kit (StemCell Technologies, Inc.) was used in a negative selection procedure to deplete the lineage committed cells by using Human Progenitor Cell Enrichment Cocktail with monoclonal antibodies to the following human cell surface antigens: CD2, CD3, CD11b, CD11c, CD14, CD16, CD19, CD24, CD56, CD66b and Glycophorin A. Using the negative selection, 90% CD34⁺ cells were recovered from the raw materials. The cell composition of the recovered HSCs was summarized in Table 1.

**Table 1. Cell composition of enriched Hematopoietic Stem Cells (HSCs). Standard deviation was calculated for population means for 3 donors.**

| | Mean% | STDEV |
|---|---|---|
| Lin-CD34⁺ | 75.1 | 6.2 |
| Lin-CD34⁻CD38⁻ | 9.8 | 2.4 |
| Lin-CD34⁻CD133⁺ | 0.9 | 0.2 |
| Lin-CD34⁻CD117⁺ | 7.2 | 0.5 |

### 7.2. Example 2: Feeder Cell-Free Expansion and Differentiation of Hematopoietic Stem Cells into Natural Killer Cells

CD34⁺ cells were cultured in the following medium formulations for up to 48 days, and aliquots of cells were taken for assessment of cell count, cell viability, characterization of natural killer cell differentiation and functional evaluation.

NK1 medium: GBGM (Glycostem Based Growth Medium, Glycostem Cat# CCT-SCB500, Clear cell technology) supplemented with pen/strep (Cat# 15140, Gibco), 20 ng/mL SCF (Cat# 255-SC, R&D Systems), 10 ng/mL Flt-3 ligand (Cat# 308-FK, R&D system), 20 ng/mL TPO (Cat# 288-TP, R&D system), 20 ng/mL IL-7 (Cat# 207-IL, R&D Systems), 200 IU/mL IL-2 (Cat# 202-IL, R&D Systems) and 10 ng/mL IL-15 (Cat# 247-IL, R&D Systems).

NK2 medium: DMEM (Cat# MT-10-013-CV, Fisher):Ham's F12 (Cat# BW12-615F, Fisher) as 1:2 supplemented with 2 mM L-Glutamine (Cat# 25030, Invitrogen), 1% pen/strep, 20% human serum AB (Cat# 100-512, Gemcell), 5 ng/mL sodium selenite (Cat# S9133, Sigma), 50 µM ethanolamine (Cat# E0135, Sigma), 25 µM β-mercaptoethanol (Cat# 21985, Invitrogen), 20 mg/mL ascorbic acid (Cat# 47863, Sigma), 5 ng/mL IL-3 (Cat# 203-IL, R&D Systems), 20 ng/mL SCF, 10 ng/mL Flt-3 ligand, 20 ng/mL IL-7 and 10 ng/mL IL-15.

NK3 medium: X-vivo 20 (Cat# BW04-448Q, Fisher) supplemented with pen/strep, 10% human serum AB (Cat# 100-512, Gemcell) and 500 IU/mL IL-2.

NK2A medium: GBGM supplemented with 10% human serum AB, 1% pen/strep, 20 ng/mL SCF, 10 ng/mL Flt-3 ligand, 20 ng/mL TPO, 20 ng/mL IL-7, 200 IU/mL IL-2, 10 ng/mL IL-15 and 1.5 IU/mL heparin (Cat# H3149, Sigma).

NK2B1 medium: DMEM:Ham's F12 as 1:2 supplemented with 2 mM L-glutamine, 1% pen/strep, 20% human serum AB, 5 ng/mL sodium selenite, 50 µM ethanolamine, 25 µM β-mercaptoethanol, 20 µg/mL ascorbic acid, 5 ng/mL IL-3, 20 ng/mL SCF, 10 ng/mL Flt-3 ligand, 20 ng/mL IL-7 and 10 ng/mL IL-15.

NK2B2 medium: DMEM:Ham's F12 as 1:2 supplemented with 2mM L-glutamine, 1% pen/strep, 20% human serum AB, 5 ng/mL sodium selenite, 50 µM ethanolamine, 25 µM β-mercaptoethanol, 20 µg/mL ascorbic acid, 200 IU/mL IL-2, 20 ng/mL SCF, 10 ng/mL Flt-3 ligand, 20 ng/mL IL-7 and 10 ng/mL IL-15.

NK2C medium: RPMI 1640 (Cat# 22400105, Invitrogen) supplemented with 10% FBS (Cat# SH30070.03, Hyclone), 2 mM L-glutamine, 1% pen/strep, 50 ng/mL SCF, 50 ng/mL Flt-3 ligand, 100 IU/mL IL-2, 20 ng/mL IL-7 and 20 ng/mL IL-15.

NK2D medium: serum-free medium (StemSpan, Cat# 09650, Stem Cell Technologies, Vancouver, Canada) supplemented with 1 µM synthetic glucocorticoid dexamethosone (Dex, Cat# D4902, Sigma, St Louis, MO), 40 ng/mL insulin-like growth factor 1 (IGF-1, Cat# 291-G1-250, R&D Systems, Minneapolis, MN), 100 ng/mL SCF, 40 µg/mL lipids (cholesterol-rich lipid mix; Cat# C7305-1G, Sigma, St Louis, MO), 5 ng/mL IL-3, 200 IU/mL IL-2, 20 ng/mL IL-7 and 20 ng/mL IL-15.

Cells collected at different time points were washed two times with RPMI1640 (phenol free) and 5% FBS, labeled with fluorescence-conjugated antibodies (Tables 2 and 3) for 15 min at 4 °C, and analyzed by flow cytometry (FACSCanto, BD) and FlowJo cytometry software (Tree Star).

**Table 2. Antibodies used for cell labeling**

| **HSC-NK FACS antibody** | | |
|---|---|---|
| **Item** | **Vendor** | **Cat No.** |
| FITC anti-hu CD3 | BD | 555332 |
| APC-Cy7 anti-hu CD3 | BD | 557832 |
| APC anti-hu CD5 | BD | 555355 |
| PE anti-hu CD7 | BD | 555361 |
| FITC anti-hu CD16 | BD | 555406 |
| PE-Cy5 anti-hu CD16 | BD | 555408 |
| PE anti-hu CD56 | BD | 555516 |
| PE-CY5 CD56 (N-CAM) | BD | 555517 |
| PE CD94 | R&D | FAB-1058P |
| APC anti-hu CD117 | BD | 550412 |
| PE anti-hu CD226 | BD | 559789 |
| Isotype FITC mouse IgG1 | BD | 340755 |
| Isotype PE mouse IgG1 | BD | 340761 |
| Isotype PerCP mouse IgG1 | BD | 340762 |
| Isotype PE-CY7 mouse IgG1 | BD | 348798 |
| Isotype APC mouse IgG1 | BD | 340754 |
| Isotype APC-CY7 mouse IgG1 | BD | 348802 |
| Isotype APC mouse IgG2a | BD | 555576 |
| PE anti-hu KIR-NKAT2 (2DL3) | BD | 556071 |
| PE NKB1(3DL1) | BD | 555967 |
| APC NKG2D | BD | 558071 |
| APC NKp46 | BD | 558051 |
| Simply Cellular Compensation beads | Bangs Labs | 550 |

**Table 3. Panel of flow cytometric characterization of NK surface receptors**

| | FITC | PE | PerCP | APC | APC-Cy7 |
|---|---|---|---|---|---|
| Blank | | | | | |
| Isotype | | | | | |
| Panel 1 | CD3 | CD56 | CD16 | | |
| Panel 2 | CD16 | NKB1 | CD56 | NKG2D | CD3 |
| Panel 3 | CD16 | NKAT2 | CD56 | NKp46 | CD3 |
| Panel 4 | CD16 | CD94 | CD56 | CD117 | CD3 |
| Panel 5 | CD16 | CD226 | CD56 | | CD3 |
| Panel 6 | CD16 | CD7 | CD56 | CD5 | CD3 |

*Cytotoxicity assay using PKH26*/*TO-PRO-3 labeling.* The target tumor cells were labeled with PKH26 (Cat#PKH26GL, Sigma-Aldrich), a dye that inserts into cell plasma membrane via its lipophilic aliphatic residue, then placed in 96-well U-bottom tissue culture plates and incubated with expanded NK cells at various effector-target (E:T) ratios in 200µl RPMI 1640 supplemented with 10% FBS. Cultures were incubated for 4 hours at 37 °C in 5% CO₂. After incubation, cells were harvested and TO-PRO-3 (Invitrogen Cat# T3605), a membrane-impermeable DNA stain, was added to cultures (1 µM final concentration) followed by FACS analysis. Cytotoxicity was expressed as the percentage of dead cells (PKH26+TO-PRO-3+) within the total PKH26+ target tumor cells.

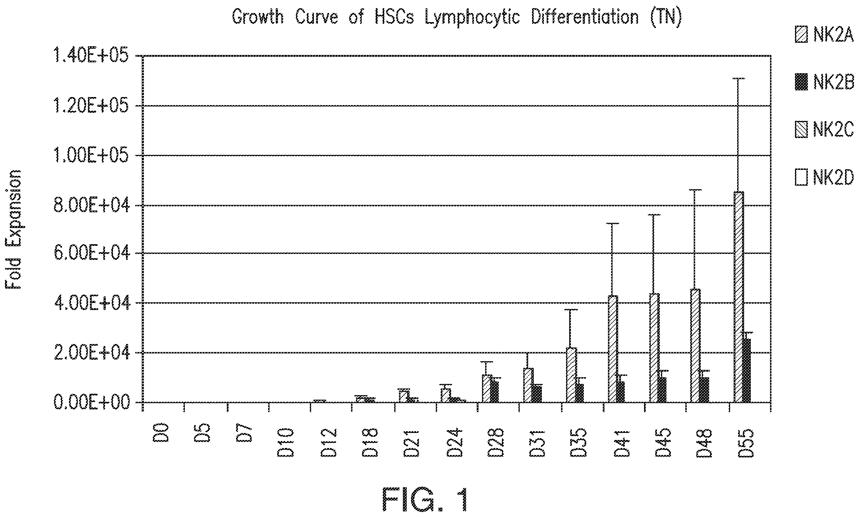

*Optimization of CD34*⁺ *cell expansion and differentiation into NK cells.* Among the medium formulations tested, NK1, NK2 and NK3, NK1 medium showed a 500-fold expansion on Day 21 (D21). Neither NK2 nor NK3 medium maintained either cell proliferation or differentiation. Further medium optimization was performed for NK1 medium and the subsequent media were named NK2A, NK2B, NK2C and NK2D. CD34⁺ cells cultured with NK2A medium showed a 10⁵-fold expansion on Day 55 (D55). Based on the results from fold expansion, differentiation, and cytotoxicity from NK1, 2 and 3 medium, a second batch of NK2A, NK2B, NK2C and NK2D medium formulations were performed and on D55, a 10⁵-fold expansion was achieved (as shown in FIG 1). NK2B medium showed an approximately 3 x 10⁴-fold expansion. Culture in NK2C medium resulted in 3 x 10²-fold expansion in 21 days, followed by declined cell viability. NK2D medium did not maintain cells through the duration of the experiment.

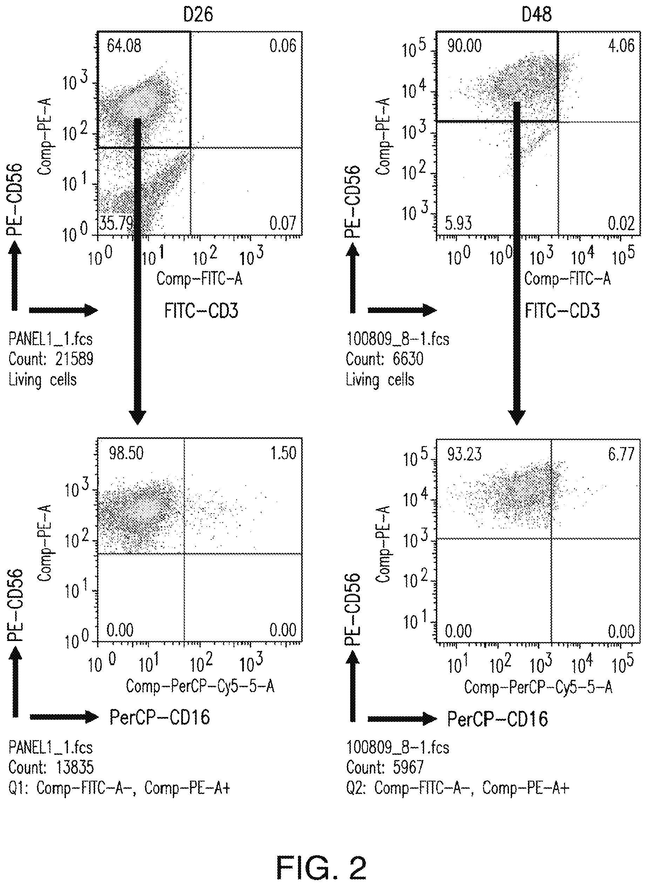

On Day 48 (D48), around 90% of the NK cells in NK2A medium were CD56⁺CD3⁻. Within the CD56⁺CD3⁻ population, over 98% of cells were CD56⁺CD16⁻ (as shown in FIG 2), while 58% expressed the activating receptor NKG2D, 68% were NKp46⁺ and 17% were CD226⁺ (as shown in Tables 4A and 4B).

**Table 4A, 4B. Phenotypic characterization of expanded NK cells on D48**

| 4A: D48 Phenotype | | | | | | | | | | | |
|---|---|---|---|---|---|---|---|---|---|---|---|
| NK2A | | | CD56+CD3- | | CD56+ CD16- | | CD56+ CD16+ | NKB1 | NKG2D | | NKAT2 |
| | Ave. | | 86.84% | | 98.44% | | 1.56% | 3.38% | 58.41% | | 1.69% |
| | STD | | 4.50% | | 0.30% | | 0.30% | 1.42% | 5.88% | | 0.22% |
| | | | | | | | | | | | |

| 4B: D48 Phenotype: CD56+CD3- | | | | | | | | | | | |
|---|---|---|---|---|---|---|---|---|---|---|---|
| NKp46 | | CD94 | | CD117 | | CD226 | | CD7 | | CD5 | |
| 67.92% | | 38.08% | | 80.53% | | 17.32% | | 34.43% | | 53.53% | |
| 5.43% | | 21.14% | | 14.27% | | 14.26% | | 8.48% | | 3.00% | |

Additionally, 97.8% of cells cultured in NK2A medium and 93.1% of cells cultured in NK2B medium were CD56⁺CD16⁻ at day 21 of cultivation.

### 7.3. Example 3: Culture of NK Cells In CNK Medium Enhances Expansion And Cytotoxicity Of NK Cells

On Day 27 (D27), CD34⁺ cells cultured in NK2A medium were further cultured in one of the following media:
- Two-stage Medium, which comprises CNK Medium and Maintenance Medium. CNK Medium is IMDM (Invitrogen) supplemented with 10% FCS (Hyclone), 200 IU/mL IL-2 (R&D Systems), 35 µg/mL transferrin (Sigma-Aldrich), 5 µg/mL insulin (Sigma-Aldrich), 2 x 10⁻⁵M ethanolamine (Sigma-Aldrich), 1 µg/mL oleic acid (Sigma-Aldrich), 1 µg/mL linoleic acid (Sigma-Aldrich), 0.2 µg/mL palmitic acid (Sigma-Aldrich), 2.5 µg/mL BSA (Sigma-Aldrich) and 0.1 µg/mL phytohemagglutinin (PHA-P, Sigma-Aldrich). CD56⁺CD3⁻ NK cells cultured in NK2A medium were resuspended at 2.5x10⁵ live cells/mL in CNK Medium in cell culture treated 24-well plates or T flasks. Mitomycin C-treated allogeneic PBMC and K562 cells (chronic myelogenous leukemia cell line) were both added to the CNK Medium as feeder cells, to a final concentration of 1 x 10⁶ per mL. NK cells were cultured for 5-6 days at 37 °C in 5% CO₂. After 5-6 days and then every 3-4 days an equal volume of Maintenance Medium (IMDM with 10% FCS, 2% Human AB serum, antibiotics, L-glutamine and 400 units of IL-2 per mL) was added to the culture.
- NK2A (PDAC) medium with mitomycin C treated CD34⁻, CD10⁺, CD105⁺, CD200⁺ tissue culture plastic-adherent placental stem cells as feeder cells;
- NK2A (MSC) medium with mitomycin C treated mesenchymal stem cell (MSC) as feeder cells; or
- Feeder-free NK2A (FF) medium as the control.

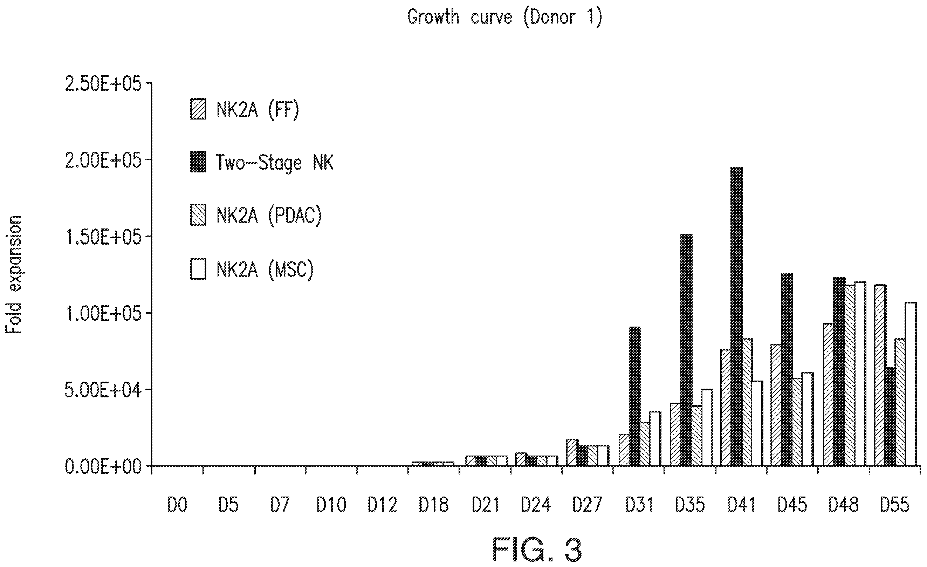

Two-stage medium enhanced the fold-expansion of the CD34⁺ cells compared to NK2A (FF), NK2A (PDAC) and NK2A (MSC), particularly between Day 27 (D27) and Day 48 (D48). *See* Figure 3.

By Day 35, the proportion of CD34⁺ cells had already decreased to approximately 4% while the proportion of CD56⁺CD3⁻ had increased to approximately 80% in the Two-stage medium. On Day 45 (D45), cells cultured in Two-stage medium showed highest cytotoxicity as compared to NK cells cultured in NK2A (FF), NK2A (PDAC) and NK2A (MSC) (as shown in Figure 3). Phenotypic characterization at Day 41 (as shown in Figure 5) showed increased expression of NKp46 and CD226 in the cells, indicating a possible explanation for the enhancement of cytotoxicity. At D41 the proportion of CD226⁺ cells increased from 0.9% ± 0.8% in NK2A medium to 13% ± 4% in Two-stage medium; the proportion of NKp46⁺ cells increased from 55.4% ± 8.7% in NK2A medium to 80% ± 7.85% in Two-Stage medium. At D48 the proportion of CD226+ cells increased from 17.3% ± 14.3% in NK2A medium to 52.3% ± 11.64% in Two-Stage medium; the proportion of NKp46⁺ cells increased from 67.9% ± 5.4% in NK2A medium to 86% ± 4% in Two-Stage medium. There was no significant difference of NKG2D expression among the conditions tested. Changes in expression of CD226 and NKp46 are shown in Table 5, below.

**Table 5. Expression of CD226 and NKp46 on NK cells cultured in NK2A (FF) and Two-Stage medium at day 41 (D41) and day 48 (D48). Standard deviation was calculated for 3 donors.**

| D41 | | NK2A (FF) | Two-Stage |
|---|---|---|---|
| CD226% | Average | 0.9% | 13% |
| | STDEV | 0.8% | 4% |
| NKp46% | Average | 55.4% | 80% |
| | STDEV | 8.7% | 7.85% |
| D48 | | NK2A (FF) | Two-Stage |
| CD226% | Average | 17.3% | 52.3% |
| | STDEV | 14.3% | 11.6% |
| NKp46% | Average | 67.9% | 86% |
| | STDEV | 5.4% | 4% |

### 7.4. Example 4: Comparison of Two-Stage Medium-Cultivated Natural Killer Cells and Natural Killer Cells Derived from Embryonic Stem Cells (ESCs)

NK cells cultured in the Two-stage medium were compared with NK cells derived from embryonic stem cells (ESCs), which were produced by the method of Woll et al., Blood 113(4):6094-6101 (2009). Specifically, a difference in expression levels of CD94 and CD117 was observed during the process of cultivation of both of the cell types. Figure 6 shows that the expression of CD117 was high in the Two-stage NK cells, or "+", from Day 7 (D7) to Day 35 (D35), while the expression of CD94 gradually increased. On Day 35 (D35), about 44% of the CD56⁺CD3⁻ (Two-step) cells were CD94⁺CD117⁺ cells, 37.6% of the CD56⁺CD3⁻ cells were CD94⁻CD117⁺ and 14.7% of the CD56⁺CD3⁻ cells were CD94⁺CD117⁻. As such, the NK cells produced by the Two-step method are distinguishable from the NK cells derived from ESCs, 78% of which remained CD117^{low/-} from Day 14 to Day 35 of the cultivation. This difference in CD117 expression is useful because CD117⁺ NK cells are cytotoxic towards tumor cell lines from various tissues, as described in Example 6, below.

These results suggest that the differentiation progression of TSNK cells is different from ESC-derived NK cells, and that TSNK cells are distinguishable from ESC derived NK cells.

### 7.5. Example 5: PDACs Enhance Fold Expansion of Cultured Natural Killer Cells in NK2A Medium

To assess the effect of CD10⁺, CD34⁻, CD105⁺, CD200⁺ tissue culture plastic-adherent placental stem cells (referred to in this Example as PDACs) on hematopoietic stem cell (HSC) differentiation to natural killer cells, HSCs were stimulated with mitomycin C-treated PDACs or bone marrow-derived mesenchymal stem cells (MSC) at a ratio of 10:1 PDACs/MSC:HSC on Day 0 and Day 21, while a feeder-free culture was used as the control. NK2A medium was used as culture medium. PDACs were found to enhance the fold expansion of cultured NK cells compared to medium alone. However, no significant difference in cytotoxicity between cells grown with or without the feeder layer was found. Cells treated with MSC showed the highest fold expansion, but the lowest cytotoxicity, as shown in Figure 7.

### 7.6. Example 6: Cytotoxic Activity of NK Cells Expanded Using Two-Stage Medium

This Example demonstrates that NK cells produced from CD34⁺ cells expanded and differentiated using the Two-stage process described above are cytotoxic to tumor cell lines.

*Lactate Dehydrogenase (LDH) release assay.* The LDH release assay was performed using CYTOTOX 96® non-radioactive cytotoxicity assay kit (Promega, Cat# G1780). In this assay, cultured NK cells derived from donor-matched human placental perfusate (HPP) and cord blood units (Combos units) were used as effector cells, while certain tumor cell line cells were used as target cells. From three units used in this study, the percentage of HPP cells was 56.6% ± 28.3%. Effector cells and target cells were placed in 96-well U-bottom tissue culture plates and incubated at various effector-target (E:T) ratios in 100 µl RPMI 1640 without phenol red (Invitrogen, Cat# 11835-030) supplemented with 2% human AB serum (Gemini, Cat# 100-512). Cultures were incubated for 4 hours at 37 °C in 5% CO₂. After incubation, 50 µl supernatant was transferred to enzymatic assay plate, LDH activity was detected as provided by the manufacturer, and absorption was measured at 490 nm in an ELISA reader (Synergy HT, Biotek). The cytotoxicity was calculated according to the following equation: % Cytotoxicity = (Sample - Effector Spontaneous - Target Spontaneous)/(Target maximum-Target Spontaneous)* 100, where "Effector Spontaneous" is a control for the spontaneous release of LDH from effector cells; "Target Spontaneous" is a control for the spontaneous release of LDH from target cells; and "Target Maximum" is a control for the maximum LDH release when essentially 100% of cells are lysed.

*Isolation of Human Placental Perfusate (HPP) CD34+ Cells and Umbilical Cord Blood (UCB) CD34+ Cells.* HPP and UCB cells were purified using either Ficoll or ammonium chloride to obtain total nucleated cells (TNCs). TNCs were then used in a positive selection procedure to isolate CD34⁺ cells using anti-CD34 beads and RoboSep following the protocol provided by the manufacturer (StemCell Technologies, Inc.) In this experiment, CD34⁺ cells were isolated with greater than 90% purity.

*Study of tumor cell susceptibility to cultured two-stage NK cells.* Tumor cell lines (Table 6), including human breast cancer (HCC2218), human colorectal adenocarcinoma (HT-29), human chronic myelogenous leukemia (CML), human acute myeloid leukemia (AML), human glioblastoma (LN-18 and U-118MG), human multiple myeloma (U266), human histiocytic lymphoma (U937), and human retinoblastoma (WERI-RB-1) were co-cultured with two-stage NK cells. The two-stage cultured NK cells included those cultured in NK2A medium for 21 days, then cultured in CNK Medium for 21 days, and those cultured in NK2A medium for 28 days and then CNK Medium for 14 days. The NK cell cytotoxicity was measured by the lactate dehydrogenase (LDH) release assay after 4-hour co-culture. At effector to target (E:T) ratio of 10:1 the latter generally showed higher cytotoxicity than the former (Table 6). Of the tumor cell lines, LN-18 was the most susceptible to NK-mediated killing, followed by K562, U937, WERI-RB-1, U-118MG, HT-29, HCC2218, KG-1 and U266.

TSNK cells therefore showed significant cytotoxicity toward various cancer cell lines. It further appears that the NK cytotoxicity can be improved by prolonging the culture period in NK2A medium from 21 days to 28 days.

**Table 6. Cytotoxicity of cultured TSNK cells targeting on tumor cell lines**

| | 21-Day NK2A Medium + 21-Day TSNK Medium | | 28-Day NK2A Medium + 14-Day TSNK Medium | |
|---|---|---|---|---|
| Tumor Lines | % Average Cytotoxicity | STDEV | % Average Cytotoxicity | STDEV |
| HCC2218 | 15.65 (n=1) | | 11.89 (n=3) | 10.2 |
| HT-29 | 20.52 (n=3) | 9.3 | 33.42 (n=3) | 20.2 |
| K562 | 69.63(n=3) | 27.6 | 80.04 (n=3) | 25.4 |
| KG-1 | 5.67 (n=3) | 3.4 | 15.41 (n=3) | 9.2 |
| LN-18 | 99.00 (n=3) | 0.0 | 99.00 (n=3) | 0.0 |
| U-118MG | 23.12 (n=3) | 6.9 | 49.16 (n=2) | 15.8 |
| U266 | 2.21 (n=1) | | 5.07 (n=2) | 3.3 |
| U937 | 46.44 (n=2) | 19.3 | 51.58 (n=2) | 23.2 |
| WERI-RB-1 | 25.30 (n=2) | 4.7 | 36.79 (n=2) | 11.3 |

| | | | | |
|---|---|---|---|---|
| *n: number of donors | | | | |

*MicroRNA Profiling of Human Placental Perfusate (HPP) CD34+ Cells and Umbilical Cord Blood (UCB) CD34*+ *Cells.* Purified donor-matched HPP and UCB CD34+ cells were subjected to microRNA (miRNA) preparation using a MIRVANA™ miRNA Isolation Kit (Ambion, Cat# 1560). CD34⁺ cells (0.5 to 1.5 x 10⁶ cells) were disrupted in a denaturing lysis buffer. The samples were then subjected to acid-phenol+chloroform extraction to isolate RNA highly enriched for small RNA species. 100% ethanol was added to bring the samples to 25% ethanol. When this lysate/ethanol mixture was passed through a glass fiber filter, large RNA species were immobilized, and the small RNA species were collected in the filtrate. The ethanol concentration of the filtrate was then increased to 55%, and the mixture was passed through a second glass fiber filter where the small RNAs became immobilized. This RNA was washed a few times, and eluted in a low ionic strength solution. The concentration and purity of the recovered small RNA was determined by measuring its absorbance at 260 and 280 nm. miRNAs found to be unique for HPP CD34+ cells in all donors tested (n=3) included hsa-miR-380, hsa-miR-512, hsa-miR-517, hsa-miR-518c, hsa-miR-519b, and hsa-miR-520a.

### 7.7. Example 7: Isolation of CD34⁺ Cells from pooled UCB and HPP

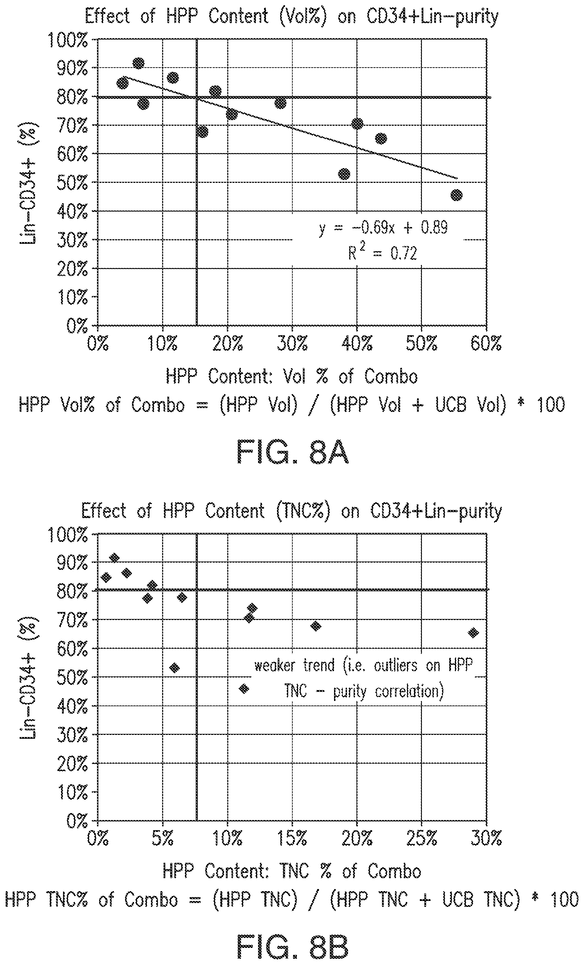

This example demonstrates the isolation of CD34⁺ cells from pooled umbilical cord blood (UCB) and human placental perfusate (HPP) (Combo). To evaluate the UCB:HPP pooling ratio, side-by-side comparisons of 3 different pooling ratios were performed as follows: (1) Full pooling: 1x UCB (full volume) + 1x HPP (full volume); (2) Partial pooling of HPP (33%): 1x UCB (full volume) + 0.33x HPP (1/3 of HPP volume); and (3) Partial pooling of HPP (10%): 1x UCB (full volume) + 0.10x HPP (1/10 of HPP volume). A total of N=3 experimental replicates were executed. The initial TNC and volumes were recorded. The pooled samples were then purified for CD34⁺ cells and CD34⁺ purity was determined post-thaw. The optimal pooling ratio was then determined graphically from the post-thaw CD34⁺ purity vs. volumetric or cell count (TNC) fractions (as a % of Combo) plots. As shown in Figure 8, the end-point CD34⁺ purity correlates well with the HPP volume content, but not as much with the HPP TNC content. Overall, UCB 85% v/v, HPP 15% v/v was found to be the optimal pooling ratio to obtain CD34⁺ cells with purity of 80% and above.

### 7.8. Example 8: Comparison of NK Cell Cultivation Using GBGM®-based media

This example demonstrates the comparison of NK cell cultivation processes using two GBGM-based media (the three-stage process and two-stage process). The two processes are summarized in Table 7. Both processes utilized GBGM as the basal medium for the differentiation of NK cells and CD34⁺ cells of placenta in origin.

**Table 7. Summary of the three-stage and two-stage processes**

| | **Three-Stage Process** | **Two-Stage Process** |
|---|---|---|
| **Cultivation Process** | **3-Stage, GBGM based, feeder-free, 35 days** | **2-Stage, GBGM in 1^{st} Stage, with K562/PBMC feeders, 35 days** |
| | • Stage 1 (9 days): GBGM with 10% human serum AB (HS), LWH heparin, TPO, SCF, IL-7, Flt3L (25 - 27 ng/mL) | • Stage 1 (21 days): GBGM with 10% human serum, heparin, TPO, SCF, IL-7, Flt3L, IL-15, (10 - 20 ng/mL), IL-2 (200 U/mL). |
| | • Stage 2 (5 days): GBGM with 10% HS, LWH heparin, SCF, IL-7, Flt3L, IL-15 (20 - 27 ng/mL) | • Stage 2 (14 days): TSNK Expansion Process in TSNK Medium, *i.e*., IMDM with 10% FBS, K562 + PBMC feeders, IL-2 (200 U/mL). |
| | • Stage 3 (21 days): GBGM with 10% HS, SCF, IL-7, Flt3L, IL-15 (20 - 27 ng/mL), IL-2 (1000 U/mL) | Media replenishment in Stage-2 with TSNK Maintenance Medium, *i.e.,* IMDM with 10% FBS, 2% HS, IL-2 (200 U/mL) |
| | Use of low-dose cytokines (IL-6, LIF, G-CSF, GM-CSF, MIP-1a) throughout, at 50 - 250 pg/mL | |

The experimental parameters are outlined as follows:

### Donor lots:

(1) CD34⁺ cells from fresh UCB: N=6
(2) CD34⁺ cells from fresh "Combo": N=2
(3) CD34+ from cryopreserved "Combo": N=8

Scale: Multiwell dishes to T-25, up to multiple T-75 flasks, 1 to 80 mL in culture volume

### Process Methods:

(1) Two-Stage process
(2) Three-Stage process

### Use of feeders:

(1) Without feeders
(2) With inactivated K562 & PBMC feeders, added to culture at Day 21

### Seeding density: 20000 - 50000 cells / mL

CD34⁺ cells from either fresh UCB or fresh combo were generated and cryopreserved with methods described in Examples 7, 10 and 11. Cultures were maintained in a 37 °C, >90% humidity, and 5% CO₂ incubator. Cell growth was monitored throughout (cell count) and medium exchanges were performed twice per week to maintain cell concentrations within the range of 5x10⁴ - 1x10⁶ per mL. Differentiation was monitored by phenotypic analysis at Day 21 and 35. When feeders were used, at Day 21 of NK cultivation, fresh 3-day cultured K562 and post-thaw allo-PBMC were inactivated with mitomycin-C (16 µg/mL, 2 hrs, 37 °C) and were added to the two-stage process conditions at 1x10⁶ per mL. The differentiating NKs were normalized to 0.5x10⁶ per mL. At Day 35, after final cell counts were performed, a flow cytometry-based cytotoxicity assay using freshly cultured and PKH labeled K562 cells (10:1 E:T ratio, 4 hr, 37 °C) was performed to evaluate NK functionality.

### Results

The median of the TNC expansion fold for CD34⁺ cells derived from fresh UCB using the two processes were comparable at Day 35. The two-stage process appeared to yield higher TNC expansion fold than the three-stage process for fresh combo-derived CD34⁺ cells. The overall TNC expansion folds were comparable for the CD34⁺ cells derived from post-thaw combo using both processes, but were significantly lower than those derived from fresh UCB or fresh combo. In all, both the three-stage and two-stage processes yielded similar cell yield at end of cultivation.

At Day 21, there were no noticeable differences of phenotype on cells originated from UCB or Combo. The two-stage process resulted in a higher percentage of CD56⁺CD3⁻ NK cells (avg. 14.7%) than the three-stage process (avg. 6.1%). The extent of differentiation (e.g., CD56⁺CD3⁻ level) appeared to be donor-dependent. The percentage of both CD3⁺CD56⁻ (T-cells) and CD3⁺CD56⁺ (NKT-like cells) populations were minimal in all cases.

At Day 35, both processes were found to be effective for differentiating NK cells, as evidenced by the high end-point CD56⁺CD3⁻ purity levels (87.2% and 90.1% for the two-stage and three-stage processes, respectively). The addition of feeders in the two-stage process appeared to enhance NK phenotypic purity (75.3% without feeders; 87.2% with feeders), while no observable benefit of feeders on NK purity was found (90.1% without feeders; 84.7% with feeders) with the three-stage process.

Cultivated NK cells maintained their CD16⁻ phenotype throughout. The two-stage process appeared to yield slightly more CD56⁺CD3⁺ cells in the absence of feeders (avg. 11.2%) than the other conditions (< 2%). The presence of PBMC and K562 feeders in both processes significantly upregulated/activated certain NK functional markers (NKp46, DNAM-1, CD94) on the NK cell population. Overall, the NK purity and functional marker expression profiles were found to be comparable between the two processes, when the feeder conditions are identical.

The functionality of cultivated NK cells, as determined by the 4 hour *in vitro* K562 cytotoxicity assay, was found to be comparable between the two processes when feeder conditions are identical. Feeder-activated NK cells were highly effective in killing K562 cells *in vitro,* with average specific lysis of 93.2% and 93.6% specific lysis for the two-stage and three-stage processes, respectively.

In summary, the two-stage and three-stage processes were found to yield comparable growth, phenotype (purity and activation markers), and *in vitro* functionality for NK cells when the same feeder condition was used. The two-stage process offers the ease and convenience of culturing NK cells compared to the three-stage process.

### 7.9. Example 9: Comparison of NK Cell Cultivation Using Various Basal Media

This study is aimed to evaluate the differentiation and expansion of CD34⁺-derived NK cells using different basal media.

The experimental conditions are summarized in Table 8. The cells were cultured as described in Example 11. All experiments were setup in the scale of multiwell dishes/T-flasks and were maintained in a 37 °C, >90% humidity, and 5% CO₂ incubator. Cell growth was monitored throughout (cell count) and medium exchanges were performed twice per week to maintain cell concentrations within the range of 5x10⁴ - 1x10⁶ per mL. Differentiation was monitored by phenotypic analysis at Day 21 and 35. At Day 21, fresh 3-day cultured K562 and post-thaw allo-PBMC were inactivated and were added to developing NK cell culture at 1x10⁶ per mL. The NK cells were normalized to 0.5x10⁶ per mL. At Day 35, after final cell counts were performed, a flow cytometry-based cytotoxicity assay using freshly cultured and PKH labeled K562 cells (10:1 E:T ratio, 4 hr, 37 °C) was carried out to evaluate NK functionality.

**Table 8. Summary of experimental conditions for evaluation of various basal media**

| | |
|---|---|
| **CD34+ donors** | 1 UCB donor |
| **Cultivation Process Method** | Three-Stage Process |
| **K562 & PBMC feeders** | • With feeders @ day 21 |
| **Basal media screened** | • GBGM (control) |
| | |
| | • AIM-V |
| | • X-VIVO 10 |
| | • X-VIVO 15 |
| | • OpTmizer |
| | • Stemspan H3000 |
| | • Cellgro |
| | • DMEM:F12 |
| | • DMEM:F12 w/ 5mM OAC (added to culture from Day 7 to Day 35) |
| **Seeding density (day 0)** | 50000 / mL |

### Growth yield

Stemspan H3000 and OpTmizer showed comparable growth yield (TNC expansion fold) to GBGM at Day 35. Cellgro, X-VIVO 15, AIM-V, X-VIVO 10, DMEM:F12, DMEM:F12 w/ 5 mM OAC showed lower growth yield than GBGM.

### Phenotypic analysis

At Day 35 (end-point), GBGM yielded about 80% purity of CD56⁺CD3⁻ cells. OpTmizer and Stemspan H3000 yielded about 50% purity of CD56⁺CD3⁻ cells. DMEM:F12 produced about 35% purity of CD56⁺CD3⁻ cells. AIM-V, X-VIVO 10, X-VIVO 15, and Cellgro media produced about <30% purity of CD56⁺CD3⁻ cells. The addition of OAC to DMEM:F12 basal medium during culture was found to greatly enhance the end-point NK purity; the percentage of CD56⁺CD3⁻ at Day 35 of culture increased from 35% to 72%.

### Cytotoxicity/Functionality

The addition of 5 mM OAC to DMEM:F12 basal medium during culture was found to greatly enhance the activation status and *in vitro* functionality of NK cells. The addition of OAC also significantly increased the level of NK activation markers NKp46, NKG2D, DNAM-1, and CD94. The *in vitro* functionality (K562 cytotoxicity) of Day-35 NK cells increased substantially as well, from 21.4% to 97.1% %.

Overall, the NK cell properties were significantly enhanced from the addition of 5 mM OAC.

### 7.10. Example 10: Storage and Cryopreservation of NK Cells

This Example demonstrates the methods of storing and cryopreserving NK cells. CD34⁺ hematopoietic stem cells isolated from human placental perfusate (HPP) and umbilical cord blood cells were expanded and differentiated into NK cells using the protocols described in the previous examples. Cells were cryopreserved right after being isolated from HPP and umbilical cord blood (at Day 0) or during the first growth phase of the NK cells (at Day 9, Day 14, Day 21 or Day 35 post isolation).

The cells were cryopreserved in the following cryopreservation formulations: Formulation 1-dextran cryo medium: 5% DMSO (Sigma Aldrich, D2650), 55% dextran (10% w/v in normal saline) (10% LMD in 0.9% sodium chloride injection, Hospira), 40% HAS (Octapharma); Formulation 2 - Trehalose cryo medium: 5% DMSO, 55% trehalose (10% w/v in normal saline), 40% HSA; Formulation 3 - CryoStor® CS2 (BioLife Solutions); Formulation 4 - CryoStor® CS5 (BioLife Solutions); Formulation 5 - CryoStor®CS10 (BioLife Solutions); Formulation 6 - Serum-free freezing media (Sigma-Aldrich, Cat# 6295); Formulation 7 - Glycerol freezing media (Sigma-Aldrich, CAT# C6039); or Formulation 8 - DMSO and serum-free freezing media (Sigma-Aldrich, CAT# 2639).

Cells collected at different time points were washed several times with culture media or saline solution. The cells were then centrifuged to obtain cell pellets. The supernatants were removed, and the cells pellets were suspended with cryopreservation media to about 1 x 10⁶ - 1.5 x 10⁷ or more cells per milliliter. The cell suspension was aliquoted to 1mL or 2 mL septum vials and incubated at 2-8 °C for approximately 10 minutes. Subsequently, the cells were frozen in a control rate freezer (Thermo) at 0.5 °C/min. Frozen vials were transferred to a cryogenic freezer for storage in liquid nitrogen vapor. Cryopreserved NK cells can be thawed quickly in a 37 °C water bath with gentle swirling of the samples until all visible ice melted. The cell samples can be diluted with pre-warmed culture media.

### 7.11. Example 11: Storage and Cryopreservation of NK Cells

This Example demonstrates another method of storing and cryopreserving NK cells. CD34⁺ hematopoietic stem cells isolated from human placental perfusate (HPP) and umbilical cord blood cells were expanded and differentiated into NK cells using the protocols described above. Cells were cryopreserved right after being isolated from HPP and umbilical cord blood (at Day 0) or during the first growth phase of the NK cells (at Day 9, Day 14, Day 21 or Day 35 post isolation).

A Cell Suspension Solution was prepared by combining Dextran-40 and HSA in the ration of 60% Dextran-40 v/v, 40% HSA (from a 25% solution) v/v).

A 2x Freezing Solution was prepared with 50% Dextran-40 v/v, 40% HSA (25% solution) v/v, 10% DMSO v/v. DMSO was first slowly added to the Dextran-40 and mixed well. Subsequently 25% solution of HSA was added to the solution slowly with mixing. The resulting solution was mixed well and brought to room temperature prior to use.

### Cryopreservation Procedure

The cell number was estimated and normalized as a cell suspension to 15x10⁶ in Cell Suspension Solution. The volume of the cell suspension was determined and an equal volume of freshly prepared 2x Freezing Solution was slowly added and mixed. The final cell suspension volume in Freezing Solution was recorded and the distributed to a number of vials. MycoAlert testing was performed on saved culture supernatants to detect mycoplasma contamination. A post-thaw test was conducted on one retain vial to determine the post-thaw viability, cell recovery and cell characterization.

### 7.12. Example 12: Analysis of cryopreserved/thawed NK Cells

*Viability assay.* NK cells cryopreserved in various formulations as in Example 10 or 11 were thawed. Cells were frozen at the density of 2 x 10⁶ - 3 x 10⁷ cells/mL. Thawed NK cells were evaluated for cell viability using the Countess® Automated Cell Counter (Invitrogen) at Day 0, Day 3 and Day 18 post thawing compared to fresh cells or prefreeze cells. Briefly, 10 µl of cell samples were mixed with 10 µl of trypan blue. The cell mixtures were pipeted into the Countess® chamber slide. The slide was inserted into the instrument and the cells were counted. Post-freeze-thaw cells showed cell viability about 80% to > 90% of viability, depending on different cryopreservation formulations.

*Apoptosis assay.* Thawed NK cells were also evaluated for apoptosis using BD AnnV/PI Apoptosis assay kit at Day 0, Day 3 and Day 18 post thawing. Briefly, cells were washed twice with 1x cold PBS and re-suspended in 1x binding buffer(BD Annexin V/PI Apoptosis Kit part number 556547 Composition of Binding buffer part number 51-66121E 0.1 M Hepes/NaOH (pH 7.4), 1.4 M NaCl, 25 mM CaCl₂. For 1x dilute 1 part 10x buffer to 9 parts of distilled water). 100 µL of cell suspension containing approximately 100,000 cells was transferred into a FACS tube. 100 µL of 1x binding buffer, 5 µL of AnnV-FITC, and 5 µL of PIPE were added to the tube. The tube was then gently vortexed and incubated in the dark for 15 minutes. Subsequently, 400 µL of 1x binding buffer was added. The samples were analyzed within 1 hour. Controls used to set up quadrants and gates were unstained cells, cells stained with AnnV-FITC only and not PI, cells stained with PI only and not AnnV. The apoptotic cells were quantified as a % of the population of events gated as "cells" on the size scatter (FSC vs SSC) plot. The post-freeze-thaw cells showed about 5-25% of dead/late apoptotic cells and 10-25% of early apoptotic cells, depending on different cryopreservation formulations. Overall, formulation 1 (5% DMSO, 55% dextran (10% w/v in normal saline), 40% HAS), formulation 2 (5% DMSO, 55% trehalose (10% w/v in normal saline), 40% HSA), formulation 4 - CryoStor® CS5 (BioLife Solutions), and formulation 5 (CryoStor®CS10 (BioLife Solutions)) showed higher cell viability and lower apoptotic cells compared to other formulations.

### 7.13. Example 13: Evaluation of In-Process Cryopreserved Cell Banking

This example demonstrates the evaluation of In-Process Cryopreserved Cell Banking. The cell culture was initiated with UCB CD34⁺ cells using the method as described by Spanholtz et al, PLoS One. 5(2):e9221 (2010) using either HS-AB or FBS as the serum source. The cell concentration was determined and adjusted, and the medium was replenished as needed. At Day 7, 9, 10 or 14, approximately 10⁶ - 3 x 10⁶ cells were removed from the cell culture, centrifuged and resuspended in cryopreservation medium (5.5% v/v Dextran-40, 10% v/v HSA, 5% v/v DMSO). The cells were frozen in a controlled-rate freezer and transferred to liquid phase nitrogen storage for cryopreservation. Approximately 1 mL cells each vial were cryopreserved at a concentration ranging from 10⁶ - 10⁷ per mL. The remaining culture was carried forward to the end-point (Day 35), referred to as "Fresh (no in-process cryopreservation)". Phenotypic analyses were performed on Day 21, 28, and 35 of the cell culture. *In vitro* functionality (K562 cytotoxicity, 10:1 E:T) was assessed at Day 35 (end-point) of the culture.

Post-thaw performance of the in-process cryopreserved culture samples (Day 9 and 14) were evaluated as follows. The cell bank vials were quickly thawed in the 37 °C water bath. The cells were then diluted with RPMI-FBS medium, centrifuged, resuspended, and seeded in culture media. Each of the culture conditions were then carried forward to Day 35, cumulative from the start of culture process. Analytics (cell count, viability, phenotypic analysis, functionality assessment) were done in the same manner as their "Fresh" counterpart.

### Results

Day 9, 10, and 14 in-process cryopreserved samples all yielded excellent post-thaw viability, regardless of in-process time point or cell concentration (96.2%-97.3% Trypan Blue negative, 86.1%-93.2% Annexin-V negative / TO-PRO-3 negative). The in-process banking did not have negative effects on end-of-culture (Day 35) yield loss. The final cell yield between either Day 9 or 14 post-thaw and "fresh" conditions are comparable, with either HS-AB or HS-AB as the serum source.

The phenotypic profiles of maturing NK cells at Day 21/28/35 time points were found to be quite comparable between the "Fresh" and "Post-Thaw" culture, respectively. The phenotypic purity (CD56⁺CD3⁻) was found to be comparable as well. The expression of certain NK functional markers (CD94, NKG2D) were slightly different due to run-to-run variability.

In the K562 cytotoxicity assay, the Day 9/14 post-thaw cultured NK cells were found to yield ∼0-20% lower specific K562 lysis readout comparing to non in-process cryopreserved cultured NK cells. However, given the expression levels of surface markers relevant to NK cytotoxic function (DNAM1, NKp46, NKG2D, etc.) were not concurrently reduced, the trend would need to be confirmed with additional donors and assay repeats. Overall, in-process cryopreservation at Day 9/14 of cultivation had minimal impact on process outcome.

### 7.14. Example 14: Development of Post-thaw medium for NK cell dosing

This example demonstrates the development of post-thaw medium for NK cell dosing in animals. The effects of injection media and cell density were tested on NK cell's viability, cytotoxicity, cell recovery and clump formation. Viability was assessed by trypan blue staining; cytotoxicity was assessed by FACS (10:1 ratio of NK:K562) and clump formulation was assessed by microscopic assay. Results are shown in Tables 9-12 for the various types of injection media and cell density.

**Table 9. Cell recovery, viability, cytotoxicity and clump formation of specific conditions tested**

| Thawing media: RPMI + 10% FBS; Injection media: PBS + 1% FBS; Cell density: 10×10⁶ cells/ ml | | | | | |
|---|---|---|---|---|---|
| | 0 hr | 1 hr | 2 hr | 3 hr | 4 hr |
| Cell Recovery | 98.8% | | | | |
| Viability | 74.3% | 69.0% | 66.1% | 63.3% | 62.4% |
| Cytotoxicity | | 60.3% | | 44.3% | |
| Clumping | None | | | | |

**Table 10. Cell recovery, viability, cytotoxicity and clump formation of specific conditions tested**

| Thawing media: RPMI + 10% FBS; Injection media: PBS + 1% FBS; Cell density: 30×10⁶ cells/ ml | | | | | |
|---|---|---|---|---|---|
| | 0 hr | 1 hr | 2 hr | 3 hr | 4 hr |
| Cell Recovery | 98.8% | | | | |
| Viability (%) | 74.3% | 69.8% | 66.5% | 64.0% | 63.6% |
| Cytotoxicity (%) | | 51.8% | | 37.9% | |
| Clumping | None | | | | |

**Table 11. Cell recovery, viability, cytotoxicity and clump formation of specific conditions tested**

| Thawing media: RPMI + 10% FBS; Injection media: Plasmalyte + 1% HSA; Cell density: 10×10⁶ cells/ ml | | | | | |
|---|---|---|---|---|---|
| | 0 hr | 1 hr | 2 hr | 3 hr | 4 hr |
| Cell Recovery | 98.8% | | | | |
| Viability | 74.9% | 76.3% | 72.0% | 70.5% | 69.4% |
| Cytotoxicity | | 59.4% | | 51.3% | |
| Clumping | None | | | | |

**Table 12. Cell recovery, viability, cytotoxicity and clump formation of specific conditions tested**

| Thawing media: RPMI + 10% FBS; Injection media: Plasmalyte + 1% HSA; Cell density: 30×10⁶ cells/ ml | | | | | |
|---|---|---|---|---|---|
| | 0 hr | 1 hr | 2 hr | 3 hr | 4 hr |
| Cell Recovery | 98.8% | | | | |
| Viability | 74.9% | 74.7% | 71.2% | 70.3% | 69.9% |
| Cytotoxicity | | 61.2% | | 53.9% | |
| Clumping | None | | | | |

The results showed that the Plasmalyte +1% HSA injection media maintained NK cells with better viability and cytotoxicity than PBS+1%FBS injection media. Cytotoxicity also decreased over time after the cells were suspended in injection media. There was no cell density effect observed on viability and cytotoxicity when cells were suspended in Plasmalyte +1% HSA. NK cells also settled down in PBS+1%FBS or Plasmalyte +1% HSA media after 1 hour; however, cells did not appear to aggregate. Finally, there was no obvious loss of cell recovery and viability observed from the freezing-thawing process.

### 7.15. Example 15: Development of Post-thaw medium for NK cell dosing

The effects of various HSA concentrations were also tested on the NK cell's viability, cytotoxicity, cell recovery and clump formation. The same methods were utilized from Example 14. Results are shown in Tables 13-16 for the various types of injection media and cell density.

**Table 13. Cell recovery, viability, cytotoxicity and clump formation of specific conditions tested**

| Thawing media: RPMI + 10% FBS; Injection media: Plasmalyte + 1% HSA; Cell density: 10×10⁶ cells/ ml | | | | | |
|---|---|---|---|---|---|
| | 0 hr | 1 hr | 2 hr | 3 hr | 4 hr |
| Cell Recovery | 82.0% | | | | |
| Viability | 75.6% | 75.3% | 74.5% | 71.8% | 71.5% |
| Cytotoxicity | | 64.4% | | 35.5% | |
| Clumping | None | | | | |

**Table 14. Cell recovery, viability, cytotoxicity and clump formation of specific conditions tested**

| Thawing media: Plasmalyte +1% HSA; Injection media: Plasmalyte +1% HSA; Cell density: 10×10⁶ cells/ ml | | | | | |
|---|---|---|---|---|---|
| | 0 hr | 1 hr | 2 hr | 3 hr | 4 hr |
| Cell Recovery | 102.4% | | | | |
| Viability (%) | 77.1% | 77.5% | 71.2% | 72.4% | 75.1% |
| Cytotoxicity (%) | | 61.48% | | 31.7% | |
| Clumping | None | | | | |

**Table 15. Cell recovery, viability, cytotoxicity and clump formation of specific conditions tested**

| Thawing media: Plasmalyte + 2.5% HSA; Injection media: Plasmalyte + 2.5% HSA; Cell density: 10×10⁶ cells/ ml | | | | | |
|---|---|---|---|---|---|
| | 0 hr | 1 hr | 2 hr | 3 hr | 4 hr |
| Cell Recovery | 91.6% | | | | |
| Viability | 83.9% | 82.6% | 78.9.0% | 79.0% | 79.3% |
| Cytotoxicity | | 71.7% | | 46.8% | |
| Clumping | None | | | | |

**Table 16. Cell recovery, viability, cytotoxicity and clump formation of specific conditions tested**

| Thawing media: Plasmalyte + 5% HSA; Injection media: Plasmalyte + 5% HSA; Cell density: 10×10⁶ cells/ ml | | | | | |
|---|---|---|---|---|---|
| | 0 hr | 1 hr | 2 hr | 3 hr | 4 hr |
| Cell Recovery | 81.2% | | | | |
| Viability | 81.8% | 80.7% | 78.2% | 78.3% | 78.6% |
| Cytotoxicity | | 64.8% | | 55.1% | |
| Clumping | None | | | | |

The results show that viability was maintained well over 4 hour post-thaw in all three injection media tested. Also, cytotoxicity decreased over time in all three injection media. However, the level of reduction was smaller in higher concentrations of HSA whereas Plasmalyte+5% HSA maintained the highest cytotoxicity. It was also observed that NK cells did not aggregate in all three injection media. Overall, Plasmalyte appears to be a better injection medium candidate than PBS.

### 7.16. Example 16: Cultivation of NK cells without IL-2

This example demonstrates cultivation of NK cells in the absence of IL-2. Cell culture was performed by the two-stage process described in Example 11. Five different concentrations of IL-2 in the first medium were tested: 0, 200, 500, 1000, 2000 U/mL.

The results indicate that developing NK cells in culture did not appear to respond to IL-2 on growth. The purity of NK cells did not appear to depend on IL-2: the NK cells differentiate into CD56⁺CD3⁻ phenotype in the absence of IL-2. The combination of IL-7, IL-15 and SCF appeared to be sufficient for *in vitro* NK cell development.

### 7.17. Example 17: Culture and Characterization of CD34+ Placental Hematopoietic Cells Towards Natural Killer (NK) Cells

This example describes a three-step cultivation process for expansion and differentiation of CD34+ placental hematopoietic cells towards a populations of natural killer (NK) cells. Cell populations obtained after 35 days of the three-step/stage culture process (termed "D35 NK cells") of this cultivation process are also described and characterized in this example.

### Materials and Methods

**Culture process for expansion and differentiation of hematopoietic stem cells (HSCs) towards NK cells.** CD34+ cells were cultured in GBGM medium (Glycostem Therapeutics; see Spanholtz, et al., PLoS One (2010) 5(2):9221) and aliquots of cells were taken for assessment of cell count, cell viability, characterization of lymphocytic differentiation and functional evaluation. To initiate *in vitro* expansion and NK lineage commitment, CD34+ cells were seeded into GBGM medium. Cells were cultured in feeder-free cultures in three stages as follows:
Stage 1 (9 days): GBGM with 10% human serum AB (HS), LWH heparin, TPO, SCF, IL-7, Flt3L (25 - 27 ng/mL);
Stage 2 (5 days): GBGM with 10% HS, LWH heparin, SCF, IL-7, Flt3L, IL-15 (20 - 27 ng/mL); and
Stage 3 (21 days): GBGM with 10% HS, SCF, IL-7, Flt3L, IL-15 (20 - 27 ng/mL), IL-2 (1000 U/mL).

**Lactate dehydrogenase (LDH) release assay.** LDH is a stable cytosolic enzyme released upon cell lysis. Assay for LDH release was conducted using the CYTOTOX 96® colorimetric cytotoxicity assay kit (Promega, Cat# G1780), and cultured D35 NK cells were used as effector cells and tumor cells were used as target cells. Effector cells and target cells were placed in 96-well U-bottom tissue culture plates and incubated at various effector-target (E:T) ratios in 100 µl RPMI 1640 without phenol red (Invitrogen, Cat# 11835-030) and supplemented with 2% human AB serum. Cultures were incubated for 4h at 37 °C in 5% CO₂. After incubation, 50 µl supernatant was transferred to enzymatic assay plates. LDH activity was detected as provided by the manufacturer, and absorption was measured at 490 nm in an ELISA reader (Synergy HT, Biotek). Cytotoxicity was calculated according to the following equation: %Cytotoxicity = (Sample - Effector Spontaneous - Target Spontaneous)/(Target maximum - Target Spontaneous) x 100.

***In vitro* cytotoxicity analysis.** Direct cytotoxicity assays were performed using the cultured D35 NK cells as effector cells and tumor cells as target cells. Tumor cells were labeled with PKH26 (Sigma-Aldrich Catalog # PKH26-GL) (see Ferlazzo G, et al, 2004, PNAS USA 101(47):16606-11; Lee-MacAry AE, et al, 2001, J Immunol Methods 252(1-2):83-92), which inserts into the cell plasma membrane due to its lipophilic aliphatic residue, then placed in 96-well U-bottom tissue culture plates and incubated with cultured D35 NK cells at various effector-to-target (E:T) ratios in 200 µl RPMI 1640 supplemented with 10% FBS. Cultures were incubated for 4h at 37 °C in 5% CO₂. After incubation, cells were harvested and TO-PRO-3 (Invitrogen Catalog # T3605), a membrane-impermeable DNA stain, was added to cultures to 1 µM final concentration followed by FACS analysis using a BD FACSCanto I. Cytotoxicity was expressed as percentage of dead cells (PKH26⁺TO-PRO-3⁺) within the total PKH26⁺ target tumor cells.

***In vivo* mouse model for biodistribution and efficacy study.** Female NSG (NOD-CgPrkdc-scid-Il2rg-tmlWjl-Sz) mice (The Jackson Laboratory; Bar Harbor, Maine) at 6∼8 weeks of age were used for the study. The in vivo efficacy study was set up as shown in Table 17.

**Table 17. Study groups for assessing in vivo D35 NK cell efficacy**

| | Group | Tumor model | # of Mice | Treatment | Dose and Dosing Route | Dosing Volume |
|---|---|---|---|---|---|---|
| Step 1 | 1 | K562 | 8 | Control | Vehicle (i.v.) | 0.4 mL |
| | 2 | K562 | 8 | Fresh NKs* | 6.0E6 (i.v.) | 0.4 mL |
| | 3 | K562 | 8 | Control | Vehicle (IT) | 0.03 mL |
| | 4 | K562 | 8 | Fresh NKs | 2.0E6 (IT) | 0.03 mL |
| | 5 | U-87MG | 8 | Control | Vehicle (i.v.) | 0.4 mL |
| | 6 | U-87MG | 8 | Fresh NKs | 6.0E6 (i.v.) | 0.4 mL |
| | 7 | U-87MG | 8 | Control | Vehicle (IT) | 0.03 mL |
| | 8 | U-87MG | 8 | Fresh NKs | 2.0E6 (IT) | 0.03 mL |
| | 9 | HCT-116 | 8 | Control | Vehicle (i.v.) | 0.4 mL |
| | 10 | HCT-116 | 8 | Fresh NKs | 6.0E6 (i.v.) | 0.4 mL |
| | 11 | HCT-116 | 8 | Control | Vehicle (IT) | 0.03 mL |
| | 12 | HCT-116 | 8 | Fresh NKs | 2.0E6 (IT) | 0.03 mL |

| | | | | | | |
|---|---|---|---|---|---|---|
| *NKs = D35 NK cells | | | | | | |

Mice were housed in microisolator housing, and quarantined for several days prior to the initiation of the study. A total of 5 ×10⁶ K562 cells or 6 ×10⁶ HCT-116 cells, or 2 ×10⁶ U087MG cells in exponential growth phase resuspended with matrigel were subcutaneously implanted into the right flank region of the mouse. Mice were monitored for tumor growth daily after cell implantation. When tumor volumes reached around 100 mm³, mice were randomized into groups using mice having tumor volumes closest to the mean value. Treatment with D35 NK cells was initiated on the day after randomization, and only one single dose of D35 NK cells was administered to the mice. The dosing route was i.v. (6 X 10⁶ NK cells per mouse) or I.T. (6 X 10⁶ NK cells per mouse) injections as described in Table 17. Tumor volumes were measured using the formula V = L × W × H × π/6, where L and W represent the longer and shorter diameters of the tumor and H represents the height of the tumor. Throughout the entire study, tumor volumes and body weight were measured twice weekly. Animals were observed for possible toxic effect from the treatment of D35 NK cells. Unscheduled sacrifices were performed when tumor volumes reached > 1,500 mm3, or loss of the original body weights exceeded 20%, or tumor ulceration occurred, or mice became moribund. At the end of the experiment, statistical analysis was performed on tumor growth rates of the control groups and each treatment group.

**Statistical analysis.** Results from different experiments are described as mean ± standard deviation of the mean (STDEV). Results were considered significant at P values of 0.05 or less.

### Results

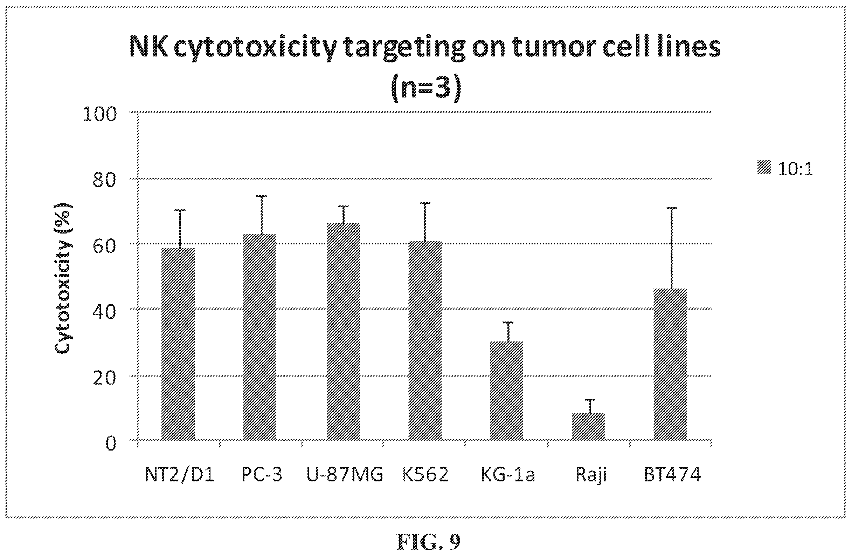

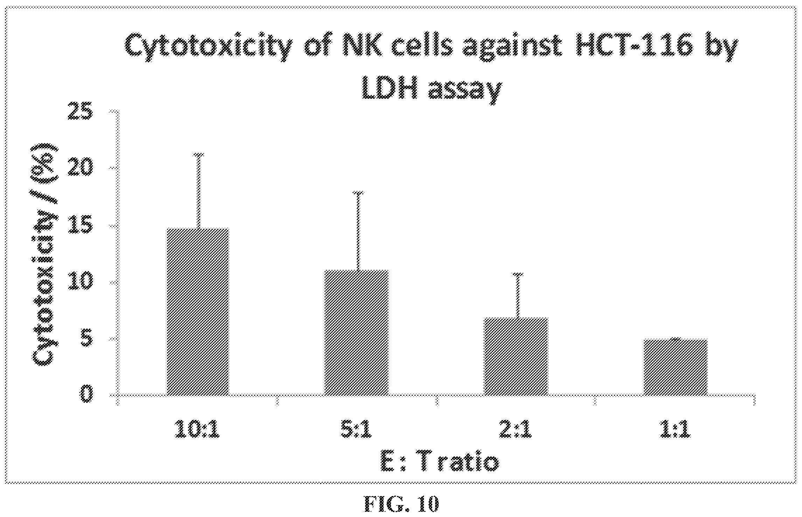

**Cytotoxicity of D35 NK cells against tumor cells *in vitro.*** Cytotoxic activity of day 35 cultured (D35) NK cell populations against tumor cell lines was assessed. As shown in Figure 9, at an effector-to-target (E:T) ratio of 10:1, cultured D35 NK cells demonstrate cytolytic activity against various tumor cell lines, including NTERA-2cl.D1 (NT2/D1, testicular carcinoma), PC-3 (prostate adenocarcinoma), U-87MG (glioblastoma), K562 (chronic myelogenous leukemia (CML)), KG-1a (acute myeloid leukemia (AML)), and BT474 (breast cancer) as detected by a FACS-based *in vitro* cytotoxicity assay in which tumor cells were labeled with PKH26-TOPRO. D35 NK cell population cytotoxicity was effective, but lowest against Raji cells (lymphoma) compared to the other cell lines tested. Using an LDH release assay, cytotoxicity of D35 NK cells against HCT-116 cells (colorectal carcinoma) was also measured, as shown in Figure 10.

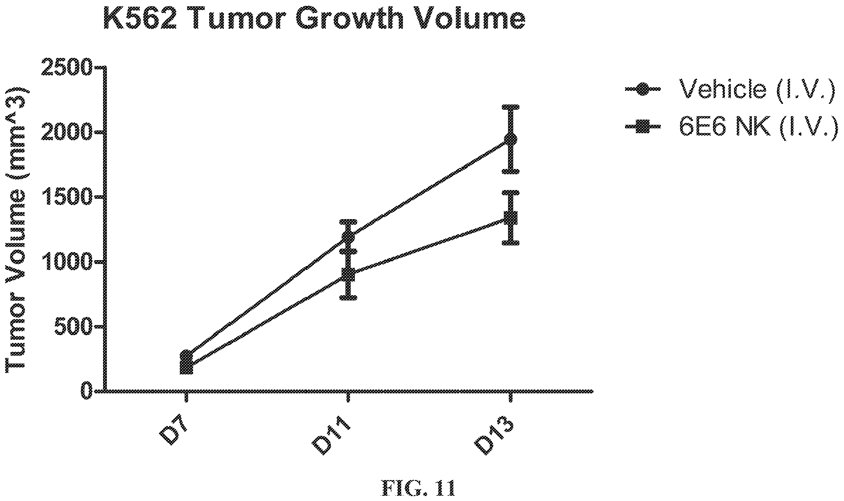

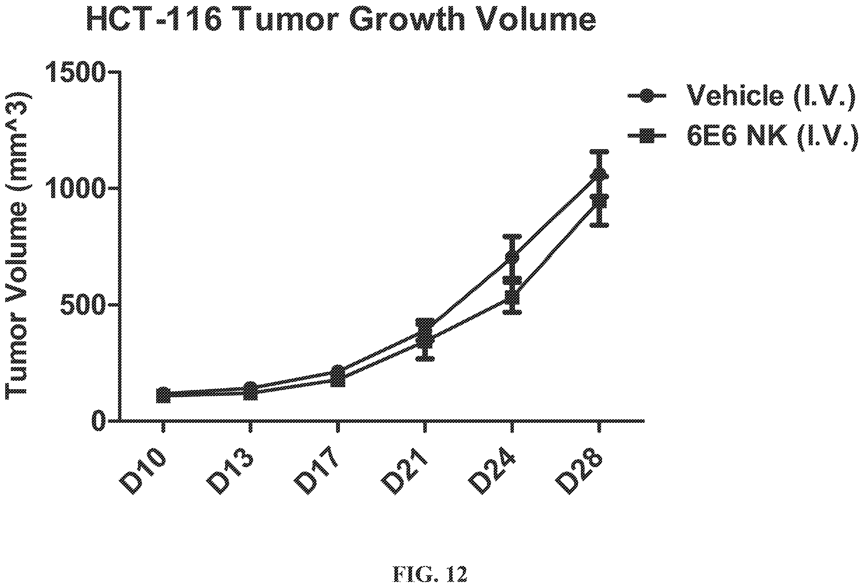

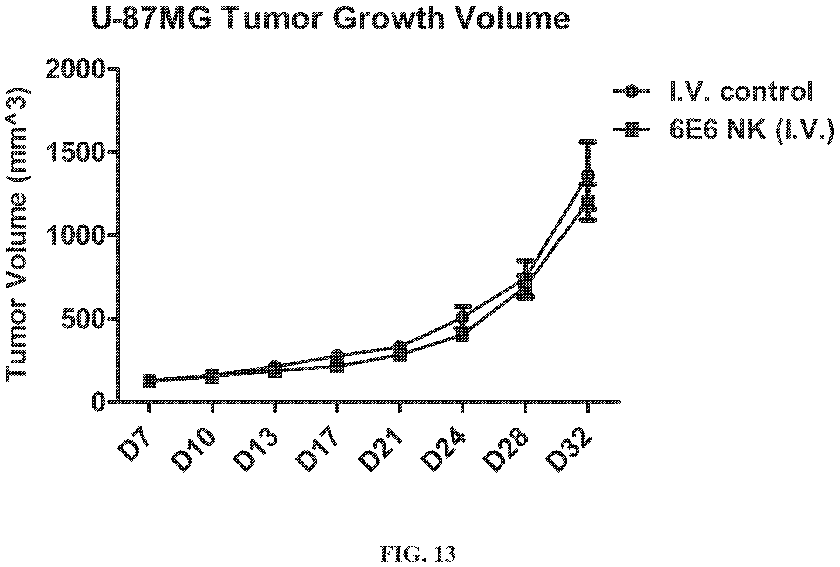

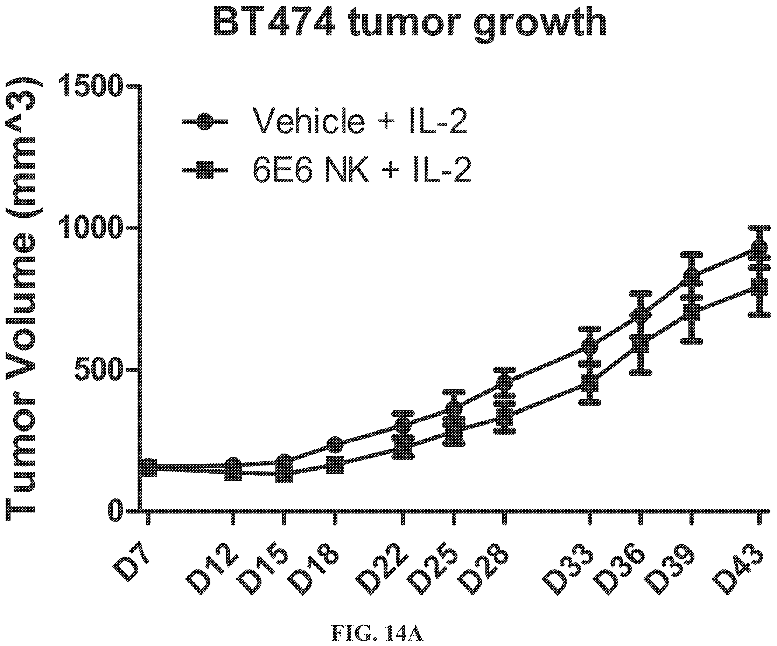

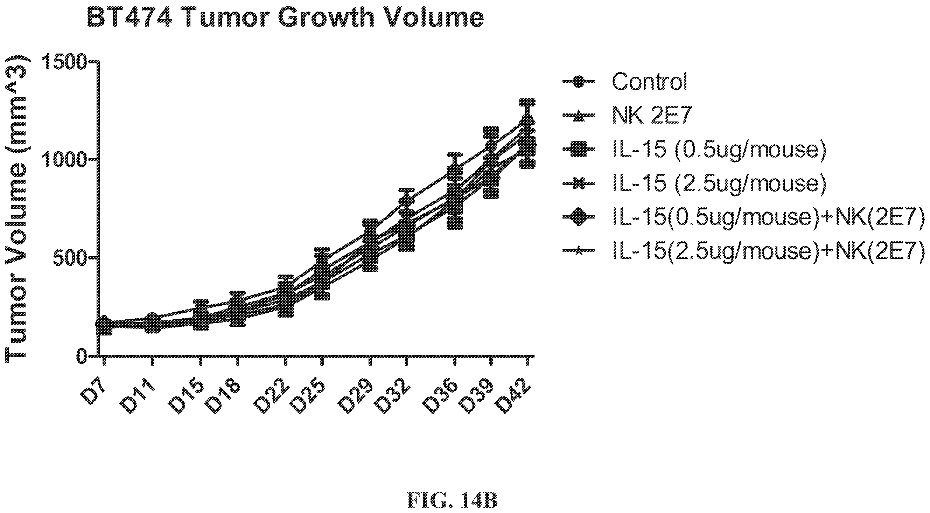

**Cytotoxicity of D35 NK cells against mouse xenografted tumors.** *In vivo* efficacy studies of D35 NK cell populations against xenograft tumors in immunodeficient mice were conducted. As shown in Figures 11-14B, single dose administrations of D35 NK cell populations exhibited tumor growth inhibition in a xenograft NSG mouse models of various tumors. Figure 11 demonstrates growth inhibition of CML (K562 cells) at Day 13. Figure 12 demonstrates growth inhibition of colorectal cancer (HCT-116 cells) at Day 28. Figure 13 demonstrates growth inhibition of glioblastoma (U-87MG cells) at Day 24. In a xenograft model of breast cancer (BT474 cells), D35 NK cell populations were administered in the presence and absence of cytokines IL-2 or IL-15. As shown in Figures 14A and 14B, the growth inhibitory effect of D35 NK cell populations was observed both in the presence and absence of IL-2 or IL-15.

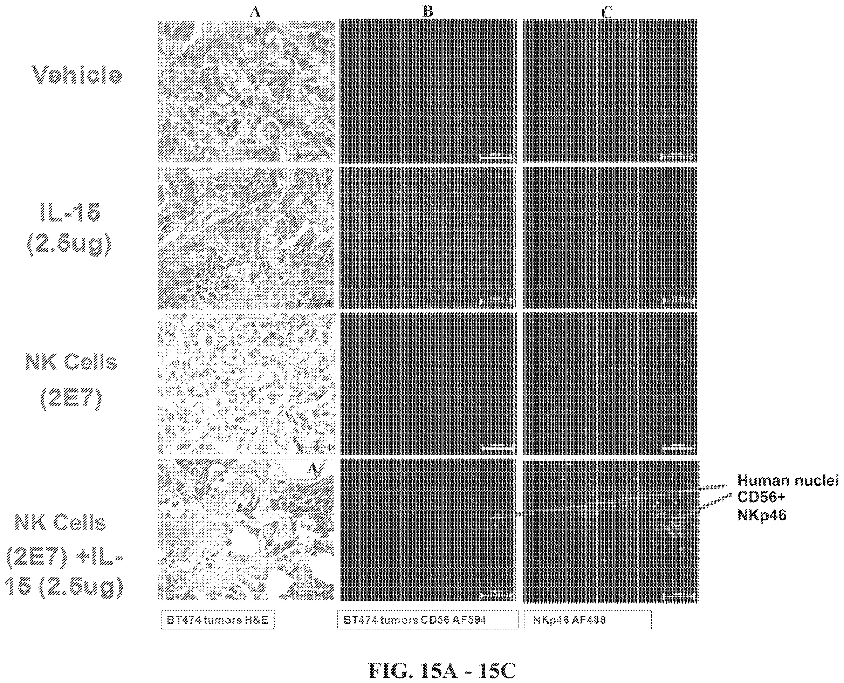

**D35 NK cell populations display tumor infiltrating ability in a BT474 xenografted NSG model.** *Ex vivo* immunohistochemistry (IHC) analysis of the BT474 xenograft from the endpoint of the D35 NK cell population-treated BT474 xenograft experiment described above (Figure 14B) reveals that D35 NK cell populations home to and infiltrate into an established BT474 tumor xenograft in NSG mice. Specifically, imaging of tumor xenograft sections revealed that necrotic tumor cells (visualized with hematoxylin and eosin (H&E) staining; Figure 15A) co-localized with tumor-infiltrated D35 NK cell populations, as detected by fluorescently labeled anti-CD56 (Figure 15B) and anti-NKp46 (Figure 15C). These results indicate that D35 NK cell populations have cytolytic activity against tumors *in vivo.*

### 7.18. Example 18: Characterization of D35 NK cell activity against Xenograft Tumors

In this example, the therapeutic efficacy of D35 NK cell populations alone and in combination with IL-2 against K562 and RPMI8226 tumors in mouse xenograft models was assessed.

### Materials and Methods

**Mice.** NSG (NOD-CgPrkdc-scid-Il2rg-tmlWjl-Sz) females from Jackson Laboratories.

**Cells.** Cells from the CML cell line K562 were injected subcutaneously with matrigel at 5 million cells/flank. Three multiple myeloma (MM) cell lines were used, RPMI8226, BT474, HL-60. Cultured D35 NK cell populations were supplied as fresh cell suspensions for immediate infusion.

**Treatment regimen.** Treatment with D35 NK cell populations prepared by a three-stage method was initiated following tumor establishment (tumor size = 100 mm³). Experimental groups were randomized based on the tumor burden distribution. Treatment groups are shown in Table 18.

**Table 18. (Groups 1-4, 9-10 = analyzed samples; Groups 5-8, 11-12 = expected samples)**

| Group | Tumor model | # of Mice | Treatment | Dose and Dosing Route | Dosing Volume |
|---|---|---|---|---|---|
| 1 | K562 | 8 | Control | Vehicle (i.v.) | 0.4 mL |
| 2 | K562 | 8 | NKs* | 6.0E6 (i.v.) | 0.4 mL |
| 3 | K562 | 8 | Control | Vehicle + IL-2 (i.v.) | 0.4 mL |
| 4 | K562 | 8 | NKs | 6.0E6 + IL-2 (i.v.) | 0.4 mL |
| | | | | | |
| 5 | RPMI822 | 8 | Control | Vehicle (i.v.) | 0.4 mL |
| 6 | RPMI822 | 8 | NKs | 6.0E6 (i.v.) | 0.4 mL |
| 7 | RPMI822 | 8 | Control | Vehicle + IL-2 (i.v.) | 0.4 mL |
| 8 | RPMI822 | 8 | NKs | 6.0E6 + IL-2 (i.v.) | 0.4 mL |
| 9 | BT474 | 8 | Control | Vehicle + IL-2 (i.v.) | 0.4 mL |
| 10 | BT474 | 8 | NKs | 6.0E6 + IL-2 (i.v.) | 0.4 mL |
| | | | | | |
| 11 | HL-60 | 8 | Control | Vehicle + IL-2 (i.v.) | 0.4 mL |
| 12 | HL-60 | 8 | NKs | 6.0E6 + IL-2 (i.v.) | 0.4 mL |

| | | | | | |
|---|---|---|---|---|---|
| * NKs = D35 NK cells | | | | | |

**Immunohistochemistry.** The following antibodies were used for immunohistochemistry: for detection of CD56, anti-NCAM clone 123C3 mouse monoclonal IgG1 from Abcam ab9272, secondary antibodies AF594 conjugated goat anti-mouse IgG₁ from Invitrogen; for detection of NKG2D, Clone ID11 mouse monoclonal IgG from Abcam ab35033, secondary antibodies AF488 conjugated goat anti-mouse IgG₁ from Invitrogen; and for detection of NKp46, goat polyclonal IgG from R&D Systems AF1850, secondary antibodies AF488 conjugated donkey anti-goat IgG from Invitrogen. Upon xenograft excision, one half of each tumor was frozen in liquid nitrogen and stored at -80 °C. The other half was immediately embedded in Optimum Cutting Temperature (OCT) media and frozen in liquid nitrogen. Five (5) µm sections were cut with a cryostat (Leica, Deerfield, IL). These sections were washed in PBS and exposed to protein blocking solution containing 2x casein solution, 5% horse serum and 0.3% Triton-X100 in PBS for 60 min at room temperature (RT). Primary antibodies diluted in blocking solution (1:50) were then applied to cells and incubated overnight at 4 °C. The next morning, samples were washed in PBS and secondary antibodies diluted in blocking solution (1:500) were applied to cells for 30 min at RT. Slides were then washed in PBS and 600 nM DAPI solution was applied for 10 minutes at RT to visualize nuclei. All slides were mounted with aqueous media for fluorescence (VECTASHIELD, Vector Laboratories). Immunohistochemistry images were captured with a NIKON Eclipse microscope model E800 equipped with a high-resolution digital camera (Nikon DXM1200F) connected to a PC equipped with NIS Elements software for image capture and archiving. Controls for these experiments were freshly prepared D35 NK cells and K562 cells prepared on cytospin slides.

### Results

After completion of the D35 NK cell population treatment schedule, xenografts were excised from the mice. One half of each tumor was immediately frozen in liquid nitrogen and stored at -80 °C. The other half was immediately embedded in Optimum Cutting Temperature (OCT) media and frozen in liquid nitrogen. 5 µm sections were cut with a cryostat (Leica, Deerfield, IL). These sections were used for hematoxylin and eosin (H&E) staining for routine histological and immunofluorescence studies.

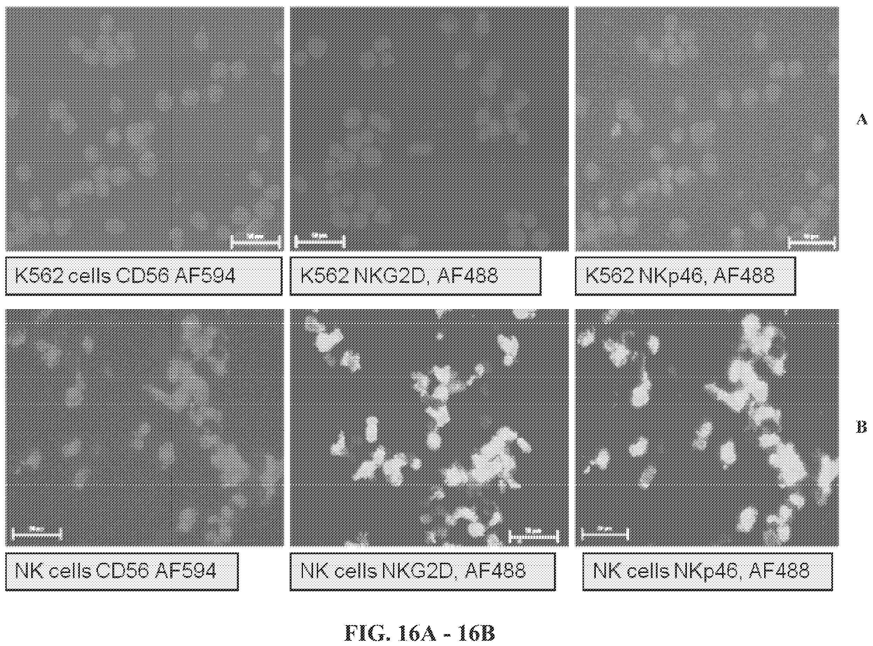

Immunofluorescence experiments were conducted in order to confirm that the antibodies are specific. As shown in Figure 16A, K562 cells do not express the CD56, NKG2D, or Nkp46 markers. As shown in Figure 16B, freshly prepared D35 NK cell populations stained positively for these three markers. No background from unspecific binding of antibodies was observed in isotype controls for each of the antibodies.

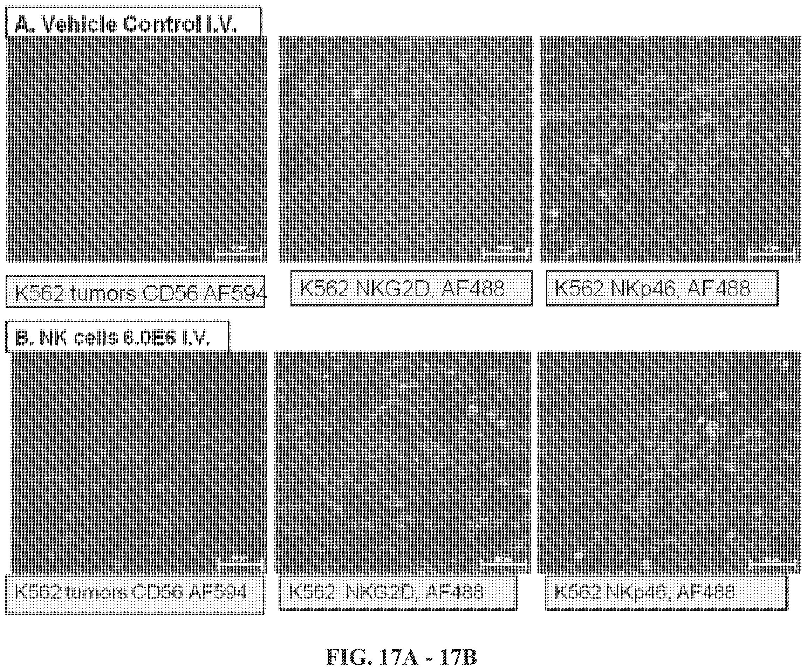

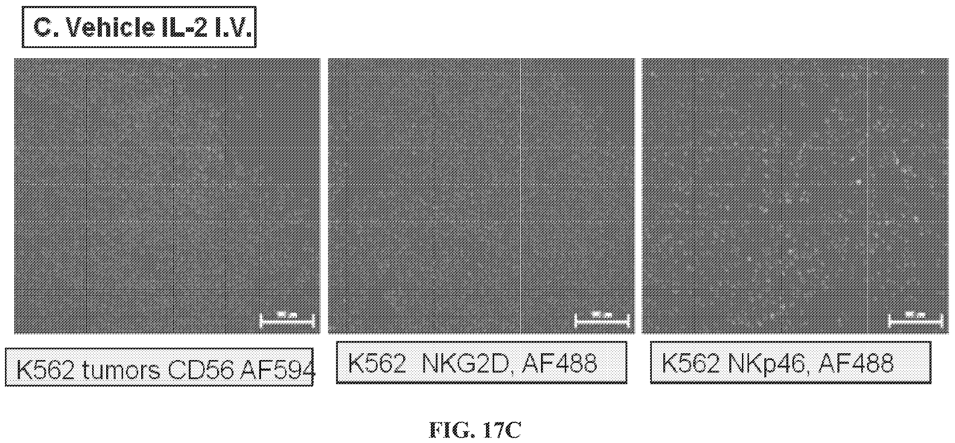

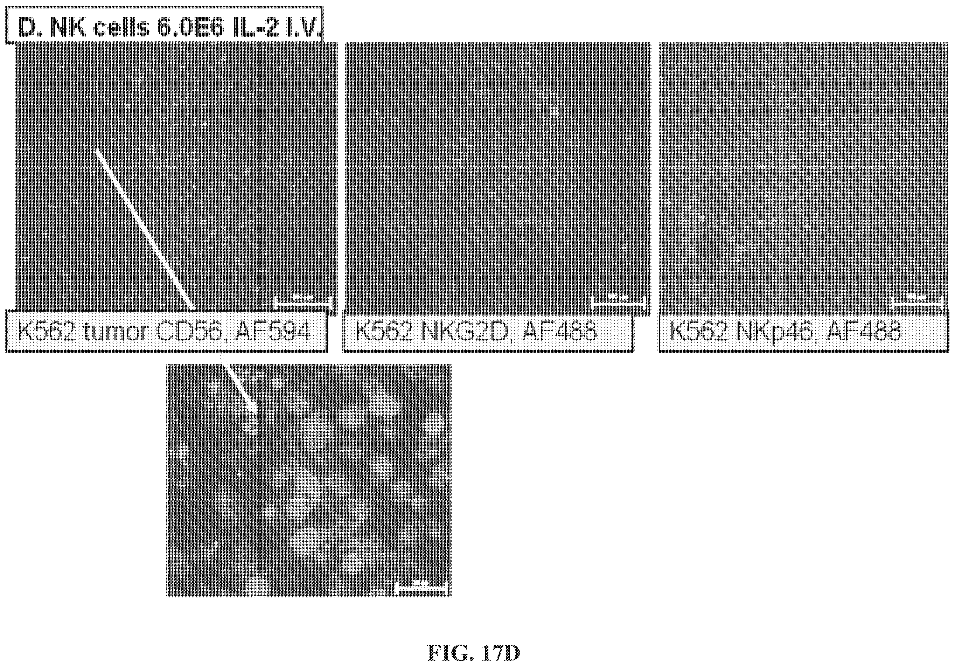

As demonstrated in Figure 17B, following infusion of a D35 NK cell population of 10⁶ cells, the cells localized to K562 tumors with high efficacy and had substantial *in vivo* survival. This was demonstrated by co-localization of the positive signal for CD56 antigen (left) with NKG2D (center). K562 tumor cell killing in the presence of cells of the D35 NK cell population and in the presence and absence of IL-2 was demonstrated via an increased number of granules (apoptotic nuclei) in the tumor cell population, as shown in Figure 17B (without IL-2) and Figure 17D (with IL-2). In contrast, no granularity was observed in vehicle controls (K562 tumor cells without the D35 NK cell population or IL-2), as shown in Figure 17A, which depicts the morphology of the growing tumor. Without wishing to be bound by any theory or mechanism, histopathological observations indicate that the regression of tumor volume following administration of the D35 NK cell population is likely due to apoptosis.

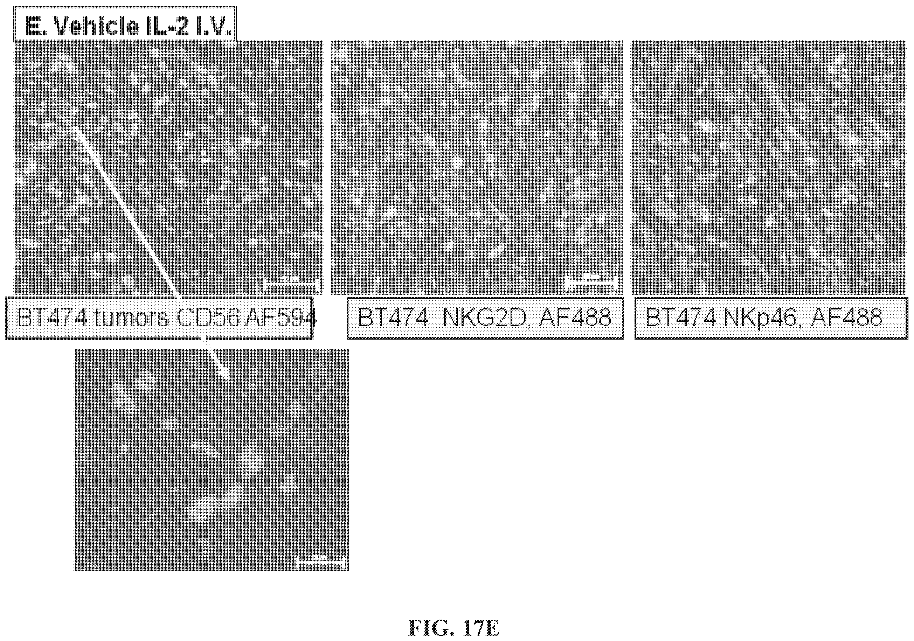

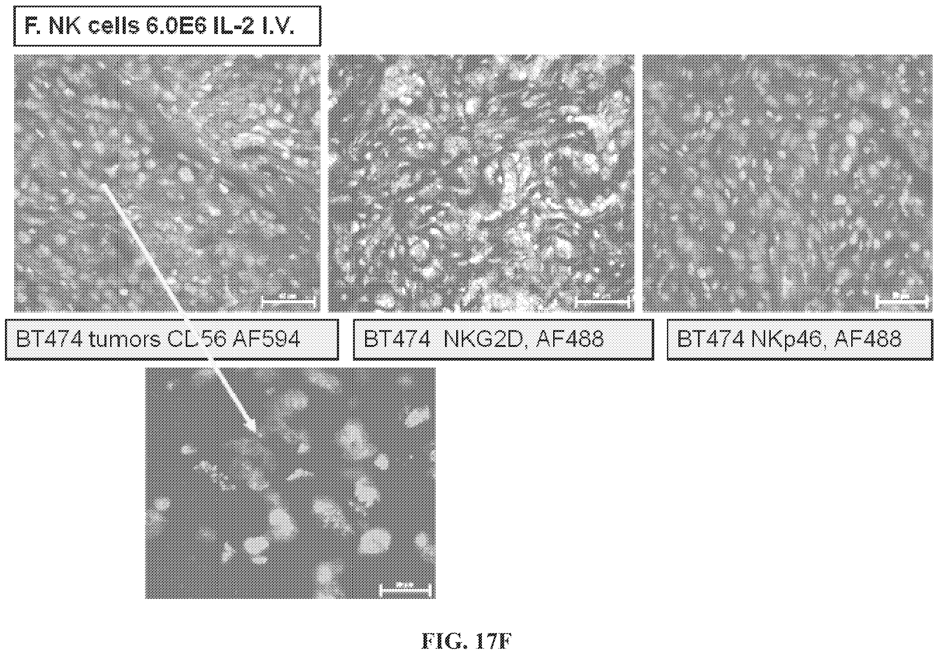

Immunohistochemistry of the D35 NK population-treated BT474 mouse xenograft tumor model demonstrates that the ability of IL-2 alone (Figure 17E) to initiate tumor cytotoxicity was increased by administration of the D35 NK cell population (Figure 17F).

These results, therefore, demonstrate that D35 NK cells exhibit cytolytic activity against various human tumor types.

### 7.19. Example 19: Characterization of D35 Cells Cultured Using 3-Stage Process in Mice

This example describes a biodistribution and phenotypic characterization study of the D35 NK cell population.

### Materials and Methods

**Expansion and differentiation of hematopoietic stem cells (HSCs) towards NK cells.** To initiate *in vitro* expansion and NK lineage commitment, CD34+ cells were seeded into GBGM medium. Cells were cultured in feeder-free cultures in the presence of low-dose (50 - 250 pg/mL) cytokines (IL-6, LIF, G-CSF, GM-CSF, MIP-1a) in three stages as described above.

***In vivo* mouse model for biodistribution and efficacy study.** Female NSG (NOD-CgPrkdc-scid-Il2rg-tmlWjl-Sz) mice (The Jackson Laboratory; Bar Harbor, Maine) at 6∼8 weeks of age were used for the study. Mice were housed in microisolator housing, and quarantined for several days prior to the initiation of the study. A total of 5 × 10⁶ K562 cells in exponential growth phase resuspended with matrigel were subcutaneously implanted into the right flank region of the mouse. Mice were monitored for tumor growth daily after cell implantation. When tumor volumes reached around 100 mm3, mice were randomized into groups using mice having tumor volumes closest to the mean value. Treatment with the D35 NK cell population was initiated on the day after randomization, and only one single dose of cells of the D35 NK cell population was administered to the mice. The dosing route was i.v. (6 X 10⁶ NK cells per mouse) or intratumoral (I.T.) (6 X 10⁶ NK cells per mouse) injections as described in Table 17. Tumor volumes were measured using the formula V = L × W × H × π/6, where L and W represent the longer and shorter diameters of the tumor and H represents the height of the tumor. Throughout the entire study, tumor volumes and body weight were measured twice weekly. Animals were observed for possible toxic effect from the treatment of the D35 NK cell population. Unscheduled sacrifices were performed when tumor volumes reached > 1,500 mm³, or loss of the original body weights exceeded 20%, or tumor ulceration occurred, or mice became moribund. At the end of the experiment, statistical analysis was performed on tumor growth rates of the control groups and each treatment group.

**Methylcellulose colony forming assay.** The methylcellulose colony forming assay, also referred to as colony forming cell (CFC) assay, was performed according to the manufacturer's protocol (StemCell Technologies, Inc.). In brief, CD34+ cell suspensions or test cells were placed into a methylcellulose medium supplemented with cytokines as indicated at different cell densities per plate. For each cell density, a triplicate assay was performed followed by 2 to 3 weeks incubation. Colony evaluation and enumeration were performed using light microscopy.

**Statistical analysis.** Results from different experiments are described as mean ± standard deviation of the mean (STDEV). Results were considered significant at P values of 0.05 or less.

### Results

**Biodistribution and persistence in mice.** The *in vivo* biodistribution and persistence of cells of the D35 NK cell population were analyzed following the adoptive transfer in NSG immunodeficient mice with transgenic expression of human IL-3, SCF and GM-CSF to model the human hematopoietic microenvironment. Animals were pre-conditioned by sublethal irradiation (260Rad) and the D35 NK cell population were administered by tail i.v. infusion (6x10⁶ cells/animal). The persistence, tissue distribution and cell surface phenotype of transferred human cells was analyzed by flow cytometry over four weeks. The animals were treated by daily injections of human recombinant IL-2 or IL-15 cytokines over the course of the study to determine the *in vivo* response of the D35 NK cell population to the clinically relevant adjuvant cytokine therapy.

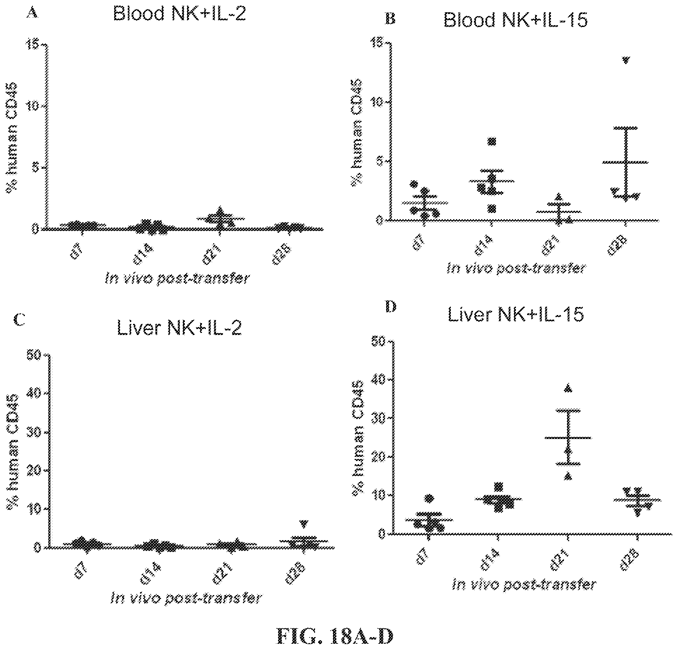

The transferred human cells were analyzed at four weekly time points following adoptive transfer (day 7, 14, 21 and 28) in peripheral blood, spleen, liver and bone marrow. Cells of the D35 NK cell population were detected by flow cytometric analysis of cell surface human CD45 expression in peripheral blood and bone marrow and in liver and spleen cell suspensions. As shown in Figure 18B and Figure 18D, in the animals receiving IL-15 adjuvant therapy, the cells of the D35 NK cell population persisted in peripheral blood at a relative frequency of 1-5% of the recipient mouse CD45+ cells throughout the whole 4-week time course. Human CD45+ cells reached a frequency of up to 20% relative to the recipient mouse CD45+ cell population in the liver at 21 days post transfer in IL-15 treated animals (Figure 18D), indicating liver-specific homing of D35 NK cells. In the same animals, cells of the D35 NK cell population were also readily detectable in the spleen and bone marrow throughout the whole 4-week time course. In the animals receiving IL-2 adjuvant therapy (Figure 18A and Figure 18C), human cells were detected at significantly lower levels compared to mice that were co-administered IL-15 (Figure 18B and Figure 18D), indicating that IL-15 enhances *in vivo* survival and /or persistence of cells of the D35 NK cell population.

These results demonstrate that cells of the D35 NK cell population persist for at least four weeks in mice.

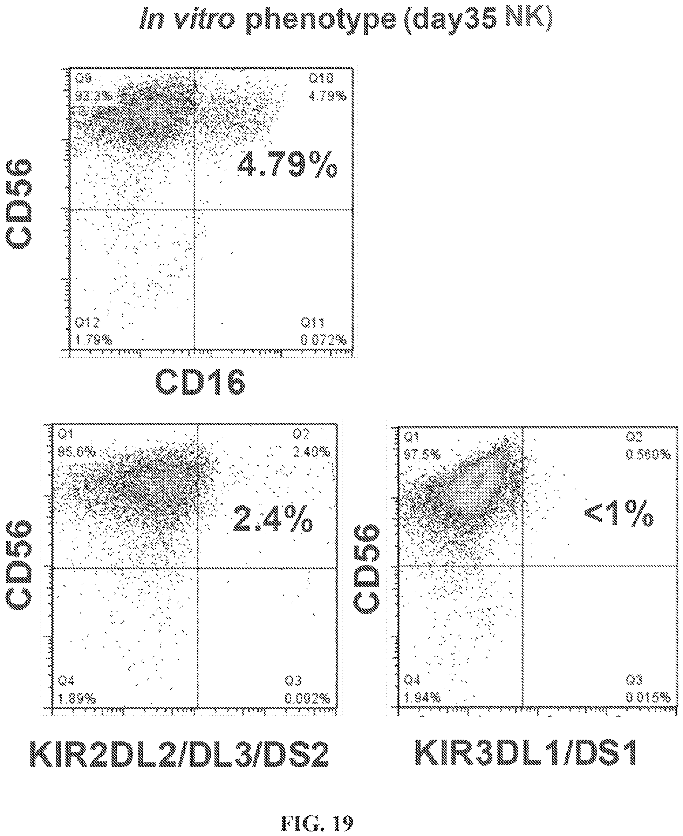

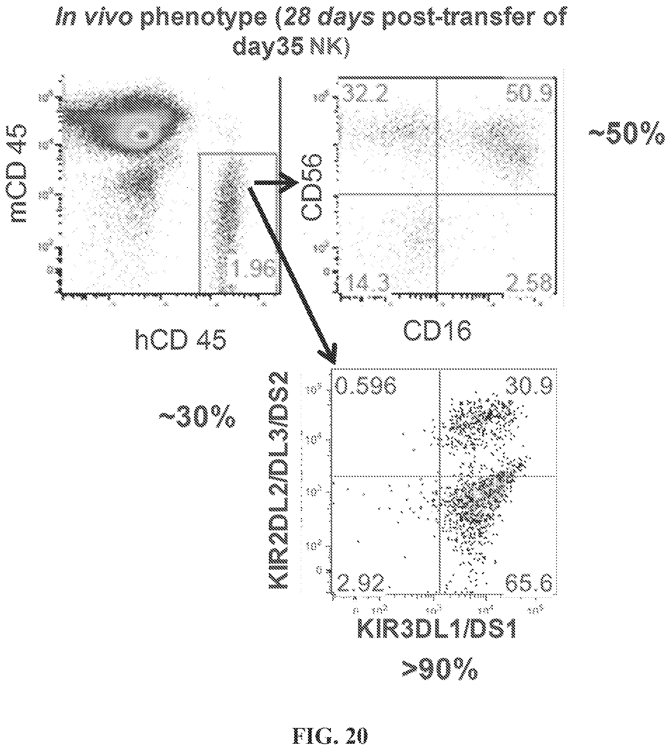

**Phenotypic and functional maturation of Three-Stage D35 NK cells in mice.** The *ex vivo* detected cells of the D35 NK cell population from the biodistribution study described above were analyzed for the expression of CD 16, a functionally relevant marker of NK cell maturation. As shown in Figure 19, cells of the D35 NK cell population *in vitro* have low levels (less than 5%) of CD56+CD16+, CD56+KIR2DL2/DL3/DS2+, and CD56+KIR3DL1/DS1 cells, consistent with the immature NK phenotype generated from expanded placental CD34+ stem cells. As shown in Figure 20, in IL-15 treated NSGS mice, cells of the D35 NK cell population show a progressive increase of CD16 expression *in vivo,* reaching up to 50% of CD56+CD16+, and over 90% CD56+KIR+. Moreover, as shown in the study above, cells of the D35 NK cell population were detected in peripheral blood at four weeks following adoptive transfer. These results together provide evidence for functional maturation of cells of the D35 NK cell population *in vivo.*

These results demonstrate that a substantial fraction of immature (CD16-) cells of the D35 NK cell population become CD16+ *in vivo,* indicating phenotypic and functional maturation.

**Antitumor activity of the D35 NK Cell Population.** In this experiment, a genetically induced leukemia model (reviewed in Mulloy et al., Cell Cycle. 2008 Nov 1;7(21):3314-9. Epub 2008 Nov 8) was used to test the specific cytotoxic activity of the D35 NK cell population against allogeneic leukemia-initiating cells and a normal non-transformed umbilical cord blood ("UCB") CD34+ cell control. Cytotoxic activity was measured using two different methods.

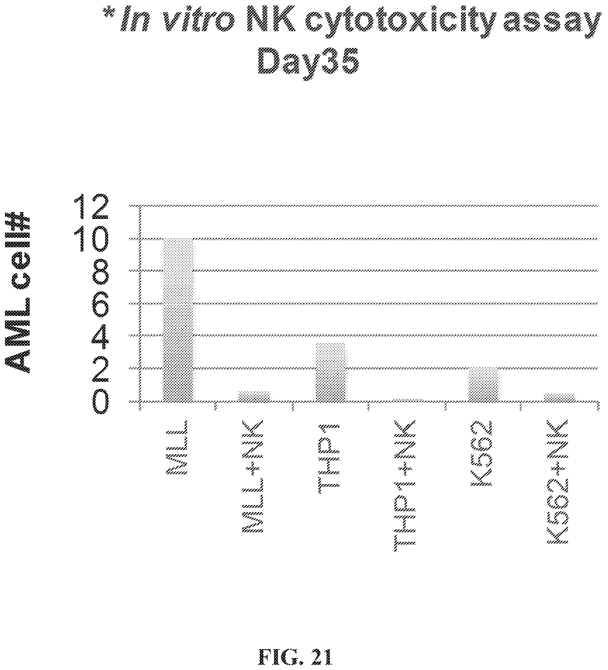

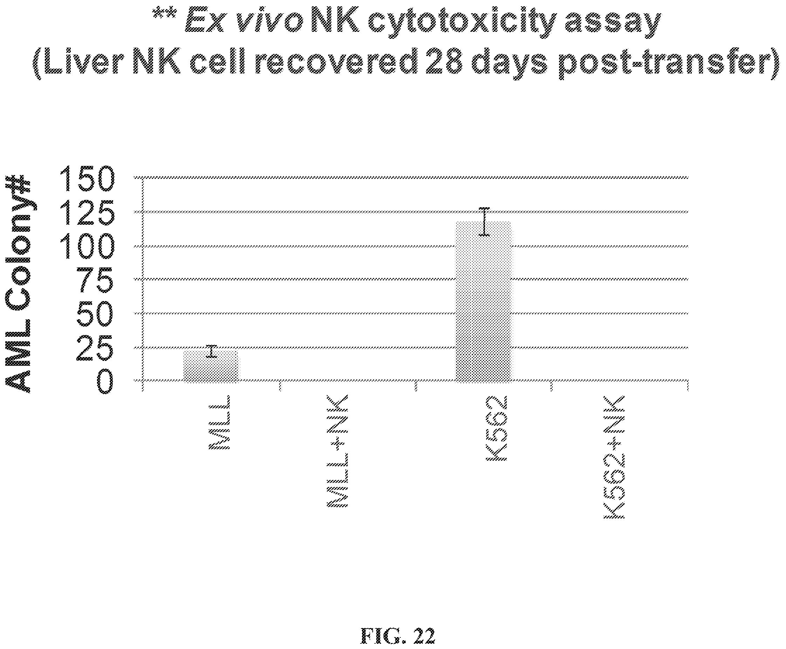

Briefly, in the first method, normal and leukemic progenitor cells were co-cultured in a liquid assay and D35 NK cell-induced cytotoxicity was measured by counting the number of remaining target cells in the coculture post 4-hour incubation. In the second method, normal CD34+ and leukemic progenitor cells were co-cultured with the D35 NK cell population for 4h and then plated into secondary colony forming cell assays in methylcellulose media. The methylcellulose colony formation assay was used as a readout of the specific cytotoxic activity of the D35 NK cell population against normal and leukemic stem/progenitor cells. The results, shown in Figures 21 and 22, demonstrate complete inhibition of the leukemic progenitor activity by co-culture with the D35 NK cell population and no effect on normal allogeneic CD34+ progenitors.

These results demonstrate that the D35 NK cell population exhibits cytotoxicity against engineered UCB CD34+ cells expressing MLL-AF9 fusion protein (these engineered CD34+ cells can develop into leukemia after transplantation into immunodeficient mice) but not normal UCB CD34+ cells evident by elimination of clonogenicity of the leukemic progenitors.

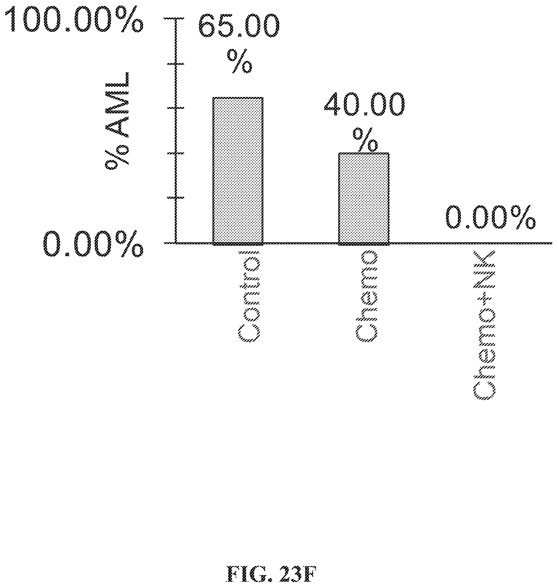

**The D35 NK cell population Suppresses Xenotransplant Disease.** A genetically induced leukemia model was utilized in an experiment to assess *in vivo* cytotoxicity of the D35 NK cell population. As schematized in Figure 23A, following transplantation of UCB CD34+ cells transduced with the MLL-AF9 fusion gene, mice were treated with chemotherapy (DA: cytarabine (50 mg/kg)/doxorubicin (1.5 mg/kg)) at day 8, day 9 and day 10. Cells of the D35 NK cell population (6 X 10⁶ cells/mouse) and IL-15 were administered at day 11. FACS analysis of GFP-expressing MLL cells was performed to measure the frequency of leukemic cells remaining at the experimental endpoint, defined as day 58 post-transplantation unless the mice died earlier. Representative graphs are shown for the control groups, *i.e.,* mice administered PBS (Figure 23B) or IL-15 (Figure 23C), or those groups administered either IL-15 and chemotherapy alone (Figure 23D) or the combination of the D35 NK cell population plus IL-15 and chemotherapy (Figure 23E). As shown in Figure 23F, at day 58, 65% of the mice in the control group were positive for AML cells; in the group treated with chemotherapy, 40% of the mice were positive for AML cells; and in the group treated with both chemotherapy and the D35 NK cell population, no mice were positive for AML cells.

These results demonstrate that the D35 NK cell population suppresses the *in vivo* relapse of leukemic disease following chemotherapy in a human xenotransplant leukemia model.

### 7.20. Example 20: Generation and Characterization of Natural Killer Progenitor Cell Populations

This example describes a three-step cultivation process for expansion and differentiation of CD34+ placental hematopoietic cells towards a natural killer (NK) progenitor cell population. NK progenitor cell populations obtained after 21 days of this cultivation process are also described and characterized in this example.

### Materials and Methods

**Culture process for expansion and differentiation of hematopoietic stem cells (HSCs) towards NK progenitor cell populations.** CD34+ cells were cultured in GBGM medium (Glycostem Therapeutics; see Spanholtz, et al., PLoS One (2010) 5(2):9221; GBGM is an animal-derived component free medium based on IMDM and contains human-derived or human recombinant proteins) and aliquots of cells were taken for assessment of cell count, cell viability, characterization of lymphocytic differentiation and functional evaluation. To initiate *in vitro* expansion and NK lineage commitment, CD34+ cells were seeded into GBGM medium. Cells were cultured in feeder-free cultures in the presence of low-dose (50 - 250 pg/mL) cytokines (IL-6, LIF, G-CSF, GM-CSF, MIP-1a) in three stages as follows:
Stage 1 (9 days): GBGM with 10% human serum AB (HS), LWH heparin, TPO, SCF, IL-7, Flt3L (25 - 27 ng/mL);
Stage 2 (5 days): GBGM with 10% HS, LWH heparin, SCF, IL-7, Flt3L, IL-15 (20 - 27 ng/mL); and
Stage 3 (7 days): GBGM with 10% HS, SCF, IL-7, Flt3L, IL-15 (20 - 27 ng/mL), IL-2 (1000 U/mL).

***In vivo* mouse model for engraftment study.** Female NSG (NOD-CgPrkdc-scid-Il2rg-tmlWjl-Sz) mice (The Jackson Laboratory; Bar Harbor, Maine) at 6∼8 weeks of age were used for the study. Mice were housed in microisolator housing, and quarantined for several days prior to the initiation of the study. Treatment with cells of the D21 NK progenitor cell population was initiated on the day after randomization, and only one single dose of the D21 NK progenitor cell population was administered to the mice. The dosing route was i.v. (6 X 10⁶ cells of the D21 NK progenitor cell population per mouse) or I.T. (6 X 10⁶ D21 NK progenitor cells per mouse) injections as described in Table 17. Tumor volumes were measured using the formula V = L × W × H × π/6, where L and W represent the longer and shorter diameters of the tumor and H represents the height of the tumor. Throughout the entire study, tumor volumes and body weight were measured twice weekly. Animals were observed for possible toxic effect from the treatment of the D21 NK progenitor cell population. Unscheduled sacrifices were performed when tumor volumes reached > 1,500 mm3, or loss of the original body weights exceeded 20%, or tumor ulceration occurred, or mice became moribund. At the end of the experiment, statistical analysis was performed on tumor growth rates of the control groups and each treatment group.

**Chromium (⁵¹Cr) release cytotoxicity.** Effector cells (cells of the D21 NK progenitor cell population) were seeded onto round-bottom 96-well plates in 100 µl aliquots/well assay medium (RPMI1640+10% FBS+P/S) at appropriate concentrations. Target tumor cells were incubated with 10 µCi sodium chromate for 60 min at 37 oC, followed by three washes with assay medium. The ⁵¹Cr labeled tumor cells were then added to the effector cells at the indicated effector-to-target (E:T) ratios. Incubation was performed for 4h at 37 oC in 5% CO₂ incubators. All cultures were performed in triplicate and % cytotoxicity was calculated according to the formula: 100 x (test ⁵¹Cr release - spontaneous ⁵¹Cr release) / (maximum ⁵¹Cr release - spontaneous ⁵¹Cr release), where maximum and spontaneous counts were calculated from triplicates of target cells incubated with 2% Triton X and in assay medium, respectively.

**Statistical analysis.** Results from different experiments are described as mean ± standard deviation of the mean (STDEV). Results were considered significant at P values of 0.05 or less.

### Results

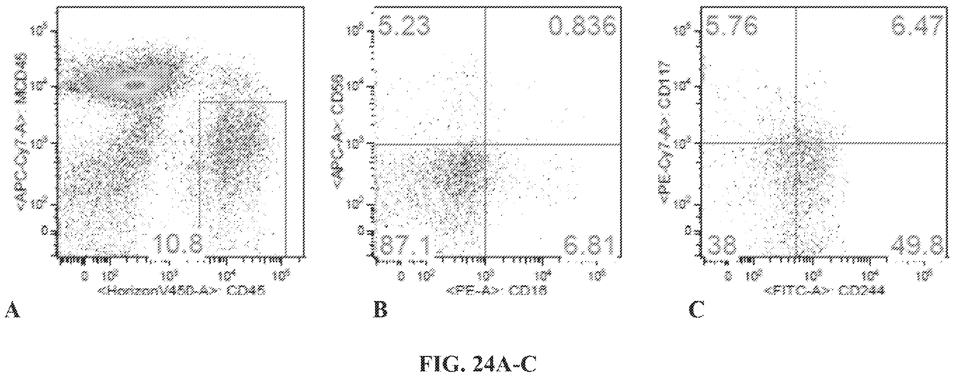

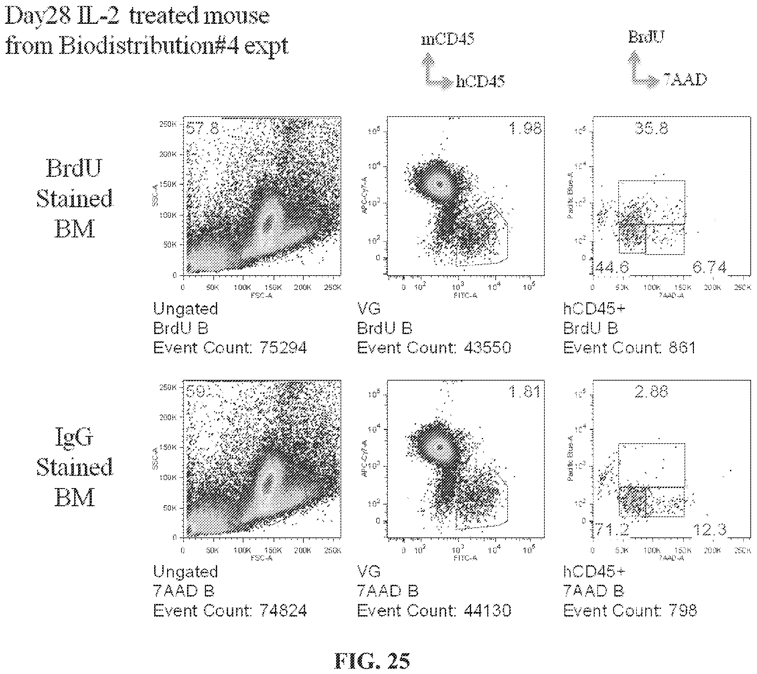

The *in vivo* biodistribution analysis of the D21 NK progenitor cell population demonstrated a high level of human cell persistence in the bone marrow, reaching a frequency of >10% relative to the recipient mouse CD45+ cells four weeks following adoptive transfer, as shown in Figure 24A. The bone marrow engrafting cells were CD56-CD16- (Figure 24B), expressed low CD117 and were >50% for the marker 2B4 (CD244) (Figure 24C), indicating an NK progenitor cell phenotype. This phenotypic population was also detected following adoptive transfer of D35 NK cells, but with a ∼10-fold lower frequency. The bone marrow engrafting D21 NK progenitor cell population was tested for *in vivo* cell cycle entry by overnight incorporation of BrdU to label the *in vivo* S phase cell cycle entry of cells by de novo DNA synthesis. As shown in Figure 25, cells of the D21 NK progenitor cell population derived from the bone marrow underwent a high rate (>35% BrdU+ cells) of *in vivo* cycling, indicating the active turnover of this population consistent with their observed *in vivo* expansion.

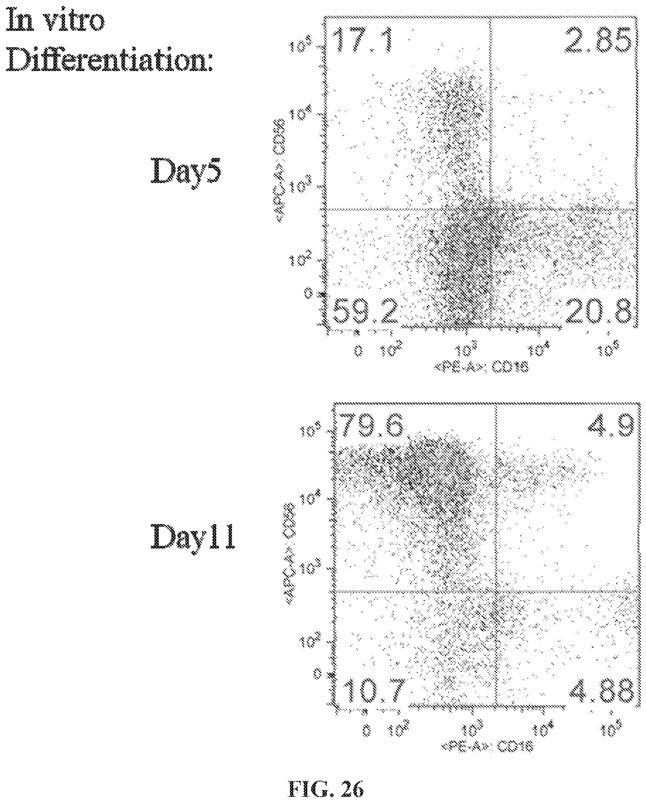

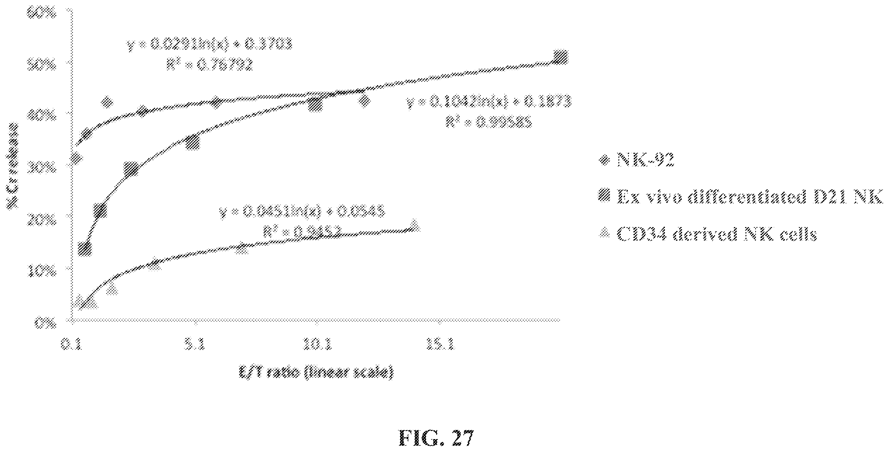

The bone marrow engrafting CD56-CD16- D21 NK progenitor cell population cells recovered from bone marrow samples 28 days post administration were purified by FACS sorting and cultured *ex vivo* in NK cell culture media (IMDM with 10% FBS, 2% HS, IL-2 (200 U/mL)) in order to determine their NK differentiation potential. As shown in Figure 26, cells of the D21 NK progenitor cell population grown in NK culture medium can be differentiated into CD56+CD16+ phenotypically mature NK cells. These *ex vivo* differentiated CD56+ NK cells derived from the bone marrow engrafting cells of the D21 NK progenitor cell population were also tested in a chromium (⁵¹CR) release cytotoxicity assay against K562 target cells. Cytotoxic activity of differentiated CD56+ NK cells derived from cells of the D21 NK progenitor cell population was compared to the NK-92 cell line as well as human CD34+ cell derived NK cells generated by *in vitro* stromal co-culture. As shown in Figure 27, differentiated CD56+ NK cells derived from cells of the D21 NK progenitor cell population showed similar or greater cytotoxic activity compared to both positive control NK cell types at equivalent effector-to-target cell ratios.

Collectively, these *ex vivo* analyses of the bone marrow engrafting population of the D21 NK progenitor cell population is consistent with their NK-cell functional differentiation potential, indicating an ability of these cells to sustain *in vivo* self-renewal upon transfer.

In conclusion, this example demonstrates that NK progenitor cell populations exhibit the ability to migrate and engraft in bone marrow. These bone marrow-engrafting cells are positive for 2B4 (CD244), suggesting that they are NK progenitor cells.

### 7.21. Example 21: Comparison of NK progenitor cell populations and NK cell populations

This example compares the phenotypes of cells of the D21 NK progenitor cell population and cells of the D35 NK cell population cultured using the 3-step process described above. In an *in vitro* profiling experiment using FACS, based on five replicates, 29.9% +/-13.4% of cells of the D21 NK progenitor cell population were CD56+CD3- compared to 88.5% +/-11.2% of cells of the D35 NK cell population. Additionally, 12.3% +/- 5.6% of cells of the D21 NK progenitor cell population were NKp46+ compared to 49.0% +/-20.4% of cells of the D35 NK cell population.

### 7.22. Example 22: Comparison of D21 NK progenitor cell populations and D35 NK cell populations

In this example, the results and conclusions from the experiments described in Examples 17-21 are summarized. These results demonstrate that a cultivation method to generate human NK cell populations from CD34+ cells isolated from donor-matched cord blood and human placental derived stem cells, which were obtained from full-term human placenta, has been successfully established. This cultivation method is feeder-free, and is based on progenitor cell expansion followed by differentiation toward NK cell populations supported by cytokines including thrombopoietin, stem cell factor, Flt3 ligand, IL-7, IL-15 and IL-2. A graded progression from populations comprising CD34+ hematopoietic progenitor cells (or hematopoietic stem cells, HSCs) toward populations comprising NK cells ultimately results in an NK cell population with an approximately 90% CD3-CD56+ phenotype and is associated with an average 10,000-fold expansion achieved over 35 days. The resulting cell population is CD16- and expresses low levels of KIRs, indicating that the population comprises a predominantly immature NK cell phenotype. Notably, however, the cell population also shows active in vitro cytotoxicity against a broad range of tumor cell line targets.

The in vivo persistence, maturation and functional activity of the HSC-derived NK cell population was assessed in immunodeficient (NSG) mice engineered to express the human cytokines SCF, GM-CSF and IL-3 (NSGS mice). Human IL-2 or IL-15 was injected intraperitoneally three times per week to test the effect of cytokine supplementation on the in vivo transferred NK cell population. The presence and detailed immunophenotype of the NK cell population was assessed in peripheral blood (PB), bone marrow (BM), spleen and liver samples at 7-day intervals up to 28 days post-transfer. Without cytokine supplementation, very few cells from this NK cell population were detectable at any time-point. Administration of IL-2 resulted in a detectable but modest enhancement of persistence of cells from this human NK cell population. The effect of IL-15 supplementation was significantly greater, leading to the robust persistence of transferred NK population cells in circulation, and a likely specific homing and expansion in the liver of recipient mice.

Following in vivo transfer, a significant fraction of human CD56+ cells expressed CD16 and KIRs, indicating full physiologic NK differentiation. Human CD56+ cells isolated ex vivo efficiently killed K562 targets in in vitro cytotoxicity assays. In contrast to peripheral blood, spleen and liver, BM contained a substantial portion of human cells that were CD56/CD16 double negative (DN) but positive for CD244 and CD117, indicating a residual progenitor function in the CD56- fraction of the CD34+ derived cell product. The BM engrafting population was higher in NK cell population cultures at earlier stages of expansion, but was preserved in the day 35-cultured product. The frequency of these cells in the BM increased over time, and showed continued cycling based on in vivo BrdU labeling 28 days post-transfer, suggesting a significant progenitor potential in vivo. Interestingly, DN cells isolated from BM could be efficiently differentiated ex vivo to mature CD56+CD16+ NK cells with in vitro cytotoxic activity against K562. In vivo, these BM engrafting cells may provide a progenitor population to produce a mature NK cell pool in humans.

The in vivo activity of HSC-derived NK cell populations was further explored using a genetically engineered human AML xenograft model of minimal residual disease (MRD). Data from these experiments indicate significant suppression of AML relapse in animals receiving the NK cell populations following chemotherapy.

### 7.23. Example 23: Telomere Length of D21 and D35 NK Cells

In this example, D21 and D35 cells cultured using the three-stage process described above were assessed for DNA telomere length.

Prior to mitotic division, cellular DNA is duplicated by a DNA polymerase. This enzyme does not replicate the very ends of the chromosomes - the telomere region. Because of this, the telomeres get shortened after each cell division, and thus telomeres act as a "mitotic clock." Telomeres function to keep chromosome ends intact. In normal cells, telomere shortening leads to cell senescence. Vertebrate telomeres are composed of 6 nucleotides (TTAGGG) in repeats of a few hundred to several thousand. Telomere length is species specific, ranging from 5-20 kilobases (kb) in humans to 20-150 kb in mice.

### 7.23.1. Materials and Methods

***Telomere PNA-FITC method.*** Detection of the telomeric sequences of cells were assessed using the Telomere PNA Kit/FITC for Flow Cytometry (Cat# K5327, DAKO, an Agilent Technologies company). The probe of this kit does not recognize subtelomeric sequences, and in contrast to traditional telomere restriction fragment (TRF) measurements, this method allows an estimation of the telomere length without inclusion of subtelomeres.

***Control Cells.*** The following control cells were used: HL60 cell line, NK92 cell line, PBMC derived NK cells, CCRF-CEM, NTERA2, and NHDF-adult fibroblast cell line.

***Analytic methods.*** Samples were analyzed by flow cytometry using the log scale FL1-H for probe fluorescence and the linear scale FL3-H for DNA staining. Forward scatter side scatter, FL1-H and FL3-H values were saved. (In a variation, FL3-A and FL#W values can also be saved.)

***Calculations.*** RTL = (mean FL1 sample cells with probe - mean FL1 samples cells without probe) x DNA index of control cells x100 / (mean FL1 control cells with probe - mean FL1 control cells without probe) x DNA index of sample cells.

### 7.23.2. Results and Discussion

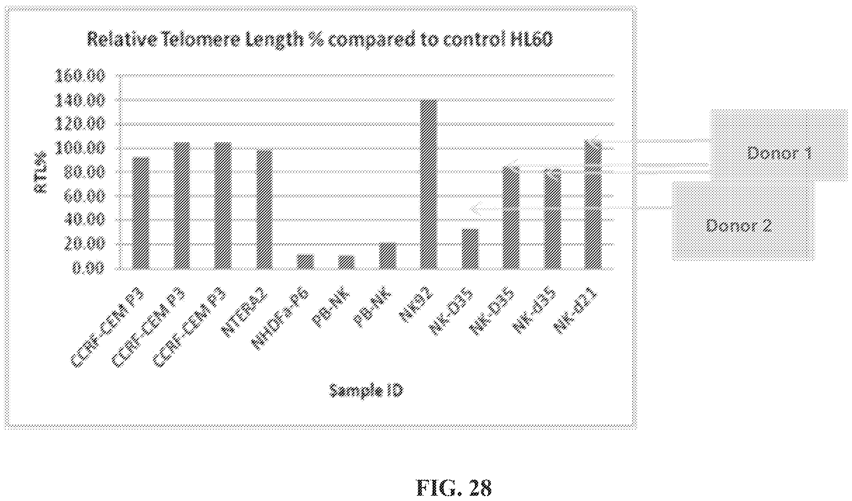

The Telomere PNA-FITC method was utilized to measure telomere length of day 21 and day 35 NK cells cultured using the three-stage process described herein in comparison to CCRF-CEM (T lymphoblastoid cell line), HL60 (promyeloblast cell line), NTERA2 (testicular embryonal carcinoma cell line), NHDF (normal human dermal fibroblast), NK92 cell line, and peripheral blood (PB) NK cells. The expected telomere length for CCRF-CEM is approximately 15-20 kb, and for HL60 is approximately 15 kb. Due to inter-assay variability, the test results were expressed as relative telomere length (RTL) compared to HL60 (%RTL = Telomere Length_{TEST}/ Telomere Length_{HL60} x 100). As shown in Figure 28, NK92 cells have a longer telomere length than day 21 NK cells (NK-D21) and day 35 NK cells (NK-D35). Commercially available PB NK cells derived from two donors were shown to have a shorter telomere length compared to NK-D21 and NK-D35 from two different donors. These results indicate that NK-D21 and NK-D35 cells are more immature and less differentiated than PB NK cells, and therefore may have a greater potential for further expansion and differentiation than PB NK cells.

### Equivalents:

The present invention is not to be limited in scope by the specific embodiments described herein. Indeed, various modifications of the invention in addition to those described will become apparent to those skilled in the art from the foregoing description and accompanying figures. Such modifications are intended to fall within the scope of the appended claims.

All references cited herein are incorporated herein by reference in their entirety and for all purposes to the same extent as if each individual publication, patent or patent application was specifically and individually indicated to be incorporated by reference in its entirety for all purposes. The citation of any publication is for its disclosure prior to the filing date and should not be construed as an admission that the present invention is not entitled to antedate such publication by virtue of prior invention.

The following numbered paragraphs (paras.) contain further statements of various aspects of the present invention:-
1. An isolated population of natural killer (NK) progenitor cells, wherein said NK progenitor cells are produced according to the following method: (i) culturing hematopoietic stem cells or progenitor cells in a first medium comprising Flt3L, TPO, SCF, IL-7, G-CSF, IL-6 and GM-CSF, (ii) subsequently culturing said cells in a second medium comprising Flt3L, SCF, IL-15, and IL-7, IL-17 and IL-15, G-CSF, IL-6 and GM-CSF, and (iii) subsequently culturing said cells in a third medium comprising SCF, IL-15, IL-7, IL-2, G-CSF, IL-6 and GM-CSF.
2. The isolated population of NK progenitor cells of para 1, wherein the duration of culturing step (i) is 7-9 days, wherein the duration of culturing step (ii) is 5-7 days, and wherein the duration of culturing step (iii) is 5-9 days.
3. The isolated population of NK progenitor cells of para 1, wherein the duration of culturing step (i) is 7-9 days, wherein the duration of culturing step (ii) is 5-7 days, and wherein the duration of culturing step (iii) is 21-35 days.
4. The isolated population of NK progenitor cells of paras 1, 2, or 3 wherein the hematopoietic stem or progenitor cells used in the method are CD34⁺.
5. The isolated population of NK progenitor cells of paras 1, 2, or 3 wherein the hematopoietic stem or progenitor cells comprise hematopoietic stem or progenitor cells from human placental perfusate and hematopoietic stem or progenitor cells from umbilical cord, wherein said placental perfusate and said umbilical cord blood are from the same placenta.
6. The isolated population of NK progenitor cells of any one of paras 1-5, wherein CD34- cells comprise more than 80% of the total population at the end of step (i).
7. The isolated population of NK progenitor cells of any one of paras 1-6, wherein said population comprises no more than 40% CD3-CD56+ cells
8. The isolated population of NK progenitor cells of any one of paras 1-7, wherein said population comprises cells which are CD52+ CD117+.
9. An isolated natural killer (NK) progenitor cell population, wherein said population comprises no more than 40% CD3-CD56+ cells.
10. An isolated natural killer (NK) progenitor cell population, wherein said population comprises cells which are CD52+ CD117+.
11. A pharmaceutical composition comprising the isolated population of NK progenitor cells of any one of paras 1-10.
12. A method of suppressing the proliferation of tumor cells comprising bringing the tumor cells into proximity with the isolated population of NK progenitor cells of any one of claims 1-10 or the composition of para 11.
13. The method of para 12, wherein said tumor cells are primary ductal carcinoma cells, glioblastoma cells, leukemia cells, acute T cell leukemia cells, chronic myeloid lymphoma (CML) cells, acute myelogenous leukemia cells, chronic myelogenous leukemia (CML) cells, lung carcinoma cells, colon adenocarcinoma cells, histiocytic lymphoma cells, multiple myeloma cells, colorectal carcinoma cells, colorectal adenocarcinoma cells, prostate cancer cells, or retinoblastoma cells.
14. A method of treating hematologic cancer in a subject in need thereof, comprising administering to said subject the isolated population of NK progenitor cells of any one of claims 1-10 or the composition of para 11.
15. The method of para 14, wherein said hematologic cancer is acute myeloid leukemia.

## Claims

1. An isolated population of natural killer (NK) progenitor cells, wherein said NK progenitor cells are produced according to the following method: (i) culturing hematopoietic stem cells or progenitor cells in a first medium comprising Flt3L, TPO, SCF, IL-7, G-CSF, IL-6 and GM-CSF, (ii) subsequently culturing said cells in a second medium comprising Flt3L, SCF, IL-15, and IL-7, IL-17 and IL-15, G-CSF, IL-6 and GM-CSF, and (iii) subsequently culturing said cells in a third medium comprising SCF, IL-15, IL-7, IL-2, G-CSF, IL-6 and GM-CSF.

2. The isolated population of NK progenitor cells of claim 1, wherein the duration of culturing step (i) is 7-9 days, wherein the duration of culturing step (ii) is 5-7 days, and wherein the duration of culturing step (iii) is 5-9 days.

3. The isolated population of NK progenitor cells of claim 1, wherein the duration of culturing step (i) is 7-9 days, wherein the duration of culturing step (ii) is 5-7 days, and wherein the duration of culturing step (iii) is 21-35 days.

4. The isolated population of NK progenitor cells of claim 1, 2, or 3 wherein the hematopoietic stem or progenitor cells used in the method are CD34⁺.

5. The isolated population of NK progenitor cells of claim 1, 2, or 3 wherein the hematopoietic stem or progenitor cells comprise hematopoietic stem or progenitor cells from human placental perfusate and hematopoietic stem or progenitor cells from umbilical cord, wherein said placental perfusate and said umbilical cord blood are from the same placenta.

6. The isolated population of NK progenitor cells of any one of claims 1-5, wherein CD34- cells comprise more than 80% of the total population at the end of step (i).

7. The isolated population of NK progenitor cells of any one of claims 1-6, wherein said population comprises no more than 40% CD3-CD56+ cells

8. The isolated population of NK progenitor cells of any one of claims 1-7, wherein said population comprises cells which are CD52+ CD117+.

9. An isolated natural killer (NK) progenitor cell population, wherein said population comprises no more than 40% CD3-CD56+ cells.

10. An isolated natural killer (NK) progenitor cell population, wherein said population comprises cells which are CD52+ CD117+.

11. A pharmaceutical composition comprising the isolated population of NK progenitor cells of any one of claims 1-10.

12. A method of suppressing the proliferation of tumor cells comprising bringing the tumor cells into proximity with the isolated population of NK progenitor cells of any one of claims 1-10 or the composition of claim 11.

13. The method of claim 12, wherein said tumor cells are primary ductal carcinoma cells, glioblastoma cells, leukemia cells, acute T cell leukemia cells, chronic myeloid lymphoma (CML) cells, acute myelogenous leukemia cells, chronic myelogenous leukemia (CML) cells, lung carcinoma cells, colon adenocarcinoma cells, histiocytic lymphoma cells, multiple myeloma cells, colorectal carcinoma cells, colorectal adenocarcinoma cells, prostate cancer cells, or retinoblastoma cells.

14. A method of treating hematologic cancer in a subject in need thereof, comprising administering to said subject the isolated population of NK progenitor cells of any one of claims 1-10 or the composition of claim 11.

15. The method of claim 14, wherein said hematologic cancer is acute myeloid leukemia.
